# EUROPEAN PATENT APPLICATION

(11) **EP 4 748 843 A2**
(43) Date of publication of application: **27.05.2026**
(21) Application number: 26169517.5
(22) Date of filing: 13.07.2023
(51) Int. Cl.: C07D 491/22

(54) **ANTIBODY-DRUG CONJUGATE, AND PREPARATION METHOD THEREFOR AND USE THEREOF**

(30) Priority: 14.07.2022 CN 202210838614
(62) Divisional of application: 23839013.2
(71) Applicant: Medilink Therapeutics (Suzhou) Co., Ltd., Suzhou, Jiangsu 215000 (CN)
(72) Inventor: Long, Kai, Shanghai 201210 (CN)
(74) Representative: karo IP

(57) **Abstract**

A bioactive conjugate, and a preparation method therefor and the use thereof. Specifically, disclosed are an antibody-drug conjugate as represented by formula (XV), a preparation method therefor and the use thereof in the prevention and/or treatment of diseases related to abnormal cell activity, including, but not limited to, the use thereof in the prevention and/or treatment of tumor diseases.

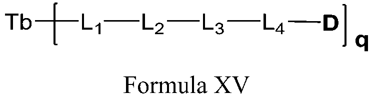

## Description

### TECHNICAL FIELD

The present disclosure belongs to the field of medical technology and relates to a variety of antibodies, an antibody-drug conjugate and a preparation method therefor, as well as a use thereof in the prevention and/or treatment of diseases related to abnormal cell activity, including, but not limited to, the use thereof in the prevention and/or treatment of tumor diseases.

### BACKGROUND

Chemotherapy using cytotoxin was once the standard treatment for cancer, but highly lethal cytotoxic molecules can kill normal cells and cause serious toxic side effects. Targeted anti-tumor drugs have become a hot topic in the field of cancer research today because they have both targeting properties and anti-tumor activity. However, due to the issue of target selectivity of targeted drugs, they often cause relatively significant toxic side effects, thereby limiting the therapeutic effect of targeted drugs. Biological macromolecule drugs, such as antibodies or antibody fragments, although highly targeted, have limited therapeutic effects on solid tumors or have no therapeutic effect at all. ADC is a conjugate of an antibody and a small molecule drug, which combines the targeting effect of antibodies with the activity of bioactive molecules. ADC has become a biological missile with promising advantages of efficacy and safety. The antibody guides the ADC to bind to target cells, followed by cellular internalization, and the small molecule drug is released inside the cells by enzymatic hydrolysis under the action of specific enzymes to treat diseases.

ADC drugs have developed very rapidly in recent years, and there are 14 types of ADCs on the market. ADCs targeting many targets, such as the antibody/ADC drugs targeting DLL3, are in the clinical research stage. For many targets, developing differentiated, higher-quality, and safer monoclonal antibodies or ADC drugs can provide broader and better drug options for patients with tumor, and also has broad market prospects.

### CONTENT OF THE PRESENT INVENTION

In order to improve the therapeutic effecacy of antibody-drug conjugates (ADCs), reduce drug toxicity side effects, and increase the therapeutic window, the present disclosure provides an antibody-drug conjugate of formula (XV) or a pharmaceutically acceptable salt or solvate thereof, which includes a bioactive molecule (drug molecule), a linker, and a targeting moiety, wherein the targeting moiety is connected to the linker via an active group (such as a sulfhydryl group) to form the antibody-drug conjugate. The present disclosure further develops a variable light chain domain (VL) and/or variable heavy chain (VH) domain containing anti-DLL3, wherein the variable light chain domain and/or variable heavy chain domain antibody is derived from a humanized antibody.

Thus, in a first aspect of the present disclosure, the present disclosure provides an antibody-drug conjugate of formula XV, 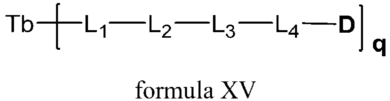
or a stereoisomer of the antibody-drug conjugate, a prodrug thereof, a pharmaceutically acceptable salt thereof, or a pharmaceutically acceptable solvate thereof,
wherein
Tb is an anti-DLL3 antibody or an antigen-binding fragment thereof;
q is a drug-to-antibody conjugation ratio;
D is a bioactive molecular fragment;
L₁ is an extension unit;
L₂ is absent or a connected unit;
L₃ is selected from an amino acid residue or a short peptide consisting of 2 to 10 amino acid residues;
L₄ is absent or present; when L₄ is present, L₄ is selected from 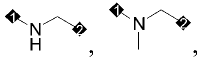 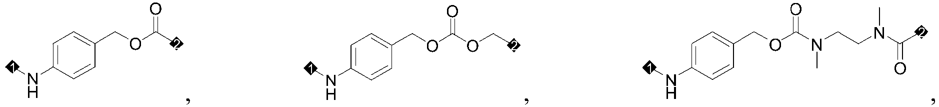 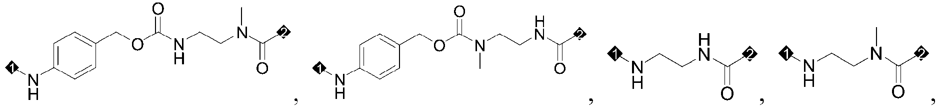 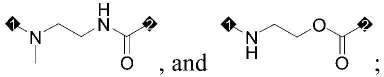 position 1 is attached to L₃ and position 2 is attached to D.

In addition, it should also be noted that for "position 1 of L₁ is attached to Tb via a Sulfur (S) atom", it can be understood by those skilled in the art that the position 1 of L₁ is attached to a sulfhydryl group contained in Tb (e.g., antibody) after a disulfide bond is broken (e.g., reduction of the disulfide bond by the reducing agent TCEP can break the disulfide bond to generate the sulfhydryl group -SH), that is, -S- between L₁ and Tb is not an additional extraneous sulfur atom. For example, in 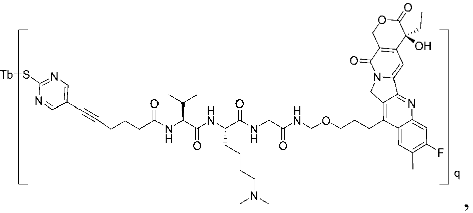 -Sis not an additional extraneous sulfur atom, but is formed by linking the sulfhydryl group contained in Tb to position 1 of L₁ such as 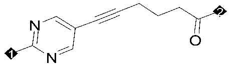 after the disulfide bond is broken.

The extension unit is a component of a antibody-drug conjugate, a drug-linker conjugate, or a linker, and its function is to connect the remaining part of the antibody-drug conjugate that binds to the target or the remaining part of the linker. The extension unit can connect Tb unit to L₂ (if present) or L₃. Specific examples include but are not limited to (where position 1 is attached to the antibody portion binding to the target and position 2 is attached to L₂ or L₃):

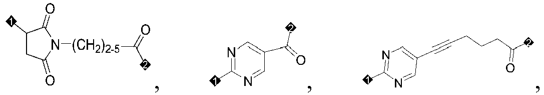

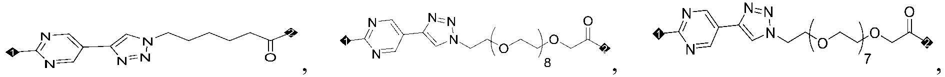

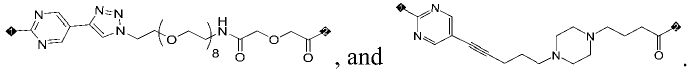

In some embodiments, L₁ is selected from: 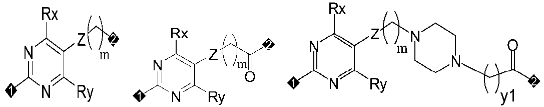 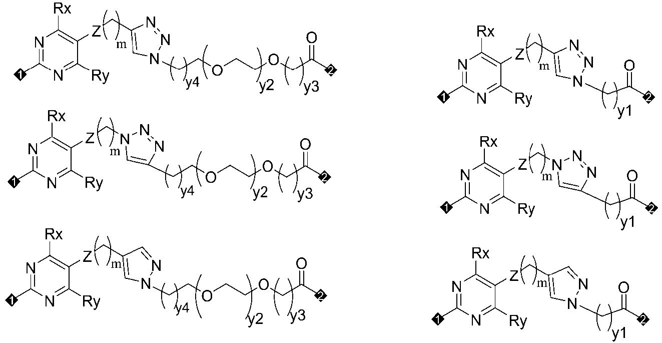 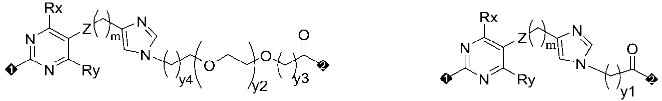 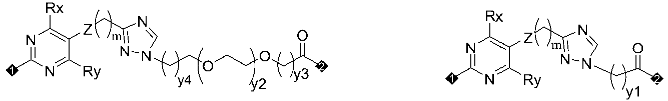 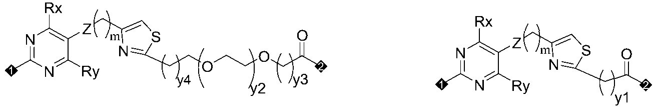 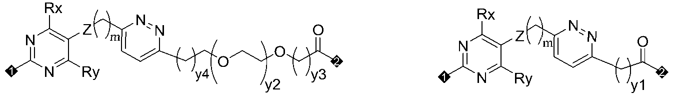 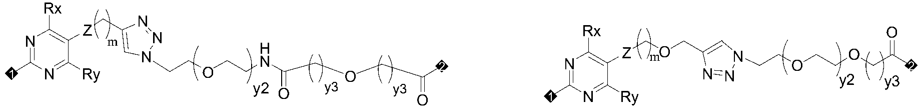 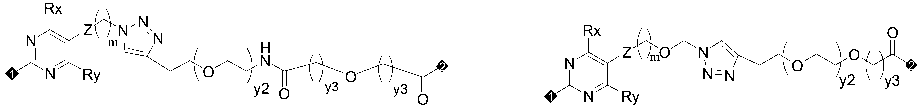 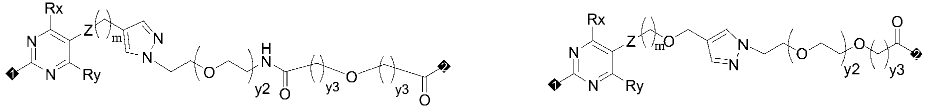 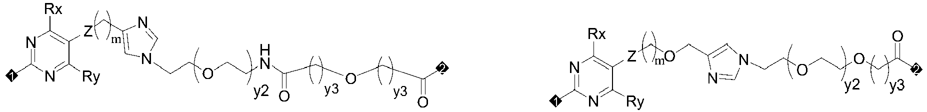 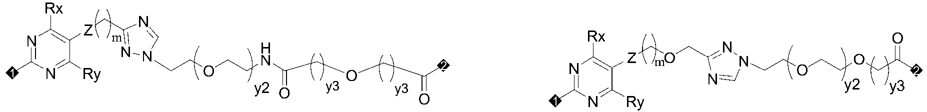 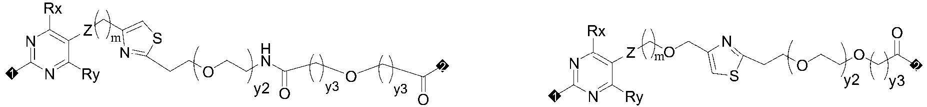 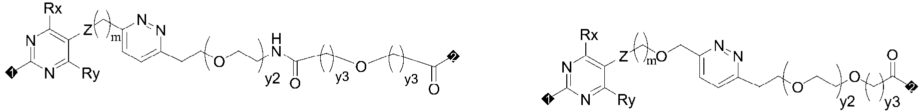 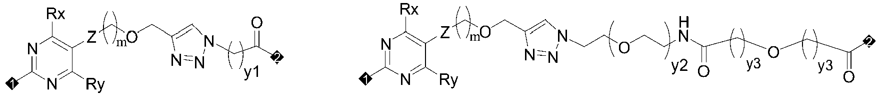 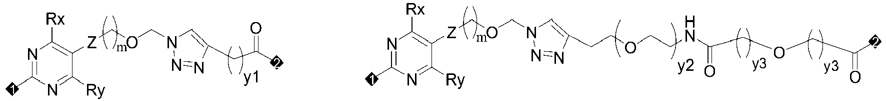 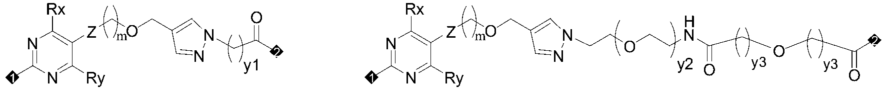 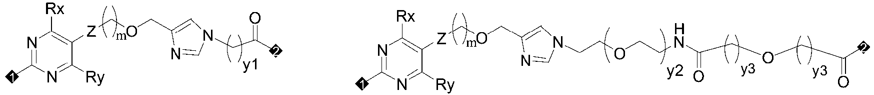 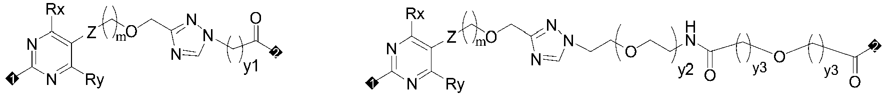 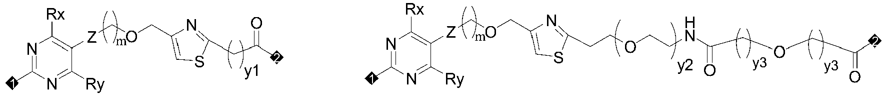 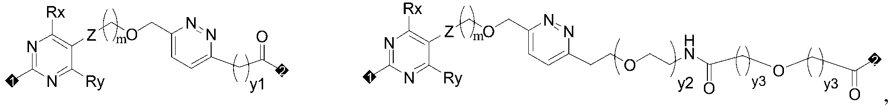
each Z is independently selected from a direct bond, a carbon-carbon triple bond, a carbon-carbon double bond, C6-10 aryl, 5- to 10-membered heteroaryl, amido, sulfonamido, imino, and CF₂;
Rx and Ry are independently selected from H and C1-4 alkyl;
each m is independently selected from 0, 1, 2, 3, 4, 5, and 6;
y1, y2, y3, and y4 are independently selected from any integer between 0 and 20;
position 1 is attached to Tb via a Sulfur (S) atom and position 2 is attached to L₂ or L₃.

The connected unit is a component of an antibody-drug conjugate, a drug-linker conjugate, or a linker, and its function is to connect the extension unit to an amino acid residue or a fragment or short peptide consisting of 1 to 10 amino acid residues. The connected unit, when present, can connect L₁ to L₃. Specific examples include but are not limited to (where position 1 is attached to the extension unit and position 2 is attached to L₃):

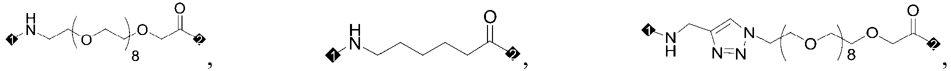

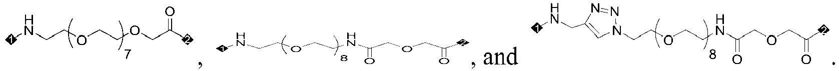

In some embodiments, L₂ is absent or present; when L₂ is present, L₂ is selected from:

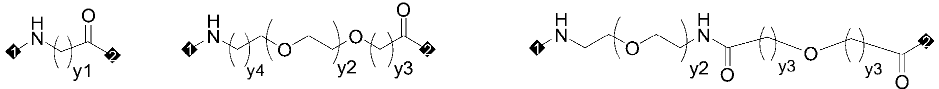

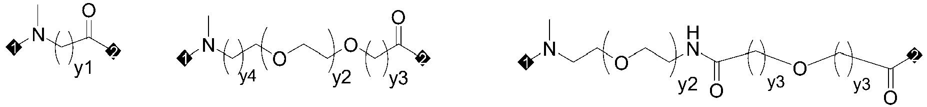

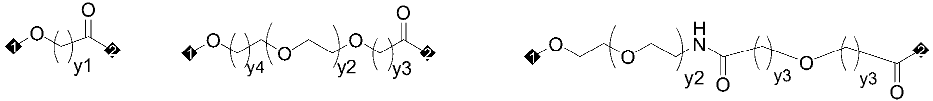

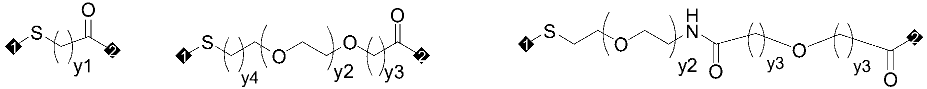

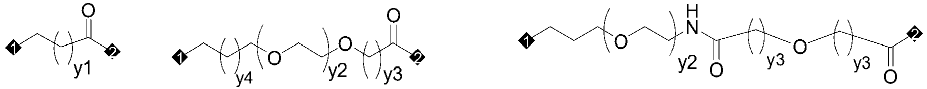

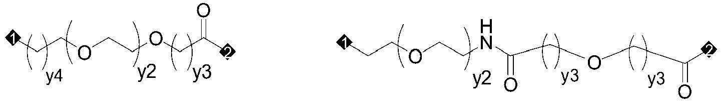

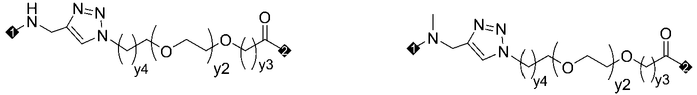

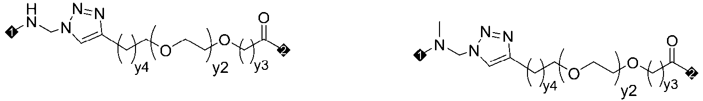

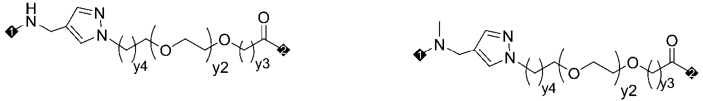

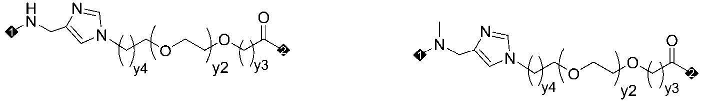

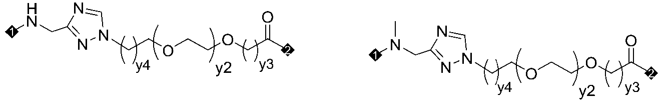

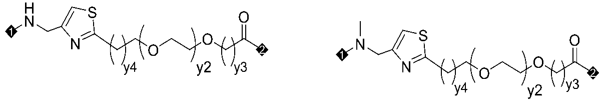

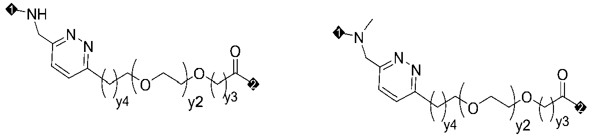

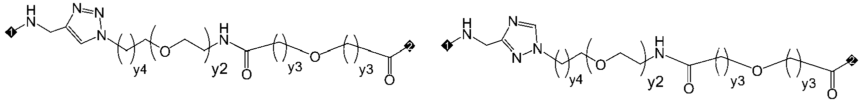

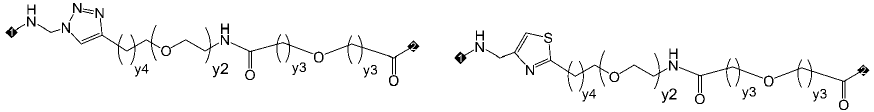

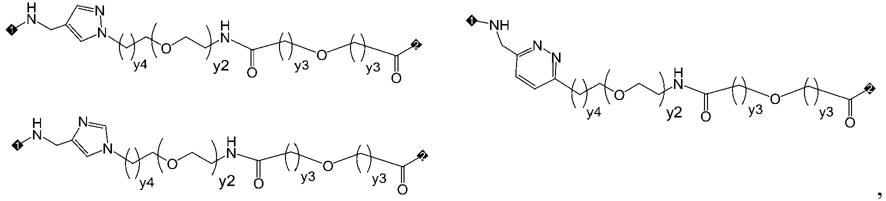

y1, y2, y3, and y4 are independently selected from any integer between 0 and 20; position 1 is attached to L₁ and position 2 is attached to L₃.

In some embodiments, L₁ is

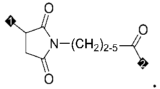

In some embodiments, L₁ is selected from 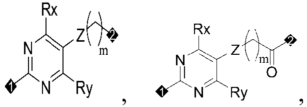 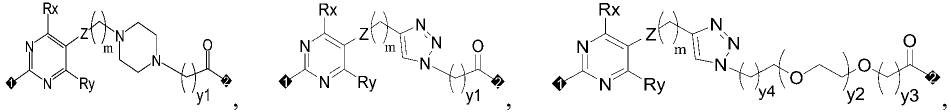 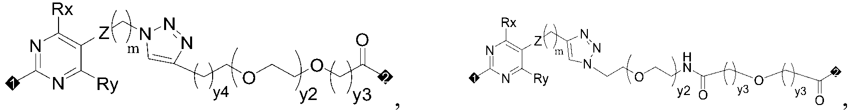 and 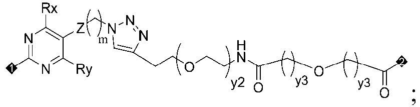 each Z is independently selected from a direct bond, a carbon-carbon triple bond, a carbon-carbon double bond, C6-10 aryl, 5- to 10-membered heteroaryl, and amido (preferably selected from a direct bond, a carbon-carbon triple bond, and a carbon-carbon double bond); Rx and Ry are independently selected from H and C1-4 alkyl; each m is independently selected from 0, 1, 2, 3, 4, 5, and 6; y1 is selected from any integer between 1 and 6 (such as 4, 5, and 6); each y2 is independently selected from any integer between 0 and 15 (such as 6 to 15); each y3 is independently selected from 1, 2, and 3; each y4 is independently selected from 0 and 1; position 1 is attached to Tb via a Sulfur (S) atom and position 2 is attached to L₂ or L₃.

In some embodiments, L₁ is selected from 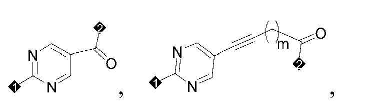 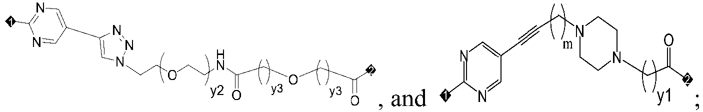 m is selected from 2, 3, and 4; y1 is selected from any integer between 1 and 6 (such as 4, 5, and 6); each y2 is independently selected from any integer between 0 and 10 (such as 6 to 10); each y3 is independently selected from 1 or 2; position 1 is attached to Tb via a Sulfur (S) atom and position 2 is attached to L₂ or L₃.

In some embodiments, L₁ is selected from

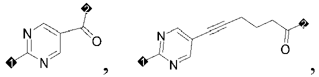

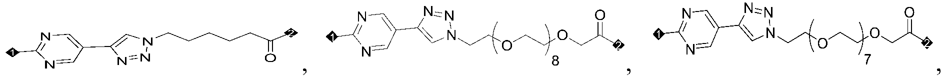

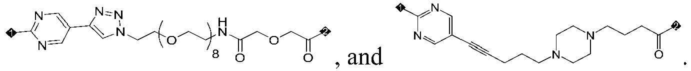

In some embodiments, L₁ is selected from 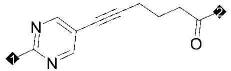 and 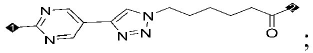 position 1 is attached to Tb via a Sulfur (S) atom and position 2 is attached to L₂ or L₃.

In some embodiments, L₂ is absent or present; when L₂ is present, L₂ is selected from 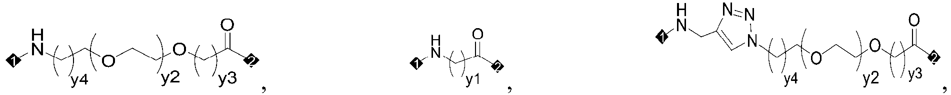 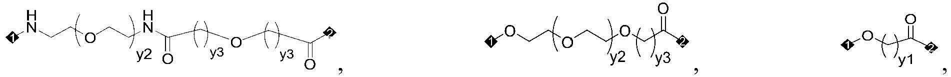 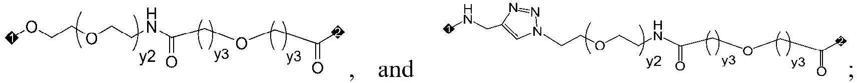 y1 is selected from any integer between 1 and 6 (such as 4, 5, and 6); each y2 is independently selected from any integer between 0 and 10 (such as 6 to 10); each y3 is independently selected from 1 or 2; each y4 is independently selected from 0 or 1; position 1 is attached to L₁ and position 2 is attached to L₃.

In some embodiments, L₂ is absent or present; when L₂ is present, L₂ is selected from 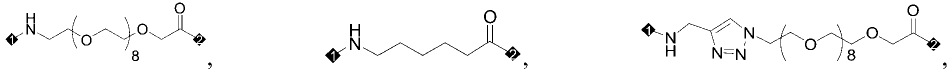 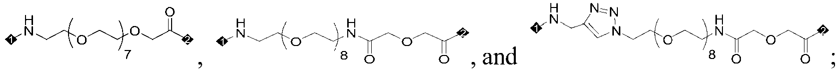 position 1 is attached to L₁ and position 2 is attached to L₃.

In some embodiments, L₂ is absent.

In some embodiments, L₂ is selected from

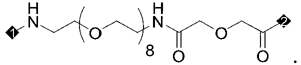

In some embodiments, L₃ is selected from an amino acid residue or a short peptide consisting of 2 to 10 amino acid residues; the amino acid residue is selected from a natural amino acid residue, an unnatural amino acid residue, or an amino acid residue represented by AA¹ or a stereoisomer thereof.

In some embodiments, L₃ is selected from amino acid residues Val, D-Val, Cit, Phe, Lys, Lys(Ac), Leu, Gly, Ala, Asn, Asp, Arg, and AA¹, or a short peptide consisting of 2 to 10 amino acid residues selected from Val, Cit, Phe, Lys, D-Val, Leu, Gly, Ala, Asn, Asp, and AA¹.

In some embodiments, L₃ is selected from Val, Cit, Phe, Lys, D-Val, Leu, Gly, Ala, Asn, AA¹, Val-Cit, Cit-Val, Cit-Ala, Val-Ala, Lys-Val, Val-Lys(Ac), Phe-Lys, Phe-Lys(Ac), Ala-Ala, Val-AA¹, Ala-AA¹, Gly-AA¹, AA¹-Gly, Ala-Ala-Ala, Ala-Ala-Asn, Ala-Ala-Asp, Val-AA¹-Gly, Ala-AA¹-Gly, Gly-AA¹-Gly, Lys-Ala-Ala-Asn, Lys-Ala-Ala-Asp, Gly-Phe-Gly, Gly-Gly-Phe-Gly, D-Val-Leu-Lys, Gly-Gly-Arg, Ala-Ala-Asn, Gly-Gly-Phe, Val-Lys-Gly, Val-Lys-Gly-Gly, Val-Lys, and Lys-Ala-Asn.

In some embodiments, L₃ is selected from AA¹, AA¹-Gly, Val-Cit, Val-AA¹-Gly, AA¹-Ala-Asn, and Gly-Gly-Phe-Gly.

In some embodiments, L₃ is selected from AA¹ and Val-AA¹-Gly.

In some embodiments, L₃ is selected from Val-AA¹-Gly.

In some embodiments, L₃ is selected from 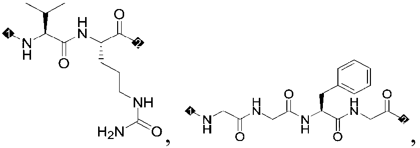 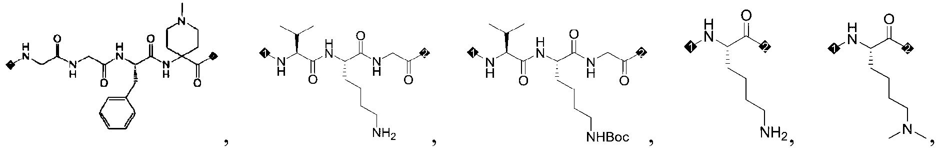 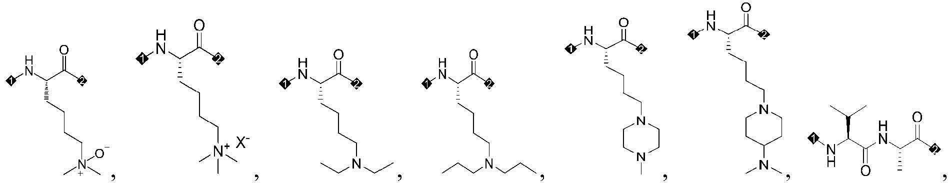 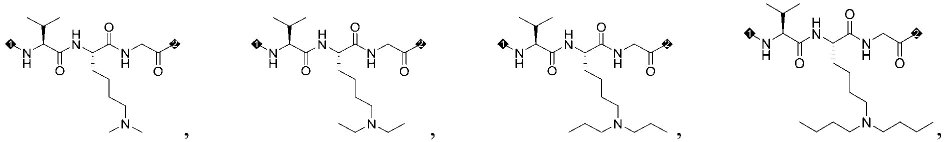 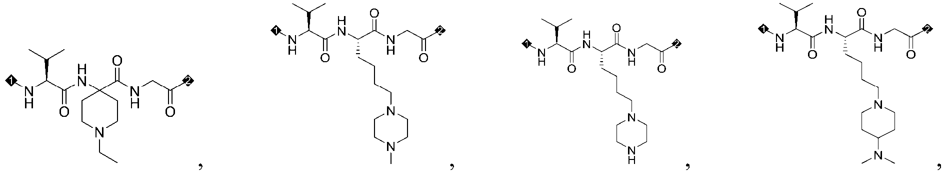 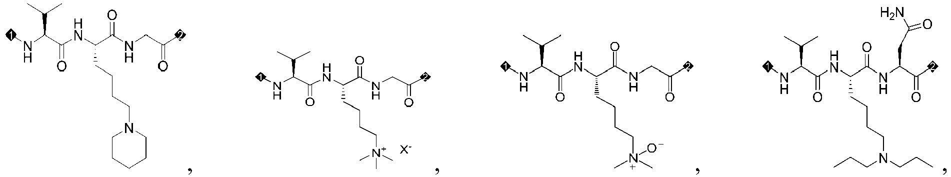 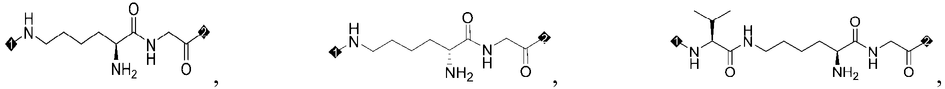 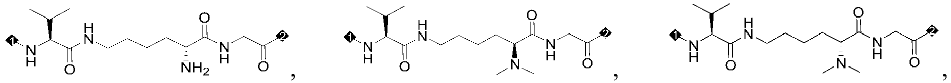 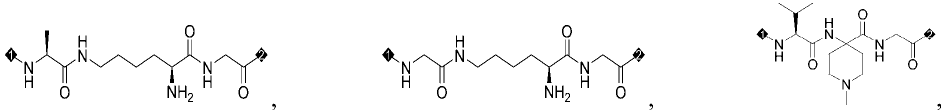 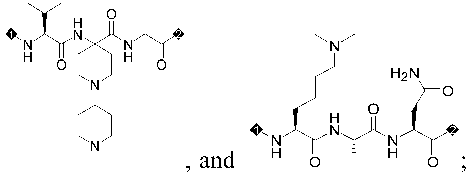 X is selected from a halide ion, a carboxylateion, a sulfate ion, a bisulfate ion, and OH⁻; position 1 is attached to L₁ or L₂ and position 2 is attached to L₄ or D.

In some embodiments, L₃ is selected from 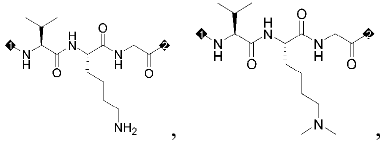 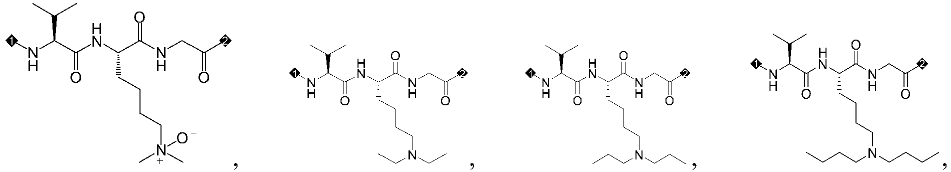 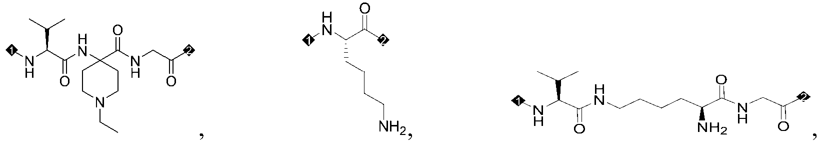 and 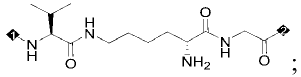 position 1 is attached to L₁ or L₂ and position 2 is attached to L₄ or D.

In some embodiments, L₃ is selected from 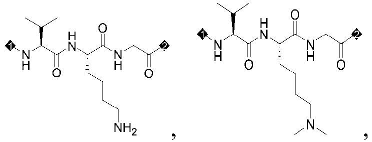 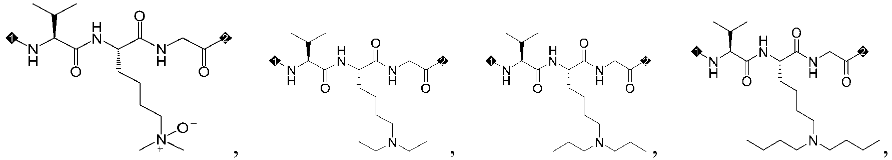 and 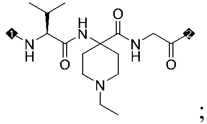 position 1 is attached to L₁ or L₂ and position 2 is attached to L₄ or D.

In some embodiments, the amino acid residue represented by AA¹ has a structure as follows, 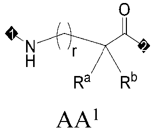 wherein
R^{a} and R^{b} are each independently selected from H, 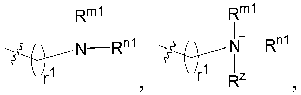 and 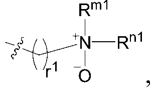 and R^{a} and R^{b} are not both H;
or, R^{a} and R^{b}, together with the carbon atom to which they are both attached, form a 4- to 10-membered heterocyclic ring; the 4- to 10-membered heterocyclic ring is optionally substituted by one or more R⁰;
r and r¹ are each independently selected from any integer between 0 and 20;
R^{m1} and Rⁿ¹ are each independently selected from H, C1-6 alkyl, C3-6 cycloalkyl, and -COOR^{x1};
R^{x1} is selected from C1-6 alkyl;
or, R^{m1} and Rⁿ¹, together with the nitrogen atom to which they are both attached, form a 4- to 10-membered heterocyclic ring; the 4- to 10-membered heterocyclic ring is optionally substituted by one or more R^{0'};
R^{z} is selected from C1-6 alkyl;
R⁰ and R^{0'} are each independently selected from C1-6 alkyl, C3-6 cycloalkyl, - NR^{m2}Rⁿ², and optionally C1-6 alkyl-substituted 4- to 10-membered heterocyclyl;
R^{m2} and Rⁿ² are each independently selected from H and C1-6 alkyl.

In some embodiments, either one of R^{a} and R^{b} is H and the other is selected from

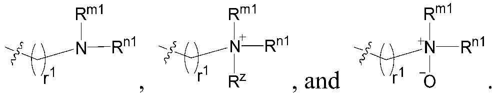

In some embodiments, either one of R^{a} and R^{b} is H and the other is selected from

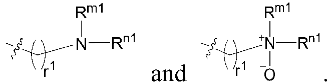

In some embodiments, R^{a} and R^{b}, together with the carbon atom to which they are both attached, form a 5- to 6-membered heterocyclic ring substituted by R⁰.

In some embodiments, R^{a} and R^{b}, together with the carbon atom to which they are both attached, form a piperidine ring substituted by R⁰ or a piperazine ring substituted by R⁰.

In some embodiments, R^{a} and R^{b}, together with the carbon atom to which they are both attached, form a piperidine ring substituted by R⁰.

In some embodiments, R^{a} and R^{b}, together with the carbon atom to which they are both attached, form 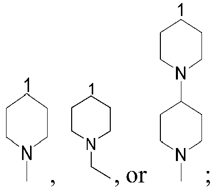 the carbon atom marked with number 1 is the carbon atom to which R^{a} and R^{b} are both attached.

In some embodiments, R^{a} and R^{b}, together with the carbon atom to which they are both attached, form 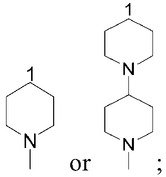 the carbon atom marked with number 1 is the carbon atom to which R^{a} and R^{b} are both attached.

In some embodiments, r and r¹ are each independently selected from 0, 1, 2, 3, 4, and 5.

In some embodiments, r and r¹ are each independently selected from 0 and 4.

In some embodiments, either one of r and r¹ is 0 and the other is 4.

In some embodiments, R^{m1} and Rⁿ¹ are each independently selected from H, methyl, ethyl, *n*-propyl, *n*-butyl, -COOCH₃, -COOCH₂CH₃, -COOCH₂CH₂CH₃, -COOCH(CH₃)₂, - COOC(CH₃)₃, and -COOCH₂CH₂CH₂CH₃.

In some embodiments, R^{m1} and Rⁿ¹ are each independently selected from H, C1-6 alkyl, C3-6 cycloalkyl, and *tert*-butoxycarbonyl.

In some embodiments, R^{m1} and Rⁿ¹ are each independently selected from H and C1-6 alkyl.

In some embodiments, R^{m1} and Rⁿ¹ are each independently selected from H, methyl, ethyl, and n-propyl.

In some embodiments, in r and r¹, when r is 4 and r¹ is 0, then R^{m1} and Rⁿ¹ are each independently selected from H and C1-6 alkyl (such as H, methyl); when r is 0 and r¹ is 4, then R^{m1} and Rⁿ¹ are each independently selected from C1-6 alkyl (such as methyl, ethyl, and *n-*propyl), preferably selected from C2-6 alkyl (such as ethyl and *n*-propyl).

In some embodiments, R^{m1} and Rⁿ¹, together with the nitrogen atom to which they are both attached, form a 5- to 6-membered heterocyclic ring optionally substituted by R^{0'}.

In some embodiments, R^{m1} and Rⁿ¹, together with the nitrogen atom to which they are both attached, form a piperidine ring optionally substituted by R^{0'} or a piperazine ring optionally substituted by R^{0'}.

In some embodiments, R^{m1} and Rⁿ¹, together with the nitrogen atom to which they are both attached, form 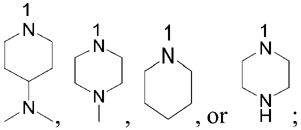 the nitrogen atom marked with number 1 is the nitrogen atom to which R^{m1} and Rⁿ¹ are both attached.

In some embodiments, R^{z} is methyl.

In some embodiments, R⁰ and R^{0'} are each independently selected from C1-6 alkyl, - NR^{m2}Rⁿ², and optionally C1-6 alkyl-substituted 5- to 6-membered heterocyclyl.

In some embodiments, R⁰ is selected from C1-6 alkyl and C1-6 alkyl-substituted 5- to 6-membered heterocyclyl; the 5- to 6-membered heterocyclyl is selected from piperidinyl and piperazinyl.

In some embodiments, R⁰ is selected from methyl, ethyl, and methyl-substituted 5- to 6-membered heterocyclyl; the 5- to 6-membered heterocyclyl is piperidinyl.

In some embodiments, R⁰ is selected from methyl and methyl-substituted 5- to 6-membered heterocyclyl; the 5- to 6-membered heterocyclyl is piperidinyl.

In some embodiments, R⁰ is selected from methyl, ethyl, and

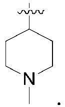

In some embodiments, R⁰ is selected from methyl and

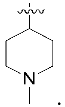

In some embodiments, R^{0'} is selected from C1-6 alkyl and -NR^{m2}ZRⁿ².

In some embodiments, R^{0'} is selected from methyl and -NR^{m2}Rⁿ².

In some embodiments, R^{m2} and Rⁿ² are methyl.

In some embodiments, the amino acid residue represented by AA¹ is selected from 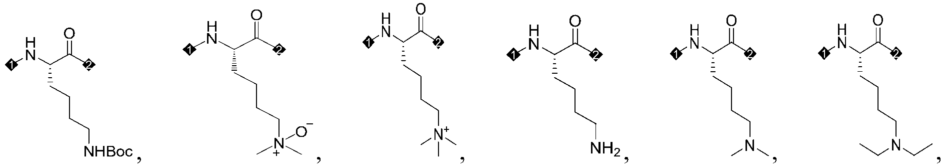 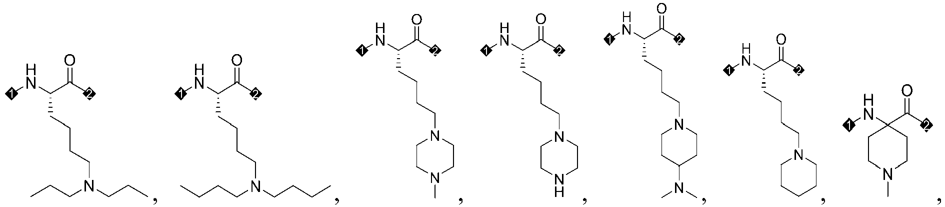 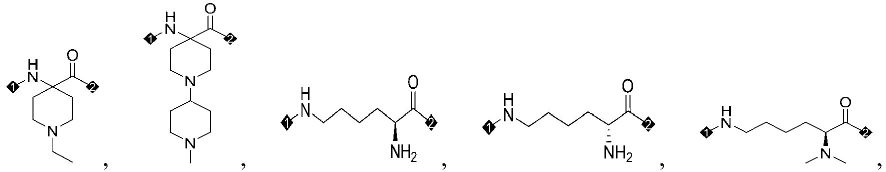 and

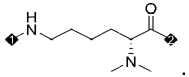

In some embodiments, the amino acid residue represented by AA¹ is selected from

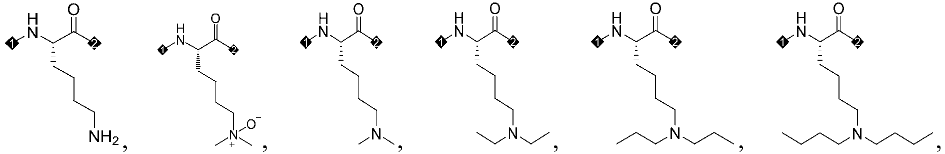

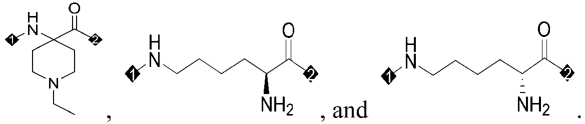

In some embodiments, the amino acid residue represented by AA¹ is selected from 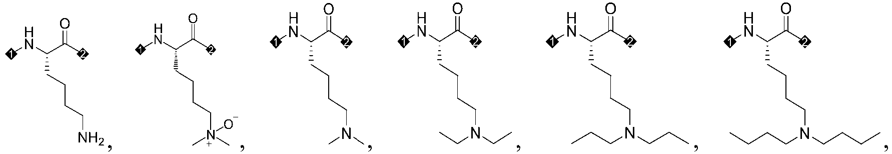 and

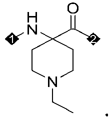

In some embodiments, L₄ is absent or present; when L₄ is present, L₄ is 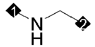 or 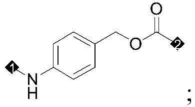 position 1 is attached to L₃ and position 2 is attached to D.

In some embodiments, L₄ is absent.

In some embodiments, L₄ is selected from 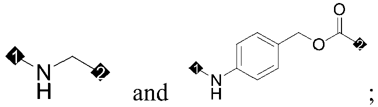 position 1 is attached to L₃ and position 2 is attached to D.

In some embodiments, L₄ is selected from 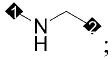 position 1 is attached to L₃ and position 2 is attached to D.

In some embodiments, the structure of 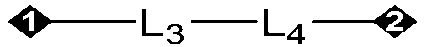 is selected from the following structural fragments:

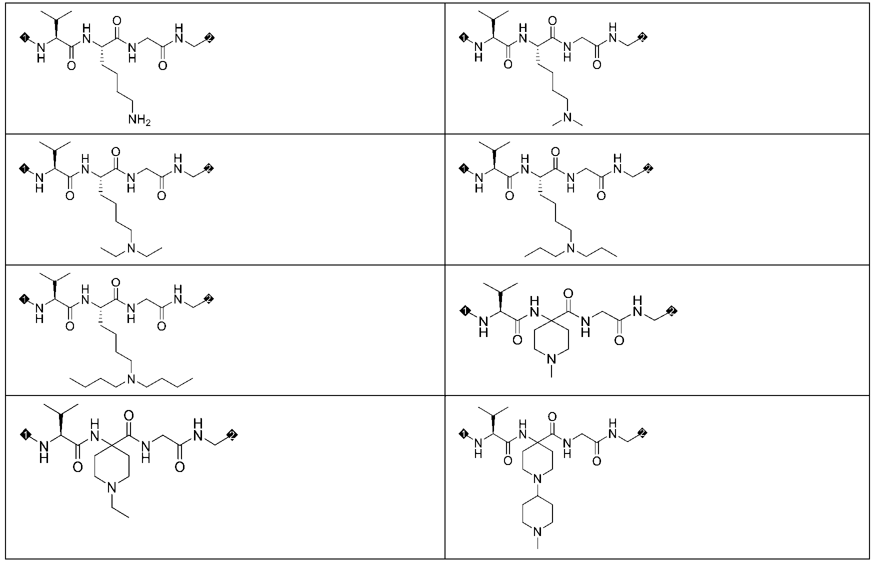

In some embodiments, q is selected from any value between 0.1 and 16.0; in preferred embodiments, q is selected from any integer between 0.1 and 16.0.

In some embodiments, q is selected from any value between 0.1 and 8.0; in preferred embodiments, q is selected from any integer between 0.1 and 8.0.

In some embodiments, q is selected from any value between 2 and 8.

In some embodiments, q is selected from any value between 3 and 8.

In some embodiments, q is selected from any value between 4 and 8.

In some embodiments, q is selected from any value between 6 and 8.

In some embodiments, q is selected from any integer between 2 and 8.

In some embodiments, q is selected from any integer between 3 and 8.

In some embodiments, q is selected from any integer between 4 and 8.

In some embodiments, q is selected from any integer between 6 and 8.

In some embodiments, q is selected from 1, 2, 3, 4, 5, 6, 7, 8, 9, 10, 11, and 12.

In some embodiments, q is selected from 2, 4, 6, and 8.

In the present disclosure, the bioactive molecule fragment refers to a part (fragment or group) of an antibody-drug conjugate (also known as ADC) that, as is known in the art, are capable of forming a bioactive drug (*e*.*g*., a small molecule cytotoxic drug, including the group of the drug that has lost an atom or atomic group) or a derivative thereof (*e*.*g*., a precursor thereof) after the linker is cleaved/degraded/enzymatically cleaved within tumor tissue or tumor cells. For the avoidance of ambiguity, the "drug" not only refers to the "medicine" that has been approved by the medical regulatory authorities, but also includes any molecule with potential therapeutic biological activity in clinic or in research and development and academic research.

In some embodiments, D is a molecular fragment with anti-tumor bioactivity.

In some embodiments, D is a molecular fragment with anti-tumor bioactivity, wherein the bioactive molecule is selected from a cytotoxic agent or a derivative thereof, such as a DNA topoisomerase inhibitor (*e*.*g*., a camptothecin bioactive molecule, such as camptothecin, DXD, camptothecin with a modified substituent, or DXD with a modified substituent) or a tubulin inhibitor (*e*.*g*., an MMAF tubulin inhibitor or an MMAE tubulin inhibitor).

In some embodiments, the antibody-drug conjugate has a structure of formula I: 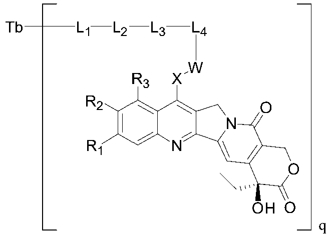 wherein
R₁ and R₂ are each independently selected from H, halogen, -OH, optionally substituted C1-6 alkyl, and optionally substituted C1-6 alkoxy, or,
R₁ and R₂, together with the carbon atom to which they are attached, form a 5- to 7-membered carbocyclic ring or a 5- to 7-membered heterocyclic ring, and the heterocyclic ring comprises one or more O, S, N, carbonyl, sulfinyl, sulfonyl, or any combination thereof;
R₃ is selected from H, halogen, -OH, -NH₂, optionally substituted C1-6 alkyl, and optionally substituted C1-6 alkoxy, or,
R₃ and X, together with the carbon atom to which they are attached, form a 5- to 7-membered carbocyclic ring or a 5- to 7-membered heterocyclic ring, and the heterocyclic ring comprises one or more O, S, N, carbonyl, sulfinyl, sulfonyl, or any combination thereof, or
R₃ and R₂, together with the carbon atom to which they are attached, form a 5- to 7-membered carbocyclic ring or a 5- to 7-membered heterocyclic ring, and the heterocyclic ring comprises one or more O, S, N, carbonyl, sulfinyl, sulfonyl, or any combination thereof;
W is absent or present; when W is present, W is selected from -O-, -S-, -NR₄-, 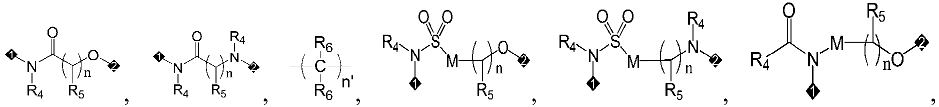 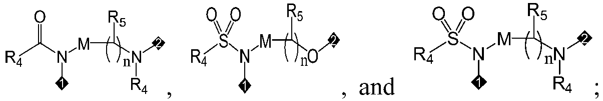 position 1 is attached to X and position 2 is attached to L₄ or L₃;
X is selected from a direct bond, optionally substituted -O-(CH₂)ₙ₃-, -N(R₄)-(CH₂)ₙ₃-, -S-(CH₂)ₙ₃-, carbonyl-(CH₂)ₙ₃, -SO₂-(CH₂)ₙ₃-, 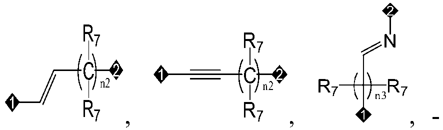 (CH₂)ₙₗ-, C3-6 cycloalkyl, C6-10 aryl, 5- to 10-membered heteroaryl, and 4- to 10-membered heterocyclyl; position 1 is attached to a parent ring and position 2 is attached to W or L₄; the substituent is selected from one or more C1-4 alkyl groups or C3-6 cycloalkyl groups; or multiple C1-4 alkyl groups, together with the carbon atom to which they are simultaneously attached, form a C3-6 cycloalkyl group;
each M is independently selected from a direct bond and -CR^{5a}R^{5b}-;
R₄, R₃, R^{5a}, R^{5b}, R₆, and R₇ are each independently selected from H, optionally substituted C1-4 alkyl, optionally substituted C1-4 alkoxy, and optionally substituted C3-6 cycloalkyl;
n, n', n1, n2, and n3 are each independently selected from any integer between 0 and 6;
L₄ is absent or present; when L₄ is present, L₄ is selected from 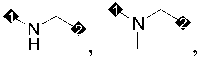 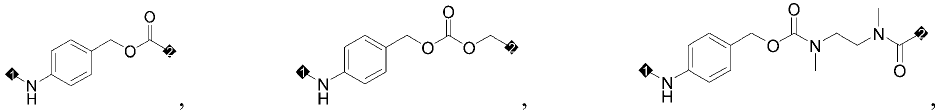 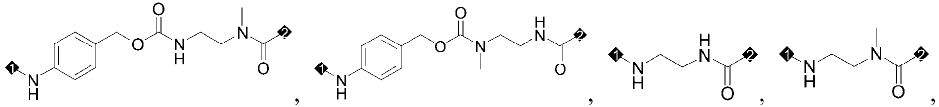 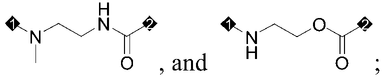 position 1 is attached to L₃ and position 2 is attached to W or X.
Tb, L₁, L₂, L₃, and q have the meanings provided above and in any embodiments specifically described herein.

In some embodiments, R₁ and R₂ are each independently selected from H, halogen, and C1-4 alkyl.

In some embodiments, R₁ and R₂, together with the carbon atom to which they are attached, form a 5- to 6-membered heterocyclic ring, and the heterocyclic ring comprises 1, 2, or 3 O, S, N, or any combination thereof.

In some embodiments, R₁ is selected from H and halogen, and R₂ is selected from H and C1-4 alkyl.

In some embodiments, R₁ and R₂, together with the carbon atom to which they are attached, form 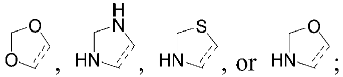 the dotted line indicates the position where the heterocyclic ring is fused with a benzene ring.

In some embodiments, R₁ is H or F, and R₂ is H or methyl.

In some embodiments, R₁ is F, R₂ is methyl; or, R₁ and R₂, together with the carbon atom to which they are attached, form

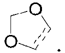

In some embodiments, R₁ is F, and R₂ is methyl.

In some embodiments, R₁ and R₂, together with the carbon atom to which they are attached, form

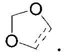

In some embodiments, R₃ is selected from H and C1-4 alkyl.

In some embodiments, R₃ and X, together with the carbon atom to which they are attached, form a 5- to 6-membered carbocyclic ring.

In some embodiments, R₃ is H; or, R₃ and X, together with the carbon atom to which they are attached, form 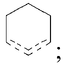 the dotted line indicates the position where the carbocyclic ring is fused with a benzene ring and a pyridine ring.

In some embodiments, R₃ is H.

In some embodiments, W is absent or present; when W is present, W is selected from -O-, -S-, -NR₄-, 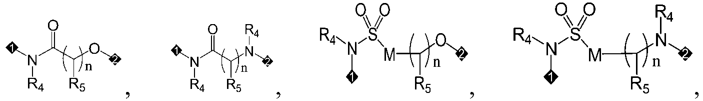 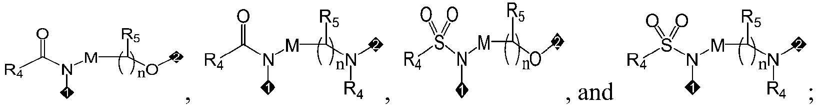 position 1 is attached to X and position 2 is attached to L₄ or L₃.

In some embodiments, W is absent or present; when W is present, W is selected from -O-, -S-, -NR₄-, 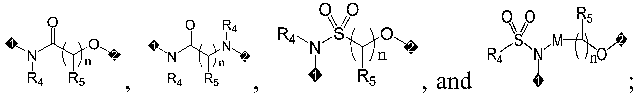 position 1 is attached to X and position 2 is attached to L₄ or L₃.

In some embodiments, W is selected from -O-, -NR₄-, and 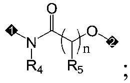 position 1 is attached to X and position 2 is attached to L₄ or L₃.

In some embodiments, W is selected from -O- and -NR₄-; position 1 is attached to X and position 2 is attached to L₄ or L₃.

In some embodiments, X is selected from optionally substituted -(CH₂)ₙ₁-, 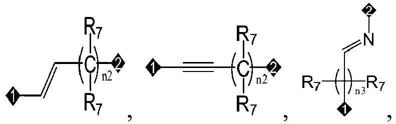 C6-10 aryl, 5- to 10-membered heteroaryl, and 4- to 10-membered heterocyclyl; position 1 is attached to a parent ring and position 2 is attached to W or L₄; the substituent is selected from 1 or 2 C1-4 alkyl groups; or 2 C1-4 alkyl groups, together with the carbon atom to which they are simultaneously attached, form a C3-6 cycloalkyl group.

In some embodiments, X is selected from optionally substituted 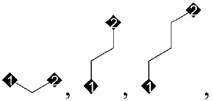 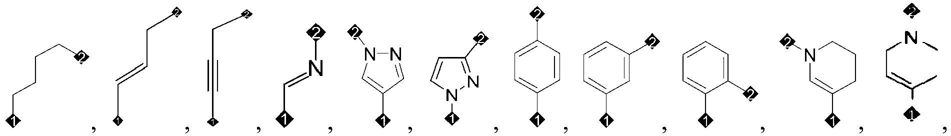 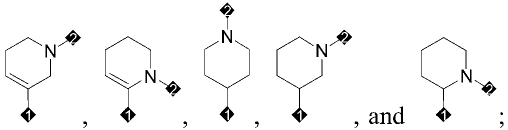 position 1 is attached to a parent ring and position 2 is attached to W or L₄; the substituent is selected from 1 or 2 C1-4 alkyl groups (such as methyl); or 2 C1-4 alkyl groups (such as methyl), together with the carbon atom to which they are simultaneously attached, form a C3-6 cycloalkyl group (such as cyclopropyl).

In some embodiments, X is selected from 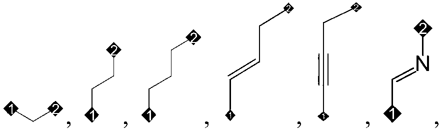 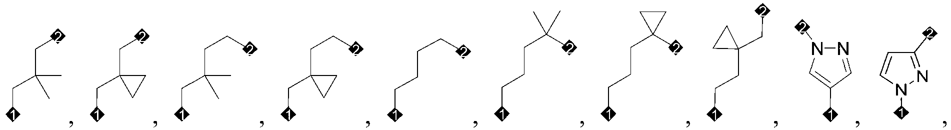 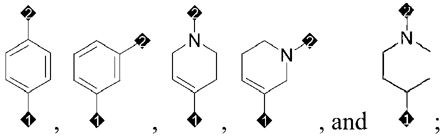 position 1 is attached to a parent ring and position 2 is attached to W or L₄.

In some embodiments, X is selected from 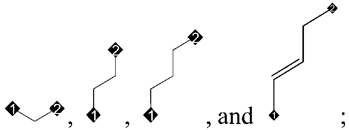 position 1 is attached to a parent ring and position 2 is attached to W.

In some embodiments, when W is absent, then X is selected from 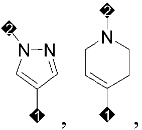 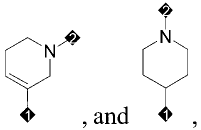 position 1 is attached to a parent ring and position 2 is attached to L₄; when W is present, then X is selected from 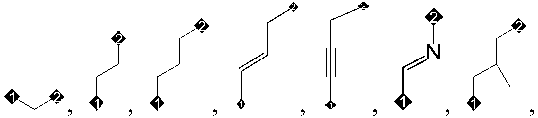 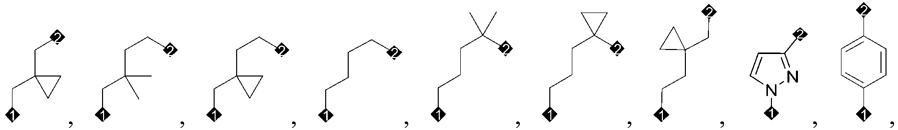 and 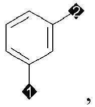 position 1 is attached to a parent ring and position 2 is attached to W.

In some embodiments, W is selected from -O-, -NR₄-, and 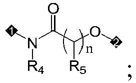 position 1 is attached to X and position 2 is attached to L₄ or L₃; X is selected from 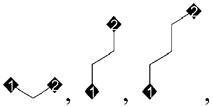 and 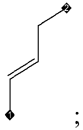 position 1 is attached to a parent ring and position 2 is attached to W.

In some embodiments, R₄ and R₅ are each independently selected from H, C1-4 alkyl, and C3-6 cycloalkyl.

In some embodiments, each R₄ is independently selected from H, C1-4 alkyl, and C3-6 cycloalkyl, and R₃ is H.

In some embodiments, each R₄ is independently selected from H, methyl, ethyl, *n-*propyl, isopropyl, *tert*-butyl, and cyclopropyl, and R₅ is H.

In some embodiments, R^{5a} and R^{5b} are each independently selected from H and C1-4 alkyl.

In some embodiments, R^{5a} and R^{5b} are each independently selected from H and methyl.

In some embodiments, each R₇ is independently selected from H and C1-4 alkyl.

In some embodiments, R₇ is H.

In some embodiments, n is selected from 1, 2, and 3.

In some embodiments, n is 1.

In some embodiments, n1 is selected from 1, 2, 3, and 4.

In some embodiments, n2 is 1.

In some embodiments, n3 is 0.

In some embodiments, L₃ is selected from 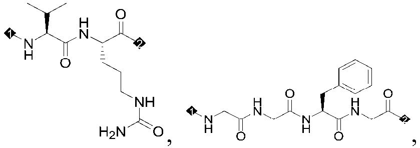 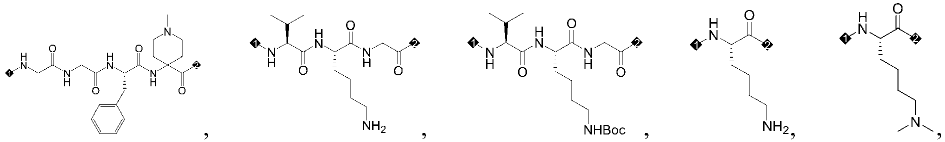 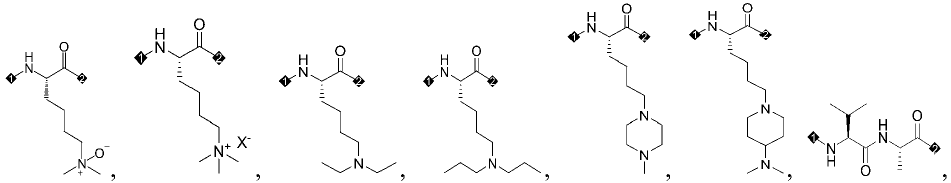 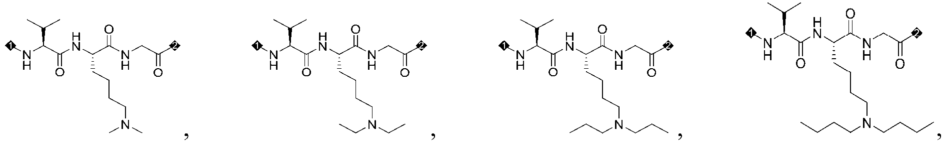 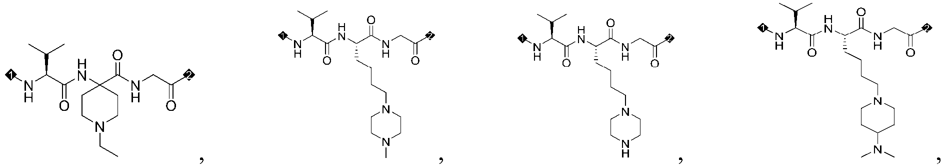 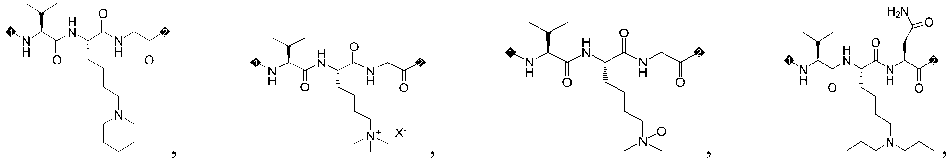 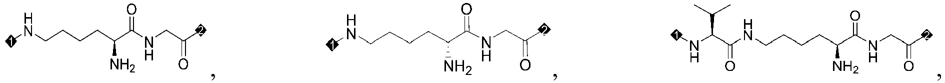 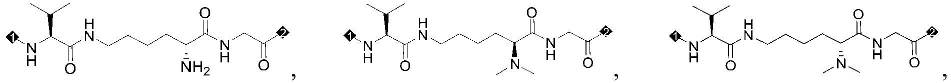 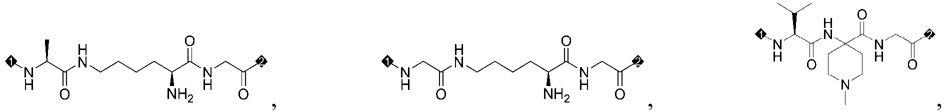 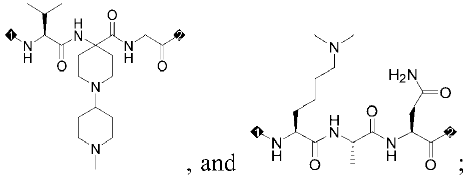 X⁻ is selected from a halide ion, a carboxylate ion, a sulfate ion, a bisulfate ion, and OH⁻; position 1 is attached to L₁ or L₂ and position 2 is attached to L₄ or W.

In some embodiments, L₃ is selected from 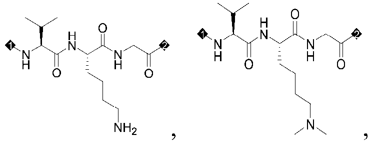 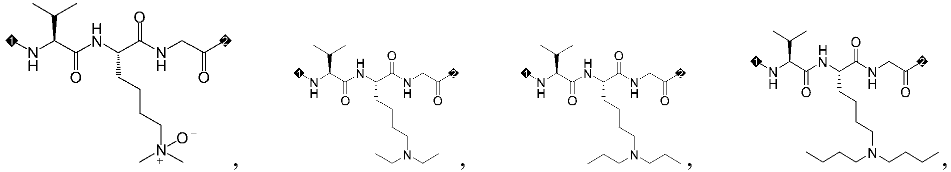 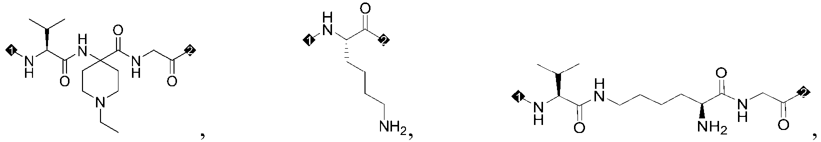 and 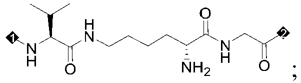 position 1 is attached to L₁ or L₂ and position 2 is attached to L₄ or W.

In some embodiments, L₃ is selected from 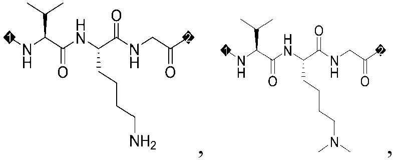 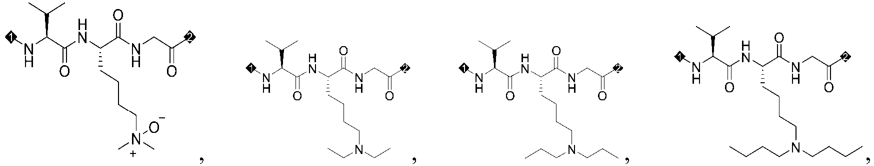 and 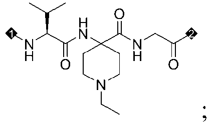 position 1 is attached to L₁ or L₂ and position 2 is attached to L₄ or W.

In some embodiments, L₄ is absent or present; when L₄ is present, L₄ is 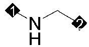 or 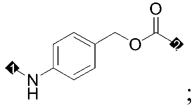 position 1 is attached to L₃ and position 2 is attached to W or X.

In some embodiments, L₄ is absent.

In some embodiments, L₄ is selected from 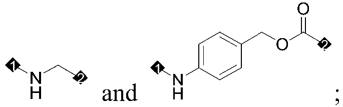 position 1 is attached to L₃ and position 2 is attached to W or X.

In some embodiments, L₄ is selected from 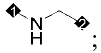 position 1 is attached to L₃ and position 2 is attached to W or X.

It should be noted that, as mentioned above, W is absent or present. Therefore, when W is absent, position 1 of L₄ is attached to L₃ and position 2 is attached to X; when W is present, position 1 of L₄ is attached to L₃ and position 2 is attached to W. The following connection relationship of L₄ can be understood with reference to the foregoing content.

In some embodiments, the structure of 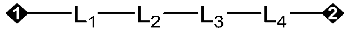 is selected from the following structural fragments:

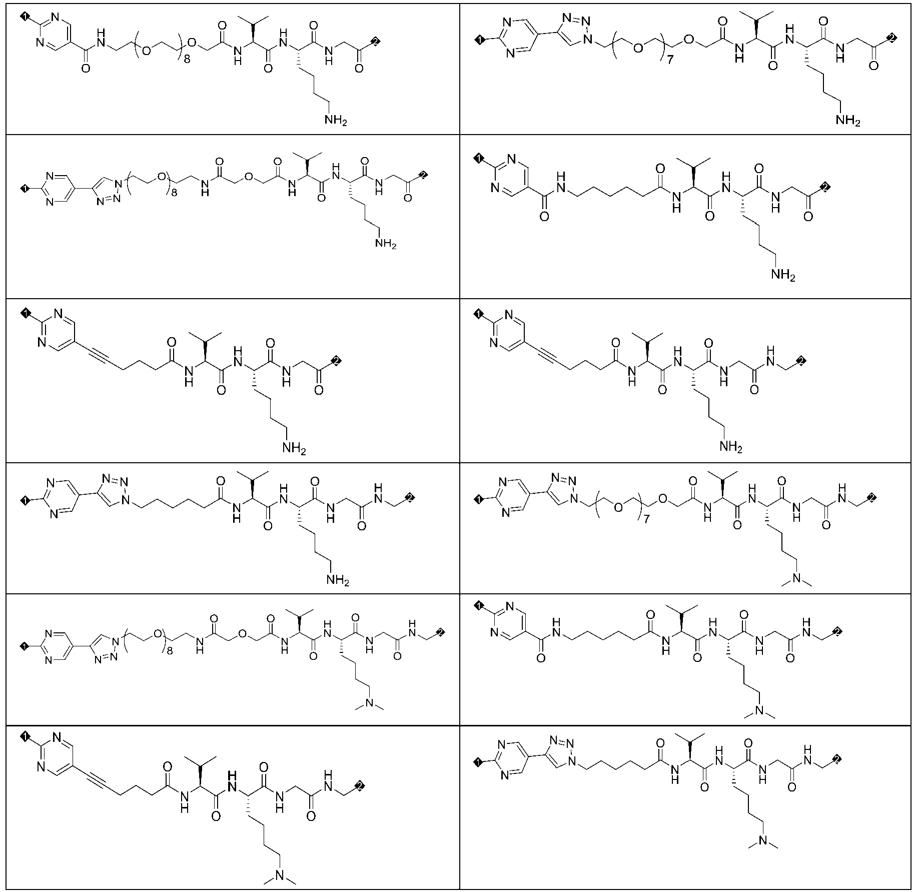

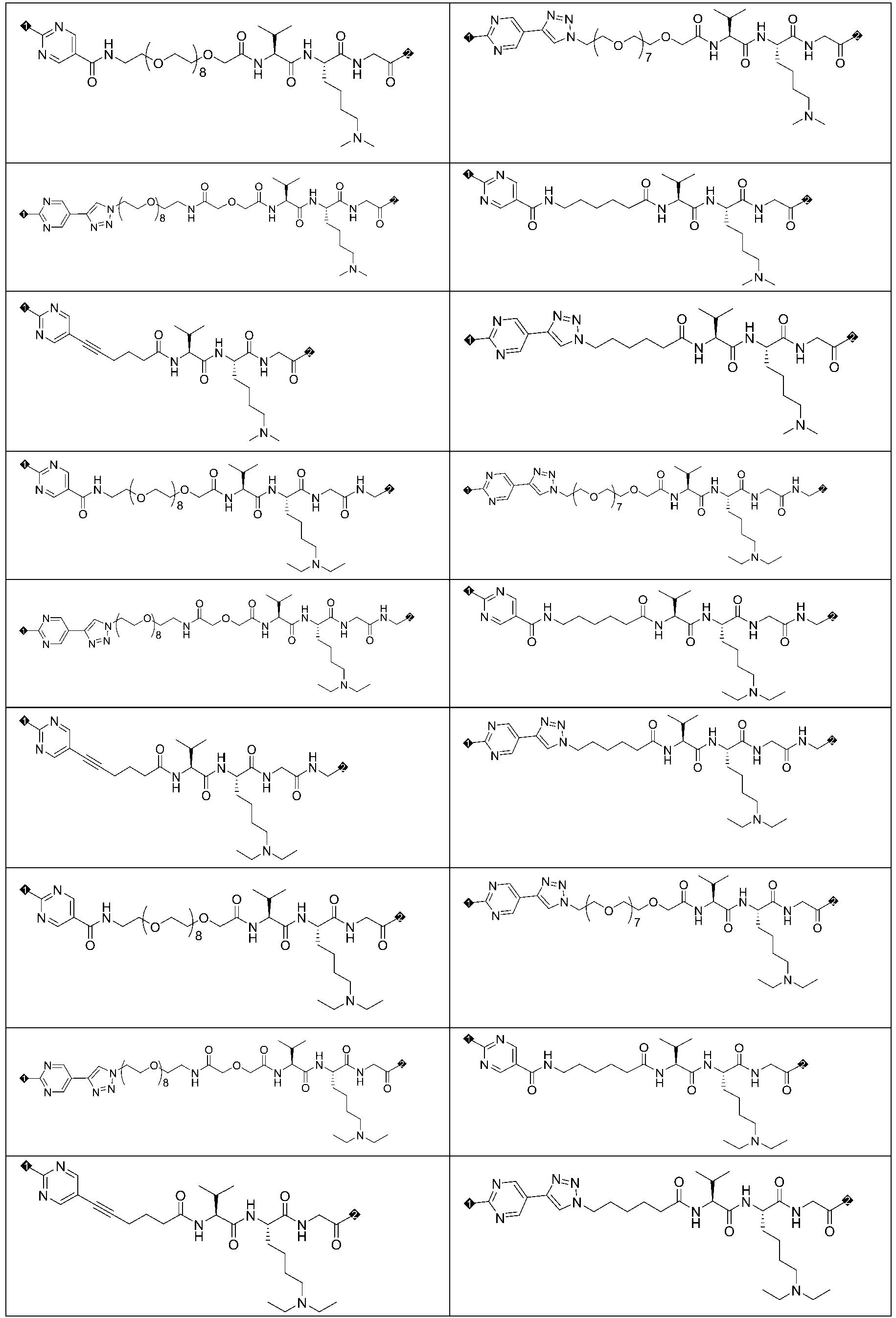

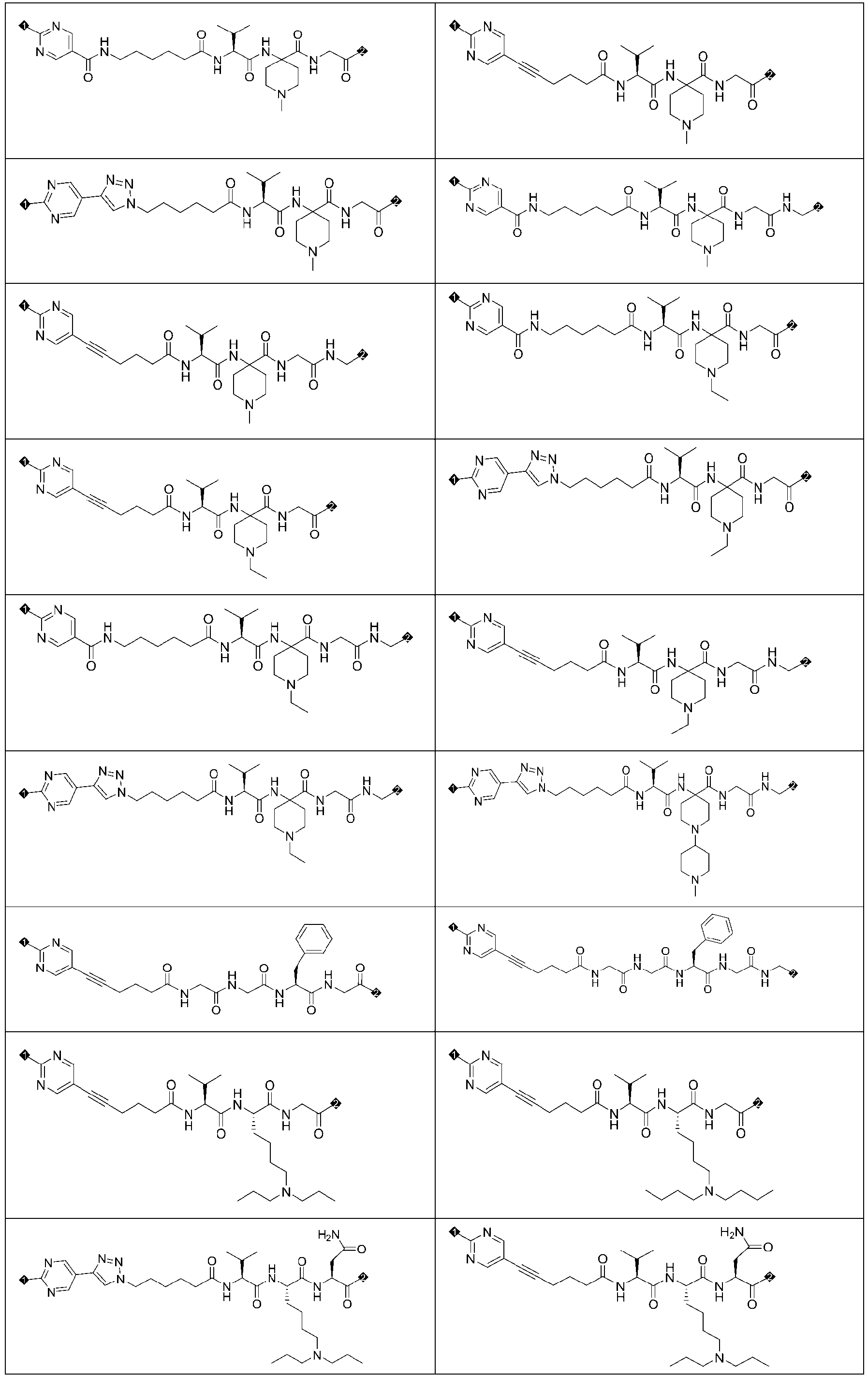

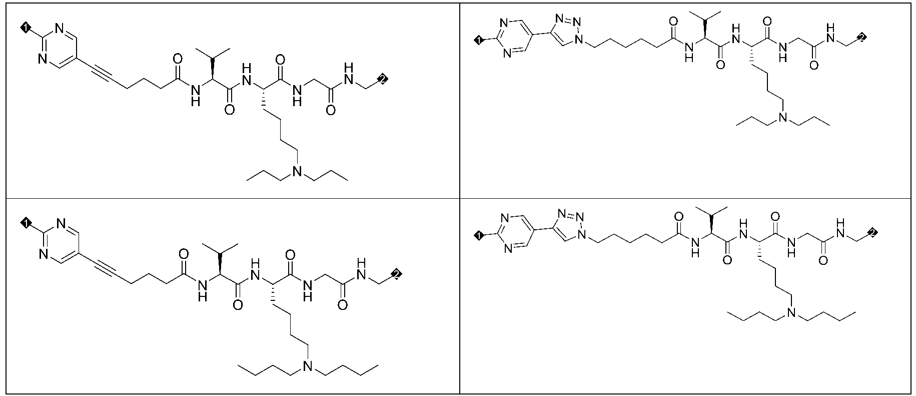

wherein position 1 is attached to Tb and position 2 is attached to W.

In some embodiments, D is the structural fragment shown in 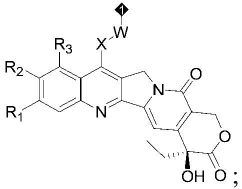 position 1 is attached to L₃ or L₄; for example, 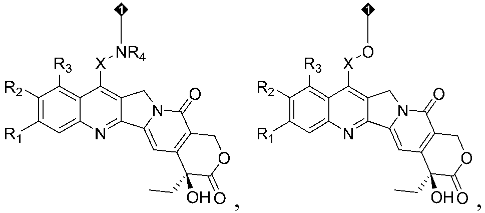 and

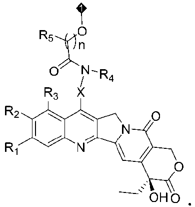

In some embodiments, the structure of 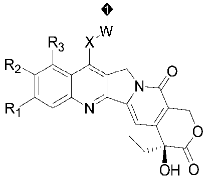 is selected from the following structural fragments: 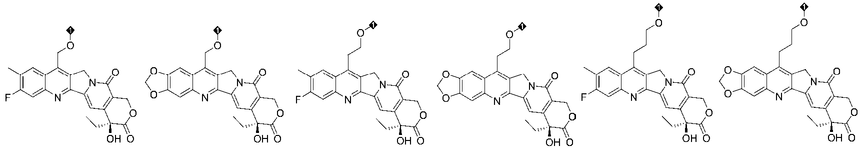 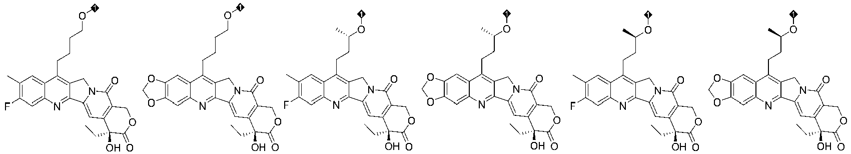 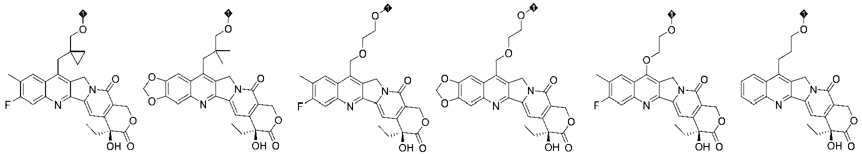 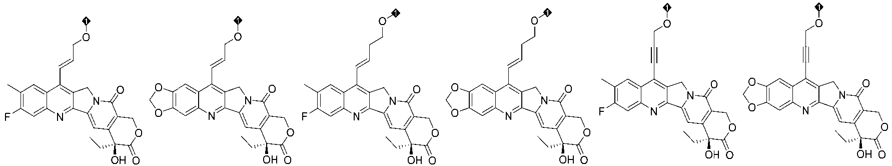 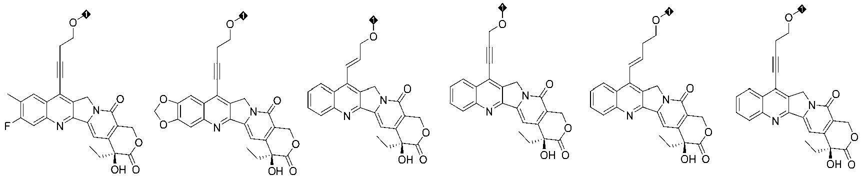 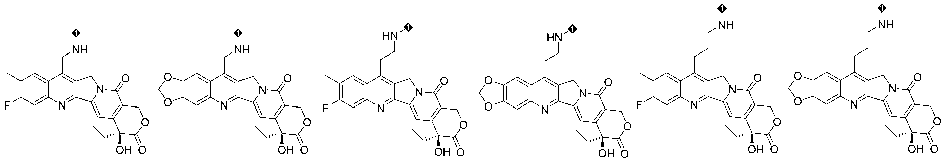 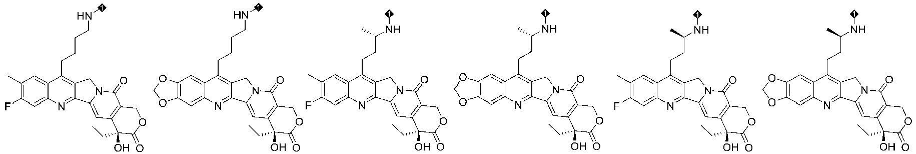 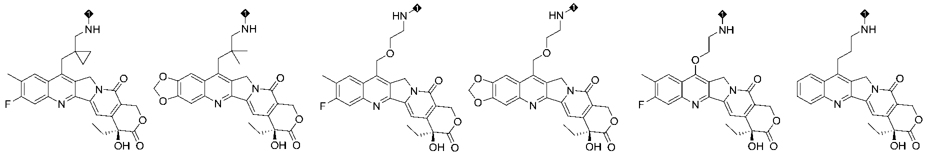 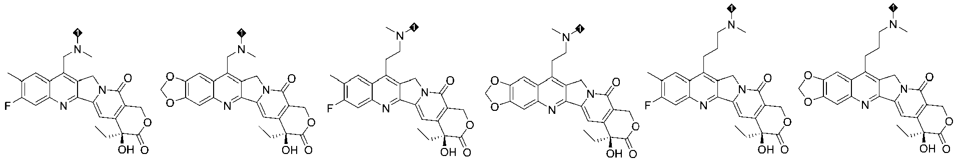 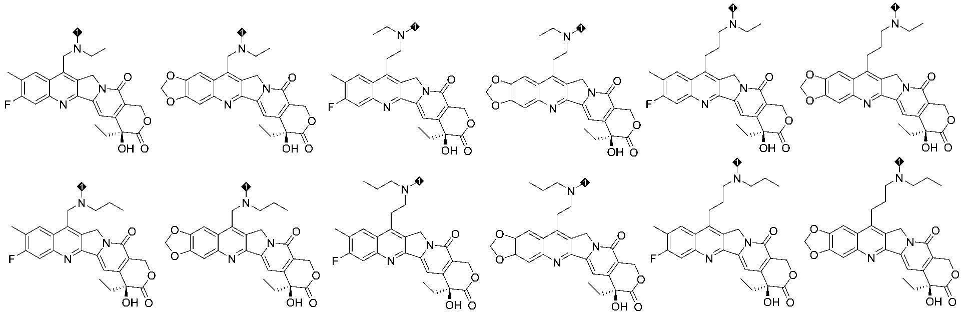 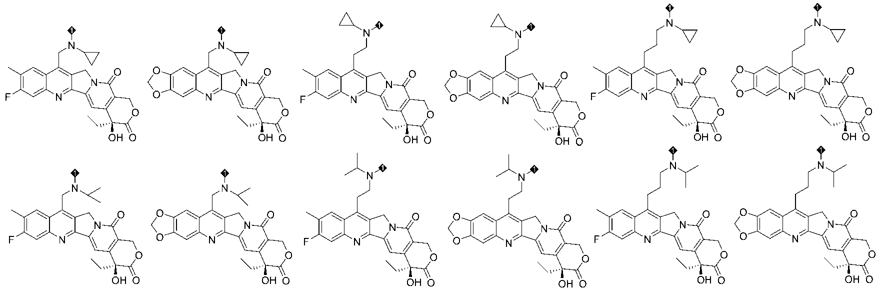 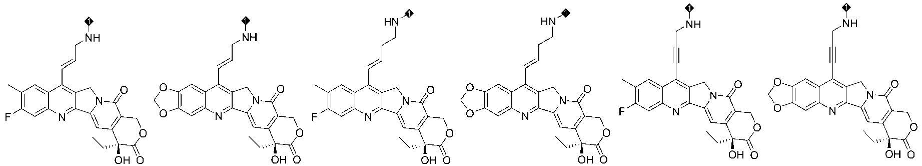 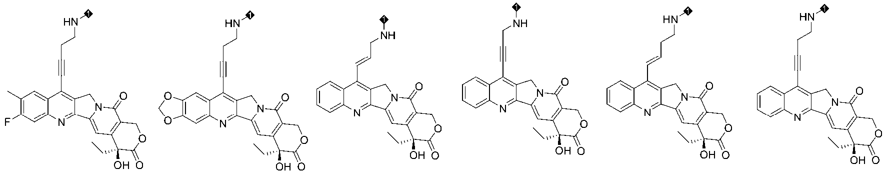 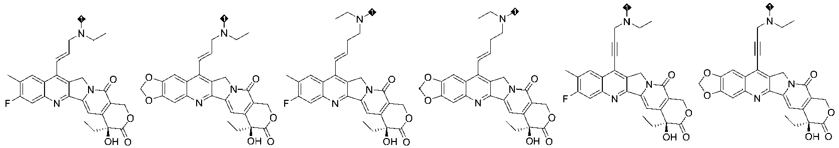 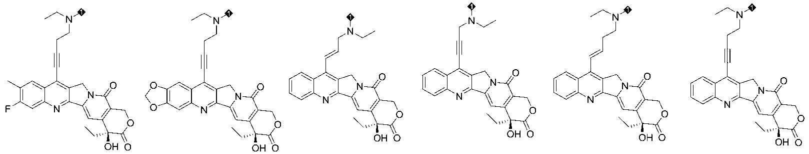 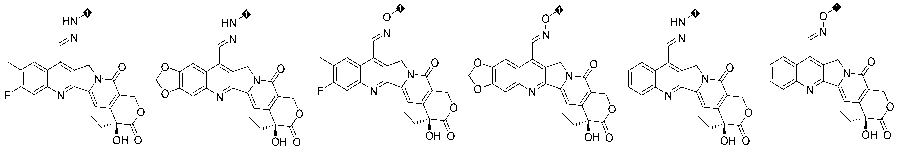 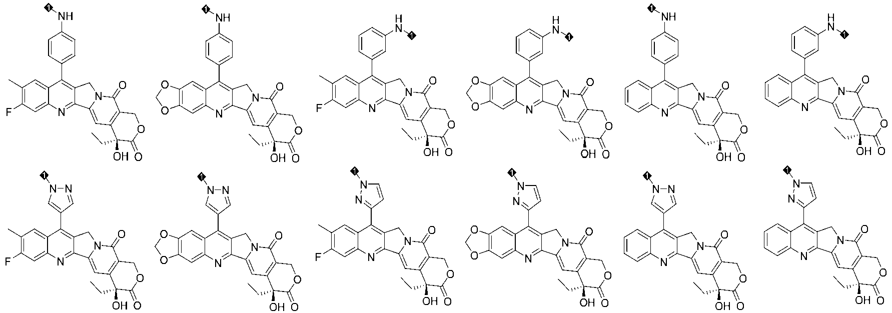 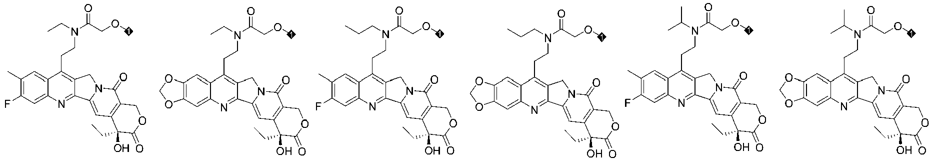 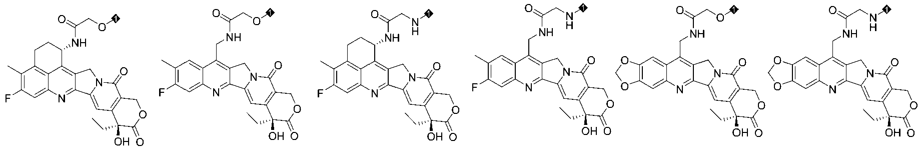 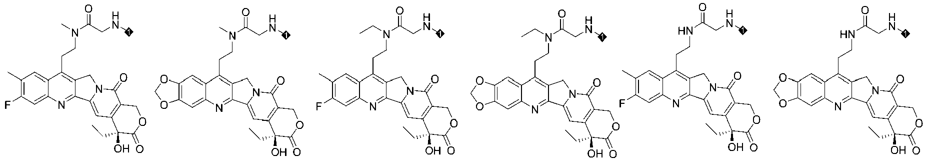 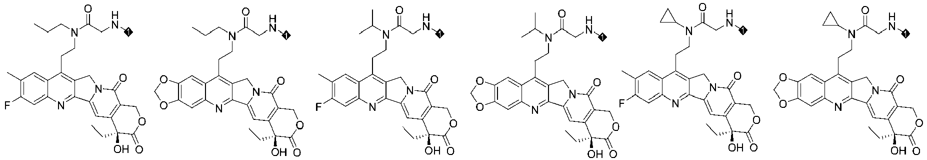 wherein position 1 is attached to L₄; when L₄ is absent, position 1 is attached to L₃.

In some embodiments,
W is absent or present; when W is present, W is selected from -O-, -S-, -NR₄-, 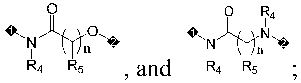 for example, absent, -O-, -NR₄-, or 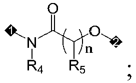 R₄ and R₃ are each independently selected from H and C1-4 alkyl; n is independently selected from 0, 1, 2, 3, and 4;
X is selected from 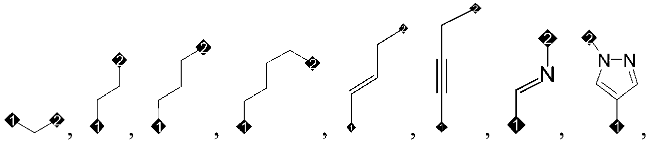 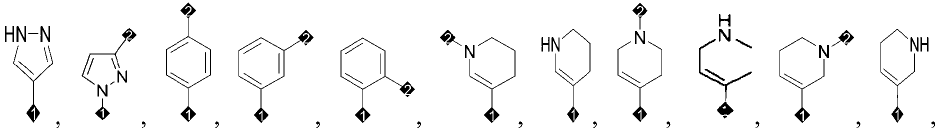 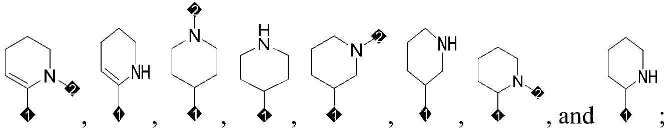
R₁ is selected from H and halogen, and R₂ is selected from H and C1-4 alkyl; or, R₁ and R₂, together with the carbon atom to which they are attached, form 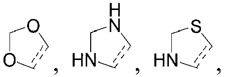 or 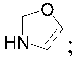 the dotted line indicates the position where the heterocyclic ring is fused with a benzene ring;
R₃ is selected from H and C1-4 alkyl; or, R₃ and X, together with the carbon atom to which they are attached, form a 5- to 6-membered carbocyclic ring;
preferably, W is absent or present; when W is present, W is selected from -O-, -NR₄-(for example, -NH-, -N(CH₃)-, and -N(C₂H₅)-), 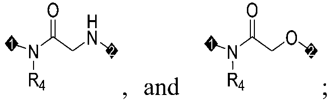 R₄ is independently selected From H, methyl, ethyl, isopropyl, *n*-propyl, *tert*-butyl and cyclopropyl;
when W is absent, X is selected from 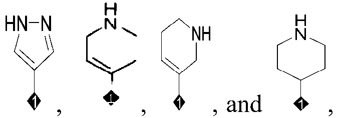 position 1 is attached to a parent ring; when W is present, X is selected from 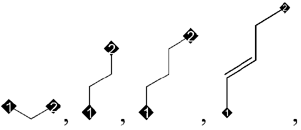 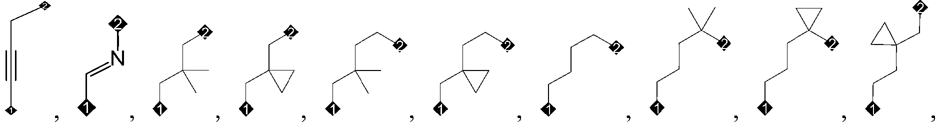 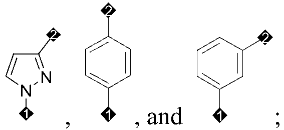
in AA¹ 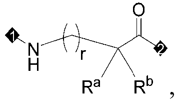 r is selected from 0, 1, 2, 3, 4, and 5;
either one of R^{a} and R^{b} is H and the other is selected from 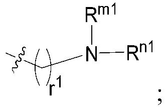 or, R^{a} and R^{b}, together with the carbon atom to which they are both attached, form a 5- to 6-membered heterocyclic ring substituted by R⁰;
R₁ is selected from H and halogen, and R₂ is selected from H and C1-4 alkyl; or, R₁ and R₂, together with the carbon atom to which they are attached, form 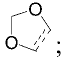
R₃ is selected from H and C1-4 alkyl; or, R₃ and X, together with the carbon atom to which they are attached, R₃ and X, together with the carbon atom to which they are attached, form 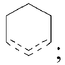
more preferably, W is selected from -O- and -NR₄-; position 1 is attached to X and position 2 is attached to L₄ or L₃;
X is selected from 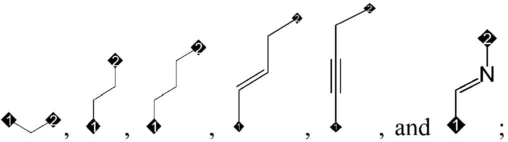 position 1 is attached to a parent ring and position 2 is attached to W; 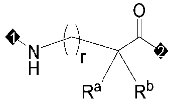
is 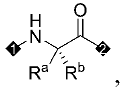 for example, 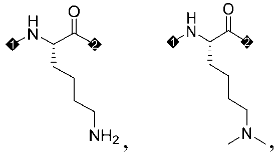 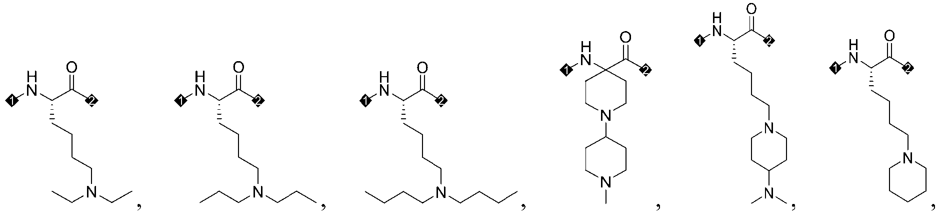 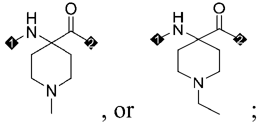
R₁ is F,
R₂ is methyl; or, R₁ and R₂, together with the carbon atom to which they are attached, form 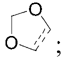
R₃ is H.

In some embodiments, the antibody-drug conjugate has a structure of formula I-1: 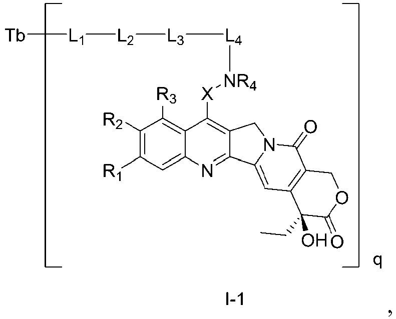 wherein Tb, L₁, L₂, L₃, L₄, X, R₁, R₂, R₃, R₄, and q have the meanings provided above and in any embodiments specifically described herein.

In some embodiments, the antibody-drug conjugate has a structure of formula I-1A or I-1B: 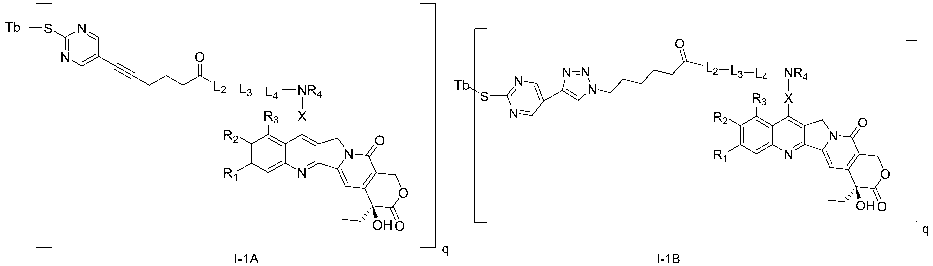 wherein Tb, L₂, L₃, L₄, X, R₁, R₂, R₃, R₄, and q have the meanings provided above and in any embodiment specifically described herein.

In some embodiments, the antibody-drug conjugate has a structure of formula I-2: 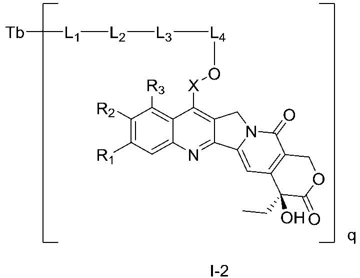 wherein Tb, L₁, L₂, L₃, L₄, X, R₁, R₂, R₃, and q have the meanings provided above and in any embodiments specifically described herein.

In some embodiments, the antibody-drug conjugate has a structure of formula I-2A or I-2B: 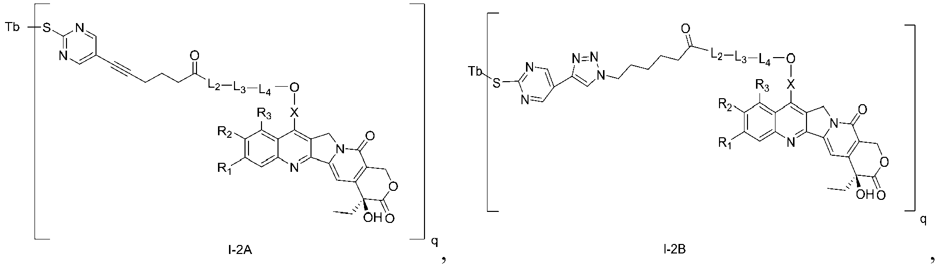 wherein Tb, L₂, L₃, L₄, X, R₁, R₂, R₃, and q have the meanings provided above and in any embodiments specifically described herein.

In some embodiments, the antibody-drug conjugate has a structure of formula I-3: 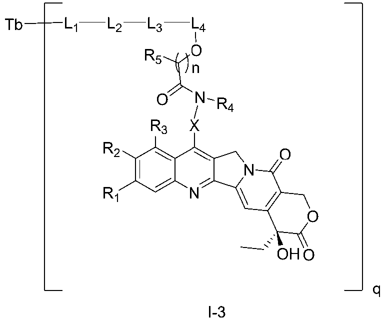 wherein Tb, L₁, L₂, L₃, L₄, X, R₁, R₂, R₃, R₄, R₅, n, and q have the meanings provided above and in any embodiments specifically described herein.

In some embodiments, the antibody-drug conjugate has a structure of formula I-3A or I-3B: 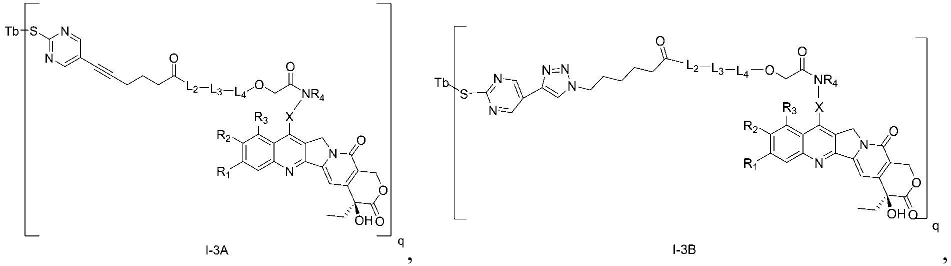 wherein Tb, L₂, L₃, L₄, X, R₁, R₂, R₃, R₄, and q have the meanings provided above and in any embodiments specifically described herein.

In some embodiments, the antibody-drug conjugate has a structure of formula I-A: 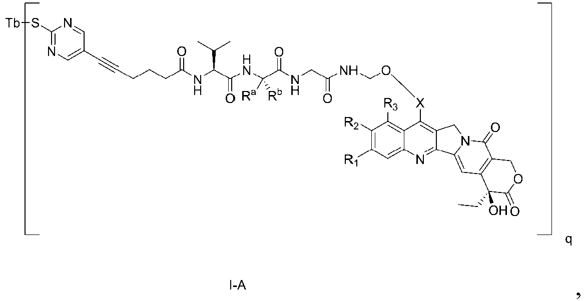 wherein Tb, X, R₁, R₂, R₃, R^{a}, R^{b}, and q have the meanings provided above and in any embodiments specifically described herein.

In some embodiments, the antibody-drug conjugate has a structure of formula I-B: 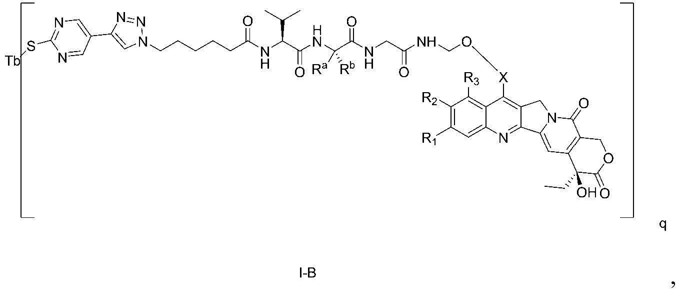 wherein Tb, X, R₁, R₂, R₃, R^{a}, R^{b}, and q have the meanings provided above and in any embodiments specifically described herein.

In some embodiments, the antibody-drug conjugate is selected from the following:

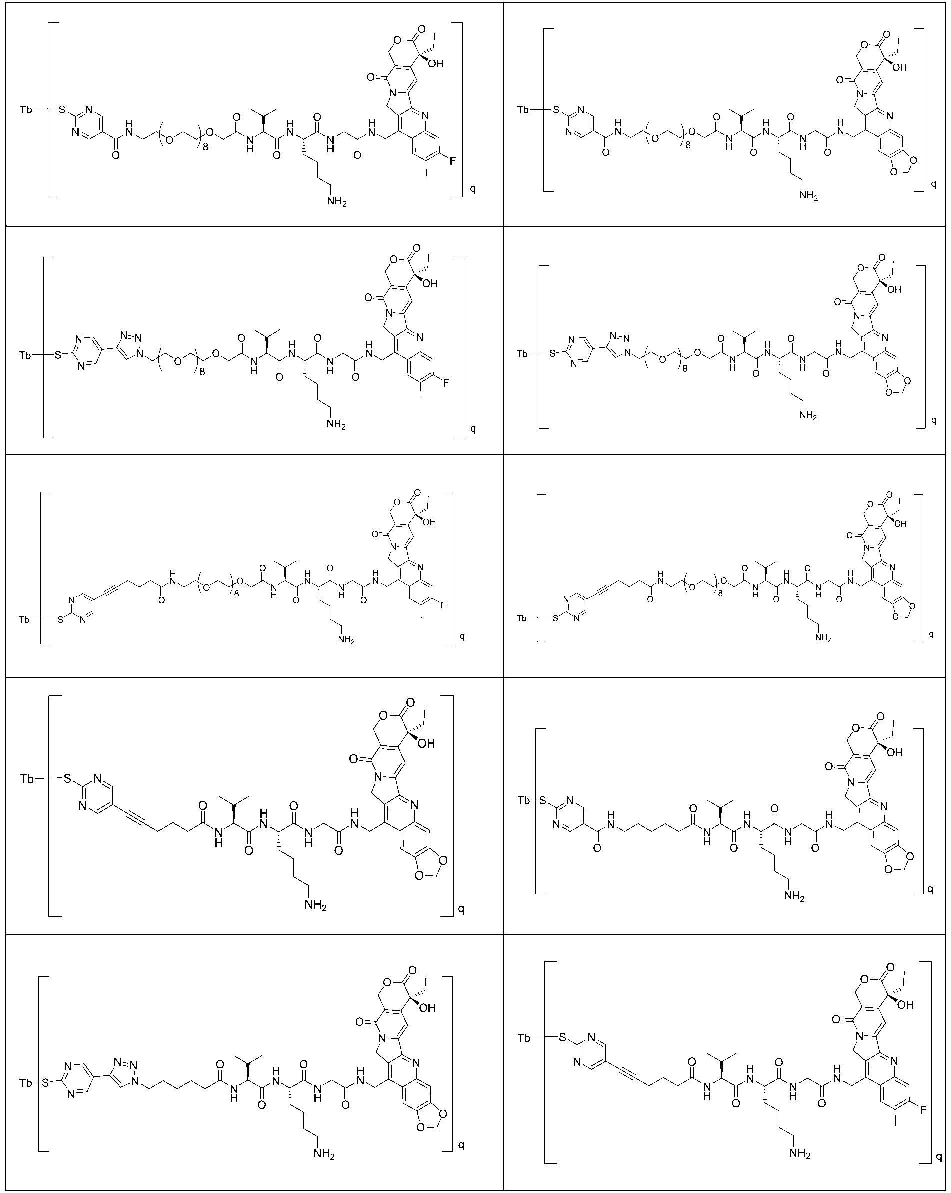

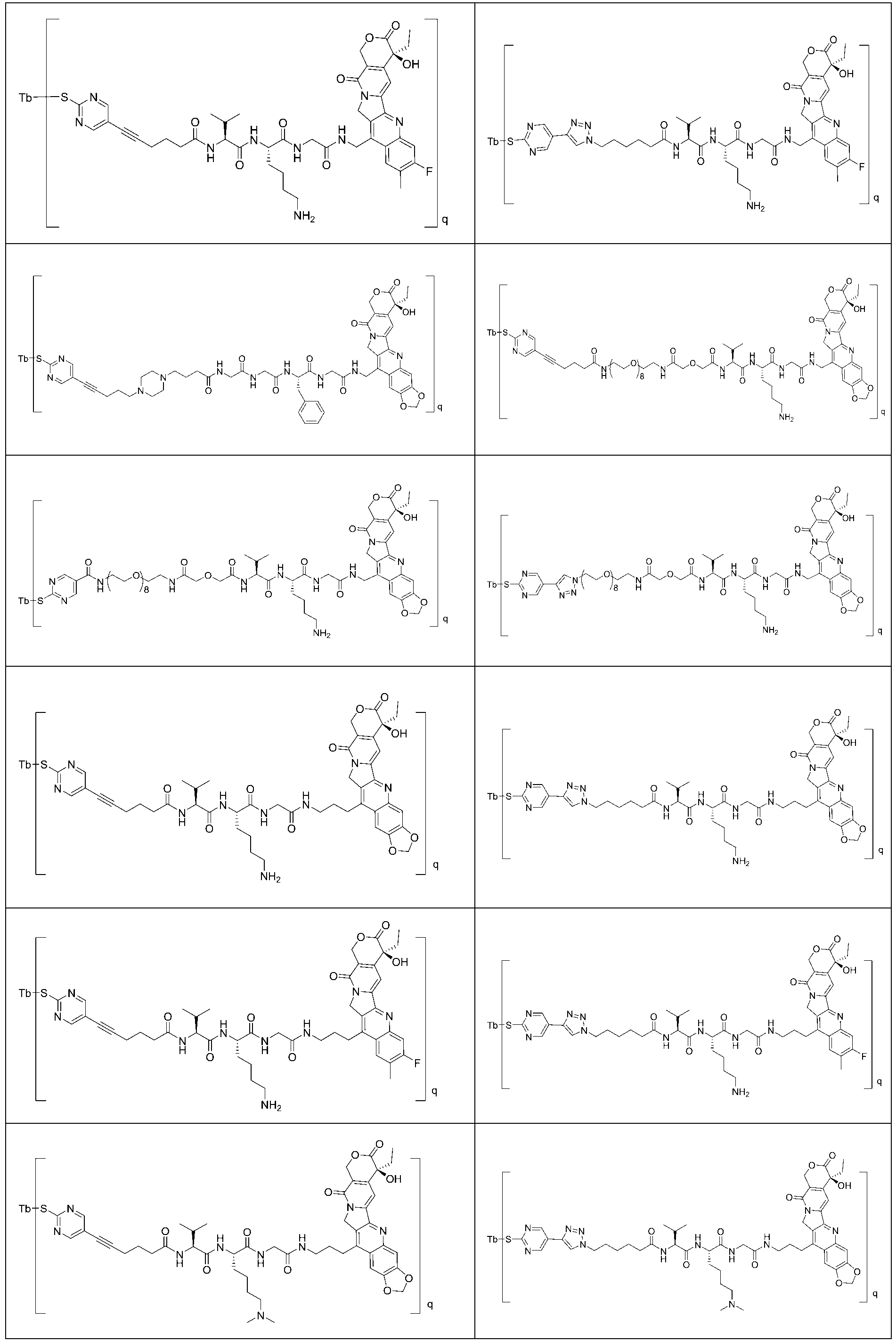

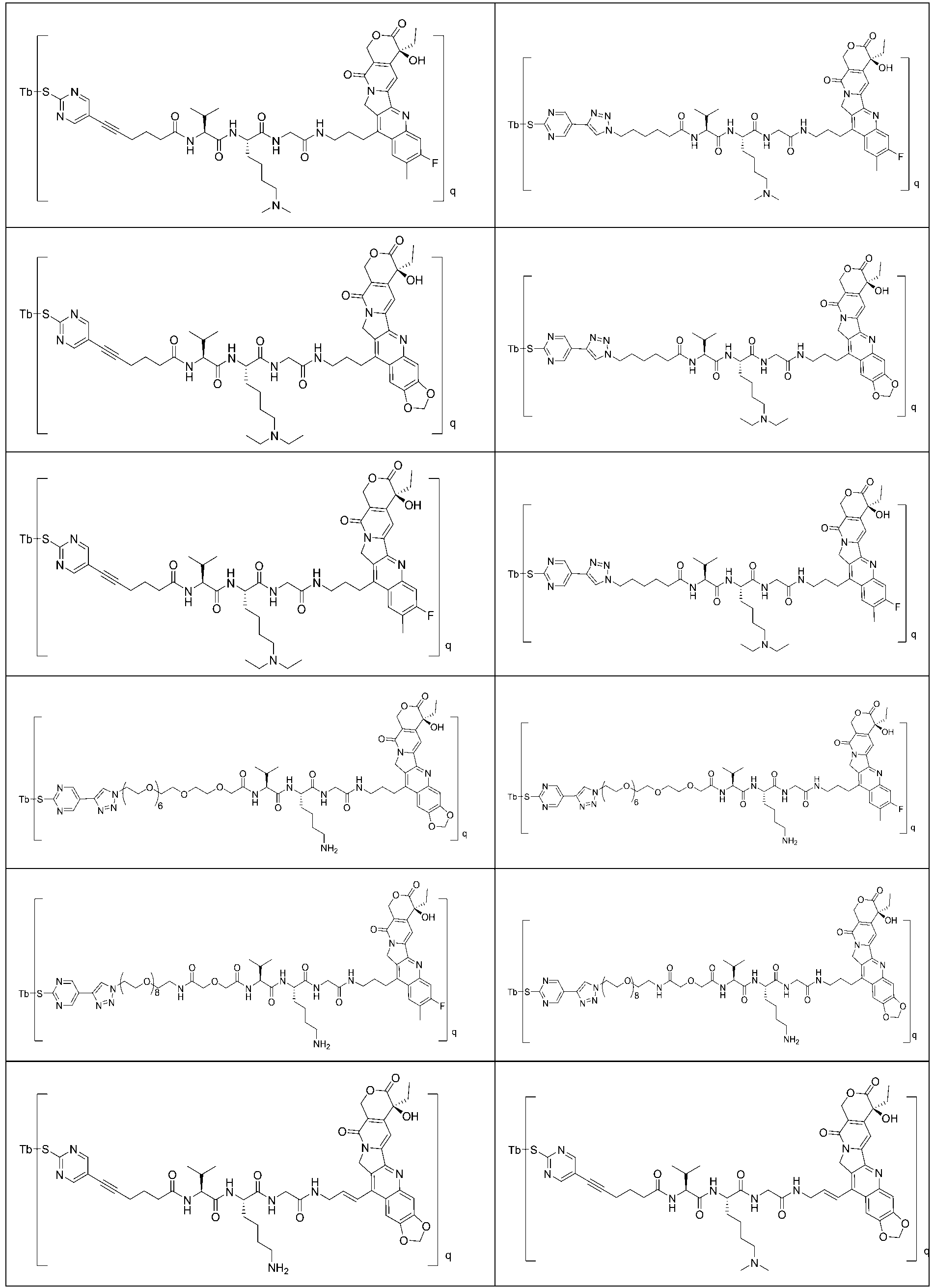

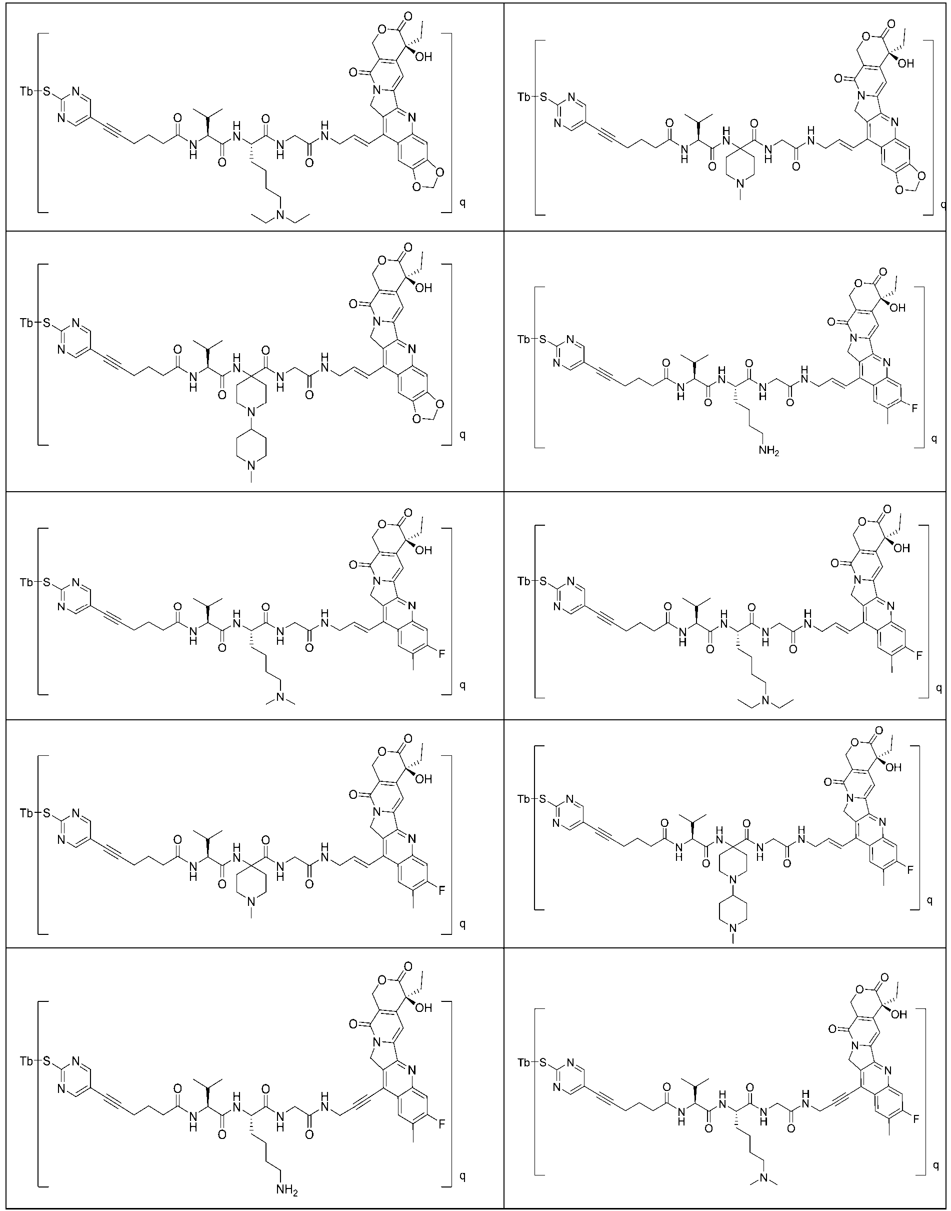

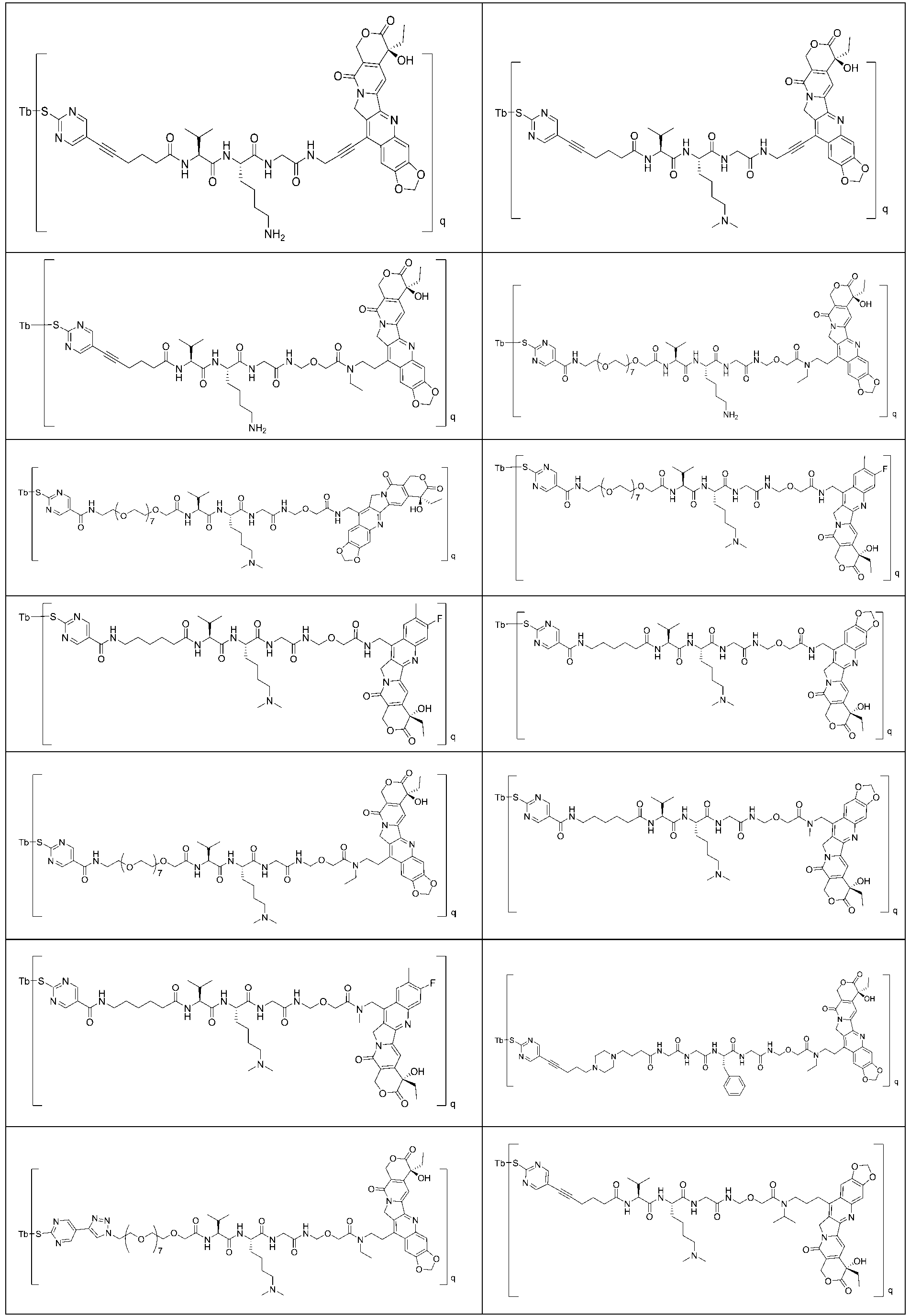

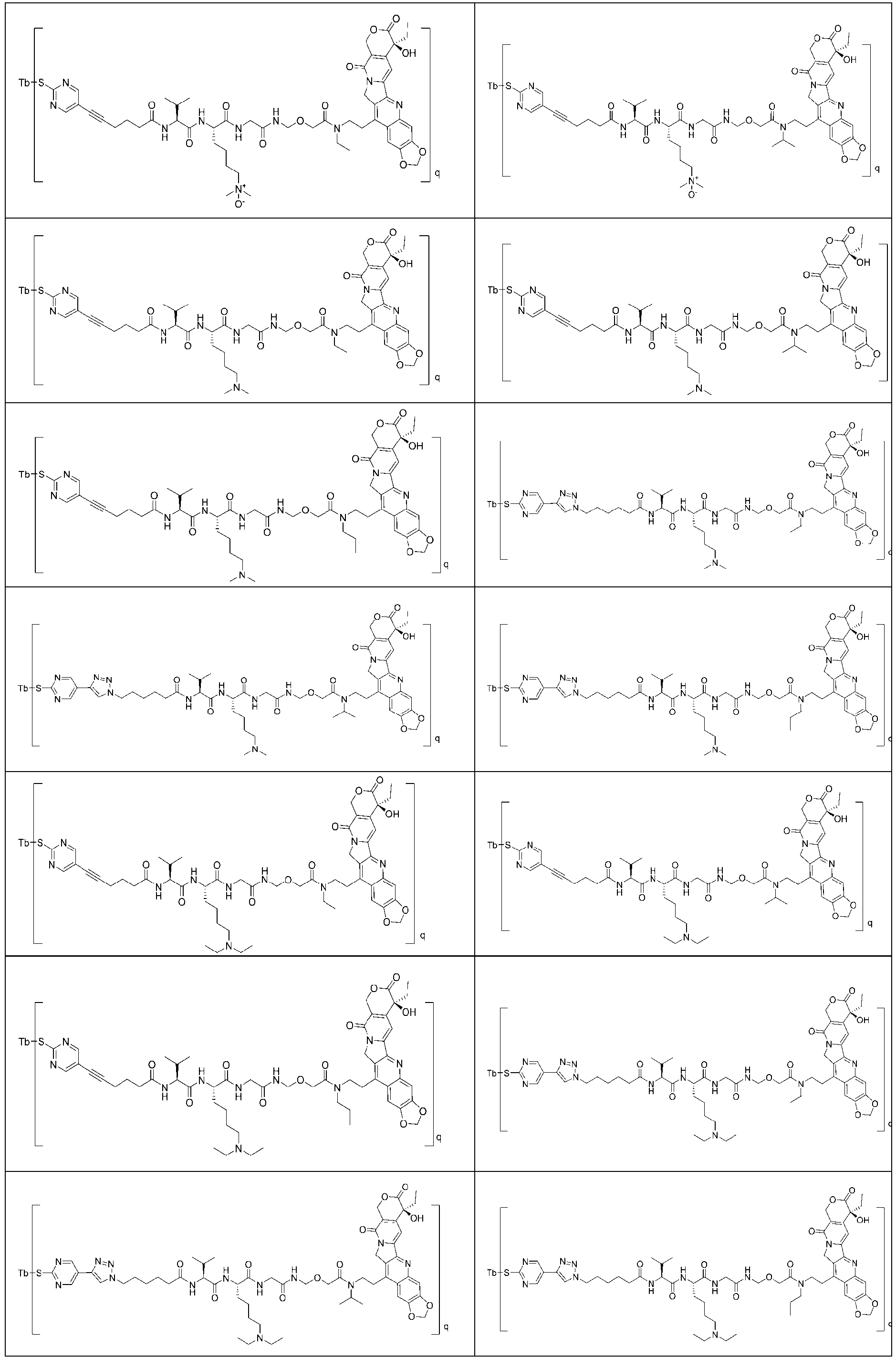

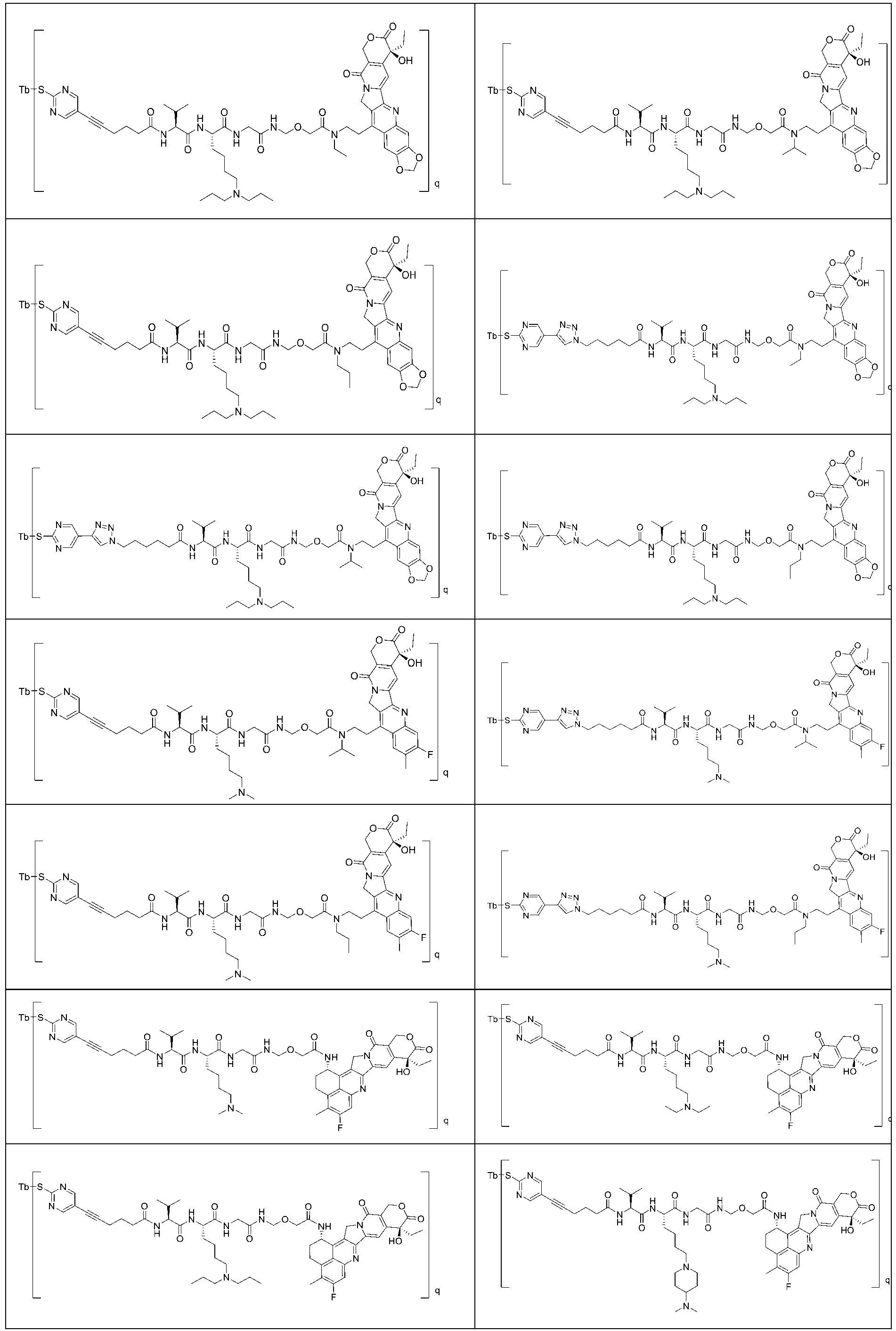

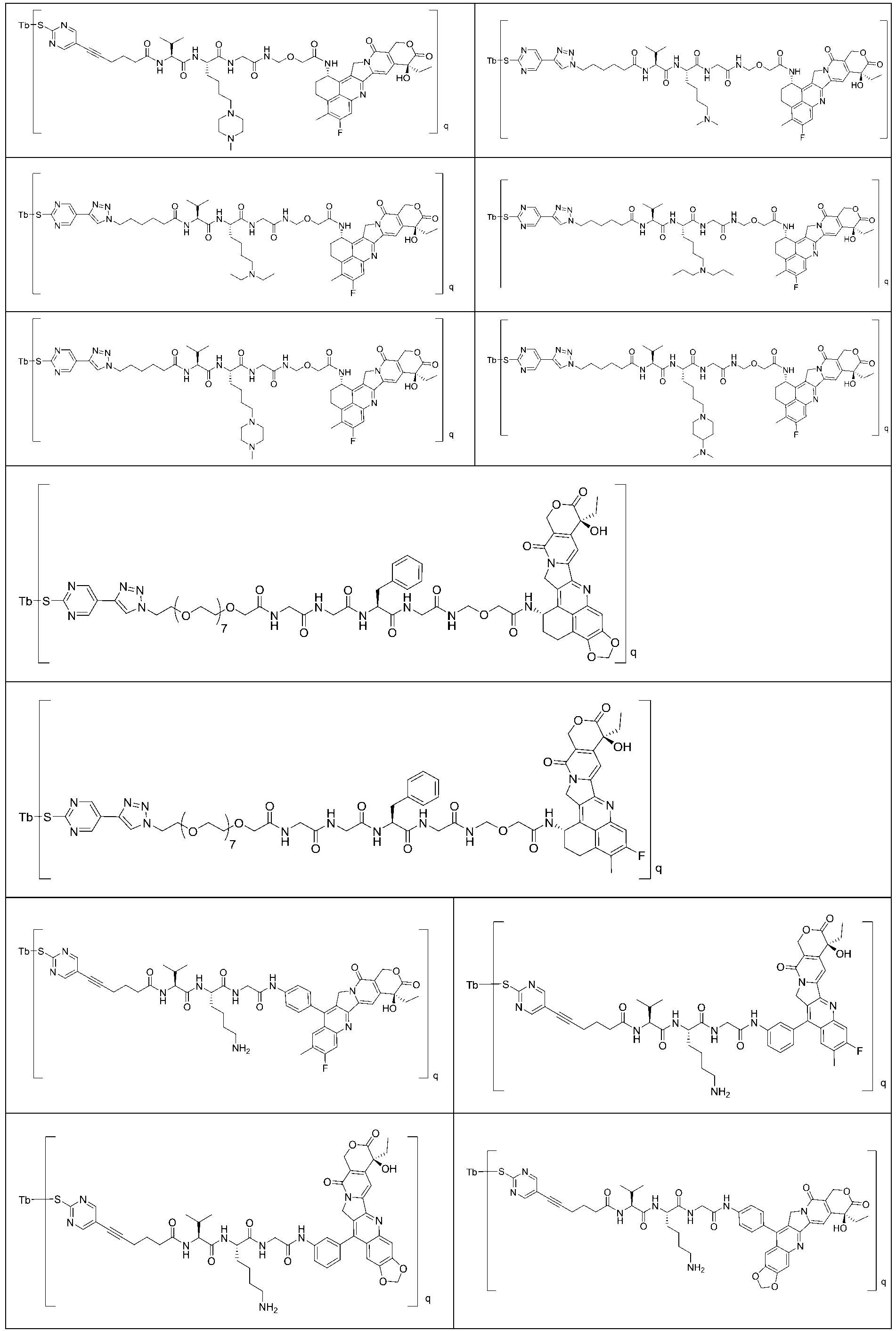

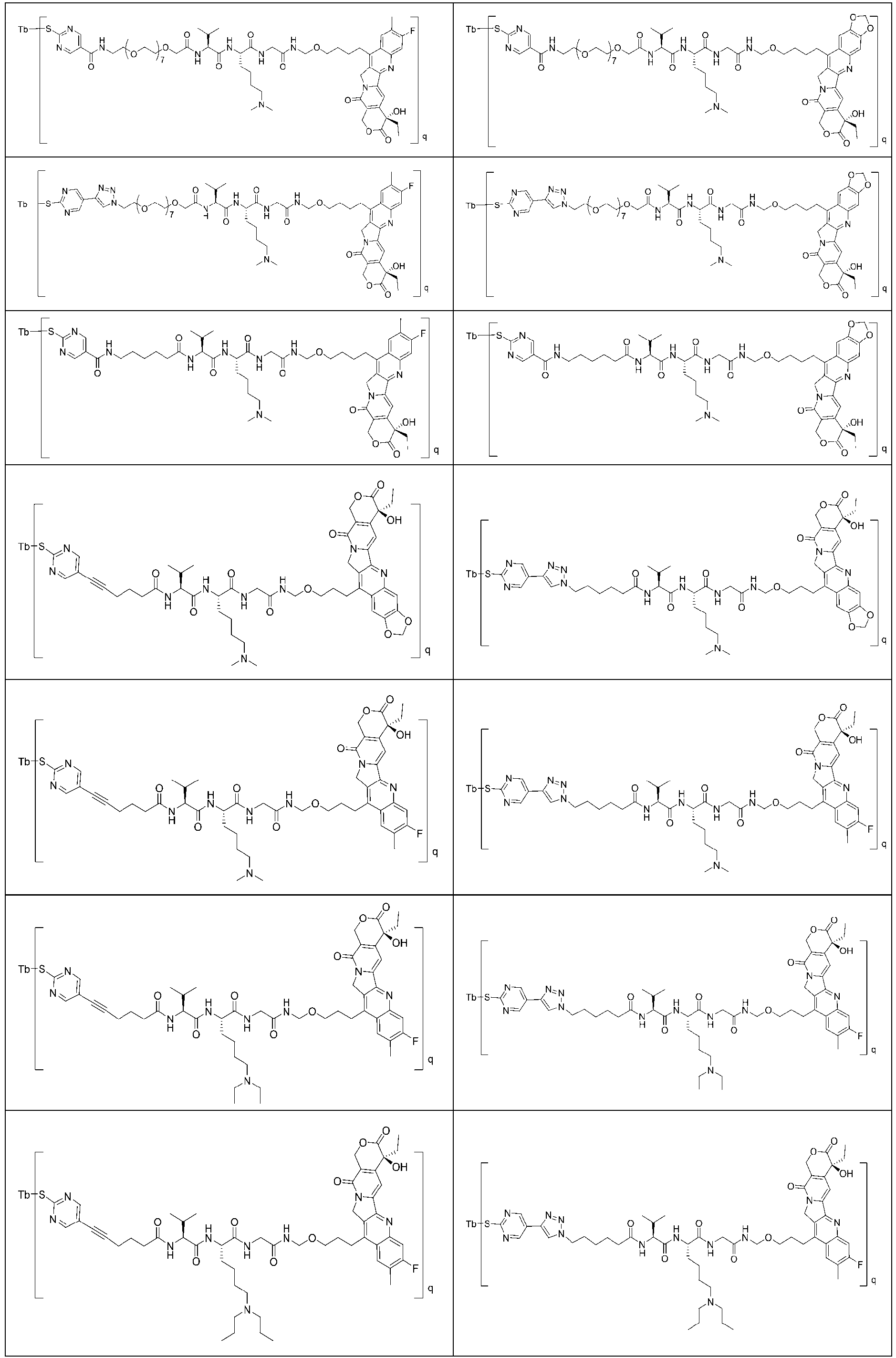

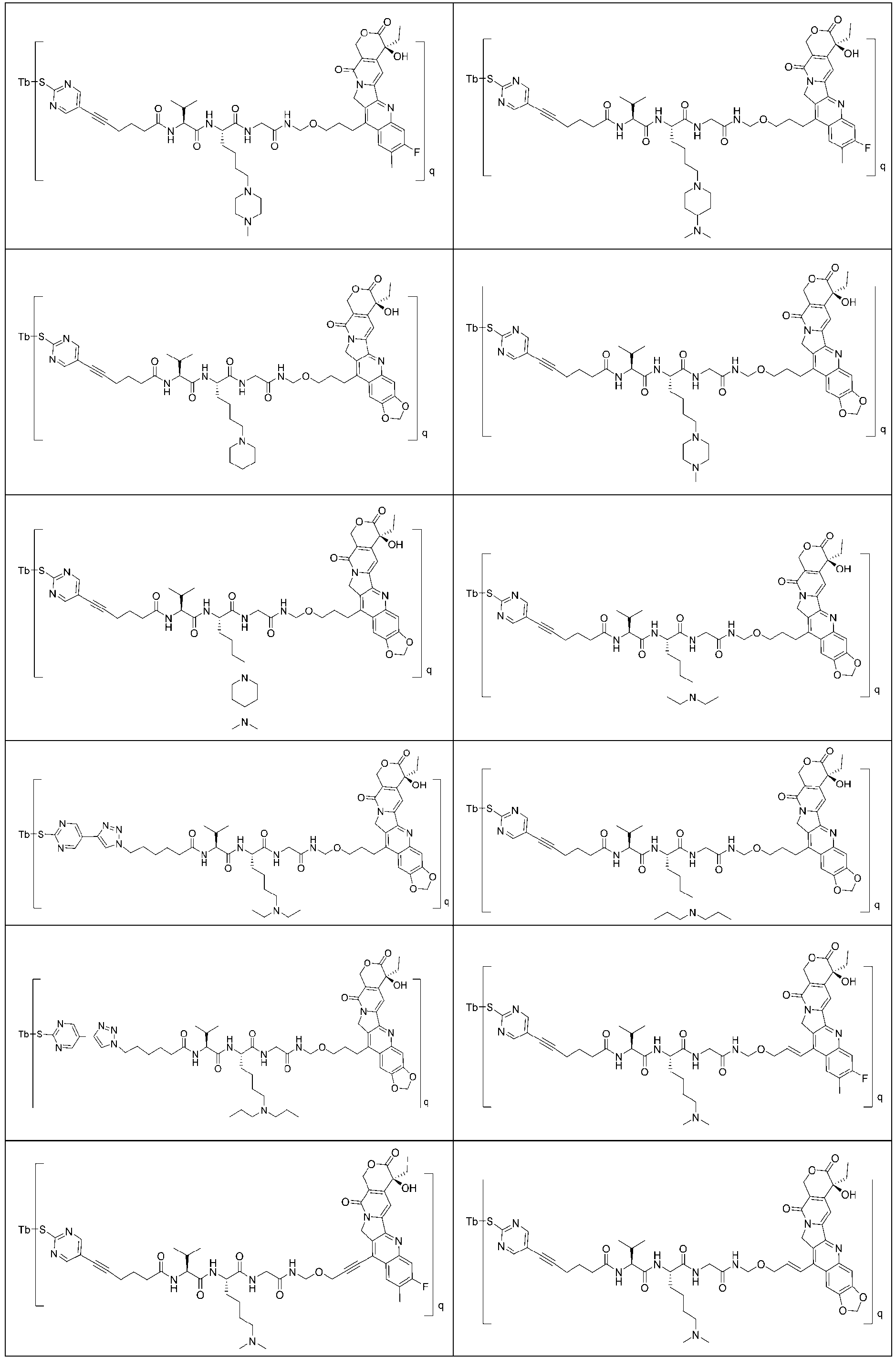

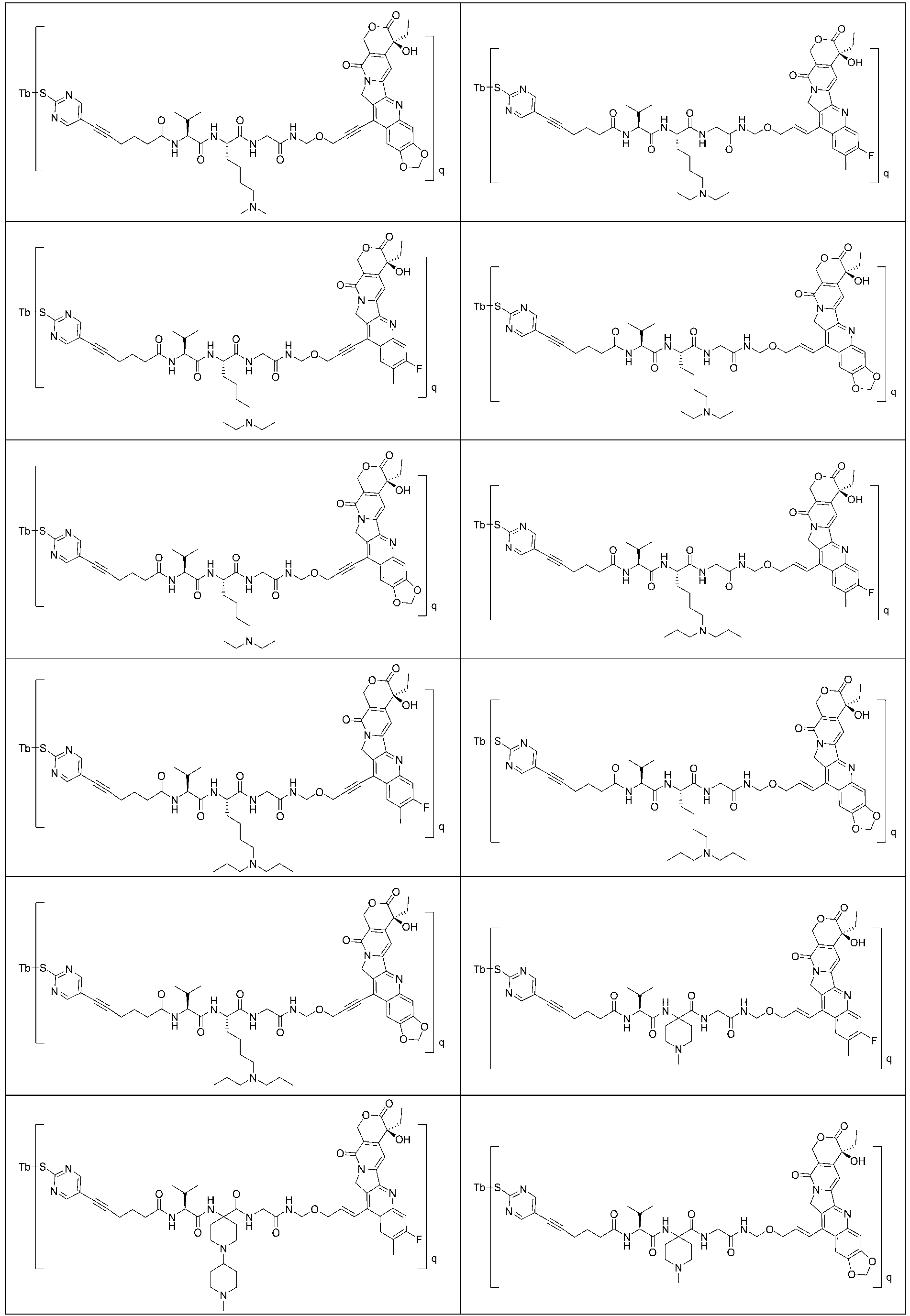

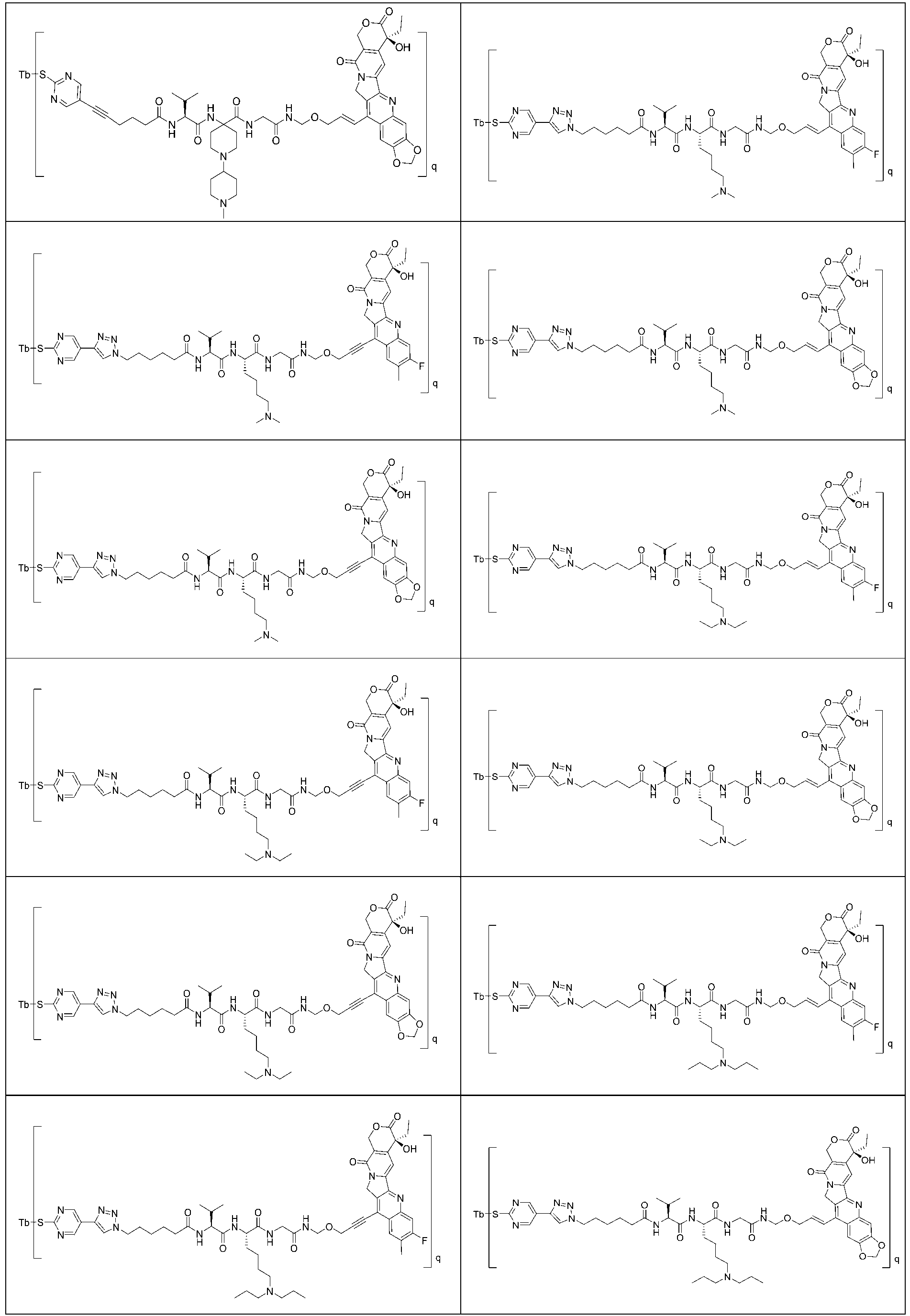

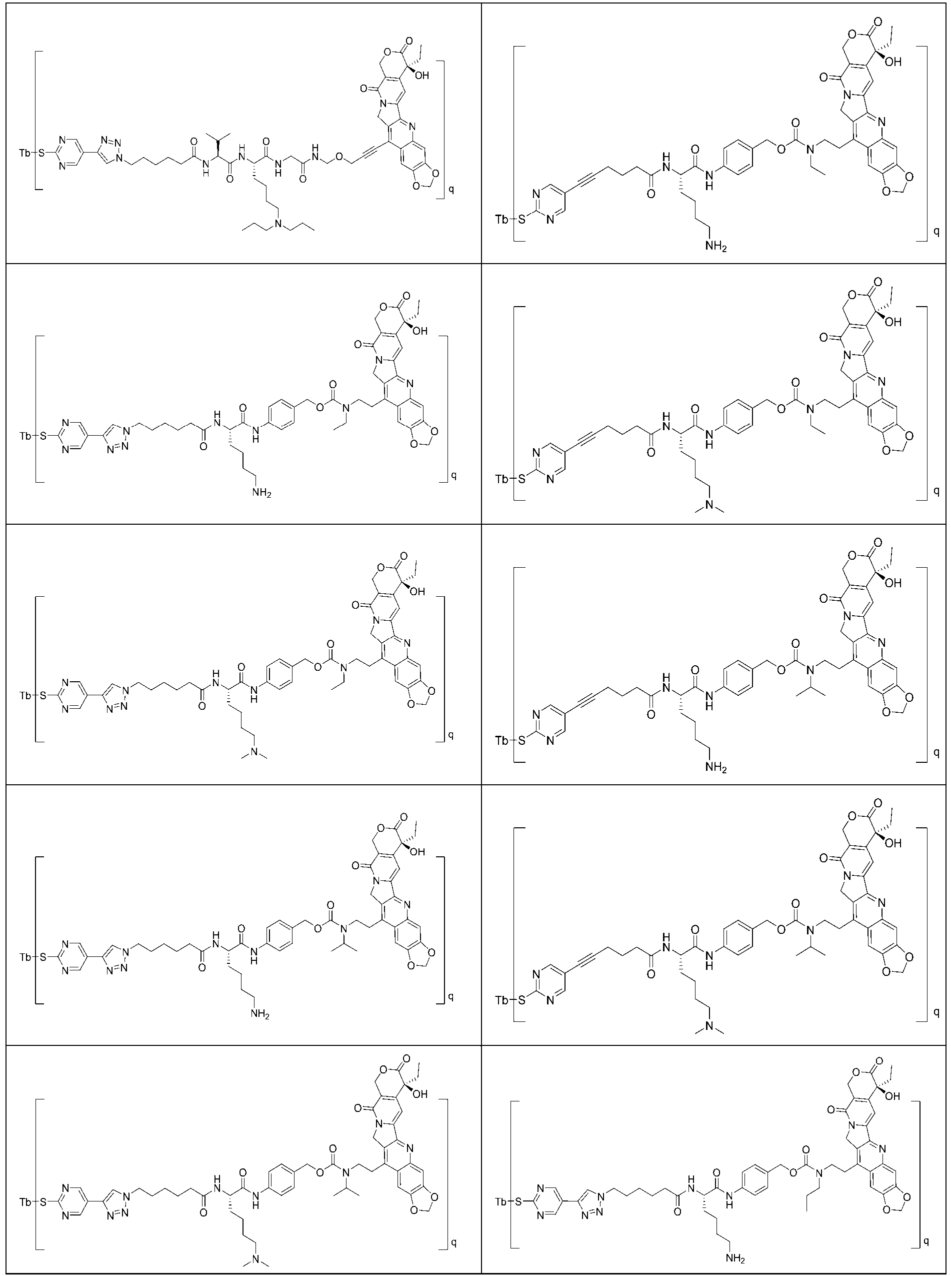

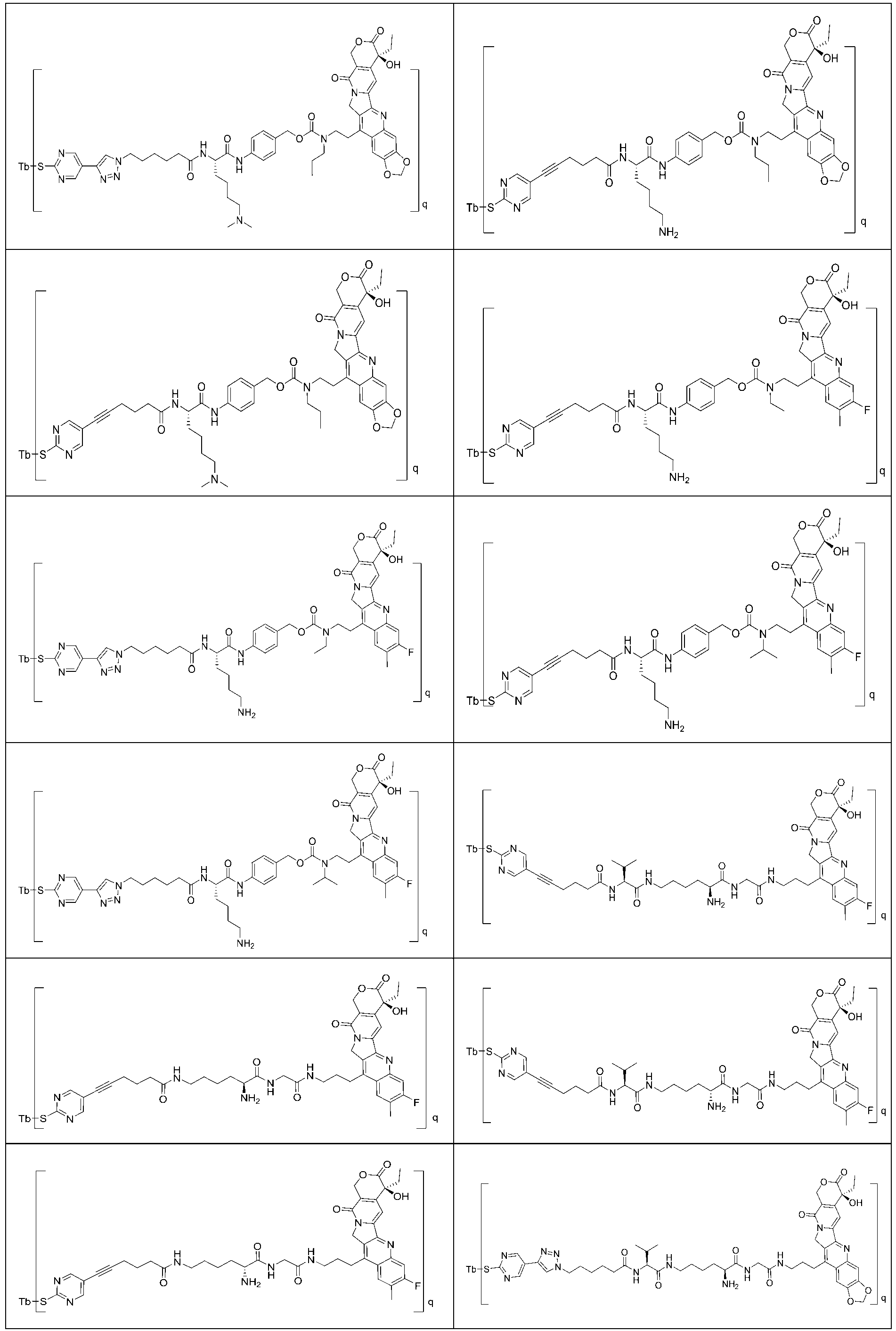

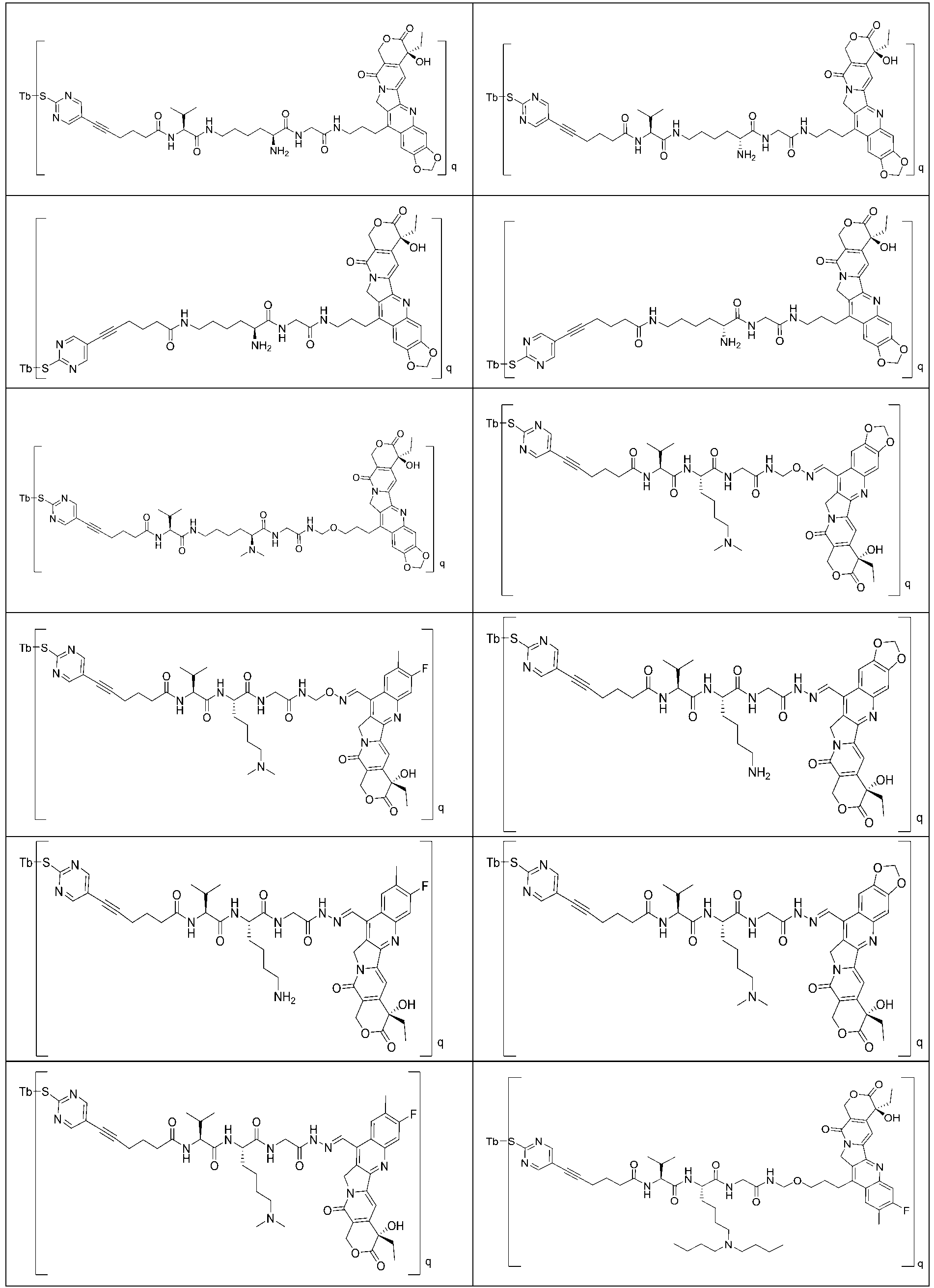

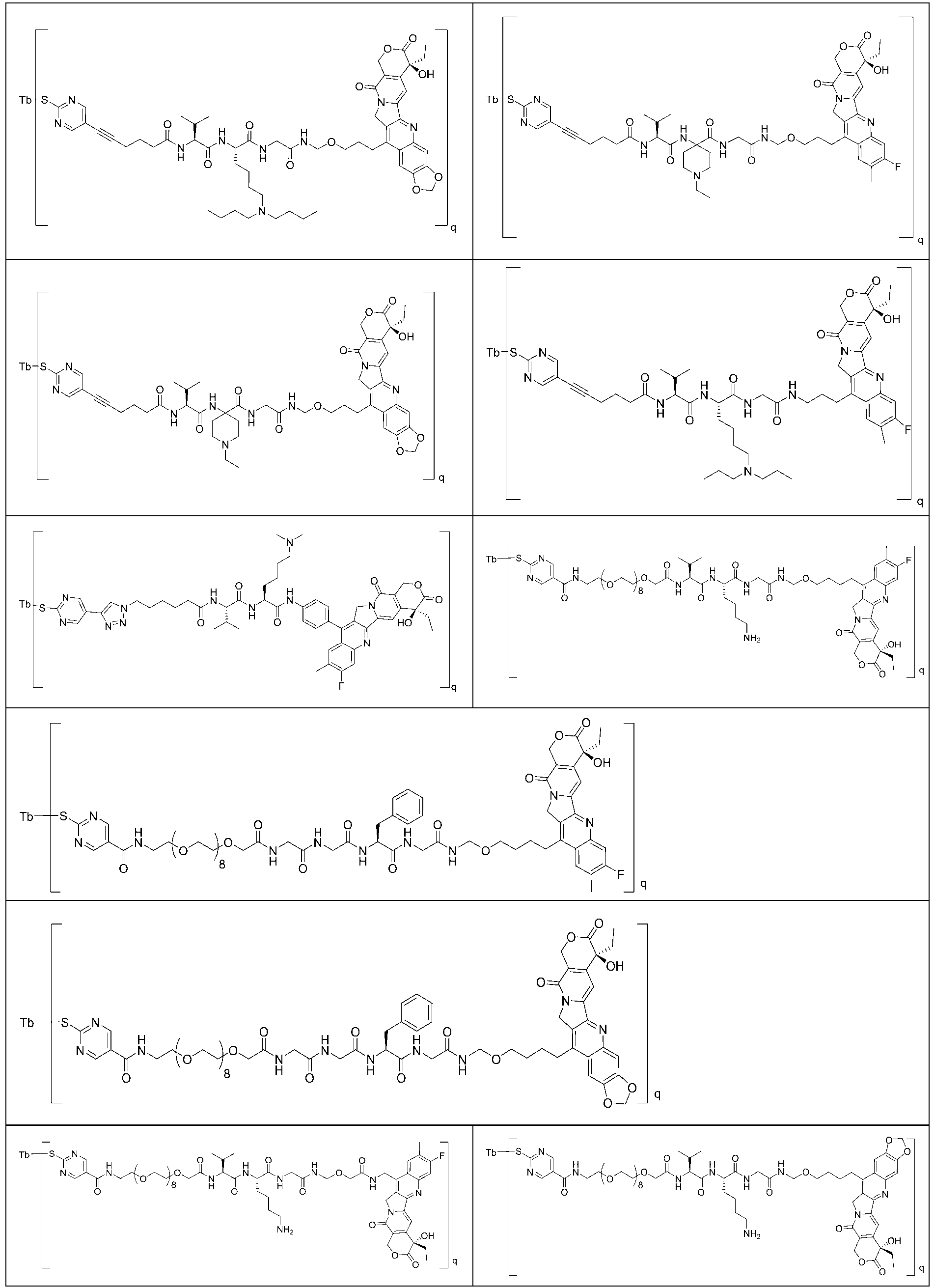

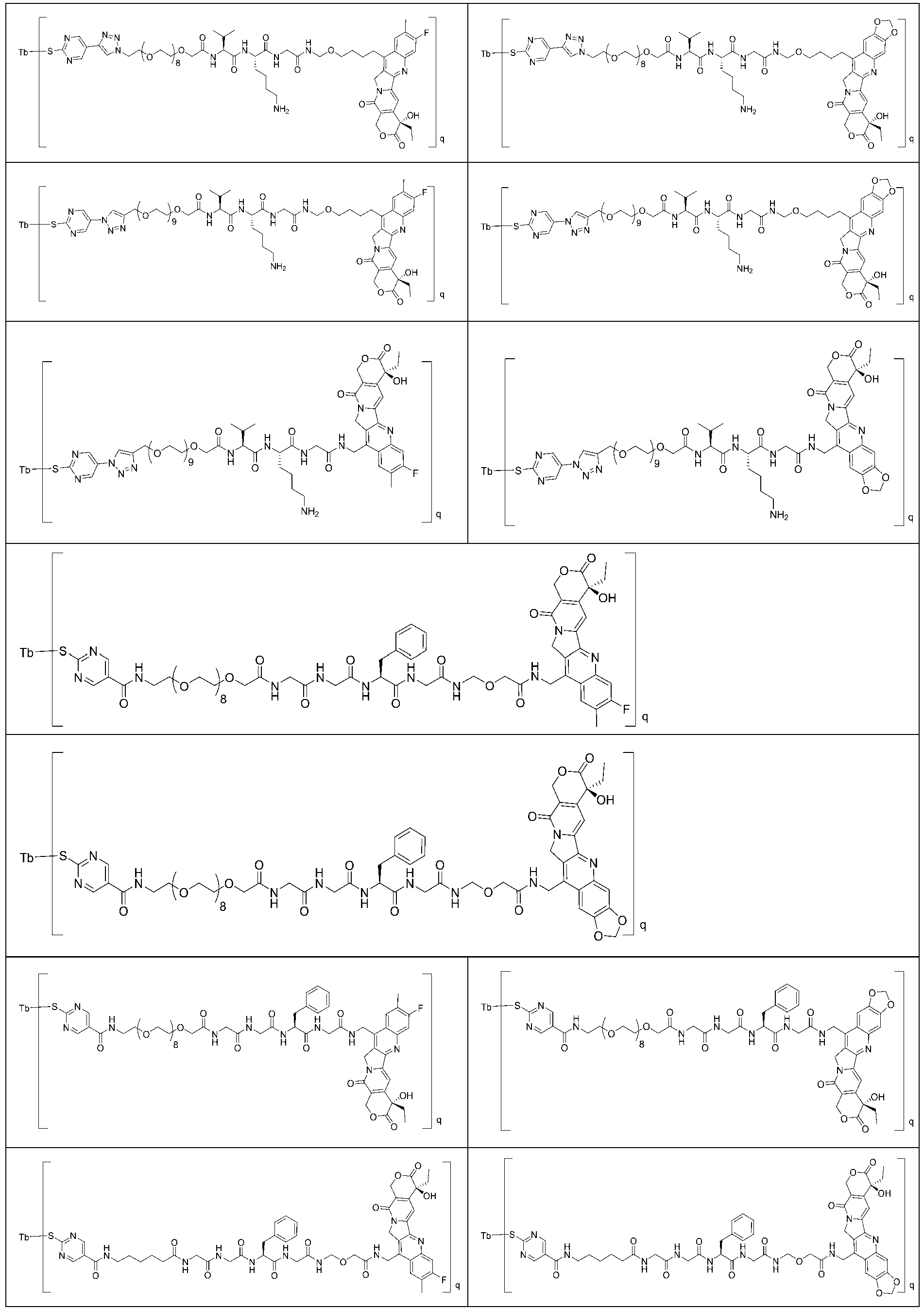

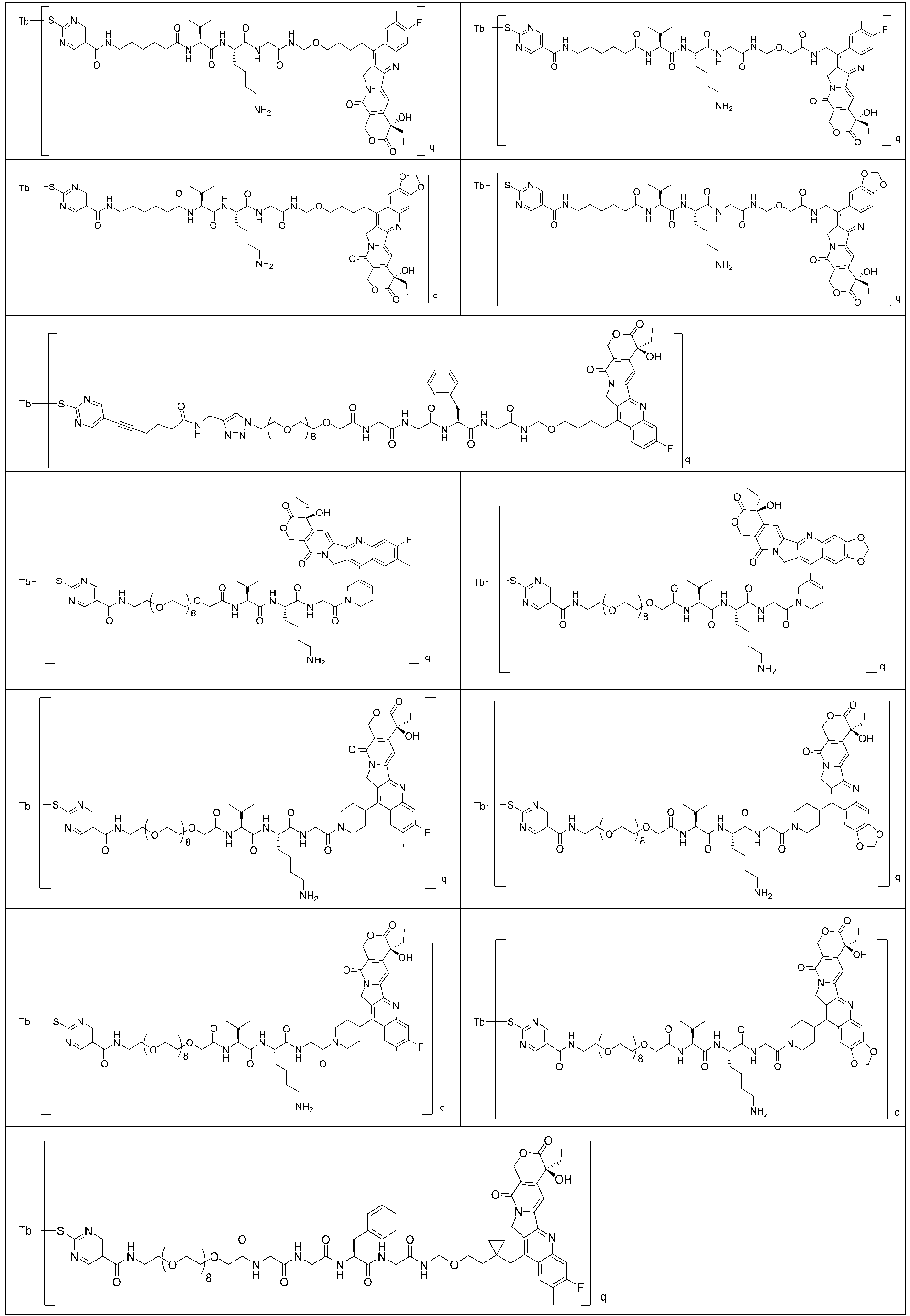

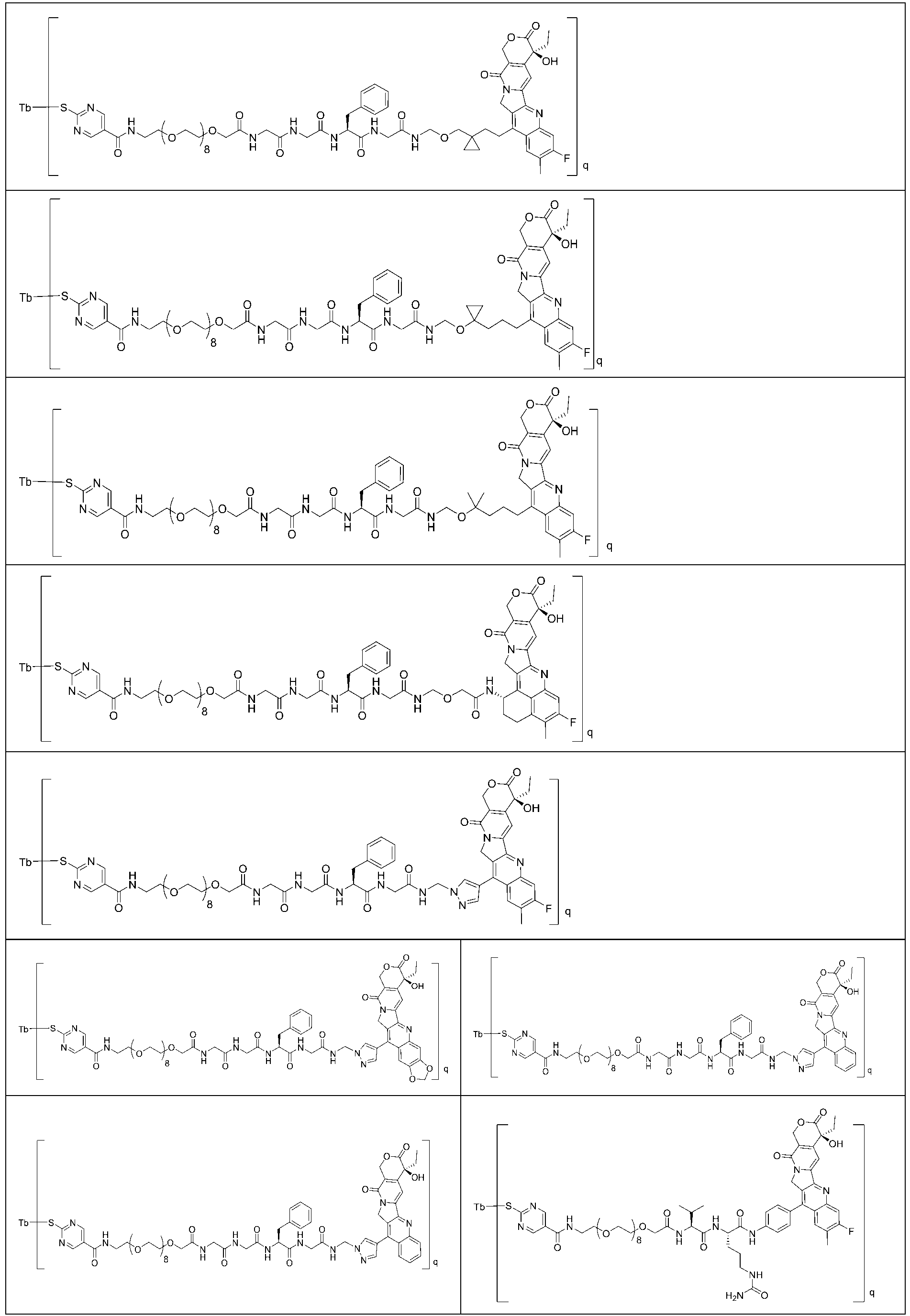

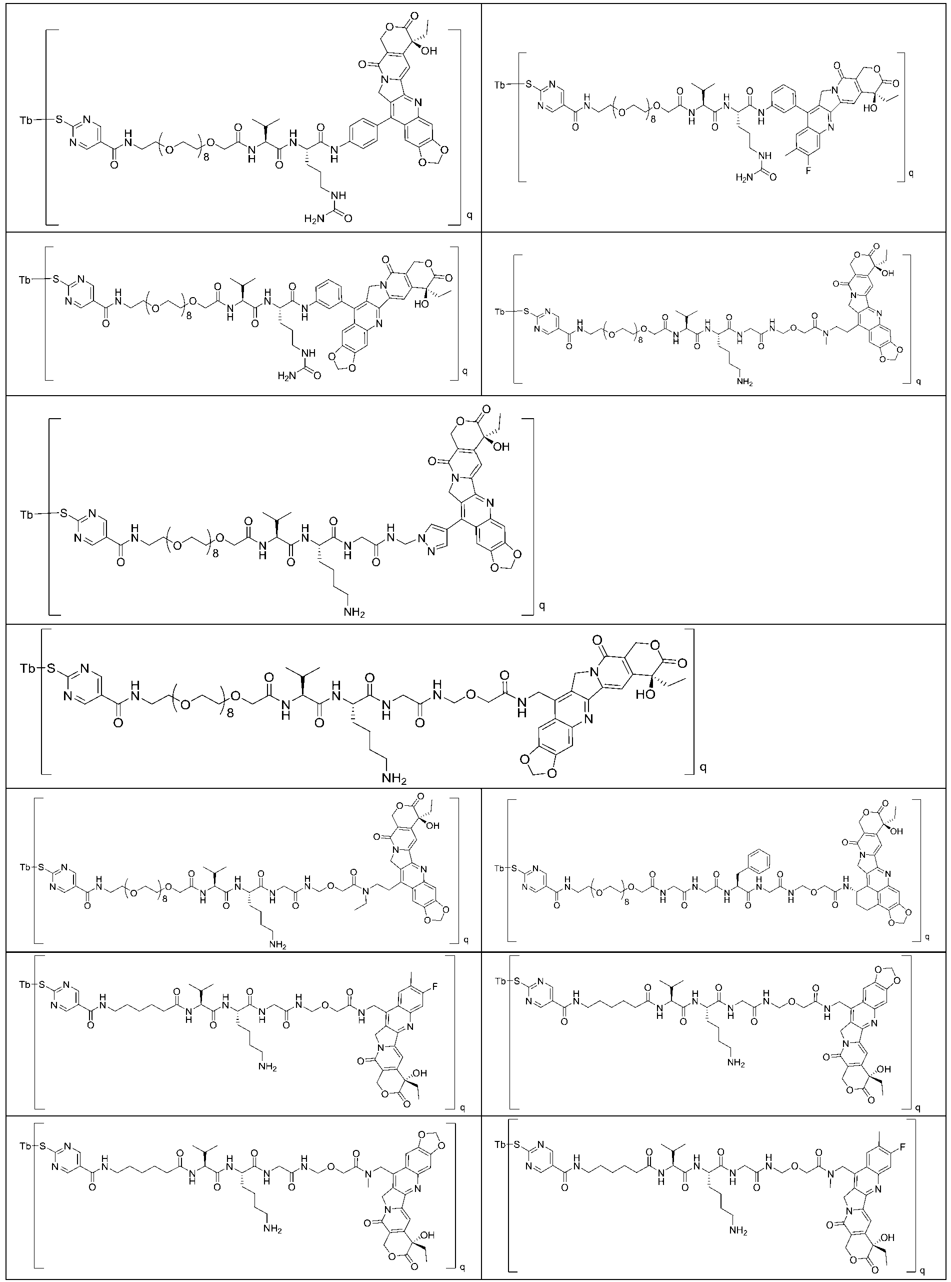

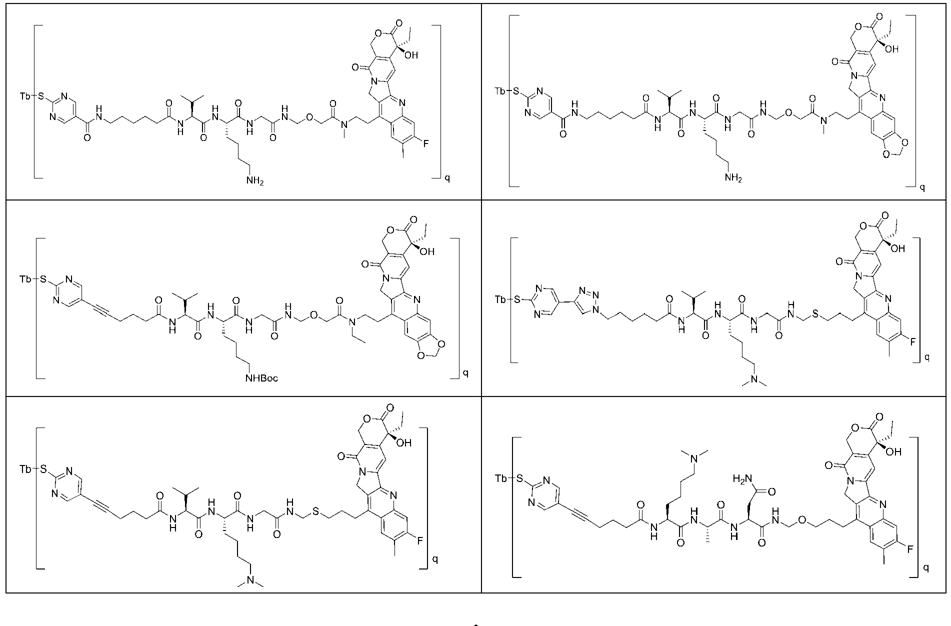

In some embodiments, Tb is an anti-DLL3 antibody or an antigen-binding fragment thereof.

In some embodiments, the antibody or the antigen-binding fragment thereof and a monoclonal antibody or an antigen-binding fragment thereof comprise Fab, Fab', F(ab')₂, Fd, Fv (for example, scFv), dAb, a complementarity determining region fragment, a non-human antibody, a humanized antibody, a chimeric antibody, a fully human antibody, a probody, a monoclonal antibody, a bispecific antibody, or a multi-specific antibody.

In some embodiments, Tb is an anti-DLL3 antibody or an antigen-binding fragment thereof with endocytosis activity, without endocytosis activity, or with weak endocytosis activity.

In some embodiments, Tb is an anti-DLL3 antibody or an antigen-binding fragment thereof with endocytosis activity.

In some embodiments, Tb is an anti-DLL3 antibody or an antigen-binding fragment thereof without endocytosis activity or with weak endocytosis activity.

In some embodiments, Tb is an anti-DLL3 antibody or an antigen-binding fragment thereof.

In some embodiments, the anti-DLL3 antibody is a non-human antibody, a humanized antibody, a chimeric antibody, or a fully human antibody.

In some embodiments, the anti-DLL3 antibody is a monoclonal antibody, a bispecific antibody, or a multi-specific antibody.

In some embodiments, the anti-DLL3 antibody or the antigen-binding fragment thereof is a monoclonal antibody or an antigen-binding fragment thereof.

In some embodiments, the anti-DLL3 antibody or the antigen-binding fragment thereof is selected from: rovalpituzumab or an antigen-binding fragment thereof, tarlatamab or an antigen-binding fragment thereof, antibody 10F2F3, antibody 87F7F10, antibody 55C11E4, antibody 59B10D3, antibody 6F11C10, antibody 55C11E4-Hz1, antibody 55C11E4-Hz2, antibody 59B10D3-Hz, antibody 10F2F3-Hz, antibody 87F7F10-Hz, antibody 6F11C10-Hz1, and antibody 6F11C10-Hz2.

In some preferred embodiments, the anti-DLL3 antibody is selected from murine antibodies 10F2F3, 87F7F10, 55C11E4, 59B10D3, and 6F11C10, or humanized antibodies thereof.

In some preferred embodiments, the anti-DLL3 antibody is selected from: antibody 55C11E4-Hz1, antibody 55C11E4-Hz2, antibody 59B10D3-Hz, antibody 10F2F3-Hz, antibody 87F7F10-Hz, antibody 6F11C10-Hz1, and antibody 6F11C10-Hz2.

The Tb antibody or the antigen-binding fragment thereof can be prepared by various methods known in the art, such as through genetic engineering and recombinant technology. For example, a DNA molecule encoding the heavy and light chain genes of the antibodies of the present disclosure are obtained by chemical synthesis or PCR amplification. The resulting DNA molecule is inserted into an expression vector and then transfected into host cells. The transfected host cells are then cultured under specific conditions to express the antibodies of the present disclosure.

In some preferred embodiments, for the above-described antibody-drug conjugate, Tb is an anti-DLL3 antibody or an antigen-binding fragment thereof described in the second aspect.

In a second aspect, the present disclosure provides an anti-DLL3 antibody or an antigen-binding fragment thereof.

In some embodiments, the present disclosure provides an anti-DLL3 antibody or an antigen-binding fragment thereof, and the antibody or the antigen-binding fragment thereof comprises a complementarity determining region (CDR) as follows:

HCDR1 or a variant of the sequence thereof, HCDR2 or a variant of the sequence thereof, and HCDR3 or a variant of the sequence thereof, comprised in the heavy chain variable region (VH) shown in SEQ ID NO: 1, 2, 5, 6, 7, 10, 11, 14, 15, 18, 19, or 20; and/or

LCDR1 or a variant of the sequence thereof, LCDR2 or a variant of the sequence thereof, and LCDR3 or a variant of the sequence thereof, comprised in the light chain variable region (VL) shown in SEQ ID NO: 3, 4, 8, 9, 12, 13, 16, 17, 21, or 22.

In certain embodiments, the anti-DLL3 antibody or the antigen-binding fragment thereof comprises: an antibody and an antigen-binding fragment thereof containing HCDR1, HCDR2, HCDR1, LCDR1, LCDR2, and LCDR3, and binding to the same DLL3 epitope as any one of the antibodies described in the previous embodiments. In certain embodiments, the anti-DLL3 antibody or the antigen-binding fragment thereof comprises: an antibody and an antigen-binding fragment thereof containing HCDR1, HCDR2, HCDR1, LCDR1, LCDR2, and LCDR3, and competing with any one of the antibodies described in the previous embodiments for binding to DLL3.

In certain embodiments, the anti-DLL3 antibody or the antigen-binding fragment thereof is an antibody and an antigen-binding fragment thereof that binds to the same DLL3 epitope as 55C11E4-hz2, 55C11E4-hz1, 6F11C10-Hz1, or 6F11C10-Hz2.

In certain embodiments, the anti-DLL3 antibody or the antigen-binding fragment thereof is an antibody and an antigen-binding fragment thereof that competes with 55C11E4-hz2, 55C11E4-hz1, 6F11C10-Hz1, or 6F11C10-Hz2 for binding to DLL3.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 1 or 2; and/or LCDR1 or a variant of the sequence thereof, LCDR2 or a variant of the sequence thereof, and LCDR3 or a variant of the sequence thereof, comprised in the VL shown in SEQ ID NO: 3 or 4.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 5, 6, or 7; and/or LCDR1 or a variant of the sequence thereof, LCDR2 or a variant of the sequence thereof, and LCDR3 or a variant of the sequence thereof, comprised in the VL shown in SEQ ID NO: 8 or 9.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 10 or 11; and/or LCDR1 or a variant of the sequence thereof, LCDR2 or a variant of the sequence thereof, and LCDR3 or a variant of the sequence thereof, comprised in the VL shown in SEQ ID NO: 12 or 13.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 14 or 15; and/or LCDR1 or a variant of the sequence thereof, LCDR2 or a variant of the sequence thereof, and LCDR3 or a variant of the sequence thereof, comprised in the VL shown in SEQ ID NO: 16 or 17.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 18, 19, or 20; and/or LCDR1 or a variant of the sequence thereof, LCDR2 or a variant of the sequence thereof, and LCDR3 or a variant of the sequence thereof, comprised in the VL shown in SEQ ID NO: 21 or 22.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 1; and/or LCDR1 or a variant of the sequence thereof, LCDR2 or a variant of the sequence thereof, and LCDR3 or a variant of the sequence thereof, comprised in the VL shown in SEQ ID NO: 3.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 2; and/or LCDR1 or a variant of the sequence thereof, LCDR2 or a variant of the sequence thereof, and LCDR3 or a variant of the sequence thereof, comprised in the VL shown in SEQ ID NO: 4.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 5; and/or LCDR1 or a variant of the sequence thereof, LCDR2 or a variant of the sequence thereof, and LCDR3 or a variant of the sequence thereof, comprised in the VL shown in SEQ ID NO: 8.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 6; and/or LCDR1 or a variant of the sequence thereof, LCDR2 or a variant of the sequence thereof, and LCDR3 or a variant of the sequence thereof, comprised in the VL shown in SEQ ID NO: 9.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 7; and/or LCDR1 or a variant of the sequence thereof, LCDR2 or a variant of the sequence thereof, and LCDR3 or a variant of the sequence thereof, comprised in the VL shown in SEQ ID NO: 9.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 10; and/or LCDR1 or a variant of the sequence thereof, LCDR2 or a variant of the sequence thereof, and LCDR3 or a variant of the sequence thereof, comprised in the VL shown in SEQ ID NO: 12.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 11; and/or LCDR1 or a variant of the sequence thereof, LCDR2 or a variant of the sequence thereof, and LCDR3 or a variant of the sequence thereof, comprised in the VL shown in SEQ ID NO: 13.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 14; and/or LCDR1 or a variant of the sequence thereof, LCDR2 or a variant of the sequence thereof, and LCDR3 or a variant of the sequence thereof, comprised in the VL shown in SEQ ID NO: 16.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 15; and/or LCDR1 or a variant of the sequence thereof, LCDR2 or a variant of the sequence thereof, and LCDR3 or a variant of the sequence thereof, comprised in the VL shown in SEQ ID NO: 17.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 18; and/or LCDR1 or a variant of the sequence thereof, LCDR2 or a variant of the sequence thereof, and LCDR3 or a variant of the sequence thereof, comprised in the VL shown in SEQ ID NO: 21.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 19; and/or LCDR1 or a variant of the sequence thereof, LCDR2 or a variant of the sequence thereof, and LCDR3 or a variant of the sequence thereof, comprised in the VL shown in SEQ ID NO: 22.

In certain embodiments, the anti-DLL3 antibody or the antigen-binding fragment thereof comprises HCDR1 or a variant of the sequence thereof, HCDR2 or a variant of the sequence thereof, and HCDR3 or a variant of the sequence thereof, comprised in the VH shown in SEQ ID NO: 20; and/or LCDR1 or a variant of the sequence thereof, LCDR2 or a variant of the sequence thereof, and LCDR3 or a variant of the sequence thereof, comprised in the VL shown in SEQ ID NO: 22.

In certain preferred embodiments, the variant of the sequence is a CDR that has one or more amino acid substitutions, deletions, or additions (for example, 1, 2, or 3 amino acid substitutions, deletions, or additions) compared to its source CDR.

In certain preferred embodiments, the substitution is a conservative substitution.

Preferably, the CDR is defined according to the AbM, Chothia, Kabat, or IMGT numbering system.

In one aspect, the present disclosure provides an anti-DLL3 antibody or an antigen-binding fragment thereof, wherein the antibody or the antigen-binding fragment thereof comprises: a heavy chain variable region (VH) and/or a light chain variable region (VL).

In certain embodiments, the anti-DLL3 antibody or the antigen-binding fragment thereof of the present disclosure comprises a heavy chain variable region (VH) and/or a light chain variable region (VL) as follows, wherein CDR is defined by the Kabat numbering system:
(a) a heavy chain variable region (VH) comprising 3 CDRs as follows: HCDR1 with a sequence of SEQ ID NO: 23 or a sequence having one or more amino acid substitutions, deletions, or additions (for example, 1, 2, or 3 amino acid substitutions, deletions, or additions) compared to the sequence of SEQ ID NO: 23; HCDR2 with a sequence of SEQ ID NO: 24 or a sequence having one or more amino acid substitutions, deletions, or additions (for example, 1, 2, or 3 amino acid substitutions, deletions, or additions) compared to the sequence of SEQ ID NO: 24; and HCDR3 with a sequence of SEQ ID NO: 25 or a sequence having one or more amino acid substitutions, deletions, or additions (for example, 1, 2, or 3 amino acid substitutions, deletions, or additions) compared to the sequence of SEQ ID NO: 25; and/or,
   a light chain variable region (VL) comprising 3 CDRs as follows: LCDR1 with a sequence of SEQ ID NO: 26 or a sequence having one or more amino acid substitutions, deletions, or additions (for example, 1, 2, or 3 amino acid substitutions, deletions, or additions) compared to the sequence of SEQ ID NO: 26; LCDR2 with a sequence of SEQ ID NO: 27 or a sequence having one or more amino acid substitutions, deletions, or additions (for example, 1, 2, or 3 amino acid substitutions, deletions, or additions) compared to the sequence of SEQ ID NO: 27; and LCDR3 with a sequence of SEQ ID NO: 28 or a sequence having one or more amino acid substitutions, deletions, or additions (for example, 1, 2, or 3 amino acid substitutions, deletions, or additions) compared to the sequence of SEQ ID NO: 28;
(b) a heavy chain variable region (VH) comprising 3 CDRs as follows: HCDR1 with a sequence of SEQ ID NO: 34 or a sequence having one or more amino acid substitutions, deletions, or additions (for example, 1, 2, or 3 amino acid substitutions, deletions, or additions) compared to the sequence of SEQ ID NO: 34; HCDR2 with a sequence of SEQ ID NO: 35 or 36 or a sequence having one or more amino acid substitutions, deletions, or additions (for example, 1, 2, or 3 amino acid substitutions, deletions, or additions) compared to the sequence of SEQ ID NO: 35 or 36; and HCDR3 with a sequence of SEQ ID NO: 37 or a sequence having one or more amino acid substitutions, deletions, or additions (for example, 1, 2, or 3 amino acid substitutions, deletions, or additions) compared to the sequence of SEQ ID NO: 37; and/or,
   a light chain variable region (VL) comprising 3 CDRs as follows: LCDR1 with a sequence of SEQ ID NO: 38 or a sequence having one or more amino acid substitutions, deletions, or additions (for example, 1, 2, or 3 amino acid substitutions, deletions, or additions) compared to the sequence of SEQ ID NO: 38; LCDR2 with a sequence of SEQ ID NO: 39 or a sequence having one or more amino acid substitutions, deletions, or additions (for example, 1, 2, or 3 amino acid substitutions, deletions, or additions) compared to the sequence of SEQ ID NO: 39; and LCDR3 with a sequence of SEQ ID NO: 40 or a sequence having one or more amino acid substitutions, deletions, or additions (for example, 1, 2, or 3 amino acid substitutions, deletions, or additions) compared to the sequence of SEQ ID NO: 40;
(c) a heavy chain variable region (VH) comprising 3 CDRs as follows: HCDR1 with a sequence of SEQ ID NO: 46 or a sequence having one or more amino acid substitutions, deletions, or additions (for example, 1, 2, or 3 amino acid substitutions, deletions, or additions) compared to the sequence of SEQ ID NO: 46; HCDR2 with a sequence of SEQ ID NO: 47 or a sequence having one or more amino acid substitutions, deletions, or additions (for example, 1, 2, or 3 amino acid substitutions, deletions, or additions) compared to the sequence of SEQ ID NO: 47; and HCDR3 with a sequence of SEQ ID NO: 48 or a sequence having one or more amino acid substitutions, deletions, or additions (for example, 1, 2, or 3 amino acid substitutions, deletions, or additions) compared to the sequence of SEQ ID NO: 48; and/or,
   a light chain variable region (VL) comprising 3 CDRs as follows: LCDR1 with a sequence of SEQ ID NO: 49 or a sequence having one or more amino acid substitutions, deletions, or additions (for example, 1, 2, or 3 amino acid substitutions, deletions, or additions) compared to the sequence of SEQ ID NO: 49; LCDR2 with a sequence of SEQ ID NO: 50 or a sequence having one or more amino acid substitutions, deletions, or additions (for example, 1, 2, or 3 amino acid substitutions, deletions, or additions) compared to the sequence of SEQ ID NO: 50; and LCDR3 with a sequence of SEQ ID NO: 51 or a sequence having one or more amino acid substitutions, deletions, or additions (for example, 1, 2, or 3 amino acid substitutions, deletions, or additions) compared to the sequence of SEQ ID NO: 51;
(d) a heavy chain variable region (VH) comprising 3 CDRs as follows: HCDR1 with a sequence of SEQ ID NO: 57 or a sequence having one or more amino acid substitutions, deletions, or additions (for example, 1, 2, or 3 amino acid substitutions, deletions, or additions) compared to the sequence of SEQ ID NO: 57; HCDR2 with a sequence of SEQ ID NO: 58 or a sequence having one or more amino acid substitutions, deletions, or additions (for example, 1, 2, or 3 amino acid substitutions, deletions, or additions) compared to the sequence of SEQ ID NO: 58; and HCDR3 with a sequence of SEQ ID NO: 59 or a sequence having one or more amino acid substitutions, deletions, or additions (for example, 1, 2, or 3 amino acid substitutions, deletions, or additions) compared to the sequence of SEQ ID NO: 59; and/or,
   a light chain variable region (VL) comprising 3 CDRs as follows: LCDR1 with a sequence of SEQ ID NO: 60 or a sequence having one or more amino acid substitutions, deletions, or additions (for example, 1, 2, or 3 amino acid substitutions, deletions, or additions) compared to the sequence of SEQ ID NO: 60; LCDR2 with a sequence of SEQ ID NO: 61 or a sequence having one or more amino acid substitutions, deletions, or additions (for example, 1, 2, or 3 amino acid substitutions, deletions, or additions) compared to the sequence of SEQ ID NO: 61; and LCDR3 with a sequence of SEQ ID NO: 62 or a sequence having one or more amino acid substitutions, deletions, or additions (for example, 1, 2, or 3 amino acid substitutions, deletions, or additions) compared to the sequence of SEQ ID NO: 62;
(e) a heavy chain variable region (VH) comprising 3 CDRs as follows: HCDR1 with a sequence of SEQ ID NO: 68 or a sequence having one or more amino acid substitutions, deletions, or additions (for example, 1, 2, or 3 amino acid substitutions, deletions, or additions) compared to the sequence of SEQ ID NO: 68; HCDR2 with a sequence of SEQ ID NO: 69 or 70 or a sequence having one or more amino acid substitutions, deletions, or additions (for example, 1, 2, or 3 amino acid substitutions, deletions, or additions) compared to the sequence of SEQ ID NO: 69 or 70; and HCDR3 with a sequence of SEQ ID NO: 71 or a sequence having one or more amino acid substitutions, deletions, or additions (for example, 1, 2, or 3 amino acid substitutions, deletions, or additions) compared to the sequence of SEQ ID NO: 71; and/or,
   a light chain variable region (VL) comprising 3 CDRs as follows: LCDR1 with a sequence of SEQ ID NO: 72 or a sequence having one or more amino acid substitutions, deletions, or additions (for example, 1, 2, or 3 amino acid substitutions, deletions, or additions) compared to the sequence of SEQ ID NO: 72; LCDR2 with a sequence of SEQ ID NO: 73 or a sequence having one or more amino acid substitutions, deletions, or additions (for example, 1, 2, or 3 amino acid substitutions, deletions, or additions) compared to the sequence of SEQ ID NO: 73; and LCDR3 with a sequence of SEQ ID NO: 74 or a sequence having one or more amino acid substitutions, deletions, or additions (for example, 1, 2, or 3 amino acid substitutions, deletions, or additions) compared to the sequence of SEQ ID NO: 74.

In certain embodiments, the anti-DLL3 antibody or the antigen-binding fragment thereof of the present disclosure comprises a heavy chain variable region (VH) and/or a light chain variable region (VL) as follows, wherein CDR is defined according to the Kabat numbering system:
(a) the VH comprises: HCDR1 with the sequence of SEQ ID NO: 23, HCDR2 with the sequence of SEQ ID NO: 24, HCDR3 with the sequence of SEQ ID NO: 25; and/or
   the VL comprises: LCDR1 with the sequence of SEQ ID NO: 26, LCDR2 with the sequence of SEQ ID NO: 27, LCDR3 with the sequence of SEQ ID NO: 28;
(b) the VH comprises: HCDR1 with the sequence of SEQ ID NO: 34, HCDR2 with the sequence of SEQ ID NO: 35 or 36, HCDR3 with the sequence of SEQ ID NO: 37; and/or
   the VL comprises: LCDR1 with the sequence of SEQ ID NO: 38, LCDR2 with the sequence of SEQ ID NO: 39, LCDR3 with the sequence of SEQ ID NO: 40;
(c) the VH comprises: HCDR1 with the sequence of SEQ ID NO: 46, HCDR2 with the sequence of SEQ ID NO: 47, HCDR3 with the sequence of SEQ ID NO: 48; and/or
   the VL comprises: LCDR1 with the sequence of SEQ ID NO: 49, LCDR2 with the sequence of SEQ ID NO: 50, LCDR3 with the sequence of SEQ ID NO: 51;
(d) the VH comprises: HCDR1 with the sequence of SEQ ID NO: 57, HCDR2 with the sequence of SEQ ID NO: 58, HCDR3 with the sequence of SEQ ID NO: 59; and/or
   the VL comprises: LCDR1 with the sequence of SEQ ID NO: 60, LCDR2 with the sequence of SEQ ID NO: 61, LCDR3 with the sequence of SEQ ID NO: 62;
   or,
(e) the VH comprises: HCDR1 with the sequence of SEQ ID NO: 68, HCDR2 with the sequence of SEQ ID NO: 69 or 70, HCDR3 with the sequence of SEQ ID NO: 71; and/or
   the VL comprises: LCDR1 with the sequence of SEQ ID NO: 72, LCDR2 with the sequence of SEQ ID NO: 73, LCDR3 with the sequence of SEQ ID NO: 74.

In certain embodiments, the anti-DLL3 antibody or the antigen-binding fragment thereof of the present disclosure comprises a heavy chain variable region (VH) and/or a light chain variable region (VL) as follows, wherein CDR is defined by the IMGT numbering system:
(a) a heavy chain variable region (VH) comprising 3 CDRs as follows: HCDR1 with a sequence of SEQ ID NO: 29 or a sequence having one or more amino acid substitutions, deletions, or additions (for example, 1, 2, or 3 amino acid substitutions, deletions, or additions) compared to the sequence of SEQ ID NO: 29; HCDR2 with a sequence of SEQ ID NO: 30 or a sequence having one or more amino acid substitutions, deletions, or additions (for example, 1, 2, or 3 amino acid substitutions, deletions, or additions) compared to the sequence of SEQ ID NO: 30; and HCDR3 with a sequence of SEQ ID NO: 31 or a sequence having one or more amino acid substitutions, deletions, or additions (for example, 1, 2, or 3 amino acid substitutions, deletions, or additions) compared to the sequence of SEQ ID NO: 31; and/or,
   a light chain variable region (VL) comprising 3 CDRs as follows: LCDR1 with a sequence of SEQ ID NO: 32 or a sequence having one or more amino acid substitutions, deletions, or additions (for example, 1, 2, or 3 amino acid substitutions, deletions, or additions) compared to the sequence of NO: 32; LCDR2 with a sequence that is FAS or a sequence having one or more amino acid substitutions, deletions, or additions (for example, 1, 2, or 3 amino acid substitutions, deletions, or additions) compared to the sequence that is FAS; and LCDR3 with a sequence of SEQ ID NO: 28 or a sequence having one or more amino acid substitutions, deletions, or additions (for example, 1, 2, or 3 amino acid substitutions, deletions, or additions) compared to the sequence of SEQ ID NO: 28;
(b) a heavy chain variable region (VH) comprising 3 CDRs as follows: HCDR1 with a sequence of SEQ ID NO: 41 or a sequence having one or more amino acid substitutions, deletions, or additions (for example, 1, 2, or 3 amino acid substitutions, deletions, or additions) compared to the sequence of SEQ ID NO: 41; HCDR2 with a sequence of SEQ ID NO: 42 or a sequence having one or more amino acid substitutions, deletions, or additions (for example, 1, 2, or 3 amino acid substitutions, deletions, or additions) compared to the sequence of SEQ ID NO: 42; and HCDR3 with a sequence of SEQ ID NO: 43 or a sequence having one or more amino acid substitutions, deletions, or additions (for example, 1, 2, or 3 amino acid substitutions, deletions, or additions) compared to the sequence of SEQ ID NO: 43; and/or,
   a light chain variable region (VL) comprising 3 CDRs as follows: LCDR1 with a sequence of SEQ ID NO: 44 or a sequence having one or more amino acid substitutions, deletions, or additions (for example, 1, 2, or 3 amino acid substitutions, deletions, or additions) compared to the sequence of NO: 44; LCDR2 with a sequence that is YAS or a sequence having one or more amino acid substitutions, deletions, or additions (for example, 1, 2, or 3 amino acid substitutions, deletions, or additions) compared to the sequence that is YAS; and LCDR3 with a sequence of SEQ ID NO: 40 or a sequence having one or more amino acid substitutions, deletions, or additions (for example, 1, 2, or 3 amino acid substitutions, deletions, or additions) compared to the sequence of SEQ ID NO: 40;
(c) a heavy chain variable region (VH) comprising 3 CDRs as follows: HCDR1 with a sequence of SEQ ID NO: 52 or a sequence having one or more amino acid substitutions, deletions, or additions (for example, 1, 2, or 3 amino acid substitutions, deletions, or additions) compared to the sequence of SEQ ID NO: 52; HCDR2 with a sequence of SEQ ID NO: 53 or a sequence having one or more amino acid substitutions, deletions, or additions (for example, 1, 2, or 3 amino acid substitutions, deletions, or additions) compared to the sequence of SEQ ID NO: 53; and HCDR3 with a sequence of SEQ ID NO: 54 or a sequence having one or more amino acid substitutions, deletions, or additions (for example, 1, 2, or 3 amino acid substitutions, deletions, or additions) compared to the sequence of SEQ ID NO: 54; and/or,
   a light chain variable region (VL) comprising 3 CDRs as follows: LCDR1 with a sequence of SEQ ID NO: 55 or a sequence having one or more amino acid substitutions, deletions, or additions (for example, 1, 2, or 3 amino acid substitutions, deletions, or additions) compared to the sequence of NO: 55; LCDR2 with a sequence that is YTS or a sequence having one or more amino acid substitutions, deletions, or additions (for example, 1, 2, or 3 amino acid substitutions, deletions, or additions) compared to the sequence that is YTS; and LCDR3 with a sequence of SEQ ID NO: 51 or a sequence having one or more amino acid substitutions, deletions, or additions (for example, 1, 2, or 3 amino acid substitutions, deletions, or additions) compared to the sequence of SEQ ID NO: 51;
(d) a heavy chain variable region (VH) comprising 3 CDRs as follows: HCDR1 with a sequence of SEQ ID NO: 63 or a sequence having one or more amino acid substitutions, deletions, or additions (for example, 1, 2, or 3 amino acid substitutions, deletions, or additions) compared to the sequence of SEQ ID NO: 63; HCDR2 with a sequence of SEQ ID NO: 64 or a sequence having one or more amino acid substitutions, deletions, or additions (for example, 1, 2, or 3 amino acid substitutions, deletions, or additions) compared to the sequence of SEQ ID NO: 64; and HCDR3 with a sequence of SEQ ID NO: 65 or a sequence having one or more amino acid substitutions, deletions, or additions (for example, 1, 2, or 3 amino acid substitutions, deletions, or additions) compared to the sequence of SEQ ID NO: 65; and/or,
   a light chain variable region (VL) comprising 3 CDRs as follows: LCDR1 with a sequence of SEQ ID NO: 66 or a sequence having one or more amino acid substitutions, deletions, or additions (for example, 1, 2, or 3 amino acid substitutions, deletions, or additions) compared to the sequence of NO: 66; LCDR2 with a sequence that is WAS or a sequence having one or more amino acid substitutions, deletions, or additions (for example, 1, 2, or 3 amino acid substitutions, deletions, or additions) compared to the sequence that is WAS; and LCDR3 with a sequence of SEQ ID NO: 62 or a sequence having one or more amino acid substitutions, deletions, or additions (for example, 1, 2, or 3 amino acid substitutions, deletions, or additions) compared to the sequence of SEQ ID NO: 62;
(e) a heavy chain variable region (VH) comprising 3 CDRs as follows: HCDR1 with a sequence of SEQ ID NO: 75 or a sequence having one or more amino acid substitutions, deletions, or additions (for example, 1, 2, or 3 amino acid substitutions, deletions, or additions) compared to the sequence of SEQ ID NO: 75; HCDR2 with a sequence of SEQ ID NO: 76 or 77 or a sequence having one or more amino acid substitutions, deletions, or additions (for example, 1, 2, or 3 amino acid substitutions, deletions, or additions) compared to the sequence of SEQ ID NO: 76 or 77; and HCDR3 with a sequence of SEQ ID NO: 78 or a sequence having one or more amino acid substitutions, deletions, or additions (for example, 1, 2, or 3 amino acid substitutions, deletions, or additions) compared to the sequence of SEQ ID NO: 78; and/or,
   a light chain variable region (VL) comprising 3 CDRs as follows: LCDR1 with a sequence of SEQ ID NO: 79 or a sequence having one or more amino acid substitutions, deletions, or additions (for example, 1, 2, or 3 amino acid substitutions, deletions, or additions) compared to the sequence of NO: 79; LCDR2 with a sequence that is LAS or a sequence having one or more amino acid substitutions, deletions, or additions (for example, 1, 2, or 3 amino acid substitutions, deletions, or additions) compared to the sequence that is LAS; and LCDR3 with a sequence of SEQ ID NO: 74 or a sequence having one or more amino acid substitutions, deletions, or additions (for example, 1, 2, or 3 amino acid substitutions, deletions, or additions) compared to the sequence of SEQ ID NO: 74;

In certain embodiments, the anti-DLL3 antibody or the antigen-binding fragment thereof of the present disclosure comprises a heavy chain variable region (VH) and/or a light chain variable region (VL) as follows, wherein CDR is defined by the IMGT numbering system:
(a) the VH comprises: HCDR1 with the sequence of SEQ ID NO: 29, HCDR2 with the sequence of SEQ ID NO: 30, HCDR3 with the sequence of SEQ ID NO: 31; and/or
   the VL comprises: LCDR1 with the sequence of SEQ ID NO: 32, LCDR2 with the sequence that is FAS, LCDR3 with the sequence of SEQ ID NO: 28;
(b) the VH comprises: HCDR1 with the sequence of SEQ ID NO: 41, HCDR2 with the sequence of SEQ ID NO: 42, HCDR3 with the sequence of SEQ ID NO: 43; and/or
   the VL comprises: LCDR1 with the sequence of SEQ ID NO: 44, LCDR2 with the sequence that is YAS, LCDR3 with the sequence of SEQ ID NO: 40;
(c) the VH comprises: HCDR1 with the sequence of SEQ ID NO: 52, HCDR2 with the sequence of SEQ ID NO: 53, HCDR3 with the sequence of SEQ ID NO: 54; and/or
   the VL comprises: LCDR1 with the sequence of SEQ ID NO: 55, LCDR2 with the sequence that is YTS, LCDR3 with the sequence of SEQ ID NO: 51;
(d) the VH comprises: HCDR1 with the sequence of SEQ ID NO: 63, HCDR2 with the sequence of SEQ ID NO: 64, HCDR3 with the sequence of SEQ ID NO: 65; and/or
   the VL comprises: LCDR1 with the sequence of SEQ ID NO: 66, LCDR2 with the sequence that is WAS, LCDR3 with the sequence of SEQ ID NO: 62;
   or,
(e) the VH comprises: HCDR1 with the sequence of SEQ ID NO: 75, HCDR2 with the sequence of SEQ ID NO: 76 or 77, HCDR3 with the sequence of SEQ ID NO: 78; and/or
   the VL comprises: LCDR1 with the sequence of SEQ ID NO: 79, LCDR2 with the sequence that is LAS, LCDR3 with the sequence of SEQ ID NO: 74.

In certain embodiments, the anti-DLL3 antibody or the antigen-binding fragment thereof of the present disclosure comprises the following heavy chain variable region (VH) and/or light chain variable region (VL), wherein at least one CDR in the heavy chain variable region (VH) and/or light chain variable region (VL) contains a mutation compared to the CDRs defined by the aforementioned Kabat numbering system or IMGT numbering system, and the mutation is one or more amino acid substitutions, deletions, additions, or any combination thereof (for example, 1, 2, or 3 amino acid substitutions, deletions, additions, or any combination thereof), and still retain the binding activity to DLL3.

Preferably, the substitution described in the present disclosure is a conservative substitution.

In certain embodiments, the antibody or the antigen-binding fragment thereof binds to human DLL3, monkey DLL3, and/or rat DLL3.

In certain embodiments, the VH of the anti-DLL3 antibody or the antigen-binding fragment thereof of the present disclosure comprises a framework region (FR) derived from a heavy chain variable region (VH) of a human immunoglobulin, and/or the VL of the antibody or the antigen-binding fragment thereof comprises a framework region (FR) derived from a light chain variable region (VL) of a human immunoglobulin. Thus, in certain embodiments, the anti-DLL3 antibody or the antigen-binding fragment thereof of the present disclosure is a fully human antibody. In certain embodiments, the anti-DLL3 antibody or the antigen-binding fragment thereof of the present disclosure is a humanized antibody.

In certain embodiments, the anti-DLL3 antibody or the antigen-binding fragment thereof of the present disclosure comprises:
(a) a heavy chain framework region of a human immunoglobulin or a variant thereof, wherein the variant has at most 20 conservative amino acid substitutions compared to the amino acid sequence encoded by the germline antibody gene from which the variant is derived (for example, at most 20, at most 15, at most 10, or at most 5 conservative amino acid substitutions; for example, 1, 2, 3, 4, 5, 6, 7, 8, 9, or 10 conservative amino acid substitutions); and/or
(b) a light chain framework region of a human immunoglobulin or a variant thereof, wherein the variant has at most 20 conservative amino acid substitutions compared to the amino acid sequence encoded by the germline antibody gene from which the variant is derived (for example, at most 20, at most 15, at most 10, or at most 5 conservative amino acid substitutions; for example, 1, 2, 3, 4, 5, 6, 7, 8, 9, or 10 conservative amino acid substitutions).

In certain embodiments, the anti-DLL3 antibody or the antigen-binding fragment thereof of the present disclosure is humanized to a degree of at least 75%, at least 80%, at least 85%, at least 90%, at least 91%, at least 92%, at least 93% %, at least 94%, at least 95%, at least 96%, at least 97%, at least 98%, or at least 99%.

In certain embodiments, the anti-DLL3 antibody or the antigen-binding fragment thereof of the present disclosure comprises:
(a) a heavy chain variable region (VH), which comprises or consists of an amino acid sequence selected from the following:
   (i) a sequence shown in SEQ ID NO: 1, 2, 5, 6, 7, 10, 11, 14, 15, 18, 19, or 20;
   (ii) a sequence having one or more amino acid substitutions, deletions, additions, or any combination thereof (for example, 1, 2, 3, 4, 5, 6, 7, 8, 9, or 10 amino acid substitutions, deletions, additions, or any combination thereof) compared to the sequence shown in SEQ ID NO: 1, 2, 5, 6, 7, 10, 11, 14, 15, 18, 19, or 20; or
   (iii) a sequence having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity compared to the sequence shown in SEQ ID NO: 1, 2, 5, 6, 7, 10, 11, 14, 15, 18, 19, or 20;
      and/or
(b) a light chain variable region (VL), which comprises or consists of an amino acid sequence selected from the following:
   (iv) a sequence shown in SEQ ID NO: 3, 4, 8, 9, 12, 13, 16, 17, 21, or 22;
   (v) a sequence having one or more amino acid substitutions, deletions, additions, or any combination thereof (for example, 1, 2, 3, 4, 5, 6, 7, 8, 9, or 10 amino acid substitutions, deletions, additions, or any combination thereof) compared to the sequence shown in SEQ ID NO: 3, 4, 8, 9, 12, 13, 16, 17, 21, or 22; or
   (vi) a sequence having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity compared to the sequence shown in SEQ ID NO: 3, 4, 8, 9, 12, 13, 16, 17, 21, or 22.

In certain embodiments, the anti-DLL3 antibody or the antigen-binding fragment thereof of the present disclosure comprises a VH shown in SEQ ID NO: 1 or 2, and/or, a VL shown in SEQ ID NO: 3 or 4.

In certain embodiments, the anti-DLL3 antibody or the antigen-binding fragment thereof of the present disclosure comprises a VH shown in SEQ ID NO: 5, 6, or 7, and/or, a VL shown in SEQ ID NO: 8 or 9.

In certain embodiments, the anti-DLL3 antibody or the antigen-binding fragment thereof of the present disclosure comprises a VH shown in SEQ ID NO: 10 or 11, and/or, a VL shown in SEQ ID NO: 12 or 13.

In certain embodiments, the anti-DLL3 antibody or the antigen-binding fragment thereof of the present disclosure comprises a VH shown in SEQ ID NO: 14 or 15, and/or, a VL shown in SEQ ID NO: 16 or 17.

In certain embodiments, the anti-DLL3 antibody or the antigen-binding fragment thereof of the present disclosure comprises a VH shown in SEQ ID NO: 18, 19, or 20, and/or, a VL shown in SEQ ID NO: 21 or 22.

In certain embodiments, the anti-DLL3 antibody or the antigen-binding fragment thereof of the present disclosure comprises a VH having at least 70%, at least 80%, at least 85%, at least 90%, at least 91%, at least 92%, at least 93%, at least 94%, at least 95%, at least 96%, at least 97%, at least 98%, or at least 99% sequence identity compared to the VH in any one of the above five groups; and/or a VL having at least 70%, at least 80%, at least 85%, at least 90%, at least 91%, at least 92%, at least 93%, at least 94%, at least 95%, at least 96%, at least 97%, at least 98%, or at least 99% sequence identity compared to the VL of the respective group; or

In certain embodiments, the anti-DLL3 antibody or the antigen-binding fragment thereof of the present disclosure comprises a VH with one or more amino acid substitutions, deletions, additions, or any combination thereof, for example, 1, 2, 3, 4, 5, 6, 7, 8, 9, or 10 amino acid substitutions, deletions, additions, or any combination thereof, compared to the VH in any one of the above five groups; and/or a VL with one or more amino acid substitutions, deletions, or additions, or any combination thereof, for example, 1, 2, 3, 4, 5, 6, 7, 8, 9, or 10 amino acid substitutions, deletions, or additions, or any combination thereof, compared to the VL of the respective group; preferably, the substitutions are conservative substitutions.

In certain embodiments, the anti-DLL3 antibody or the antigen-binding fragment thereof of the present disclosure comprises:
(a) a VH shown in the sequence of SEQ ID NO: 1 and a VL shown in the sequence of SEQ ID NO: 3;
(b) a VH shown in the sequence of SEQ ID NO: 2 and a VL shown in the sequence of SEQ ID NO: 4;
(c) a VH shown in the sequence of SEQ ID NO: 5 and a VL shown in the sequence of SEQ ID NO: 8;
(d) a VH shown in the sequence of SEQ ID NO: 6 and a VL shown in the sequence of SEQ ID NO: 9;
(e) a VH shown in the sequence of SEQ ID NO: 7 and a VL shown in the sequence of SEQ ID NO: 9;
(f) a VH shown in the sequence of SEQ ID NO: 10 and a VL shown in the sequence of SEQ ID NO: 12;
(g) a VH shown in the sequence of SEQ ID NO: 11 and a VL shown in the sequence of SEQ ID NO: 13;
(h) a VH shown in the sequence of SEQ ID NO: 14 and a VL shown in the sequence of SEQ ID NO: 16;
(i) a VH shown in the sequence of SEQ ID NO: 15 and a VL shown in the sequence of SEQ ID NO: 17;
(j) a VH shown in the sequence of SEQ ID NO: 18 and a VL shown in the sequence of SEQ ID NO: 21;
(k) a VH shown in the sequence of SEQ ID NO: 19 and a VL shown in the sequence of SEQ ID NO: 22;
(l) a VH shown in the sequence of SEQ ID NO: 20 and a VL shown in the sequence of SEQ ID NO: 22;
(m) a heavy chain variable region (VH) and a light chain variable region (VL), wherein the heavy chain variable region (VH) and the light chain variable region (VL) independently have at least 70%, at least 80%, at least 85%, at least 90%, at least 91%, at least 92%, at least 93%, at least 94%, at least 95%, at least 96%, at least 97%, at least 98%, or at least 99% sequence identity compared to the VH and VL in any one of the groups (a) to (1), respectively; or
(n) a heavy chain variable region (VH) and a light chain variable region (VL), wherein the heavy chain variable region (VH) and the light chain variable region (VL) independently have one or more amino acid substitutions, deletions, additions, or any combination thereof (for example, 1, 2, 3, 4, 5, 6, 7, 8, 9, or 10 amino acid substitutions, deletions, additions, or any combination thereof) compared to the VH and VL in any one of the groups (a) to (1), respectively. Preferably, the substitution is a conservative substitution.

In certain embodiments, the anti-DLL3 antibody of the present disclosure is a chimeric antibody, a humanized antibody, or a fully human antibody. In certain embodiments, the anti-DLL3 antibody or the antigen-binding fragment thereof of the present disclosure is selected from Fab, Fab', (Fab')₂, a Fv fragment such as scFv or disulfide-linked Fv (dsFv), a diabody, and a multi-specific antibody. In certain embodiments, the anti-DLL3 antibody of the present disclosure is scFv.

In certain embodiments, the heavy chain of the anti-DLL3 antibody or the antigen-binding fragment thereof of the present disclosure comprises a heavy chain constant region (CH) of a human immunoglobulin or a variant thereof, wherein the variant has at most 50 conservative amino acid substitutions compared to the wild-type sequence from which the variant is derived (for example, at most 45, at most 40, at most 35, at most 30, at most 25, at most 20, at most 15, at most 10, or at most 5 conservative amino acid substitutions; for example, 1, 2, 3, 4, 5, 6, 7, 8, 9, or 10 conservative amino acid substitutions). In certain embodiments, the light chain of the anti-DLL3 antibody or the antigen-binding fragment thereof of the present disclosure comprises a light chain constant region (CL) of a human immunoglobulin or a variant thereof, wherein the variant has at most 50 conservative amino acid substitutions compared to the wild-type sequence from which the variant is derived (for example, at most 45, at most 40, at most 35, at most 30, at most 25, at most 20, at most 15, at most 10, or at most 5 conservative amino acid substitutions; for example, 1, 2, 3, 4, 5, 6, 7, 8, 9, or 10 conservative amino acid substitutions).

In some embodiments, the constant region is altered, *e.g.,* mutated, to modify the properties of the anti-DLL3 antibody molecule (*e.g.,* to alter one or more of the following characteristics: Fc receptor binding, antibody glycosylation, the number of cysteine residues, effector cell function, or complement function). Functional changes can be produced by replacing at least one amino acid residue with a different residue in the constant region of an antibody, *e.g.,* altering the affinity of the antibody for an effector ligand (such as FcR or complement C1q), thus altering (*e.g.,* decreasing) effector function. The Fc region of antibodies mediates several important effector functions, such as ADCC, antibody-dependent cellular phagocytosis (ADCP), and CDC.

In certain embodiments, the anti-DLL3 antibody or the antigen-binding fragment thereof of the present disclosure has a heavy chain constant region (CH), which is selected from, for example, the heavy chain constant regions of IgG1, IgG2, IgG3, IgG4, IgM, IgA1, IgA2, IgD, and IgE; specifically selected from, for example, the heavy chain constant regions of IgG1, IgG2, IgG3, and IgG4, and more specifically selected from the heavy chain constant region of IgG1 (for example, human IgG1). In some embodiments, the heavy chain constant region of human IgG1 is shown in SEQ ID NO: 82. In certain embodiments, the antibody or the antigen-binding fragment thereof of the present disclosure has a light chain constant region, which is selected from, for example, a κ or λ light chain constant region, preferably a κ light chain constant region (for example, a human κ light chain constant region). In some embodiments, the light chain constant region has a sequence shown in SEQ ID NO: 83.

In certain embodiments, the anti-DLL3 antibody or the antigen-binding fragment thereof comprises a CH shown in SEQ ID NO: 82 or a variant thereof, the variant having at most 20 conservative amino acid substitutions compared to SEQ ID NO: 82 (for example, at most 20, at most 15, at most 10, or at most 5 conservative amino acid substitutions, for example, 1, 2, 3, 4, 5, 6, 7, 8, 9, or 10 conservative amino acid substitutions); or having at least 70%, at least 80%, at least 85%, at least 90%, at least 91%, at least 92%, at least 93%, at least 94%, at least 95%, at least 96%, at least 97%, at least 98%, or at least 99% sequence identity compared to SEQ ID NO: 70.

In some embodiments, the anti-DLL3 antibody or the antigen-binding fragment thereof comprises a light chain constant region or a variant thereof. In some embodiments, the light chain constant region comprises a κ light chain constant region. In some embodiments, the light chain constant region comprises a light chain constant region (CL) shown in SEQ ID NO: 83 or a variant thereof, the variant having at most 20 conservative amino acid substitutions compared to SEQ ID NO: 83 (for example, at most 20, at most 15, at most 10, or at most 5 conservative amino acid substitutions, for example, 1, 2, 3, 4, 5, 6, 7, 8, 9, or 10 conservative amino acid substitutions); or having at least 70%, at least 80%, at least 85%, at least 90%, at least 91%, at least 92%, at least 93%, at least 94%, at least 95%, at least 96%, at least 97%, at least 98%, or at least 99% sequence identity compared to SEQ ID NO: 80.

In some embodiments, the anti-DLL3 antibody or the antigen-binding fragment thereof comprises a heavy chain constant region (CH) shown in SEQ ID NO: 82 and a light chain constant region (CL) shown in SEQ ID NO: 83.

In certain embodiments, the anti-DLL3 antibody or the antigen-binding fragment thereof of the present disclosure comprises:
(a) a heavy chain comprising or consisting of an amino acid sequence selected from the following:
   (i) a sequence comprising a VH shown in SEQ ID NO: 6 or 7 and a CH shown in SEQ ID NO: 82;
   (ii) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (i); and
(b) a light chain comprising an amino acid sequence selected from the following:
   (iv) a sequence comprising a VL shown in SEQ ID NO: 9 and a CL shown in SEQ ID NO: 83;
   (v) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (iv).

In some embodiments, the substitution described in (ii) or (v) is a conservative substitution.

In certain embodiments, the anti-DLL3 antibody or the antigen-binding fragment thereof of the present disclosure comprises:
(a) a heavy chain comprising an amino acid sequence selected from the following:
   (i) a sequence comprising a VH shown in SEQ ID NO: 19 or 20 and a CH shown in SEQ ID NO: 82;
   (ii) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (i); and
(b) a light chain comprising an amino acid sequence selected from the following:
   (iv) a sequence comprising a VL shown in SEQ ID NO: 22 and a CL shown in SEQ ID NO: 83;
   (v) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (iv).

In some embodiments, the substitution described in (ii) or (v) is a conservative substitution.

In some embodiments, the substitution described in (ii) or (v) is a conservative substitution.

In certain embodiments, the anti-DLL3 antibody or the antigen-binding fragment thereof of the present disclosure comprises a heavy chain and a light chain;
the heavy chain comprises or consists of the following sequences:
   (i) a sequence shown in SEQ ID NO: 33;
   (ii) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (i); and
the light chain comprises or consists of the following sequences:
   (iv) a sequence shown in SEQ ID NO: 56;
   (v) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (iv);
preferably, the substitution described in (ii) or (v) is a conservative substitution.

In certain embodiments, the anti-DLL3 antibody or the antigen-binding fragment thereof of the present disclosure comprises a heavy chain and a light chain;
the heavy chain comprises or consists of the following sequences:
   (i) a sequence shown in SEQ ID NO: 45;
   (ii) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (i); and
the light chain comprises or consists of the following sequences:
   (iv) a sequence shown in SEQ ID NO: 56;
   (v) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (iv);
preferably, the substitution described in (ii) or (v) is a conservative substitution.

In certain embodiments, the anti-DLL3 antibody or the antigen-binding fragment thereof of the present disclosure comprises a heavy chain and a light chain;
the heavy chain comprises or consists of the following sequences:
   (i) a sequence shown in SEQ ID NO: 67;
   (ii) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (i); and
the light chain comprises or consists of the following sequences:
   (iv) a sequence shown in SEQ ID NO: 81;
   (v) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (iv);
preferably, the substitution described in (ii) or (v) is a conservative substitution.

In certain embodiments, the anti-DLL3 antibody or the antigen-binding fragment thereof of the present disclosure comprises a heavy chain and a light chain;
the heavy chain comprises or consists of the following sequences:
   (i) a sequence shown in SEQ ID NO: 80;
   (ii) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (i); or
   (iii) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (i); and
the light chain comprises or consists of the following sequences:
   (iv) a sequence shown in SEQ ID NO: 81;
   (v) a sequence having one or more amino acid substitutions, deletions, additions, or any combination thereof (for example, at most 50, at most 45, at most 40, at most 35, at most 30, at most 25, at most 20, at most 15, at most 10, or at most 5 amino acid substitutions, deletions, additions, or any combination thereof; for example, 1, 2, 3, 4, 5, 6, 7, 8, 9, or 10 amino acid substitutions, deletions, additions, or any combination thereof) compared to the sequence shown in (iv); or
   (vi) a sequence having at least 80%, at least 85%, at least 90%, at least 91%, at least 92%, at least 93%, at least 94%, at least 95%, at least 96%, at least 97%, at least 98%, or at least 99% sequence identity compared to the sequence shown in (iv);
preferably, the substitution described in (ii) or (v) is a conservative substitution.

In certain embodiments, the anti-DLL3 antibody or the antigen-binding fragment thereof, wherein the antibody comprises a heavy chain and a light chain selected from the group consisting of:
(a) a heavy chain comprising a VH shown in SEQ ID NO: 6 or 7 and a CH shown in SEQ ID NO: 82, and a light chain comprising a VL shown in SEQ ID NO: 9 and a CL shown in SEQ ID NO: 83;
(b) a heavy chain comprising a VH shown in SEQ ID NO: 19 or 20 and a CH shown in SEQ ID NO: 82, and a light chain comprising a VL shown in SEQ ID NO: 22 and a CL shown in SEQ ID NO: 83.

In certain embodiments, the antibody provided by the present disclosure is a multi-specific antibody that binds DLL3 as well as one or more other antigens. In certain preferred embodiments, the multi-specific antibody is a bispecific antibody, a trispecific antibody, or a tetraspecific antibody. In some embodiments, the multi-specific antibody of the present disclosure comprises the anti-DLL3 antibody or the antigen-binding fragment thereof, and an additional antibody or fragment thereof or antibody analog.

### Antibody derivatives of the present disclosure

The anti-DLL3 antibody or the antigen-binding fragment thereof of the present disclosure can be derivatized, for example, linked to another molecule (*e.g.,* another polypeptide or protein). Generally, derivatization (*e.g.,* labeling) of an antibody or an antigen-binding fragment thereof does not adversely affect its binding to DLL3. Accordingly, the anti-DLL3 antibody or the antigen-binding fragment thereof of the present disclosure is also intended to include such derivatized forms. For example, the anti-DLL3 antibody or the antigen-binding fragment thereof of the present disclosure can be linked (by chemical coupling, genetic fusion, non-covalent linkage, or other means) to one or more other molecular moieties, such as another antibody (*e.g.,* to form a bispecific antibody), a detection reagent, a pharmaceutical reagent, and/or a protein or polypeptide (*e.g.,* an avidin or a polyhistidine tag) that is capable of mediating the binding of the antibody or the antigen-binding fragment thereof to another molecule.

One type of derivatized antibody (*e.g.,* bispecific antibody) is produced by cross-linking 2 or more antibodies (of the same type or of different types). Methods for obtaining bispecific antibodies are well known in the art, examples of which include, but are not limited to, chemical cross-linking methods, cell engineering methods (hybridoma methods), or genetic engineering methods.

Another type of derivatized antibody is a labeled antibody. For example, the antibody or the antigen-binding fragment thereof of the present disclosure can be linked to a detectable label. The detectable label described in the present disclosure can be any substance that can be detected by fluorescence, spectrometry, photochemistry, biochemistry, immunology, electrical, optical, or chemical means. Such labels are well known in the art, examples of which include, but are not limited to, enzymes (*e.g.,* horseradish peroxidase, alkaline phosphatase, β-galactosidase, urease, or glucose oxidase), radionuclides (*e.g.,* ³H, ¹²⁵I, ³⁵S, ¹⁴C, or ³²P), fluorescent dyes (*e.g.,* fluorescein isothiocyanate (FITC), fluorescein, tetramethylrhodamine isothiocyanate (TRITC), phycoerythrin (PE), Texas Red, rhodamine, quantum dots, or cyanine dye derivatives (*e.g.,* Cy7 or Alexa 750)), acridinium ester compounds, magnetic beads (*e.g.,* Dynabeads^{®}), calorimetric markers such as colloidal gold or colored glass or plastic (*e.g.,* polystyrene, polypropylene or latex) beads, and biotin for binding to avidin (*e.g.,* streptavidin) modified with the above mentioned markers. Patents teaching the use of such markers include, but are not limited to, U.S. Patent Nos. 3,817,837; 3,850,752; 3,939,350; 3,996,345; 4,277,437; 4,275,149; and 4,366,241 (all incorporated herein by reference). Detectable labels as described above can be detected by methods known in the art. For example, radioactive labels can be detected using photographic film or a scintillation calculator, and fluorescent markers can be detected using a light detector to detect emitted light. Enzyme markers are generally detected by providing a substrate for the enzyme and detecting the reaction product generated by the enzyme's action on the substrate, and calorimetric markers are detected by simply visualizing a colored marker. In certain embodiments, such labels can be adapted for immunological assays (*e.g.,* enzyme-linked immunosorbent assay, radioimmunoassay, fluorescent immunoassay, or chemiluminescent immunoassay). In certain embodiments, detectable labels as described above can be linked to the antibodies or the antigen-binding fragments thereof of the present disclosure via linkers of varying lengths to reduce potential steric hindrance.

In addition, the antibody or the antigen-binding fragment thereof of the present disclosure can also be derivatized with chemical groups, such as polyethylene glycol (PEG), methyl, ethyl, or glycosyl groups. These groups can be used to improve the biological properties of the antibody, such as increasing the serum half-life.

### Preparation of antibodies

The antibodies of the present disclosure can be prepared by various methods known in the art, such as through genetic engineering and recombinant technology. For example, a DNA molecule encoding the heavy and light chain genes of the antibodies of the present disclosure are obtained by chemical synthesis or PCR amplification. The resulting DNA molecule is inserted into an expression vector and then transfected into host cells. The transfected host cells are then cultured under specific conditions to express the antibodies of the present disclosure.

The antigen-binding fragments of the present disclosure can be obtained by hydrolysis of intact antibody molecules (see Morimoto et al., J. Biochem. Biophys. Methods 24:107-117 (1992) and Brennan et al., Science 229:81 (1985)). Alternatively, these antigen-binding fragments can also be produced directly from recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab, or F(ab')₂ fragments can also be directly isolated from the recombinant host cell culture medium. Those of ordinary skill in the art are well aware of other techniques for preparing such antigen-binding fragments.

In a third aspect, the present disclosure provides an isolated nucleic acid molecule comprising a nucleotide sequence encoding the antibody or the antigen-binding fragment thereof of the second aspect of the present disclosure, or a heavy chain variable region and/or a light chain variable region thereof, or one or more CDRs thereof, or a multi-specific antibody of the present disclosure; in some embodiments, the nucleotide sequence is substitutable according to codon degeneracy. In certain embodiments, the nucleotide sequence is codon optimized.

In certain embodiments, the isolated nucleic acid molecule of the present disclosure comprises: (i) a first nucleic acid and a second nucleic acid encoding, respectively, a heavy chain variable region and a light chain variable region of the antibody or the antigen-binding fragment thereof of the second aspect of the present disclosure, or (ii) a first nucleic acid encoding, respectively, a heavy chain variable region and a heavy chain constant region and a second nucleic acid encoding, respectively, a light chain variable region and a light chain constant region of the antibody or the antigen-binding fragment thereof of the second aspect of the present disclosure, or (iii) a first nucleic acid and a second nucleic acid encoding, respectively, a heavy chain and a light chain of the antibody or the antigen-binding fragment thereof of the second aspect of the present disclosure. In certain embodiments, the first nucleic acid and the second nucleic acid comprise nucleic acids having a degenerate sequence or a sequence that is substantially identical to any of the first nucleic acids and second nucleic acids described in (i)-(iii) above. In certain embodiments, the degenerate sequences or substantially identical sequence refers to a sequence having at least about 85%, 90%, 95%, 99%, or higher sequence identity compared to the nucleic acid molecules described in (i)-(iii), a sequence having one or more nucleotide substitutions, or a sequence differing by no more than 3, 6, 15, 30, or 45 nucleotides.

In a fourth aspect, there is provided a vector (*e.g.,* a cloning vector or an expression vector) comprising the isolated nucleic acid molecule of the third aspect of the present disclosure. In certain embodiments, the vector of the present disclosure is a cloning vector or an expression vector. In certain embodiments, the vector of the present disclosure is, for example, a plasmid, a cosmid, a phage, a lentivirus, etc. In certain embodiments, the vector is capable of expressing the antibody or the antigen-binding fragment thereof of the present disclosure in a subject (*e.g.,* a mammal, such as a human).

In a fifth aspect, there is provided a host cell comprising the isolated nucleic acid molecule of the third aspect of the present disclosure or the vector of the fourth aspect of the present disclosure. The host cell may be a eukaryotic cell (*e.g.,* mammalian cell, insect cell, or yeast cell) or a prokaryotic cell (*e.g.,* E. coli). Suitable eukaryotic cells include, but are not limited to, NS0 cells, SP2/0 cells, Vero cells, Hela cells, COS cells, CHO cells, HEK293 cells, BHK cells, and MDCKII cells. Suitable insect cells include, but are not limited to, Sf9 cells. In certain embodiments, the host cell of the present disclosure is a mammalian cell, such as CHO (*e.g.,* CHO-K1, CHO-S, CHO DXB11, or CHO DG44).

In a sixth aspect, there is provided a method for preparing the antibody or the antigen-binding fragment thereof of the second aspect of the present disclosure, or the multi-specific antibody of the present disclosure, which comprises culturing the host cell of the present disclosure under conditions that allow the expression of the antibody or the antigen-binding fragment thereof, or the multi-specific antibody, and recovering the antibody or the antigen-binding fragment thereof from a cultured host cell culture.

### Antibody-drug conjugate

In a seventh aspect, there is provided an anti-DLL3 antibody-drug conjugate, a stereoisomer thereof, a prodrug thereof, a pharmaceutically acceptable salt thereof, or a pharmaceutically acceptable solvate thereof, wherein the antibody is the aforementioned antibody or antigen-binding fragment thereof that binds to DLL3.

In some embodiments, the antibody-drug conjugate is selected from: 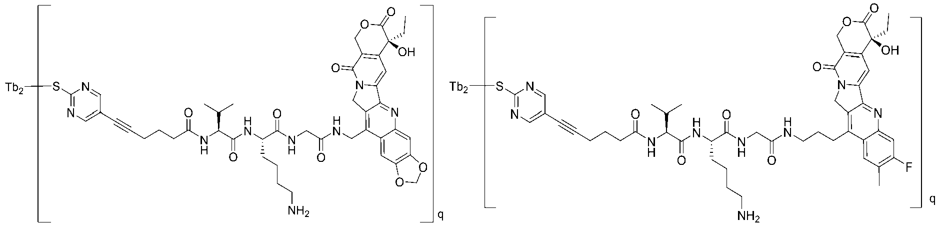 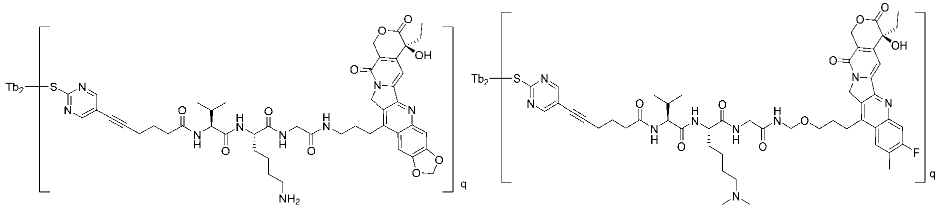 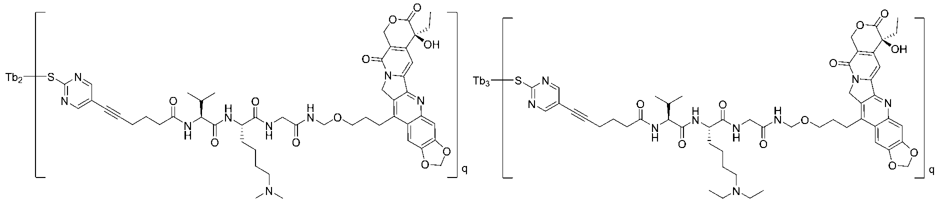 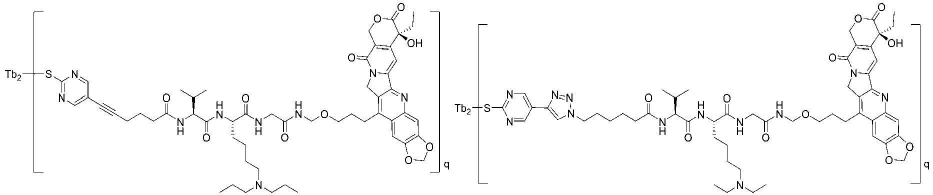 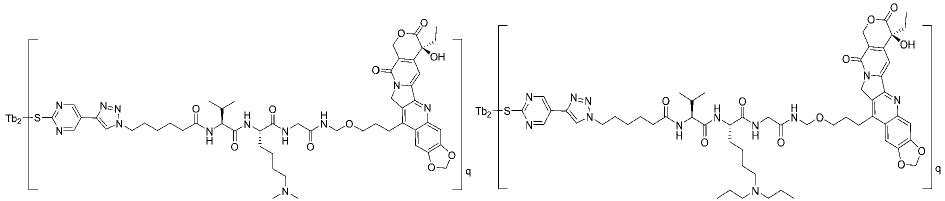 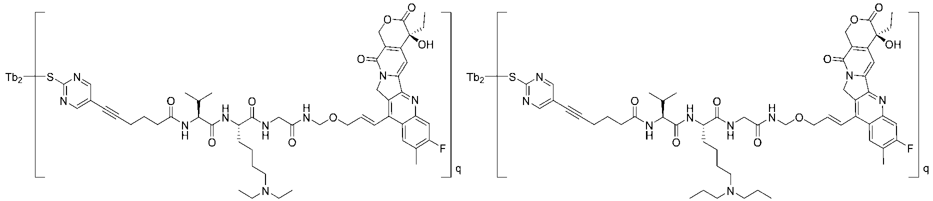 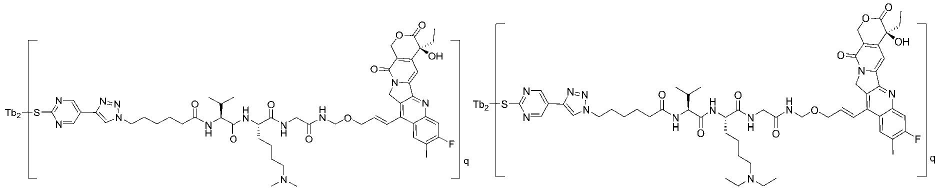 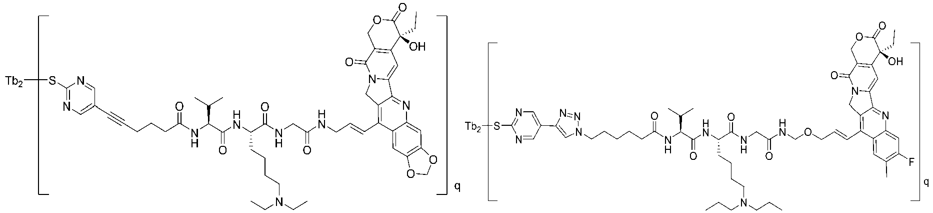 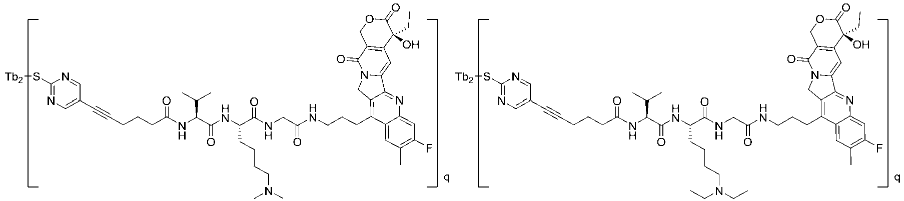 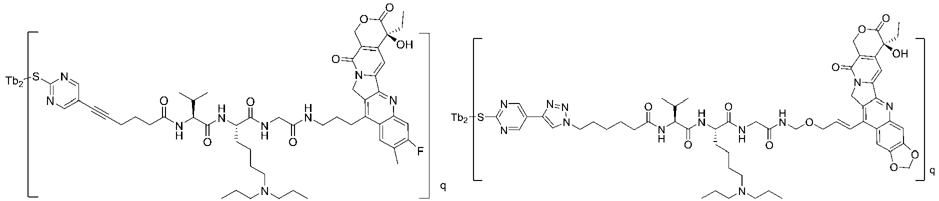 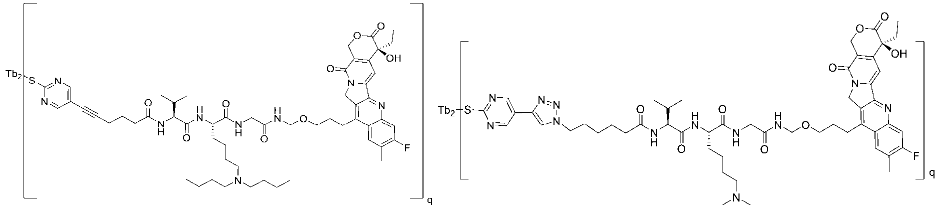 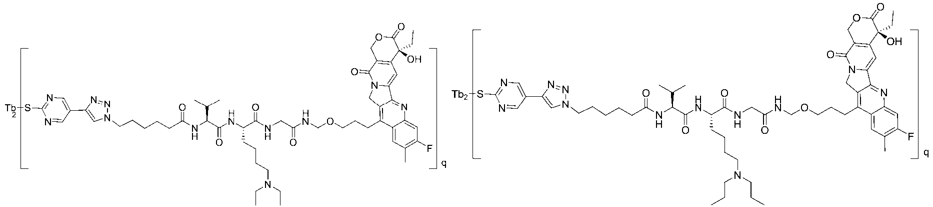 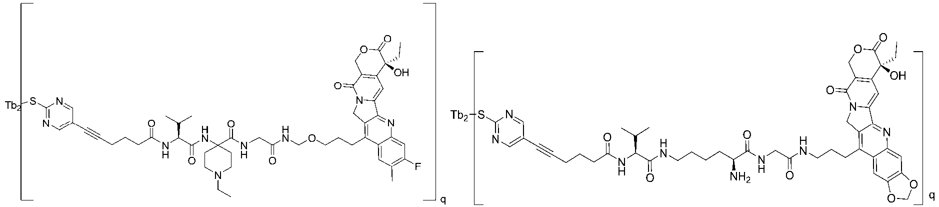 wherein
Tb₂ is an anti-DLL3 antibody or an antigen-binding fragment thereof, for example, rovalpituzumab, tarlatamab, 55C11E4-Hz1, 55C11E4-Hz2, 59B10D3-Hz, 10F2F3-Hz, 87F7F10-Hz, C2, 6F11C10-Hz1, and 6F11C10-Hz2 antibodies.
q is selected from any value between 0.1 and 16.0, preferably any value between 2 and 8. In some embodiments, q is 1, 2, 3, 4, 5, 6, 7, 8, 9, or 10. In some preferred embodiments, q is 2, 4, 6, or 8.

### Preparation of antibody-drug conjugate

In an eighth aspect of the present disclosure, the present disclosure provides a method for preparing the aforementioned antibody-drug conjugate, which comprises:
performing a conjugation reaction between Tb and a drug-linker conjugate of formula III 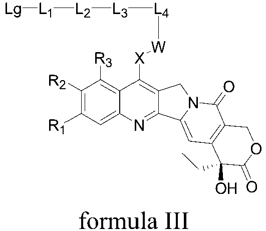
in a suitable solvent and under suitable conditions;
wherein
Tb has the meaning provided above and in any embodiments specifically described herein;
R₁, R₂, R₃, X, W, L₁, L₂, L₃, and L₄ have the meanings provided above and in any embodiments specifically described herein.
Lg is a leaving group, and Lg is selected from halogen, sulfonyl, a tertiary amine group (Me₃N⁺, Et₃N⁺), a diazonium group, -OMs, MeSO₂-, and CF₃SO₃-.

In some embodiments, Lg is selected from F, Cl, and MeSO₂-.

In some embodiments, Lg is selected from F and MeSO₂-.

In some embodiments, the method comprises a step of performing a conjugation reaction between Tb and the drug-linker conjugate of formula III 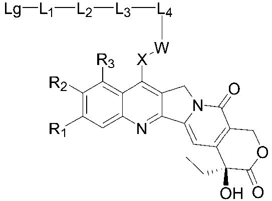 in a suitable solvent and under suitable conditions to form a C-S bond.

In some embodiments, the molar ratio of the Tb to the drug-linker conjugate is 1:(1-20), for example, 1:2, 1:3, 1:4, 1:5, 1:6, 1:7, 1:8, 1:9, 1:10, 1:12, 1:14, 1:16, 1:18, 1:(10-20), 1:(12-20), 1:(14-20), 1:(16-20), or 1:(18-20).

In some embodiments, the conjugation reaction is carried out in water and/or an organic solvent.

In some embodiments, the organic solvent is selected from *N,N-*dimethylformamide, dimethyl sulfoxide, *N*-methylpyrrolidone, nitriles (for example, acetonitrile), alcohols (for example, methanol or ethanol), or any combination thereof.

In some embodiments, the method further comprises a step of purifying the conjugate product.

In some embodiments, the conjugate product is purified by a chromatography method.

In some embodiments, the chromatography method comprises one or more of ion exchange chromatography, hydrophobic chromatography, reversed-phase chromatography, or affinity chromatography.

In a ninth aspect of the present disclosure, the present disclosure provides a group of antibody-drug conjugates, comprising the aforementioned antibody-drug conjugate of the present disclosure, a stereoisomer thereof, a prodrug thereof, a pharmaceutically acceptable salt thereof, or a pharmaceutically acceptable solvate thereof, wherein the antibody-drug conjugates have one, two, or more q values.

In some embodiments, when the antibody-drug conjugates in the group have one q value, the q value is equal to the DAR value.

In some embodiments, when the antibody-drug conjugates in the group have two or more q values, the proportion of the antibody-drug conjugate with a specific q value among all the antibody-drug conjugates in the group is greater than 50%, 60%, 70%, 80%, 90%, 95%, 96%, 97%, 98%, or 99%.

In some embodiments, the drug-to-antibody ratio (DAR) of the antibody-drug conjugates in the group is selected from an integer or decimal from 1 to 10.

In some embodiments, the drug-to-antibody ratio (DAR) of the group is selected from 1.5 to 2.5, 3.5 to 4.5, 5.5 to 6.5, and 7.5 to 8.5;

In some embodiments, the drug-to-antibody ratio (DAR) of the group is selected from about 2.0, 4.0, 6.0, and 8.0;

In some embodiments, the drug-to-antibody ratio (DAR) of the antibody-drug conjugates in the group is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, and 9.

In some embodiments, the group comprises an ADC with a distribution of DARs from 1 to 8, such as 1.5, 2, 4, 6, and 8 (*i.e.,* 1.5, 2, 4, 6, and 8 payload species). It should be noted that degradation products can be generated, such that the group may also comprise a DAR of 1, 3, 5, and 7. Furthermore, the group may also have a DAR greater than 8. The antibody-drug conjugate is produced by reduction of interchain disulfide followed by conjugation. In some embodiments, the antibody-drug conjugate comprises both of the following: an antibody-drug conjugate having a DAR of 4 or less (*i.e.,* the payload species are 4 or less) and an antibody-drug conjugate having a DAR of 6 or more (*i.e.,* the payload species are 6 or more).

In a tenth aspect of the present disclosure, the present disclosure provides a pharmaceutical composition comprising the antibody-drug conjugate in the aforementioned first or seventh aspect, a stereoisomer thereof, a prodrug thereof, a pharmaceutically acceptable salt thereof, or a pharmaceutically acceptable solvate thereof, the anti-DLL3 antibody or the antigen-binding fragment thereof in the second aspect, the nucleic acid described in the third aspect, the vector described in the fourth aspect, the host cell described in the fifth aspect, or the group of antibody-drug conjugates in the ninth aspect, and optionally one or more pharmaceutical excipients.

In certain embodiments, the pharmaceutical composition comprises the antibody-drug conjugate in the aforementioned first or seventh aspect, a stereoisomer thereof, a prodrug thereof, a pharmaceutically acceptable salt thereof, or a pharmaceutically acceptable solvate thereof, the anti-DLL3 antibody or the antigen-binding fragment thereof in the second aspect, the nucleic acid described in the third aspect, the vector described in the fourth aspect, the host cell described in the fifth aspect, or the group of antibody-drug conjugates in the ninth aspect, the doses of all of which are in therapeutically effective amounts.

In certain embodiments, the pharmaceutical composition comprises the antibody-drug conjugate in the aforementioned first or seventh aspect, a stereoisomer thereof, a prodrug thereof, a pharmaceutically acceptable salt thereof, or a pharmaceutically acceptable solvate thereof, and a pharmaceutically acceptable carrier and/or excipient.

In certain embodiments, the pharmaceutical composition of the present disclosure comprises the group of antibody-drug conjugates in the ninth aspect, and a pharmaceutically acceptable carrier and/or excipient. In certain preferred embodiments, the pharmaceutical composition of the present disclosure comprises the above-mentioned group of antibody-drug conjugates, or comprises the aforementioned group of antibody-drug conjugates and a buffer. In certain further preferred embodiments, the pharmaceutical composition of the present disclosure further comprises an excipient and/or a surfactant.

In certain embodiments, the pharmaceutical composition of the present disclosure comprises the anti-DLL3 antibody or the antigen-binding fragment thereof of the present disclosure, and a pharmaceutically acceptable carrier and/or excipient. In certain preferred embodiments, the pharmaceutical composition of the present disclosure comprises an anti-DLL3 antibody or an antigen-binding fragment thereof, and a buffer. In certain further preferred embodiments, the pharmaceutical composition of the present disclosure further comprises an excipient and/or a surfactant.

In some embodiments, the buffer is a histidine buffer. In certain preferred embodiments, the buffer is a 20 mM histidine buffer with a pH of 6.0.

In certain embodiments, the pharmaceutical composition of the present disclosure comprises the host cell of the present disclosure, and a pharmaceutically acceptable carrier and/or excipient, wherein the host cell comprises the isolated nucleic acid molecule or vector as described above.

In certain embodiments, the pharmaceutical composition of the present disclosure comprises the multi-specific antibody of the present disclosure, and a pharmaceutically acceptable carrier and/or excipient.

In some embodiments, the drug-to-antibody ratio (DAR) in the pharmaceutical composition is selected from an integer or decimal from 1 to 10.

In some embodiments, the drug-to-antibody ratio (DAR) in the pharmaceutical composition is selected from 1.5 to 2.5, 3.5 to 4.5, 5.5 to 6.5, and 7.5 to 8.5;

In some embodiments, the drug-to-antibody ratio (DAR) in the pharmaceutical composition is selected from about 2.0, 4.0, 6.0, and 8.0;

In some embodiments, the drug-to-antibody ratio (DAR) of the antibody-drug conjugate in the pharmaceutical composition is selected from 2, 2.5, 3, 3.5, 4, 4.5, 5, 5.5, 6, 6.5, 7, 7.2, 7.4, 7.5, 7.6, 7.7, 7.8, 7.9, 8.0, 8.1, 8.2, 8.3, 8.4, 8.5, 8.7, 8.9, and 9.

In an eleventh aspect, there is provided a use of the antibody or the antigen-binding fragment thereof of the present disclosure in the preparation of a kit for detecting the presence or level of DLL3 in a sample. In another aspect, the present disclosure provides a diagnostic or therapeutic kit comprising one or more of the following: the antibody or the antigen-binding fragment thereof, the nucleic acid, the vector, the host cell, the multi-specific antibody, the antibody-drug conjugate, the group of antibody-drug conjugates, or the pharmaceutical composition described in the present disclosure. Optionally, the diagnostic or therapeutic kit further comprises instructions for use.

In a twelfth aspect of the present disclosure, the present disclosure provides a use of the aforementioned antibody-drug conjugate composition or the aforementioned pharmaceutical composition in the manufacture of a medicament for the treatment and/or prevention of a disease (*e.g.,* a cancer disease) associated with abnormal cell activity. The antibody-drug conjugate composition or the aforementioned pharmaceutical composition may be in a therapeutically effective amount.

In some embodiments, there is provided a use of the anti-DLL3 or the antigen-binding fragment thereof, the nucleic acid, the vector, the host cell, or the multi-specific antibody of the present disclosure in the manufacture of a medicament, wherein the medicament is used for modulating (inhibiting or blocking) the activity of DLL3.

In some embodiments, there is provided a use of the anti-DLL3 or the antigen-binding fragment thereof, the nucleic acid, the vector, the host cell, or the multi-specific antibody of the present disclosure in the manufacture of a medicament, wherein the medicament is used for the treatment or prevention of a disease associated with the activity of DLL3.

In some embodiments, there is provided a use of the anti-DLL3 or the antigen-binding fragment thereof, the nucleic acid, the vector, the host cell, the antibody-drug conjugate, or the multi-specific antibody of the present disclosure in the manufacture of a medicament, wherein the medicament is used for the treatment or prevention of tumors associated with the activity of DLL3.

In some embodiments, there is provided a use of the antibody-drug conjugate in the aforementioned first or seventh aspect of the present disclosure, a stereoisomer thereof, a prodrug thereof, a pharmaceutically acceptable salt thereof, or a pharmaceutically acceptable solvate thereof, the anti-DLL3 antibody or the antigen-binding fragment thereof in the second aspect, the nucleic acid described in the third aspect, the vector described in the fourth aspect, the host cell described in the fifth aspect, the group of antibody-drug conjugates in the ninth aspect, or the pharmaceutical composition in the tenth aspect in the manufacture of a medicament, wherein the medicament is used for the treatment or prevention of a tumor.

In some embodiments, there is provided a use of the antibody-drug conjugate in the aforementioned first or seventh aspect of the present disclosure, a stereoisomer thereof, a prodrug thereof, a pharmaceutically acceptable salt thereof, or a pharmaceutically acceptable solvate thereof, the group of antibody-drug conjugates in the ninth aspect, or the pharmaceutical composition in the tenth aspect in the manufacture of a medicament, wherein the medicament is used for the treatment or prevention of a tumor related to a target of Tb.

In some embodiments, there is provided a use of the antibody-drug conjugate in the aforementioned first or seventh aspect of the present disclosure, a stereoisomer thereof, a prodrug thereof, a pharmaceutically acceptable salt thereof, or a pharmaceutically acceptable solvate thereof, the group of antibody-drug conjugates in the ninth aspect, or the pharmaceutical composition in the tenth aspect in the manufacture of a medicament, wherein the medicament is used for the treatment or prevention of a tumor related to DLL3.

In some preferred embodiments, there is provided a use of the antibody-drug conjugate in the aforementioned first or seventh aspect of the present disclosure, a stereoisomer thereof, a prodrug thereof, a pharmaceutically acceptable salt thereof, or a pharmaceutically acceptable solvate thereof, the anti-DLL3 antibody or the antigen-binding fragment thereof in the second aspect, the nucleic acid described in the third aspect, the vector described in the fourth aspect, the host cell described in the fifth aspect, the group of antibody-drug conjugates in the ninth aspect, or the pharmaceutical composition in the tenth aspect in the manufacture of a medicament, wherein the medicament is used for the treatment or prevention of a tumor related to DLL3

In a thirteenth aspect of the present disclosure, the present disclosure provides a method for using the antibody-drug conjugate in the aforementioned first or seventh aspect, a stereoisomer thereof, a prodrug thereof, a pharmaceutically acceptable salt thereof, or a pharmaceutically acceptable solvate thereof, the anti-DLL3 antibody or the antigen-binding fragment thereof in the second aspect, the nucleic acid described in the third aspect, the vector described in the fourth aspect, the host cell described in the fifth aspect, the group of antibody-drug conjugates in the ninth aspect, or the pharmaceutical composition in the tenth aspect for the treatment and/or prevention of a disease (*e.g.,* a tumor) associated with abnormal cell activity.

In the above twelfth and thirteenth aspects, the tumor is selected from esophageal cancer (*e.g.,* esophageal adenocarcinoma and esophageal squamous cell carcinoma), brain tumor, lung cancer (*e.g.,* small cell lung cancer, non-small cell lung cancer, or lung adenocarcinoma), squamous cell carcinoma, bladder cancer, stomach cancer, ovarian cancer, peritoneal cancer, pancreatic cancer, breast cancer, head and neck cancer, cervical cancer, endometrial cancer, colon cancer (*e.g.,* human colon adenocarcinoma), rectal cancer, colorectal cancer, liver cancer, kidney cancer, urothelial cancer, epidermal cancer, non-Hodgkin lymphoma, central nervous system tumor (*e.g.,* neuroglioma, glioblastoma multiforme, glioma, or sarcoma), prostate cancer, or thyroid cancer.

In some embodiments, the tumor is a DLL3-related tumor.

In some embodiments, the tumor is a DLL3-related tumor.

In the present disclosure, unless otherwise stated, the scientific and technical terms used herein have the meanings that are generally understood by those skilled in the art. Moreover, the cell culture, molecular genetics, nucleic acid chemistry, and immunology laboratory procedures used herein are all routine procedures widely used in the corresponding fields. Meanwhile, for a better understanding of the present disclosure, definitions and explanations of relevant terms are provided below.

As used herein, examples of the term "pharmaceutically acceptable salt" are organic acid addition salts formed from organic acids that form pharmaceutically acceptable anions, including, but not limited to, formate, acetate, propionate, benzoate, maleate, fumarate, succinate, tartrate, citrate, ascorbate, α-ketoglutarate, α-glycerophosphate, alkylsulfonate, or arylsulfonate; preferably, the alkylsulfonate is methylsulfonate or ethylsulfonate; the arylsulfonate is benzenesulfonate or *p*-toluenesulfonate. Suitable inorganic salts may also be formed, including, but not limited to, hydrochloride, hydrobromide, hydroiodate, nitrate, bicarbonate, carbonate, sulfate, phosphate, *etc.*

As used herein, the term "pharmaceutically acceptable carrier and/or excipient" refers to a carrier and/or excipient that is pharmacologically and/or physiologically compatible with a subject and the active ingredient, which is well-known in the art (see, for example, Remington's Pharmaceutical Sciences, Edited by Gennaro AR, 19th ed. Pennsylvania: Mack Publishing Company, 1995), and includes but is not limited to: a pH adjuster, a surfactant, an adjuvant, an ionic strength enhancer, a diluent, an osmolality-maintaining agent, a delayed absorption agent, and a preservative.

Pharmaceutically acceptable salts can be obtained using standard procedures well known in the art, for example, by reacting a sufficient amount of a basic compound with a suitable acid providing a pharmaceutically acceptable anion.

In the present disclosure, the pharmaceutically acceptable adjuvant refers to the excipient and additive used in the manufacture of medicaments and the formulation of prescriptions, and refers to the substances contained in a pharmaceutical preparation whose safety has been reasonably evaluated, besides the active ingredients. In addition to shaping, acting as a carrier, and improving stability, the pharmaceutically acceptable adjuvant also has important functions such as solubilization, hydrotropy, sustained and controlled release, and is an important ingredient that may affect the quality, safety, and efficacy of drugs. According to its source, the pharmaceutically acceptable adjuvant can be divided into natural product, semi-synthetic product, and full synthetic product. According to its function and use, the pharmaceutically acceptable adjuvant can be divided into: solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adherents, antioxidants, chelating agents, penetration enhancers, pH adjusters, buffers, plasticizers, surfactants, foaming agents, defoaming agents, thickeners, inclusion agents, humectants, absorbents, diluents, flocculants and deflocculants, filter aids, release blockers, *etc.* According to its route of administration, the pharmaceutically acceptable adjuvant can be divided into oral administration, injection, mucosal administration, transdermal or topical administration, nasal or oral inhalation administration, ocular administration, *etc.* The same pharmaceutically acceptable adjuvant can be used in pharmaceutical preparations with different routes of administration, and has different effects and uses.

The pharmaceutical composition can be prepared into various suitable dosage forms according to the route of administration. For example, tablets, capsules, granules, oral solutions, oral suspensions, oral emulsions, powders, tinctures, syrups, injections, suppositories, ointments, creams, pastes, ophthalmic preparations, pills, implants, aerosols, powder aerosols, sprays, *etc.* Herein, the pharmaceutical composition or suitable dosage form may contain 0.01 mg to 1000 mg of the compound of the present disclosure or a pharmaceutically acceptable salt or conjugate thereof, suitably 0.1 mg to 800 mg, preferably 0.5 mg to 500 mg, preferably 0.5 mg to 350 mg, particularly preferably 1 mg to 250 mg.

The pharmaceutical composition can be administered in the form of injection, including injection solution, sterile powder for injection, and concentrated solution for injection. Herein, useful carriers and solvents include water, Ringer's solution, and isotonic sodium chloride solution. In addition, sterile non-volatile oils can also be used as a solvent or suspending medium, such as monoglycerides or diglycerides.

The term "treatment" as used herein generally refers to obtaining a desired pharmacological and/or physiological effect. The effect may be prophylactic in terms of complete or partial prevention of a disease or its symptoms; and/or therapeutic in terms of partial or complete stabilization or cure of a disease and/or side effects due to the disease. "Treatment" as used herein encompasses any treatment of a disease in a patient, including: (a) prevention of a disease or symptom in a patient who is susceptible to the disease or symptom but has not yet been diagnosed with the disease; (b) suppression of symptoms of the disease, *i.e.,* preventing its development; or (c) alleviation of symptoms of the disease, *i.e.,* causing regression of the disease or symptom.

In the present disclosure, the term "individual" includes humans or non-human animals. Exemplary human individuals include human individuals (referred to as patients) with a disease (*e.g.,* a disease described herein) or normal individuals. In the present disclosure, the term "non-human animal" includes all vertebrates, such as non-mammals (*e.g.,* birds, amphibians, reptiles) and mammals, such as non-human primates, livestocks, and/or domesticated animals (*e.g.,* sheep, dogs, cats, cows, pigs, *etc*.)*.*

In the present disclosure, the term "effective dose" refers to the amount of a compound that, when administered, alleviates one or more symptoms of the condition being treated to some extent.

The term "bioactive substance," "bioactive molecule," or "drug molecule" in the present disclosure refers to a substance that inhibits or prevents cell function and/or causes cell death or destruction. In some embodiments of the present disclosure, the bioactive substance, bioactive molecule, or drug molecule in the conjugates is a molecule with anti-tumor bioactivity. For example: radioactive isotopes, such as radioactive isotopes of At²¹¹, I¹³¹, I¹²⁵, Y⁹⁰, Re¹⁸⁶, Re¹⁸⁸, Sm¹⁵³, Bi²¹², P³², Pb²¹², and Lu; metal complexes, such as metal platinum complexes, metal gold complexes, and oxaliplatin; glycopeptide antibiotics, such as bleomycin and pingyangmycin; DNA topoisomerase inhibitors, such as topoisomerase I inhibitors, *e.g.,* camptothecin, hydroxycamptothecin, 9-aminocamptothecin, SN-38, irinotecan, topotecan, belotecan, rubitecan, as well as topoisomerase II inhibitor, *e.g.,* actinomycin D, adriamycin, doxorubicin, duocarmycin, daunorubicin, mitoxantrone, podophyllotoxin, and etoposide; drugs that interfere with DNA synthesis, such as methotrexate, 5-fluorouracil, cytarabine, gemcitabine, mercaptopurine, pentostatin, fludarabine, cladribine, and nelarabine; drugs that act on structural proteins, such as tubulin inhibitors, *e.g.,* vinca alkaloids, vincristine, vinblastine, paclitaxel, docetaxel, and cabazitaxel; tumor signaling pathway inhibitors, such as serine/threonine kinase inhibitors, tyrosine kinase inhibitors, aspartokinase inhibitors, or histidine kinase inhibitors; also included are proteasome inhibitors, histone deacetylase inhibitors, tumor angiogenesis inhibitors, cyclin inhibitors, maytansine derivatives, calicheamicin derivatives, auristatin derivatives, pyrrolobenzodiazepines (PBD) derivatives, melphalan, mitomycin C, chlorambucil, or other active substances that inhibit tumor cell growth and promote tumor cell apoptosis and necrosis; enzymes and fragments thereof, such as nucleases; antibiotics; payloads, such as small molecule payloads or enzymatically active payloads of bacterial, fungal, plant, or animal origin, including fragments and/or variants thereof; growth inhibitors; pharmaceutical modules. The term "payload" refers to a substance capable of producing a deleterious effect on the growth or proliferation of cells.

In the present disclosure, the term "linker" refers to a fragment that links a bioactive molecule (drug molecule) to a targeting moiety.

In the present disclosure, the term "targeting moiety" refers to a moiety in a conjugate that is capable of specifically binding to a target (or portion of a target) on the cell surface. Through the interaction of the targeting moiety with the target, the conjugate can be delivered to a specific cell population.

In the present disclosure, an antibody or antigen-binding fragment thereof includes a derivatized antibody or antigen-binding fragment thereof, such as an antibody or antigen-binding fragment thereof having a sulfhydryl group, wherein the derivatization allows the antibody to have a group or ability to react with a drug-linker conjugate. The sulfhydryl group -SH can be derivatized by breaking the disulfide bond (*e.g.,* by reduction with the reducing agent TCEP).

The terms "cancer" and "tumor" are used herein with the same meaning.

The term "gene" as used herein includes not only DNA but also its mRNA, its cDNA, and its cRNA.

The term "polynucleotide" is used herein in the same meaning as nucleic acid and also includes DNA, RNA, probes, oligonucleotides, and primers.

The terms "polypeptide" and "protein" are used herein without distinction.

The term "cell" as used herein also includes cells within an individual animal and cells in culture.

The DLL3 involved in the anti-DLL3 antibodies described in the present disclosure can be a conventional DLL3 in the art, and also represents a DLL3 variant.

The term "DLL3" refers to a highly tumor-selective cell surface target mainly expressed in neural or neuroendocrine tumors, including small cell lung cancer (SCLC), large cell neuroendocrine carcinoma (LCNEC), gastrointestinal neuroendocrine tumors (GI-NEC), small cell bladder cancer (SCBC), glioblastoma multiforme, metastatic castration-resistant prostate cancer, pulmonary neuroendocrine tumors, etc. Especially for SCLC, over 80% of SCLCs show positive DLL3 expression, while it is almost absent in normal lung cancer tissue and paraneoplastic tissues. DLL3 is a type I transmembrane protein composed of 618 amino acids, with residues 27 to 492 constituting the extracellular domain, which is the target region for ADC drug targeting. The extracellular domain consists of an N-terminal DSL domain followed by six tandem EGF-like domains.

KD refers to the dissociation constant obtained from the ratio of Kd (the dissociation rate of the specific binding molecule-target protein interaction) to Ka (the association rate of the specific binding molecule-target protein interaction) (or Kd/Ka, expressed in molar concentration (M)). The KD value can be measured using methods well established in the art. A preferred method for determining the KD of the binding molecule is through the use of surface plasmon resonance, such as a biosensor system, e.g., Biacore^{™} (GE Healthcare Life Sciences) system.

As used herein, the percentage homology between two amino acid sequences is equal to the percentage identity between the two sequences. The sequence percentage identity between two sequences is a function of the number of positions shared by the sequences *(i.e.,* % homology = number of identical positions / total number of positions × 100), where the number of gaps and the length of each gap are taken into account to achieve the optimal comparison for the two sequences. Sequence comparison and determination of the percentage identity can be performed using methods commonly known in the art, and mathematical algorithms can be employed to carry out such sequence comparisons and the determination of percentage identity. For example, the algorithm described by Meyers and Miller, 1988 in Comput. Appl. Biosci. 4: 11-17 (which is incorporated into the ALIGN program, version 2.0) can be used to determine the percentage identity between amino acid sequences and/or nucleotide sequences. Additionally, the GAP program from the GCG software package available online from Accelrys (using its default parameters) can be used to determine the percentage identity between amino acid sequences or nucleotide sequences. In an embodiment, the two sequences are of equal length.

The term "epitope" refers to a portion of an antigenic polypeptide or protein that has antigenic or immunogenic activity in an animal *in vivo,* preferably a mammal. The epitopes of the antibodies or antigen-binding fragments thereof of the present disclosure can be determined using existing techniques, such as synthetic peptide methods, immunoinformatics prediction, determination of polypeptide activity, epitope peptide scanning, phage display technology, X-ray diffraction and nuclear magnetic resonance analysis, and antibody homology modeling and protein docking prediction methods. The phrase "antibodies that bind to the same epitope" as used herein indicates different antibodies that bind to a common epitope. If a second antibody binds to part of the peptide or part of the tertiary structure that the first antibody binds to, it can be determined that the first antibody and the second antibody bind to the same epitope.

In the present disclosure, the term "antibody" is used in its broadest interpretation to include intact monoclonal antibodies, polyclonal antibodies, and multi-specific antibodies (e.g., bispecific antibodies) formed from at least two intact antibodies, so long as they have the desired biological activity. In the present disclosure, "antibody" and "immunoglobulin" are used interchangeably. "Antibody molecule" or "antibody" as used herein refers to immunoglobulin molecules and immunologically active portions of immunoglobulin molecules, *i.e.,* molecules containing an antigen-binding site that immune-specifically binds to an antigen. Thus, the term antibody broadly encompasses not only an intact antibody molecule, but also fragments of the antibody as well as variants (including derivatives) of the antibody and antibody fragments. When "antibody molecule" or "antibody" is used in the same context as antigen-binding fragment, "antibody molecule" or "antibody" refers to an intact antibody molecule or a full-length antibody. The term antibody molecule as described in the present specification includes, for example, but is not limited to, single-chain Fv (scFv), Fab fragments, Fab' fragments, F(ab')2, disulfide-linked Fv (sdFv), Fv, and intact antibodies or full-length antibodies. The term "single-chain Fv" or "scFv" refers to a polypeptide comprising the VL domain of an antibody linked to the VH domain of the antibody. For example, antibodies that immune-specifically bind to DLL3 can cross-react with other antigens. Preferably, antibodies that immune-specifically bind to DLL3 do not cross-react with other antigens. Immunospecific binding can be identified, for example, by immunoassays or other methods known to those skilled in the art. "Intact" antibody or "full-length" antibody refers to a protein comprising two heavy chains (H) and two light chains (L) linked to each other by disulfide bonds, and the protein comprises: (1) for the heavy chain, a heavy chain variable region (abbreviated herein as "VH") and a heavy chain constant region containing three domains CH1, CH 2, CH3; and (2) for the light chain, a light chain variable region (abbreviated herein as "VL") and a light chain constant region containing one domain CL. The antibodies of the present disclosure include, but are not limited to, monoclonal, multi-specific, humanized or chimeric antibodies, single-chain antibodies, Fab fragments, F(ab') fragments, anti-idiotypic (anti-Id) antibodies (including, for example, anti-Id antibodies of antibodies of the present disclosure), and epitope-binding fragments of any of the above antibodies. The immunoglobulin molecules of the present disclosure may be of any type (*e.g.,* IgG, IgE, IgM, IgD, IgA, and IgY), any class (*e.g.,* IgG1, IgG2, IgG3, IgG4, IgA1, and IgA2), or any subclass of immunoglobulin. Preferably, the antibodies of the present disclosure comprise or consist of a VH domain, a VH CDR (often represented herein by HCDR), a VL domain, or a VL CDR (often represented herein by LCDR) having any one of the amino acid sequences or fragments or variants thereof described in the table of sequence and specific information thereof.

In the present disclosure, the term "monoclonal antibody" refers to an antibody derived from a population of substantially homogeneous antibodies, *i.e.,* the antibodies making up the population are identical except for a small number of natural mutations that may be present. Monoclonal antibodies have high specificity against one determinant (epitope) of an antigen, while polyclonal antibodies comprise different antibodies against different determinants (epitopes). In addition to specificity, the advantage of monoclonal antibodies is that they can be synthesized without contamination from other antibodies. The modifier "monoclonal" as used herein means that the antibody is characterized as being derived from a substantially homogeneous population of antibodies and should not be construed as needing to be prepared by a special method.

In some embodiments of the present disclosure, monoclonal antibodies further include, in particular, chimeric antibodies, *i.e.,* a portion of the heavy and/or light chain is identical or homologous to a type, a class, or a subclass of antibodies, and the remainder is identical or homologous to another type, another class, or another subclass of antibodies, so long as they have the desired biological activity (see, for example, US 4,816,567; and Morrison et al., 1984, PNAS, 81: 6851-6855). Chimeric antibodies that can be used in the present disclosure include primatized antibodies comprising variable region antigen-binding sequences and human constant region sequences from non-human primates (*e.g.,* ancient monkeys, chimpanzees, *etc*.)*.*

The term "antigen-binding fragment" refers to a portion of an antibody, preferably an antigen-binding or variable region. Examples of antibody fragments include Fab, Fab', F(ab')₂, Fd, Fv, dAb, complementarity determining region fragments, diabodies, linear antibodies, and single-chain antibody molecules. The term "antigen-binding fragment" as used herein refers to a partial fragment of an antibody that has antigen-binding activity, wherein the fragment has all or part of the functions of the antibody, including but not limited to single-chain Fv (scFv), Fab, Fab', F(ab')₂, disulfide-linked Fv (sdFv), Fv, di-scFv, *etc.* The term also includes Fab', which is a monovalent fragment of the variable region of an antibody resulting from treatment of F(ab')₂ under reducing conditions. However, the term is not limited to these molecules as long as the fragment has binding affinity for the antigen. Furthermore, these functional fragments include not only fragments obtained by treating the full-length molecule of the antibody protein with appropriate enzymes, but also proteins produced in appropriate host cells using genetically modified antibody genes.

The term "Fab'" as used herein refers to a monovalent fragment of the variable region of an antibody obtained by treating F(ab')₂ under reducing conditions as described above. However, Fab' of the present disclosure also includes Fab' produced using genetically modified antibody genes.

As used herein, the term "scFv" refers to a single polypeptide chain comprising VL and VH domains, wherein the VL and VH are linked by a linker or directly (see, *e.g.,* Bird et al., Science 242: 423-426 (1988); Huston et al., Proc. Natl. Acad. Sci. USA 85: 5879-5883 (1988); and Pluckthun, The Pharmacology of Monoclonal Antibodies, Volume 113, Roseburg and Moore, eds., Springer-Verlag, New York, pp. 269-315 (1994)). Such scFv molecules may have the general structure: NH₂-VL-linker-VH-COOH or NH₂-VH-linker-VL-COOH. Suitable linkers in the prior art consist of repeated GGGGS amino acid sequences or variants thereof. For example, a linker with the amino acid sequence (GGGGS)4 can be used, but its variants can also be used (Holliger et al. (1993), Proc. Natl. Acad. Sci. USA 90: 6444-6448). Other linkers applicable to the present disclosure are described by Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56; and Roovers et al. (2001), Cancer Immunol. In some cases, a disulfide bond may also exist between the VH and VL of the scFv. As used herein, the term "di-scFv" refers to an antibody fragment formed by the linkage of two scFvs.

As used herein, the antibody or antigen-binding fragment thereof contains variants, amino acid substitutions, deletions, or additions that still have the activity to bind the antigen.

The term "bispecific antibody", also known as "bifunctional antibody conjugate", refers to a conjugate formed by a first antibody (fragment) and a second antibody (fragment) through a coupling arm. The conjugate retains the activity of each antibody and thus has bifunctional and bispecific properties.

The term "multi-specific antibody" includes, for example, a trispecific antibody which is an antibody with three different antigen binding specificities, and a tetraspecific antibody which is an antibody with four different antigen binding specificities.

The term "intact antibody" or "full-length antibody" refers to an antibody comprising an antigen-binding variable region, a light chain constant region (CL), and a heavy chain constant region (CH1, CH2, and CH3). The constant region may be a natural sequence (*e.g.,* a human natural constant region sequence) or an amino acid sequence variant thereof. The intact antibody is preferably an intact antibody with one or more effector functions.

The term "probody" is a modified antibody comprising an antibody or antibody fragment that can specifically bind to a target thereof and can be coupled with a masking moiety, wherein the masking moiety means that the cleavage constant for the binding capacity of the antibody or antibody fragment to the target thereof is at least 100-fold, 1000-fold, or 10,000-fold greater than that for the binding capacity of the antibody or antibody fragment not coupled with a masking moiety to the target thereof.

In the present disclosure, a "humanized" form of a non-human (*e.g.,* mouse) antibody refers to a chimeric antibody that contains minimal non-human immunoglobulin sequences. Most humanized antibodies are human recipient immunoglobulins whose hypervariable region residues have been replaced with non-human (*e.g.,* mouse, rat, rabbit, or non-human primate) hypervariable region residues (donor antibodies) with the desired specificity, affinity, and functions. In some embodiments, framework region (FR) residues of human immunoglobulins are also replaced with non-human residues. Furthermore, the humanized antibody may further comprise residues not present in the recipient antibody or donor antibody. These modifications are intended to further optimize the performance of the antibody. The humanized antibody generally comprises at least one variable region, typically two variable regions, in which all or almost all of the hypervariable loops correspond to those of non-human immunoglobulins, while the FRs are full or nearly full sequences of human immunoglobulins. The humanized antibody may further comprise at least a portion of an immunoglobulin constant region (Fc, typically a human immunoglobulin Fc). For details see, for example, Jones et al., 1986, Nature, 321: 522-525; Riechmann et al., 1988, Nature, 332: 323-329; and Presta, 1992, Curr Op Struct Bwl 2: 593-596.

Intact antibodies can be divided into different "classes" based on the amino acid sequence of the heavy chain constant region. The five main classes are IgA, IgD, IgE, IgG, and IgM, several of which can also be divided into different "subclasses" (isotypes), such as IgG1, IgG2, IgG3, IgG4, IgA1, and IgA2. The heavy chain constant regions of different classes of antibodies are called α, β, ε, γ, and µ, respectively. The subunit structures and three-dimensional configurations of different classes of immunoglobulins are well known in the art.

As used herein, the CDRs contained in the antibodies or antigen-binding fragments thereof of the present disclosure can be determined according to various numbering systems known in the art. In certain embodiments, the CDRs contained in the antibodies or antigen-binding fragments thereof of the present disclosure are preferably determined using the Kabat, Chothia, AbM, or IMGT numbering systems.

As used herein, the term "framework residue region" or "FR residue" refers to those amino acid residues in an antibody variable region other than the CDR residues as defined above.

As used herein, the term "germline antibody gene" refers to an immunoglobulin-encoding gene expressed in non-lymphoid cells that has not undergone the maturation process leading to genetic rearrangement and maturation that results in the expression of a specific immunoglobulin. An advantage provided by various embodiments of the present disclosure derives from the recognition that the amino acid sequences encoded by germline antibody genes retain more of characteristic important amino acid sequence structures of individuals of an animal species than the amino acid sequences encoded by mature antibody genes. Therefore, when therapeutically applied to the species, the amino acid sequences encoded by germline antibody genes are less likely to be recognized as foreign substances by that species.

As amino acid substitutions, conservative amino acid substitutions are preferred in this specification. A conservative amino acid substitution refers to a substitution that occurs within a set of amino acids related to the side chains of the amino acids. The preferred amino acid sets are as follows: an acidic set (aspartic acid and glutamic acid); a basic set (lysine, arginine, and histidine); a non-polar set (alanine, valine, leucine, isoleucine, proline, phenylalanine, methionine, and tryptophan); and an uncharged polar family (glycine, asparagine, glutamine, cysteine, serine, threonine, and tyrosine). More preferred amino acid sets are as follows: an aliphatic hydroxyl group (serine and threonine); an amide-containing set (asparagine and glutamine); an aliphatic set (alanine, valine, leucine, and isoleucine); and an aromatic set (phenylalanine, tryptophan, and tyrosine). Such amino acid substitutions are preferably made within sets that do not compromise the properties of the substance with the original amino acid sequence.

In addition, it is known that the lysine residue at the carboxyl terminus of the heavy chain of antibodies produced in cultured mammalian cells is deleted (Journal of Chromatography A, 705: 129-134 (1995)). It is also known that two amino acid residues (glycine and lysine) at the carboxyl terminus of the heavy chain of antibodies produced in cultured mammalian cells are deleted, and that a proline residue newly located at the carboxyl terminus is amidated (Analytical Biochemistry, 360: 75-83 (2007)). However, such deletions and modifications of the heavy chain sequence do not affect the antigen-binding affinity and effector functions (activation of complement, antibody-dependent cellular cytotoxicity, *etc*.) of the antibody.

The twenty conventional amino acids involved herein have been written following conventional usage. See, for example, Immunology-A Synthesis (2nd Edition, E. S. Golub and D. R. Gren, Eds., Sinauer Associates, Sunderland, Mass. (1991)), which is incorporated herein by reference. As used herein, the terms "polypeptide" and "protein" have the same meaning and are used interchangeably. Additionally, in the present disclosure, amino acids are typically represented using the one-letter and three-letter abbreviations known in the art. For example, alanine can be represented by A or Ala; arginine can be represented by R or Arg; glycine can be represented by G or Gly; glutamine can be represented by Q or Gln.

As used herein, the term "prevention" refers to a method implemented to prevent or delay the onset of a disease or condition or symptom (*e.g.,* tumors and infectious diseases) in a subject. As used herein, the term "treatment" refers to a method implemented to obtain a beneficial or desired clinical outcome. For the purposes of the present disclosure, beneficial or desired clinical outcomes include, but are not limited to, alleviation of symptoms, reduction of disease extent, stabilization (*i.e*., prevention of further deterioration) of disease status, delaying or slowing of disease progression, improvement or alleviation of disease conditions, and relieving of symptoms (whether partial or complete), whether they are detectable or undetectable. In addition, "treatment" may also refer to prolonging survival compared with expected survival (if left untreated).

As used herein, the term "subject" refers to a mammal, such as a primate mammal, *e.g.,* a non-human primate mammal or a human. In certain embodiments, the subject (*e.g.,* human) has, or is at risk of, a tumor or infectious disease.

As used herein, the term "effective amount" refers to an amount sufficient to achieve, or at least partially achieve, the desired effect. For example, an effective amount to prevent a disease (e.g., tumors and infectious diseases) refers to an amount sufficient to prevent, stop, or delay the occurrence of a disease (*e.g.,* tumors and infectious diseases); an effective amount to treat a disease refers to an amount sufficient to cure or at least partially stop a disease and its complications in a patient who already has the disease. The determination of such an effective amount is well within the competence of those skilled in the art. For example, the amount effective for therapeutic use will depend on the severity of the disease to be treated, the overall status of the patient's immune system, the patient's general condition such as age, weight, and gender, the method of drug administration, and other treatments administered concurrently, *etc.*

As used herein, the term "effector function" refers to those biological activities attributable to the Fc region of an antibody (either a natural sequence Fc region or an amino acid sequence variant Fc region), which vary according to the antibody isotype.

The term "pharmaceutically acceptable" means that a molecule, molecular fragment, or composition that, when appropriately administered to an animal or human, do not produce adverse, allergic, or other harmful reactions. Specific examples of substances that may serve as pharmaceutically acceptable carriers or components thereof include sugars (such as lactose), starch, cellulose and derivatives thereof, vegetable oils, gelatin, polyols (such as propylene glycol), alginates, *etc.*

The term "group of antibody-drug conjugates" refers to a mixture of a set or group of antibody-drug conjugates of the present disclosure, stereoisomers thereof, prodrugs thereof, pharmaceutically acceptable salts thereof, tautomers thereof, or pharmaceutically acceptable solvates thereof, in which the q of the antibody-drug conjugates may be the same or different. In addition, it may also be referred to as an "antibody-drug conjugate mixture".

The *in vivo* therapeutic effect of antibodies and antibody-drug conjugates on cancer can be determined using experimental animals, for example, by administering antibodies to nude mice implanted with tumor cell lines expressing DLL3 and measuring any changes in the cancer cells.

In the present disclosure, amino acid substitutions in the antibody are mostly L-amino acid substitutions, but are not limited thereto. In some embodiments, one or more D-amino acids may be included in the antibody peptide chain. Peptides containing D-amino acids are more stable and less susceptible to degradation in the oral cavity, gut, or plasma than peptides containing only L-amino acids.

The monoclonal antibodies used in the present disclosure can be produced by a number of methods. For example, the monoclonal antibodies for use in the present disclosure can be obtained by hybridoma methods using cells from a number of species (including mouse, hamster, rat, and human) (see, for example, Kohler et al., 1975, Nature, 256: 495), or prepared by recombinant DNA techniques (see, for example, US 4,816,567), or isolated from phage antibody libraries (see, for example, Clackson et al., 1991, Nature, 352: 624-628; and Marks et al., 1991, Journal of Molecular Biology, 222: 581-597).

As used herein, unless otherwise expressly stated, the expressions "each ... independently selected from" and "... each independently selected from" used throughout this specification are interchangeable, and should be understood in a broad sense, which can mean that in different groups, the specific options expressed for the same or different symbols do not affect each other, and which can also mean that in the same group, the specific options expressed for the same or different symbols do not affect each other.

As used herein, the term "direct bond" means that the groups on both sides of the bond are directly connected. For example, if X is a direct bond in the compound of formula II 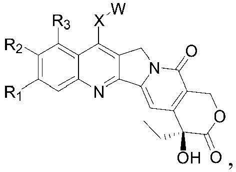 the structural formula is 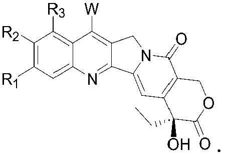 The remaining direct bonds can be understood with reference to the foregoing.

As used herein, the term "absent" means that the group is absent. For example, if W is absent in the compound of formula II 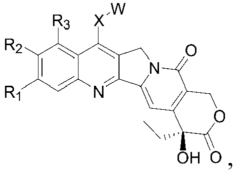 the structural formula is

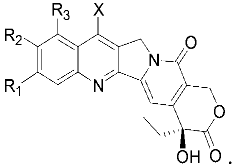

In the compound of formula II 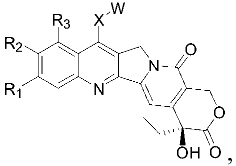 R₁ and R₂, together with the carbon atom to which they are attached, form 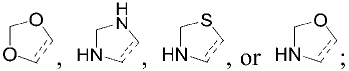 the "dotted line" bond indicates the position where the heterocyclic ring is fused with a benzene ring, for example, forming 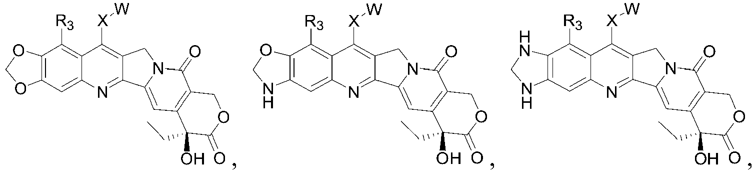 or

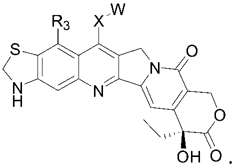

As used herein, in the drug-linker conjugate of formula III 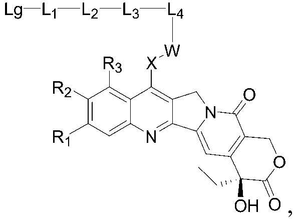 when L₄ is 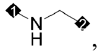 the labels 1 and 2 indicate the connection positions of L₄ to the remaining groups. Specifically, position 1 is attached to L₃, and position 2 is attached to the drug molecule, i.e., 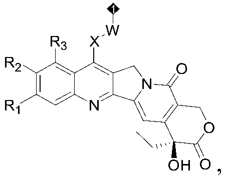 forming 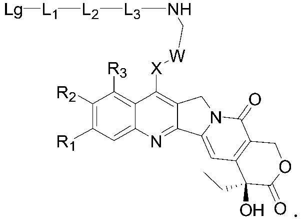 The remaining labels 1 and 2 can be understood with reference to the foregoing.

As used herein, in the compound of formula II 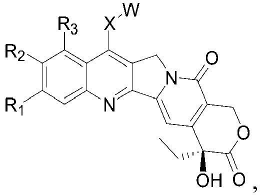 R₃ and X, together with the carbon atom to which they are attached, form 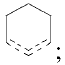 the dotted line indicates the position where the carbocyclic ring is fused with a benzene ring and a pyridine ring, forming

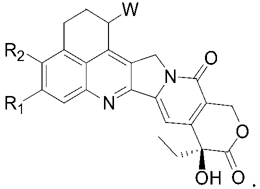

As used herein, the definition of X is, for example, "X is selected from optionally substituted 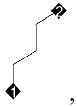 and the substituent is selected from 1 or 2 C1-4 alkyl groups (e.g., methyl)", then X may be, for example, 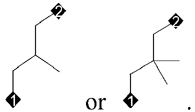 Other similar definitions of X can be understood with reference to the foregoing.

As used herein, the definition of X is, for example, "X is selected from optionally substituted 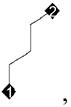 and the substituent is selected from 2 C1-4 alkyl groups (*e.g.,* methyl) that, together with the carbon atom to which they are simultaneously attached, form a C3-6 cycloalkyl group (*e.g.,* cyclopropyl)", then X may, for example, be 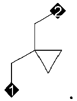 Other similar definitions of X can be understood with reference to the foregoing.

In the structure 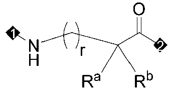 of the amino acid residue represented by AA¹, if r is 0, those skilled in the art can understand that the structure of the amino acid residue represented by AA¹ will change to

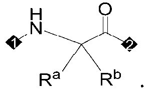

In the structure 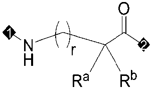 of the amino acid residue represented by AA¹, if R^{a} and R^{b}, together with the carbon atom to which they are both attached, form a 4- to 10-membered heterocyclic ring, the 4- to 10-membered heterocyclic ring is optionally substituted by one or more R⁰, wherein the term "the 4- to 10-membered heterocyclic ring is optionally substituted by one or more R⁰" means that the 4- to 10-membered heterocyclic ring may be either unsubstituted or substituted by one or more R⁰, and in the more R⁰, the definition of each R⁰ may be the same or different. Other similar definitions can be understood with reference to the foregoing.

As used herein, for example, when L₃ is selected from Lys, Val-Cit, Ala-Ala-Asn, Ala-Ala-Asp, Gly-Gly-Phe-Gly, Val-Lys-Gly, Val-Ala, and Lys-Ala-Asn, "the distal amino group of the lysine (Lys) is optionally substituted by 1, 2, or 3 substituents selected from *tert-*butoxycarbonyl, C1-6 alkyl (preferably methyl), and O" means that the distal amino group of Lys in each option of L₃ is optionally substituted by 1, 2, or 3 substituents selected from *tert-*butoxycarbonyl, C1-6 alkyl (preferably methyl), and O. Here, "the distal amino group of Lys" refers to the exposed amino group -NH₂ in the lysine residue 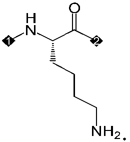 "The distal amino group of the lysine (Lys) is optionally substituted by 1, 2, or 3 substituents selected from *tert-*butoxycarbonyl, C1-6 alkyl (preferably methyl), and O" indicates that the distal amino group of lysine (Lys) may not be substituted, or may be substituted by 1, 2, or 3 substituents selected from *tert*-butoxycarbonyl, C1-6 alkyl (preferably methyl), and O. For example, it can be substituted by one *tert*-butoxycarbonyl, becoming 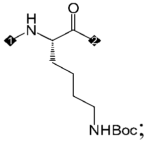 or it can be substituted by two methyl groups, becoming 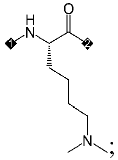 or it can be substituted by two methyl groups and one O simultaneously, becoming 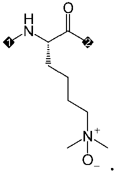 It should be noted that "the distal amino group of lysine (Lys) is substituted by O" means that the distal amino group of lysine (Lys) is oxidized, that is, becoming 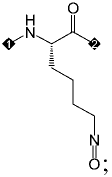 if the distal amino group is further substituted by two methyl groups, then becoming

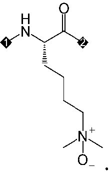

In various parts of this specification, substituents of the compounds in the present disclosure are disclosed according to group types or ranges. Specifically, the present disclosure includes each individual sub-combination of respective members within these group types or ranges. For example, the term "C1-6 alkyl" particularly refers to methyl, ethyl, C3 alkyl, C4 alkyl, C5 alkyl, and C6 alkyl as independently disclosed.

As used herein, the term "C1-6 alkyl" refers to a linear or branched alkyl group containing 1 to 6 carbon atoms, including, for example, "C1-3 alkyl" or "C1-4 alkyl", methyl, ethyl, *etc.* Specific examples include, but are not limited to: methyl, ethyl, *n*-propyl, isopropyl, *n*-butyl, isobutyl, *sec*-butyl, *tert*-butyl, pentyl, and hexyl.

As used herein, the term "C₁₋₄ alkyl" refers to a linear or branched alkyl group containing 1 to 4 carbon atoms, including, for example, "C₁₋₃ alkyl", methyl, ethyl, *etc.* Specific examples include, but are not limited to: methyl, ethyl, *n*-propyl, isopropyl, *n*-butyl, isobutyl, *sec*-butyl, and *tert*-butyl.

As used herein, the term "C₂₋₆ alkenyl" refers to a linear, branched, or cyclic alkenyl group containing at least one double bond and 2 to 6 carbon atoms, including, for example, "C₂₋₄ alkenyl", *etc.* Examples include, but are not limited to: vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl, *etc.*

As used herein, the term "C₂₋₆ alkynyl" refers to a linear or branched alkynyl group containing at least one triple bond and 2 to 6 carbon atoms, including, for example, "C₂₋₄ alkynyl", *etc.* Examples include, but are not limited to: ethynyl, propynyl, 2-butynyl, 2-pentynyl, 3-pentynyl, 4-methyl-2-pentynyl, 2-hexynyl, 3-hexynyl, 5-methyl-2-hexynyl, *etc.*

As used herein, the term "halogen" includes fluorine, chlorine, bromine, and iodine.

As used herein, the term "3- to 6-membered cycloalkyl" or "C₃₋₆ cycloalkyl" refers to a saturated cyclic alkyl group containing 3 to 6 carbon atoms, including cyclopropane (*i.e.,* cyclopropyl), cyclobutane *(i.e.,* cyclobutyl), cyclopentane *(i.e.,* cyclopentyl), and cyclohexyl.

As used herein, the term "3- to 7-membered carbocycloalkyl" or "C₃₋₇ cycloalkyl" refers to a saturated cyclic alkyl group containing 3 to 7 carbon atoms, including cyclopropyl, cyclobutyl, cyclopentyl, cyclohexyl, and cycloheptyl.

As used herein, the term "C1-6 alkoxy" refers to an alkyl group as defined above which is attached to the parent molecular moiety through an oxygen atom. Specific examples include, but are not limited to, methoxy, ethoxy, propoxy, isopropoxy, n-propoxy, isopropoxy, *n*-butoxy, isobutoxy, *tert*-butoxy, pentoxy, hexyloxy, *etc.*

As used herein, the term "C₁₋₄ alkoxy" refers to an alkyl group as defined above which is attached to the parent molecular moiety through an oxygen atom. Specific examples include, but are not limited to, methoxy, ethoxy, propoxy, isopropoxy, *n*-propoxy, isopropoxy, *n*-butoxy, isobutoxy, *tert*-butoxy, *etc.*

As used herein, the term "4- to 10-membered heterocyclyl" refers to a cyclic group containing 4 to 10 ring atoms (at least one of which is a heteroatom, such as a nitrogen atom, an oxygen atom, or a sulfur atom). The term "4- to 6-membered heterocyclyl" refers to a cyclic group containing 4 to 6 ring atoms (at least one of which is a heteroatom, such as a nitrogen atom, an oxygen atom, or a sulfur atom). Optionally, ring atoms (*e.g.,* carbon atoms, nitrogen atoms, or sulfur atoms) in the cyclic structure can be substituted by oxygen. "4- to 8-membered heterocyclyl" includes, for example, "4- to 8-membered nitrogen-containing heterocyclyl", "4- to 8-membered oxygen-containing heterocyclyl", "4- to 7-membered heterocyclyl", "4- to 7-membered oxygen-containing heterocyclyl", "4- to 7-membered heterocyclyl", "4- to 6-membered heterocyclyl", "5- to 7-membered heterocyclyl", "5- to 6-membered heterocyclyl", "5- to 6-membered nitrogen-containing heterocyclyl", including, but not limited to, oxocyclobutyl, pyrrolidinyl, tetrahydrofuryl, piperidinyl, piperazinyl, tetrahydropyranyl, homopiperazinyl, *etc.*

As used herein, the term "4- to 10-membered heterocyclic ring" refers to a ring containing 4 to 10 ring atoms (at least one of which is a heteroatom, such as a nitrogen atom, an oxygen atom, or a sulfur atom). The term "5- to 6-membered heterocyclic ring" refers to a ring containing 5 to 6 ring atoms (at least one of which is a heteroatom, such as a nitrogen atom, an oxygen atom, or a sulfur atom), including, but not limited to, pyrrolidine, tetrahydrofuran, piperidine, piperazine, tetrahydropyran, and other rings.

As used herein, the term "aryl" refers to an aromatic monocyclic or polycyclic hydrocarbon group, such as 6- to 10-membered aryl, 5- to 8-membered aryl, *etc.* Specific examples include, but are not limited to, phenyl, naphthyl, anthracenyl, phenanthryl, *etc.* The "6- to 10-membered aryl" refers to an aryl group containing 6 to 10 ring atoms. The "C6-10 aryl" refers to an aryl group containing 6 to 10 carbon atoms.

As used herein, the term "heteroaryl" refers to an aromatic cyclic group, in which at least one ring atom is a heteroatom, such as a nitrogen atom, an oxygen atom, or a sulfur atom. Optionally, ring atoms (*e.g.,* carbon atoms, nitrogen atoms, or sulfur atoms) in the cyclic structure can be substituted by oxygen. Specific examples include, but are not limited to, 5-to 10-membered heteroaryl, 5- to 6-membered heteroaryl, 5- to 10-membered nitrogen-containing heteroaryl, 6- to 10-membered oxygen-containing heteroaryl, 6- to 8-membered nitrogen-containing heteroaryl, 5- to 8-membered oxygen-containing heteroaryl, *etc.,* such as furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridonyl, 4-pyridonyl, pyrimidinyl, 1,4-dioxinyl, 2*H*-1,2-oxazinyl, 4*H*-1,2-oxazinyl, 6*H*-1,2-oxazinyl, 4*H*-1,3-oxazinyl, 6*H*-1,3-oxazinyl, 4H-1,4-oxazinyl, pyridazinyl, pyrazinyl, 1,2,3-triazinyl, 1,3,5-triazinyl, 1,2,4,5-tetrazinyl, azacycloheptatrienyl, 1,3-diazacycloheptatrienyl, azacyclooctatetraenyl, *etc.*

A bond in a structural formula represented herein by a wavy line "~~" is intended to indicate that the structure represents a *cis* or *trans* isomer, or a mixture of *cis* and *trans* isomers in any ratio.

The term "drug-to-antibody ratio" or "DAR" refers to the ratio of drug to antibody in a group (or mixture) or composition or ADC molecule, such as a small molecule payload attached to the antibody of the ADC. The DAR of an ADC can range from 1 to 16; however, depending on the number of attachment sites on the antibody, higher loadings (*e.g.,* 20) are also possible. The term DAR can be used when referring to the amount of drug loaded onto a single antibody, or alternatively, when referring to the average or mean DAR of a group of ADCs. It should be understood that the latter is typically referred to as average DAR. During the determination of the DAR value by mass spectrometry, the antibody has been reduced to isolated heavy and light chains, DAR1 represents a conjugate containing a light or heavy chain conjugated with one payload molecule; DAR2 represents a conjugate containing a light or heavy chain conjugated with two payload molecules; DAR3 represents a conjugate containing a light or heavy chain conjugated with three payload molecules.

### Beneficial effects of the present disclosure

The present disclosure, through extensive research on multiple target antibody-drug conjugates (ADCs), achieves enrichment in the tumor microenvironment, linker-specific *in vivo* enzymatic cleavage characteristics, and conjugation methods with the targeting moiety, combined with a large number of *in vivo* and *in vitro* pharmacodynamic screening verifications, resulting in a novel class of antibody bioactive molecule conjugates. Using the conjugates obtained according to the above method, a variety of surprising technical effects can be achieved as follows:
the conjugates obtained according to the above method have better solubility and excellent chemical stability, for example, avoiding the reversible Michael addition reaction caused by the traditional maleimide linkage in ADCs, so that a high drug-to-antibody ratio (DAR) can be achieved, and in some embodiments, the DAR value of the conjugate can reach 6 to 8;
the conjugates exhibit extremely high conjugation efficiency, achieving or exceeding 90% in some embodiments;
extensive research has identified a class of linkers that possess high plasma stability while being cleavable in the tumor microenvironment (both inside and outside tumor cells), thereby producing effective anti-tumor results even in tumors with low or no antigen expression;
the conjugates (ADCs) obtained according to the above method improve the exposure of the entire ADC molecule in the relatively acidic tumor environment by adjusting the physicochemical properties of the linkers and the overall ADC molecule. Therefore, the ADCs have better tumor tissue targeting, *i.e.,* the ability to enrich in the tumor microenvironment, increasing the intratumoral and blood concentration ratios of bioactive molecules and reducing mechanism-related toxicity of the ADC molecules (toxicity produced after ADCs bind to cell surface antigens in non-tumor tissues and are endocytosed, also known as "on-target toxicity"), thereby achieving a higher therapeutic index;
the conjugates obtained according to the above method exhibit high stability in systemic circulation, reduce the shedding of drug molecules in non-target tissues, and reduce the "off-target" toxicity caused by the shedding of payloads in non-target tissues;
the bioactive molecules of the conjugates have higher anti-tumor cell activity, thereby having an excellent by-stander effect; ADCs can more effectively kill tumor cells with high antigen expression as well as tumor cells with low or no antigen expression within tumor tissues;
the payload-linker of the present disclosure, using the extracellular cleavage ability of its linker in the tumor microenvironment, can be used to form an antibody-conjugated drug with an antibody without cell endocytosis ability, and such antibody-conjugated drugs still have high anti-tumor activity;
the payload-linker of the present disclosure, using the extracellular cleavage ability of its linker in the tumor microenvironment as well as its enrichment ability in the tumor microenvironment, can be used to form an antibody-conjugated drug with an antibody without cell endocytosis ability as well as an antibody without the ability to bind extracellular antigens of the tumor cell, and such antibody-conjugated drugs still have high anti-tumor activity;
the anti-DLL3 antibodies provided by the present disclosure exhibit a high degree of humanization or are fully human antibodies, thus can be safely administered to human subjects without inducing immunogenic responses.

In summary, the ADCs of the present disclosure have significant clinical value.

### BRIEF DESCRIPTION OF THE DRAWINGS

FIG. 1. *In vivo* endocytic activity assay of anti-DLL3 humanized antibodies.
FIG. 2A. Binding tests of 4 antibodies including DL301 with DLL3.
FIG. 2B. Competition experiment of 55C11E4-hz2 with 3 antibodies including DL301.
FIG. 2C. Competition experiment of 66F11C10-hz2 with 3 antibodies including DL301.
FIG. 3. *In vivo* efficacy testing of anti-human DLL3 ADC in the SHP77 CDX model.
FIG. 4. Changes in mouse body weight during administration of anti-human DLL3 ADC in the SHP77 CDX model.
FIG. 5. *In vivo* efficacy testing of anti-human DLL3 ADC in the NCI-H69 CDX model.
FIG. 6. Changes in mouse body weight during administration of anti-human DLL3 ADC in the NCI-H69 CDX model.

### DETAILED DESCRIPTION OF THE PREFERRED EMBODIMENT

The present disclosure is further described below through the description of specific embodiments, but this is not intended to limit the present disclosure. Those skilled in the art can make various modifications or improvements based on the teachings of the present disclosure without departing from the basic idea and scope of the present disclosure. If the manufacturer of the reagents or instruments used is not indicated, they are all conventional products that can be purchased commercially.

The abbreviations in the present disclosure have the following meanings:

| | | | |
|---|---|---|---|
| OMs | Methanesulfonate | FA | Formic acid |
| OTs | *p*-Toluenesulfonate | ACN | Acetonitrile |
| OTf | Trifluoromethanesulfonate | CCK8 reagent | 2-(2-Methoxy-4-nitrophenyl)-3-(4-nitrophenyl)-5-(2,4-disulfophenyl)-2*H*-tetrazolium monosodium salt |
| TBS | *tert*-Butyldimethylsilyl | FBS | Fetal bovine serum |
| MMT | *p-*Methoxyphenyl(diphenyl)methyl | DMSO | Dimethyl sulfoxide |
| PB/PBS | Phosphate buffered saline | HBTU | *O*-(Benzotriazol-1-yl)-*N,N,N',N'*-tetramethyluronium hexafluorophosphate |
| HOBT | 1-Hydroxybenzotriazole | HATU | 2-(7-Azabenzotriazol-1-yl)-*N*,*N*,*N'*,*N'*-tetramethyluronium hexafluorophosphate |
| NBS | *N*-Bromosuccinimide | TFA | Trifluoroacetic acid |
| PPTS | Pyridinium *p*-toluenesulfonate | DCM | Dichloromethane |
| THF | Tetrahydrofuran | DMF | *N,N-Dimethylformamide* |
| EDC | 1-(3-Dimethylaminopropyl)-3-ethylcarbodiimide hydrochloride | IgG | Immunoglobulin G |
| CDR | Complementarity determining region in an immunoglobulin variable region | HRP | Horseradish peroxidase |
| FR | Antibody framework region: amino acid residues in an antibody variable region excluding CDR residues | hFc | HFc fragment of human IgG antibody |
| VH | Antibody heavy chain variable region | KD | Equilibrium dissociation constant |
| VL | Antibody light chain variable region | HCDR1 | Complementarity determining region 1 in the immunoglobulin heavy chain variable region |
| AbM | The AbM CDR definition method originates from Martin's related research (Martin ACR, Cheetham JC, Rees AR (1989) Modelling antibody hypervariable loops: A combined algorithm. Proc Natl Acad Sci USA 86: 9268-9272). This definition method integrates some of the definitions of both Kabat and Chothia. | HCDR2 | Complementarity determining region 2 in the immunoglobulin heavy chain variable region |
| Kabat | Immunoglobulin comparison and numbering system proposed by Elvin A. Kabat (see, for example, Kabat et al., Sequences of Proteins of Immunological Interest, 5th Ed. Public Health Service, National Institutes of Health, Bethesda, Md., 1991). | HCDR3 | Complementarity determining region 3 in the immunoglobulin heavy chain variable region |
| Chothia | Immunoglobulin numbering system proposed by Chothia *et al.*, which is a classic rule for identifying CDR region boundaries based on the position of structural loop regions (see, for example, Chothia & Lesk (1987) J. Mol. Biol. 196:901-917; Chothia et al. (1989) Nature 342:878-883). | LCDR1 | Complementarity determining region 1 in the immunoglobulin light chain variable region |
| IMGT | Numbering system based on the international ImMunoGeneTics information system^{®} (IMGT) initiated by Lefranc *et al.*, see Lefranc et al., Dev. Comparat. Immunol. 27:55-77, 2003. | LCDR2 | Complementarity determining region 2 in the immunoglobulin light chain variable region |
| mAb | Monoclonal antibody | LCDR3 | Complementarity determining region 3 in the immunoglobulin light chain variable region |
| EC₅₀ | Concentration that produces 50% efficacy or binding | SEC-HPLC | Size-exclusion high-performance liquid chromatography |
| ELISA | Enzyme-linked immunosorbent assay | HC | Immunoglobulin heavy chain |
| PCR | Polymerase chain reaction | LC | Immunoglobulin light chain |

### Sequence and specific information thereof:

| SEQ ID No. | Name | Sequence |
|---|---|---|
| 1 | 10F2F3-58H | QVQLQQSGPVLVKPGASVKMSCKASGYTFTDYYMNW VKQSHGKSLEWIGVINPYNGGTNYSQKFKGKATLTVD KSSSTAYMELNSLTSEDSAVYYCARDSNYVGLLDYWG QGTSVTVSS |
| 2 | 10F2F3-58hzvh | QVQLVESGAEVKKPGSSVKVSCKASGYAFSSHWVNW VRQAPGQGLEWMGQIYPGNGDTNYAQKFQGRVTLTA DKSTSTAYMELSSLTSEDTAVYFCARWFAYWGQGTLV TVSS |
| 3 | 10F2F3-58L | DIVMTQSPSSLAMSVGQRVTINCKSSQSLLDGGNQKNY LAWYQQKVGQPPKLLVYFASIRESGVPDRFIGSGSGTD FTLTISSVQTEDLADYFCQQHYNTPWTFGGGTKLEIK |
| 4 | 10F2F3-58hzvl | DIQMTQSPSSLSASVGDRVTITCKSSQSLLDGGNQKNY LAWYQQKPGKPPKLLVYFASIRESGVPSRFSGSGSGTD FTLTISSLQPEDFATYYCQQHYNTPWTFGQGTKVEIK |
| 5 | 55C11E4-67H | EVQLQQSGPVLVKPGASVKMSCKASGYTFTDYYMNW VKQSHGKSLEWIGVINPYNGGTNYSQKFKGKATLTVD KSSSTAYMELNSLTSEDSAVYYCARDSNYVGLLDYWG QGTSVTVSS |
| 6 | 55C11E4-67hzvh | EVQLVQSGAEVKKPGASVKVSCKASGYTFTDYYMNW VRQAPGQRLEWMGVINPYNGGTNYSQKFQGRVTLTV DKSASTAYMELSSLRSEDTAVYYCARDSNYVGLLDYW GQGTLVTVSS |
| 7 | 55C11E4-67hzvh2 | EVQLVQSGAEVKKPGASVKVSCKASGYTFTDYYMNW VRQAPGQRLEWMGVINPYNGGTNYAQKFQGRVTLTV DKSASTAYMELSSLRSEDTAVYYCARDSNYVGLLDYW GQGTLVTVSS |
| 8 | 55C11E4-67L | DIVLTQSPATLSVTPGDSVSLSCRASQSISNNLHWYQQK SHESPRLLIKYASQSISGIPSRFSGSGSGPDFTLSIDSVET EDFGVYFCQQSNSWPYTFGGGTKLEIK |
| 9 | 55C11E4-67hzvl | DIVLTQSPATLSLSPGERATLSCRASQSISNNLHWYQQK PGQAPRLLIKYASQSISGIPARFSGSGSGPDFTLTISSLEP EDFAVYFCQQSNSWPYTFGQGTKVEIK |
| 10 | 59B10D3-69H | EVKLEESGGGLVQPGGSMKLSCAASGFTFSDAWMDW VRQSPEKGLEWIAEIRNKANNHATYYTESVKGRFTISR DDSKSSVYLQMISLRPEDTGIYYCTRNNYGSTWTYYA MDYWGQGTSVTVSS |
| 11 | 59B10D3-69hzvh | EVQLVESGGGLVQPGGSLRLSCAASGFTFSDAWMDW VRQAPGKGLEWVAEIRNKANNHATYYVDSVKGRFTIS RDDAKNSVYLQMNSLRAEDTAVYYCTRNNYGSTWTY YAMDYWGQGTLVTVSS |
| 12 | 59B10D3-69L | DIQMTQTSSSLSASLGDRVTINCRASQDISNYLNWYQQ KPDGTVKLLIYYTSRLHSGVPSRFSGSGSGTDYSLTISN LEHEDIATYFCQQGYTLPLTFGAGTNLELK |
| 13 | 59B10D3-69hzvl | DIQMTQSPSSLSASVGDRVTITCRASQDISNYLNWYQQ KPGKAVKLLIYYTSRLHSGVPSRFSGSGSGTDYTLTISS LQPEDFATYYCQQGYTLPLTFGQGTKVEIK |
| 14 | 87F7F10-62H | DVQLQESGPGLVKPSQSLSLTCSVTGYSITSDFYWNWI RQFPGNKLEWMGYITYDGSTNYDPSLKNRISITRDTSK NQFFLKLNSVTAEDTATYYCVYGDFNRHAMDYWGQG TSVTVSS |
| 15 | 87F7F10hzvh | QVQLQESGPGLVKPSETLSLTCTVSGYSITSDFYWNWI RQPPGKGLEWIGYITYDGSTNYNPSLKSRVTISRDTSKN QFSLKLSSVTAADTAVYYCVYGDFNRHAMDYWGQGT LVTVSS |
| 16 | 87F7F 10-62L | DIVMSQSPSSLAVSAGEKVTMSCRSSQSLFNSRTRKNY LAWYQQKPGQPPKVLIYWASTRESGVPDRFTGSGSGT DFTLTISSVQAEDLAVYYCEQSFYLFTFGTGTKLEI |
| 17 | 87F7F10hzvl | DIVMTQSPDSLAVSLGERATINCRSSQSLFNSRTRKNYL AWYQQKPGQPPKVLIYWASTRESGVPDRFSGSGSGTD FTLTISSLQAEDVAVYYCEQSFYLFTFGQGTKVEIK |
| 18 | 6F11C10-84H | EVHLQQSGPVLVKPGPSVKISCKASGFTFTDYYMHWV KQSHGKSLEWIGLVYPYNGDISYNQKFKGKATLTVDT SSSTAYMELNSLTSEDSAVYYCARQGNYVNNAIDYWG QGTSVTVSS |
| 19 | 6F11C10-84hzvh | EVQLVQSGAEVKKPGASVKVSCKASGFTFTDYYMHW VRQAPGQSLEWMGLVYPYNGDISYAQKFQGRVTLTV DTSISTAYMELSRLRSDDTAVYYCARQGNYVNNAIDY WGQGTLVTVSS |
| 20 | 6F11C10-84hz2vh | EVQLVQSGAEVKKPGASVKVSCKASGFTFTDYYMHW VRQAPGQSLEWMGLVYPYSGDISYAQKFQGRVTLTVD TSISTAYMELSRLRSDDTAVYYCARQGNYVNNAIDYW GQGTLVTVSS |
| 21 | 6F11C10-84L | DIVMTQSQKFMSTSVGDRVSISCKASQNVGNIIAWYQQ KPGQSPKALIYLASYRYSGVPDRFTGSGSGTDFTLTINN VQSEDLAEYFCQQYSDSPYTFGSGTKLEI |
| 22 | 6F11C10-84hzvl | DIQMTQSPSSLSASVGDRVTITCKASQNVGNIIAWYQQ KPGKSPKALIYLASYRYSGVPSRFSGSGSGTDFTLTISSL QPEDVATYFCQQYSDSPYTFGQGTKVEIK |
| 23 | 10F2F3-58hz kabat HCDR1 | SHWVN |
| 24 | 10F2F3-58hz kabat HCDR2 | QIYPGNGDTNYAQKFQG |
| 25 | 10F2F3-58hz kabat HCDR3 | WFAY |
| 26 | 10F2F3-58hz kabat LCDR1 | KSSQSLLDGGNQKNYLA |
| 27 | 10F2F3-58hz kabat LCDR2 | FASIRES |
| 28 | 10F2F3-58hz kabat/IMGT LCDR3 | QQHYNTPWT |
| 29 | 10F2F3-58hz IMGT HCDR1 | GYAFSSHW |
| 30 | 10F2F3-58hz IMGT HCDR2 | IYPGNGDT |
| 31 | 10F2F3-58hz IMGT HCDR3 | ARWFAY |
| 32 | 10F2F3-58hz IMGT LCDR1 | QSLLDGGNQKNY |
| 33 | 55C11E4 hz1 HC heavy chain | EVQLVQSGAEVKKPGASVKVSCKASGYTFTDYYMNW VRQAPGQRLEWMGVINPYNGGTNYSQKFQGRVTLTV DKSASTAYMELSSLRSEDTAVYYCARDSNYVGLLDYW GQGTLVTVSSASTKGPSVFPLAPSSKSTSGGTAALGCL VKDYFPEPVTVSWNSGALTSGVHTFPAVLQSSGLYSLS SVVTVPSSSLGTQTYICNVNHKPSNTKVDKKVEPKSCD KTHTCPPCPAPELLGGPSVFLFPPKPKDTLMISRTPEVTC VVVDVSHEDPEVKFNWYVDGVEVHNAKTKPREEQYN STYRVVSVLTVLHQDWLNGKEYKCKVSNKALPAPIEK TISKAKGQPREPQVYTLPPSRDELTKNQVSLTCLVKGF YPSDIAVEWESNGQPENNYKTTPPVLDSDGSFFLYSKL TVDKSRWQQGNVFSCSVMHEALHNHYTQKSLSLSPGK |
| / | 10F2F3-58hz IMGT LCDR2 | FAS |
| 34 | 55C11E4-67hz1/hz2 kabat HCDR1 | DYYMN |
| 35 | 55C11E4-67hz1 kabat HCDR2 | VINPYNGGTNYSQKFQG |
| 36 | 55C11E4-67hz2 kabat HCDR2 | VINPYNGGTNYAQKFQG |
| 37 | 55C11E4-67hz1/hz2 kabat HCDR3 | DSNYVGLLDY |
| 38 | 55C11E4-67hz1/hz2 kabat LCDR1 | RASQSISNNLH |
| 39 | 55C11E4-67hz1/hz2 kabat LCDR2 | YASQSIS |
| 40 | 55C11E4-67hz1/hz2 kabat/IMGT LCDR3 | QQSNSWPYT |
| 41 | 55C11E4-67hz1/hz2 IMGT HCDR1 | GYTFTDYY |
| 42 | 55C11E4-67hz1/hz2 IMGT HCDR2 | INPYNGGT |
| 43 | 55C11E4-67hz1/hz2 IMGT HCDR3 | ARDSNYVGLLDY |
| 44 | 55C11E4-67hz1/hz2 IMGT LCDR1 | QSISNN |
| 45 | 55C11E4 -67 hz2 HC heavy chain | EVQLVQSGAEVKKPGASVKVSCKASGYTFTDYYMNW VRQAPGQRLEWMGVINPYNGGTNYAQKFQGRVTLTV DKSASTAYMELSSLRSEDTAVYYCARDSNYVGLLDYW GQGTLVTVSSASTKGPSVFPLAPSSKSTSGGTAALGCL VKDYFPEPVTVSWNSGALTSGVHTFPAVLQSSGLYSLS SVVTVPSSSLGTQTYICNVNHKPSNTKVDKKVEPKSCD KTHTCPPCPAPELLGGPSVFLFPPKPKDTLMISRTPEVTC VVVDVSHEDPEVKFNWYVDGVEVHNAKTKPREEQYN STYRVVSVLTVLHQDWLNGKEYKCKVSNKALPAPIEK TISKAKGQPREPQVYTLPPSRDELTKNQVSLTCLVKGF YPSDIAVEWESNGQPENNYKTTPPVLDSDGSFFLYSKL TVDKSRWQQGNVFSCSVMHEALHNHYTQKSLSLSPGK |
| / | 55C11E4-67hz1/hz2 IMGT LCDR2 | YAS |
| 46 | 59B10D3-69hz kabat HCDR1 | DAWMD |
| 47 | 59B10D3-69hz kabat HCDR2 | EIRNKANNHATYYVDSVKG |
| 48 | 59B10D3-69hz kabat HCDR3 | NNYGSTWTYYAMDY |
| 49 | 59B10D3-69hz kabat LCDR1 | RASQDISNYLN |
| 50 | 59B10D3-69hz kabat LCDR2 | YTSRLHS |
| 51 | 59B10D3-69hz kabat/TMGT LCDR3 | QQGYTLPLT |
| 52 | 59B10D3-69hz IMGT HCDR1 | GFTFSDAW |
| 53 | 59B10D3-69hz IMGT HCDR2 | IRNKANNHAT |
| 54 | 59B10D3-69hz IMGT HCDR3 | TRNNYGSTWTYYAMDY |
| 55 | 59B10D3-69hz IMGT LCDR1 | QDISNY |
| 56 | 55C11E4 hz1/hz2 LC light chain | DIVLTQSPATLSLSPGERATLSCRASQSISNNLHWYQQK PGQAPRLLIKYASQSISGIPARFSGSGSGPDFTLTISSLEP EDFAVYFCQQSNSWPYTFGQGTKVEIKRTVAAPSVFIF PPSDEQLKSGTASVVCLLNNFYPREAKVQWKVDNALQ SGNSQESVTEQDSKDSTYSLSSTLTLSKADYEKHKVYA CEVTHQGLSSPVTKSFNRGEC |
| / | 59B10D3-69hz IMGT LCDR2 | YTS |
| 57 | 87F7F10hz kabat HCDR1 | SDFYWN |
| 58 | 87F7F10hz kabat HCDR2 | YITYDGSTNYNPSLKS |
| 59 | 87F7F10hz kabat HCDR3 | GDFNRHAMDY |
| 60 | 87F7F10hz kabat LCDR1 | RSSQSLFNSRTRKNYLA |
| 61 | 87F7F10hz kabat LCDR2 | WASTRES |
| 62 | 87F7F10hz kabat/IMGT LCDR3 | EQSFYLFT |
| 63 | 87F7F10hz IMGT HCDR1 | GYSITSDFY |
| 64 | 87F7F10hz IMGT HCDR2 | ITYDGST |
| 65 | 87F7F10hz IMGT HCDR3 | VYGDFNRHAMDY |
| 66 | 87F7F10hz IMGT LCDR1 | QSLFNSRTRKNY |
| 67 | 6F11C10 hz1 HC heavy chain | EVQLVQSGAEVKKPGASVKVSCKASGFTFTDYYMHW VRQAPGQSLEWMGLVYPYNGDISYAQKFQGRVTLTV DTSISTAYMELSRLRSDDTAVYYCARQGNYVNNAIDY WGQGTLVTVSSASTKGPSVFPLAPSSKSTSGGTAALGC LVKDYFPEPVTVSWNSGALTSGVHTFPAVLQSSGLYSL SSVVTVPSSSLGTQTYICNVNHKPSNTKVDKKVEPKSC DKTHTCPPCPAPELLGGPSVFLFPPKPKDTLMISRTPEV TCVVVDVSHEDPEVKFNWYVDGVEVHNAKTKPREEQ YNSTYRVVSVLTVLHQDWLNGKEYKCKVSNKALPAPI EKTISKAKGQPREPQVYTLPPSRDELTKNQVSLTCLVK GFYPSDIAVEWESNGQPENNYKTTPPVLDSDGSFFLYS KLTVDKSRWQQGNVFSCSVMHEALHNHYTQKSLSLSP GK |
| / | 87F7F10hz IMGT LCDR2 | WAS |
| 68 | 6F11C10-84hz1/hz2 kabat HCDR1 | DYYMH |
| 69 | 6F11C10-84hz1 kabat HCDR2 | LVYPYNGDISYAQKFQG |
| 70 | 6F11C10-84hz2 kabat HCDR2 | LVYPYSGDISYAQKFQG |
| 71 | 6F11C10-84hz1/hz2 kabat HCDR3 | QGNYVNNAIDY |
| 72 | 6F11C10-84hz1/hz2 kabat LCDR1 | KASQNVGNIIA |
| 73 | 6F11C10-84hz1/hz2 kabat LCDR2 | LASYRYS |
| 74 | 6F11C10-84hz1/hz2 kabat/IMGT LCDR3 | QQYSDSPYT |
| 75 | 6F11C10-84hz1/hz2 IMGT HCDR1 | GFTFTDYY |
| 76 | 6F11C10-84hz1 IMGT HCDR2 | VYPYNGDI |
| 77 | 6F11C10-84hz2 IMGT HCDR2 | VYPYSGDI |
| 78 | 6F11C10-84hz1/hz2 IMGT HCDR3 | ARQGNYVNNAIDY |
| 79 | 6F11C10-84hz1/hz2 IMGT LCDR1 | QNVGNI |
| 80 | 6F11C10 hz2 HC heavy chain | EVQLVQSGAEVKKPGASVKVSCKASGFTFTDYYMHW VRQAPGQSLEWMGLVYPYSGDISYAQKFQGRVTLTVD TSISTAYMELSRLRSDDTAVYYCARQGNYVNNAIDYW GQGTLVTVSSASTKGPSVFPLAPSSKSTSGGTAALGCL VKDYFPEPVTVSWNSGALTSGVHTFPAVLQSSGLYSLS SVVTVPSSSLGTQTYICNVNHKPSNTKVDKKVEPKSCD KTHTCPPCPAPELLGGPSVFLFPPKPKDTLMISRTPEVTC VVVDVSHEDPEVKFNWYVDGVEVHNAKTKPREEQYN STYRVVSVLTVLHQDWLNGKEYKCKVSNKALPAPIEK TISKAKGQPREPQVYTLPPSRDELTKNQVSLTCLVKGF YPSDIAVEWESNGQPENNYKTTPPVLDSDGSFFLYSKL TVDKSRWQQGNVFSCSVMHEALHNHYTQKSLSLSPGK |
| / | 6F11C10-84hz1/hz2 IMGT LCDR2 | LAS |
| 81 | 6F11C10 hz1/hz2 LC light chain | DIQMTQSPSSLSASVGDRVTITCKASQNVGNIIAWYQQ KPGKSPKALIYLASYRYSGVPSRFSGSGSGTDFTLTISSL QPEDVATYFCQQYSDSPYTFGQGTKVEIKRTVAAPSVF IFPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGEC |
| 82 | IgG1 CH heavy chain constant region | |
| 83 | Kappa CL light chain constant region | |

The present disclosure is described with reference to the following examples, which are intended to illustrate, but not to limit, the present disclosure.

Unless specifically stated otherwise, the molecular biology experimental methods and immunoassay methods used in the present disclosure generally refer to the methods described in J. Sambrook et al., Molecular Cloning: A Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995. Those skilled in the art appreciate that the examples describe the present disclosure by way of example and are not intended to limit the scope of protection claimed in the present disclosure.

### Preparation protocol

The structures of the compounds described in the following examples were determined by nuclear magnetic resonance (¹H NMR) or mass spectrometry (MS).

The instrument for detecting nuclear magnetic resonance (¹H NMR) is a Bruker 400 MHz NMR instrument; the test solvent is deuterated methanol (CD₃OD), deuterated chloroform (CDCl₃), or hexadeuterodimethyl sulfoxide (DMSO-d₆); the internal standard substance is tetramethylsilane (TMS).

Abbreviations in nuclear magnetic resonance (NMR) spectra used in the examples are shown below.

s: singlet, d: doublet, t: triplet, q: quartet, dd: double doublet, qd: quartet doublet, ddd: double double doublet, ddt: double double triplet, dddd: double double double doublet, m: multiplet, br: broad, J: coupling constant, Hz: hertz, DMSO-d₆: deuterated dimethyl sulfoxide. δ value is expressed in ppm.

The instrument for detecting mass spectrometry (MS) is an Agilent (ESI) mass spectrometer (model Agilent 6120B).

### I. Synthesis of bioactive molecules and intermediates used in the synthesis of "drug-linker compounds"

### A. Synthesis of bioactive molecules

### Example A1.1: Synthesis of (S)-4-ethyl-8-fluoro-4-hydroxy-9-methyl-11-(1H-pyrazol-4-yl)-1,12-dihydro-14H-pyrano[3',4':6,7]indolizino[1,2-b]quinoline-3,14(4H)-dione (A1.1)

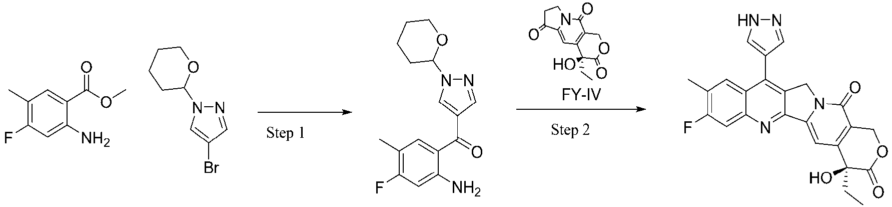

Step 1: 4-Bromo-1-(tetrahydro-2*H*-pyran-2-yl)-1*H*-pyrazole (4.6 g) was dissolved in anhydrous tetrahydrofuran (50 mL). The above solution was cooled to -78°C with dry ice-acetone under nitrogen atmosphere, and then n-butyllithium (12 mL, 2 M) was added dropwise thereto. The reaction mixture was stirred at -78°C for 20 minutes, and then methyl 2-amino-4-fluoro-5-methylbenzoate (1.83 g) was added thereto. After the addition, the reaction mixture was naturally warmed to room temperature and stirred and reacted for another 5 hours. The reaction was quenched with methanol (3 mL), then ethyl acetate (200 mL) was added thereto, and the mixture was washed with water (100 mL × 3). The organic phase was dried and then the organic solvent was removed. The residue was separated by silica gel column chromatography to obtain the target product (2-amino-4-fluoro-5-methylphenyl)(1-(tetrahydro-2*H*-pyran-2-yl)-1*H*-pyrazol-4-yl)methanone, ESI-MS (m/z): 304 [M+H]⁺.

Step 2: (*S*)-4-Ethyl-4-hydroxy-7,8-dihydro-1*H*-pyrano[3,4-*f*]indolizine-3,6,10(4*H*)-trione (2.63 g), (2-amino-4-fluoro-5-methylphenyl)(1-(tetrahydro-2*H*-pyran-2-yl)-1*H*-pyrazol-4-yl)methanone (3.03 g), and *p*-toluenesulfonic acid (1.74 g) were dissolved in dichloromethane (50 mL), then the solvent was removed, and the mixture was heated to 120°C under nitrogen atmosphere and reacted for 4 hours. The mixture was dissolved in ethyl acetate (300 mL). The organic phase was washed with water (100 mL × 2), dried, and then the organic solvent was removed. The residue was separated by silica gel column chromatography to obtain the target product (*S*)-4-ethyl-8-fluoro-4-hydroxy-9-methyl-11-(1*H-*pyrazol-4-yl)-1,12-dihydro-14*H*-pyrano[3',4':6,7]indolizino[1,2-*b*]quinoline-3,14(4*H*)-dione (A1.1). ESI-MS (m/z): 447 [M+H]⁺.

### Example A1.2: Synthesis of (S)-7-ethyl-7-hydroxy-14-(1H-pyrazol-4-yl)-10,13-dihydro-11H-[1,3]dioxolo[4,5-g]pyrano[3',4':6,7]indolizino[1,2-b]quinoline-8,11(7H)-dione (A1.2)

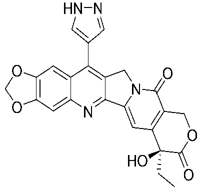

Using the same method and reaction conditions as in Example A1.1, methyl 6-aminobenzo[*d*][1,3]dioxolane-5-carboxylate was used instead of 2-amino-4-fluoro-5-methylbenzoate to obtain the target product (*S*)-7-ethyl-7-hydroxy-14-(1*H*-pyrazol-4-yl)-10,13-dihydro-11*H*-[1,3]dioxolo[4,5-g]pyrano[3',4":6,7]indolizino[1,2-*b*]quinoline-8,11(7*H*)-dione (A1.2). ESI-MS (m/z): 459 [M+H]⁺.

### Example A1.3: Synthesis of (S)-14-(3-aminophenyl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.3)

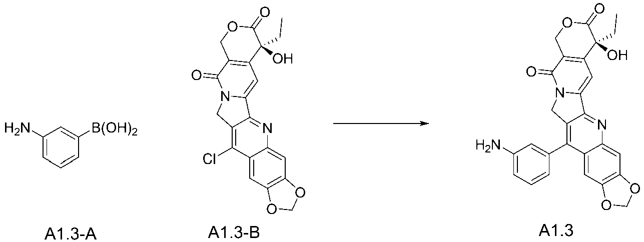

Compound (A1.3-A) (1.4 g), compound (A1.3-B) (2.2 g), Pd₂(DBA)₃ (300 mg), tricyclohexylphosphine (300 mg), and potassium acetate (1.1 g) were sequentially added to a mixed solvent of dioxane (30 mL) and water (5 mL), and the mixture was heated to 100°C, stirred and reacted for 12 hours under nitrogen atmosphere. After cooling, the reaction mixture was added with ethyl acetate (200 mL), washed with water (100 mL), dried, and then separated by silica gel column chromatography to obtain the target product (*S*)-14-(3-aminophenyl)-7-ethyl-7-hydroxy-10,13-dihydro-11*H*-[1,3]dioxolo[4,5-*g*]pyrano[3',4':6,7]indolizino[1,2-b]quinoline-8,11(7*H*)-dione (A1.3). ESI-MS (m/z): 484 [M+H]⁺.

### Example A1.4: Synthesis of (S)-14-(4-aminophenyl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.4)

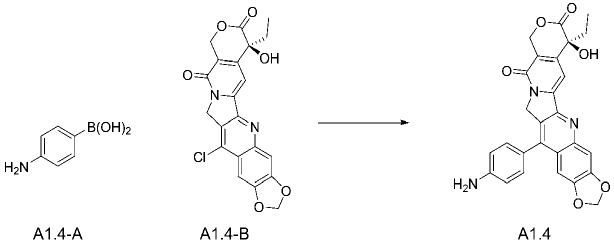

Compound (A1.4-A) (1.4 g), compound (A1.4-B) (2.2 g), Pd₂(DBA)₃ (300 mg), tricyclohexylphosphine (300 mg), and potassium acetate (1.1 g) were sequentially added to a mixed solvent of dioxane (30 mL) and water (5 mL), and the mixture was heated to 100°C, stirred and reacted for 12 hours under nitrogen atmosphere. After cooling, the reaction mixture was added with ethyl acetate (200 mL), washed with water (100 mL), dried, and then separated by silica gel column chromatography to obtain the target product (*S*)-14-(4-aminophenyl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (A1.4).
ESI-MS (m/z): 484 [M+H]⁺;
¹H NMR (400 MHz, DMSO) δ 7.56 (s, 1H), 7.34 (d, *J* = 8.0 Hz, 2H), 7.27 (s, 1H), 7.14 (s, 1H), 6.89 (d, *J* = 7.8 Hz, 2H), 6.26 (s, 2H), 5.40 (s, 2H), 5.05 (s, 2H), 1.96 - 1.78 (m, 2H), 0.88 (t, *J* = 7.2 Hz, 3H).

### Example A1.5: Synthesis of (S)-14-(3-aminopropyl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.5)

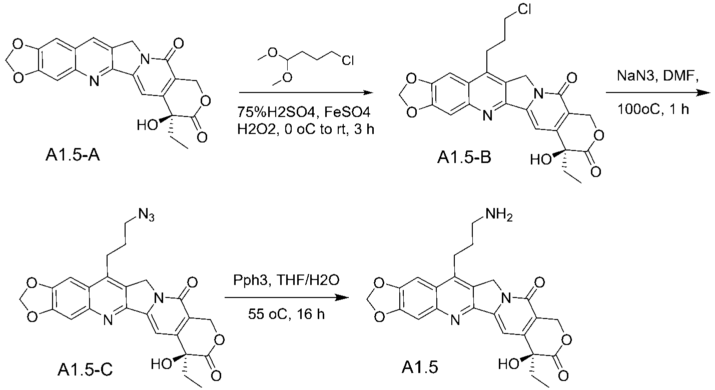

### Step 1: Synthesis of (S)-14-(3-chloropropyl)-7-ethyl-7-hydroxy-10,13-dihydro-11H-[1,3]dioxolo[4,5-g]pyrano[3',4':6,7]indolizino[1,2-b]quinoline-8,11(7H)-dione

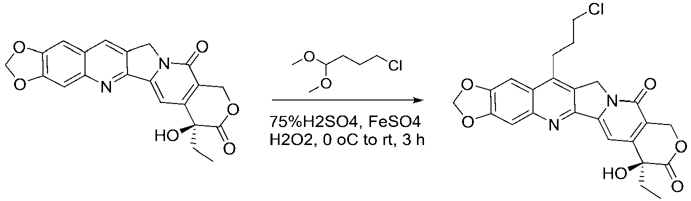

Under an ice bath, ferrous sulfate heptahydrate (570 mg of ferrous sulfate heptahydrate dissolved in 1 mL of water) and 4,4-dimethoxychlorobutane (3.89 g) were added to a 75% sulfuric acid solution (5 mL) of compound (*S*)-7-ethyl-7-hydroxy-10,13-dihydro-11*H-*[1,3]dioxolo[4,5-g]pyrano[3',4':6,7]indolizino[1,2-*b*]quinoline-8,11(7*H*)-dione (A1.5-A, 500 mg). The reaction mixture was stirred for three minutes, and then hydrogen peroxide (29%, 2.5 mL) was added dropwise thereto. The reaction mixture was stirred and reacted at 0°C for 5 minutes, then warmed to room temperature, and stirred and reacted for 3 hours. The reaction mixture was diluted with water (50 mL), extracted with ethyl acetate (80 mL × 2), and the organic phase was washed with saturated brine, dried over anhydrous sodium sulfate, and concentrated to obtain a crude product. The crude product was further purified by C18 column chromatography (acetonitrile/0.05% formic acid in water: 5% to 60%) to obtain the target compound (yellow solid, 400 mg, yield: 67%).
LCMS (ESI) [M+H]⁺: 468.9;
¹H NMR (400 MHz, DMSO-d6) δ 7.65 (s, 1H), 7.51 (s, 1H), 7.24 (s, 1H), 6.50 (s, 1H), 6.30 (s, 2H), 5.42 (s, 2H), 5.26 (s, 2H), 3.81 (d, *J* = 5.9 Hz, 2H), 3.22 (s, 2H), 1.98 (d, *J* = 6.7 Hz, 4H), 0.88 (t, *J =* 7.2 Hz, 3H).

### Step 2: Synthesis of (5)-14-(3-azidopropyl)-7-ethyl-7-hydroxy-10,13-dihydro-11H-[1,3]dioxolo[4,5-g]pyrano[3',4':6,7]indolizino[1,2-b]quinoline-8,11(7H)-dione

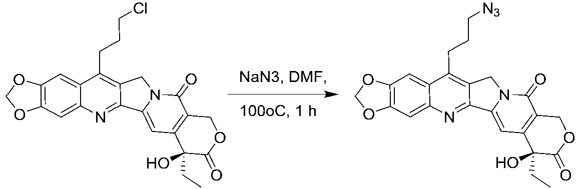

Sodium azide (284 mg) was added to a solution (3 mL) of compound (*S*)-14-(3-chloropropyl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (200 mg) in *N,N-*dimethylformamide, and the reaction mixture was stirred and reacted at 100°C for 1 hour. The reaction mixture was added with water (30 mL), extracted with ethyl acetate (60 mL × 2), and the organic phase was washed with saturated brine, dried over anhydrous sodium sulfate, and concentrated to obtain the target compound (*S*)-14-(3-azidopropyl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (yellow solid, 180 mg, yield: 88%).
LCMS (ESI) [M+H]⁺: 476;
¹H NMR (400 MHz, DMSO-d6) δ 7.65 (s, 1H), 7.51 (s, 1H), 7.24 (s, 1H), 6.48 (s, 1H), 6.29 (s, 2H), 5.42 (s, 2H), 5.25 (s, 2H), 3.53 - 3.49 (m, 2H), 3.16 - 3.12 (m, 2H), 1.93 - 1.80 (m, 4H), 0.88 (t, *J =* 7.2 Hz, 3H).

### Step 3: Synthesis of (S)-14-(3-aminopropyl)-7-ethyl-7-hydroxy-10,13-dihydro-11H-[1,3]dioxolo[4,5-g]pyrano[3',4':6,7]indolizino[1,2-b]quinoline-8,11(7H)-dione

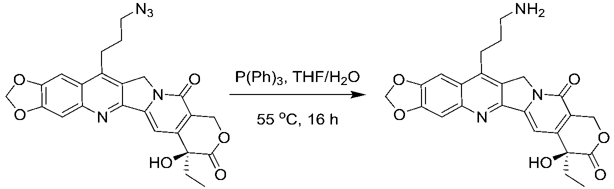

Triphenylphosphine (108 mg, 0.411 mmol) was added to a mixed solution of compound (*S*)-14-(3-azidopropyl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (130 mg, 0.274 mmol) in tetrahydrofuran (3 mL) and water (1 mL), and the reaction mixture was reacted at 55°C for 16 hours. LCMS showed the completion of the reaction. The reaction mixture was added with water (5 mL), adjusted to acidity with 2N hydrochloric acid (3 mL), and extracted with ethyl acetate (10 mL). The aqueous phase was purified by preparative high-performance liquid chromatography (acetonitrile/0.05% formic acid in water) to obtain the target compound (S)-14-(3-aminopropyl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (A1.5, yellow solid, 15 mg, yield: 12%).
LCMS (ESI) [M+H]⁺: 449.9;
¹H NMR (400 MHz, DMSO-d6) δ 7.72 (s, 3H), 7.53 (s, 1H), 7.25 (s, 1H), 6.50 (s, 1H), 6.30 (s, 2H), 5.43 (s, 2H), 5.24 (s, 2H), 3.15 (d, *J =* 6.4 Hz, 2H), 3.03 (t, *J =* 6.9 Hz, 2H), 1.96 - 1.81 (m, 4H), 0.88 (t, *J =* 7.3 Hz, 3H).

### Example A1.6: Synthesis of (S)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.6)

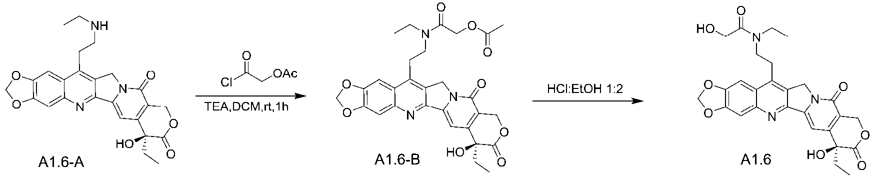

### Step 1: Preparation of (S)-2-(ethyl(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)amino)-2-oxoethyl acetate (A1.6-B):

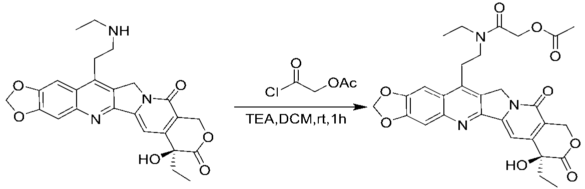

Compound (*S*)-7-ethyl-14-(2-(ethylamino)ethyl)-7-hydroxy-10,13-dihydro-11*H-*[1,3]dioxolo[4,5-*g*]pyrano[3',4':6,7]indolizino[1,2-*b*]quinoline-8,11(7*H*)-dione (A1.6-A, 50 mg), acetoxyacetyl chloride (73 mg), and triethylamine (50 mg) were sequentially added to dichloromethane (5 mL), and the reaction mixture was stirred and reacted at room temperature for 1 hour. The reaction mixture was subjected to rotary evaporation to remove the solvent to obtain 75 mg of the crude oily compound.

LCMS (ESI) [M+H]⁺: 564.2.

### Step 2: Synthesis of (S)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.6)

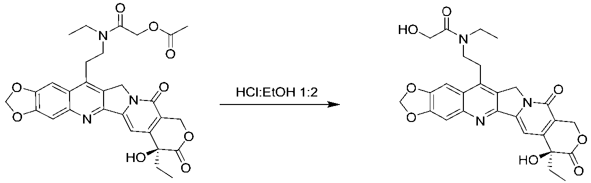

The crude compound (*S*)-2-(ethyl(2-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)ethyl)amino)-2-oxoethyl acetate (75 mg, 0.13 mmol) was dissolved in a mixed solution of concentrated hydrochloric acid and anhydrous ethanol (volume ratio of 1/2, 3 mL), and then the reaction mixture was refluxed at 85°C for 1 hour. LCMS showed the completion of the reaction. The reaction mixture was concentrated, and the crude product was purified by preparative chromatography (0.01% trifluoroacetic acid in water, acetonitrile) to obtain the target compound (*S*)-*N*-ethyl-*N*-(2-(7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)ethyl)-2-hydroxyacetamide (A1.6) (15 mg, yield: 26%) as a white solid.
LCMS (ESI) [M+H]⁺: 522.0;
¹H NMR (400 MHz, DMSO) δ 7.92 (s, 1H), 7.53 (m, 1H), 7.25 (s, 1H), 6.50 (s, 1H), 6.31 (s, 2H), 5.43 (s, 2H), 5.34 (m, 2H), 4.65 (m, 1H), 4.07 (m, 2H), 3.52 (m, 2H), 3.41 (m, 2H), 2.00 (m, 2H), 1.88 (m, 2H), 1.16 - 1.04 (m, 3H), 0.89 - 0.83 (m, 3H).

### Example A1.7: Synthesis of (S)-N-methyl-N-(2-(7-ethyl-7-hydroxy-8,11-dioxo-7,8,11,13-tetrahydro-10H-[1,3]dioxolo[4,5-g]|pyrano[3',4":6,7]indolizino[1,2-b]quinolin-14-yl)ethyl)-2-hydroxyacetamide (A1.7)

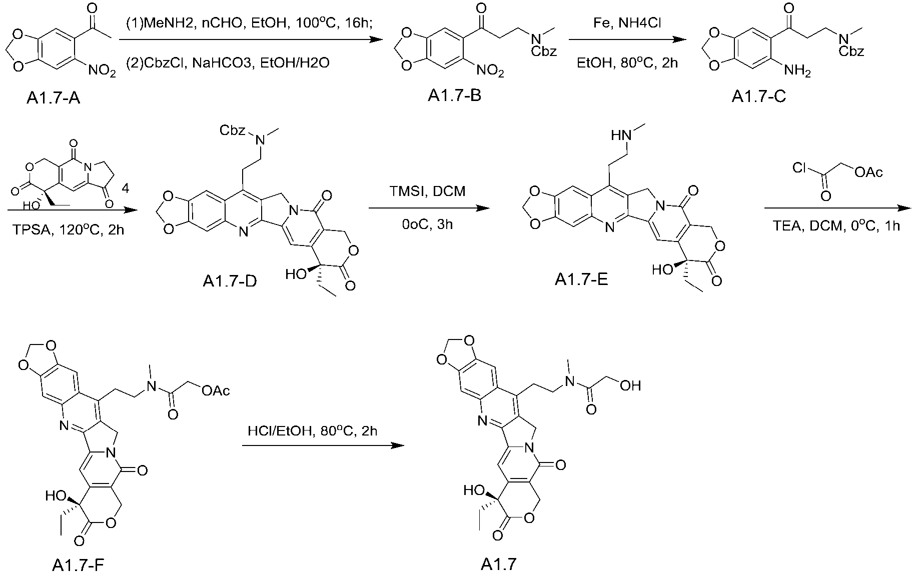

### Step 1: Preparation of compound benzyl methyl(3-(6-nitrobenzo[d][1,3]dioxol-5-yl)-3-oxopropyl)carbamate

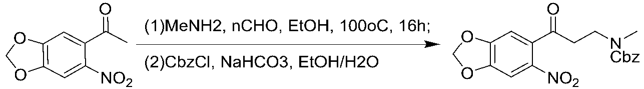

Compound 1-(6-nitrobenzo[*d*][1,3]dioxin-5-yl)ethan-1-one (A1.7-A, 500 mg), methylamine hydrochloride (1.6 g), and paraformaldehyde (714 mg) were dissolved in ethanol (8 mL), and the reaction mixture was reacted in a sealed vessel at 100°C for 16 hours. The reaction mixture was cooled to room temperature and concentrated under reduced pressure. The resulting residue was dissolved in dichloromethane (80 mL), and the organic phase was extracted with water (50 mL × 3). The pH of the resulting aqueous phase was adjusted to 9 with sodium bicarbonate, then benzyl chloroformate (513 mg, 3.0 mmol) was added thereto, and the reaction mixture was reacted at room temperature for 16 hours. The reaction mixture was extracted with ethyl acetate (30 mL × 3), and the organic phase was washed with saturated brine, dried over anhydrous sodium sulfate, subjected to suction filtration, and concentrated to obtain a crude product. The crude product was purified by C18 column chromatography (acetonitrile/0.05% formic acid in water = 5 to 95%) to obtain the target compound (A1.7-B, 180 mg, yield: 23%).

LCMS (ESI) [M+H]⁺: 387.1.

¹H NMR (400 MHz, CDCl₃) δ 7.56 (s, 1H), 7.35 (m, 5H), 7.32 (m, 1H), 6.17 (s, 2H), 5.13 (s, 2H), 3.71 (m, 2H), 3.03 (m, 3H), 2.99 - 2.86 (m, 2H).

### Step 2: Preparation of compound benzyl (3-(6-aminobenzo[d][1,3]dioxin-5-yl)-3-oxopropyl)(methyl)carbamate (A1.7-C)

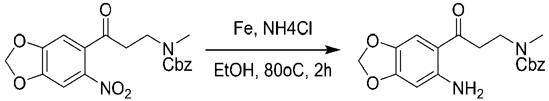

Compound benzyl methyl(3-(6-nitrobenzo[*d*][1,3]dioxol-5-yl)-3-oxopropyl)carbamate (A1.7-B) (180 mg, 0.47 mmol) was dissolved in a mixed solution of saturated ammonium chloride aqueous solution (8 mL) and ethanol (8 mL), then iron powder (130 mg) was added thereto, and the reaction mixture was stirred at 80°C for 2 hours. The reaction mixture was filtered, and the filtrate was concentrated under reduced pressure to a solid. The crude product was purified by TLC (petroleum ether: ethyl acetate = 2/1) to obtain the target compound A1.7-C (76 mg).

LCMS (ESI) [M+H]⁺: 357.0.

### Step 3: Preparation of benzyl (S)-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(methyl)carbamate (A1.7-D)

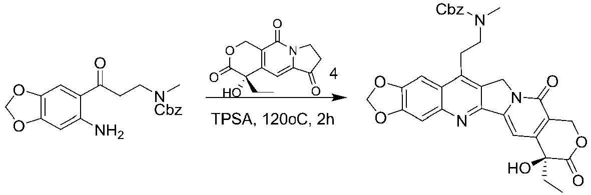

Compound benzyl (3-(6-aminobenzo[id][1,3]dioxin-5-yl)-3-oxopropyl)(methyl)carbamate (A1.7-C) (38 mg), compound (*S*)-4-ethyl-4-hydroxy-7,8-dihydro-1*H*-pyrano[3,4-*f*]indolizine-3,6,10(4*H*)-trione (29 mg), and *p*-toluenesulfonic acid (23 mg) were dissolved in a dichloromethane (5 mL) solution at room temperature. The resulting solution was clarified and mixed homogeneously, then concentrated under reduced pressure, and evacuated to vacuum with an oil pump. The reaction mixture was reacted at 120°C under vacuum for 2 hours. The reaction mixture was cooled to room temperature, added with water (30 mL), and extracted with dichloromethane (30 mL × 3). The combined organic phases were sequentially dried over anhydrous sodium sulfate, filtered, and the filtrate was concentrated under reduced pressure to obtain the target compound A1.7-D (50 mg).

LCMS (ESI) [M+H]⁺ = 584.0.

### Step 4: Preparation of (S)-7-ethyl-7-hydroxy-14-(2-(methylamino)ethyl)-10,13-dihydro-11H-[1,3]dioxolo[4,5-g]pyrano[3',4':6,7]indolizino[1,2-b]quinoline-8,11(7H)-dione (A1.7-E)

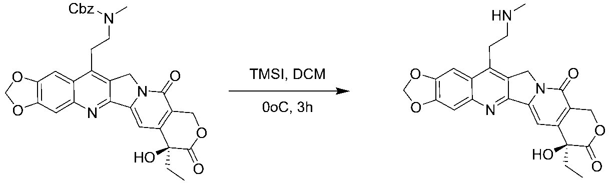

Compound benzyl (*S*)-(2-(7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)ethyl)(methyl)carbamate (A1.7-D) (50 mg) was dissolved in a dichloromethane (5 mL) solution at room temperature, and trimethylsilyl iodide (51 mg, 0.26 mmol) was added thereto at 0°C, and the reaction mixture was reacted for 3 hours. The reaction mixture was concentrated to remove the solvent, and the residue was purified by preparative high-performance liquid chromatography (acetonitrile/0.05% formic acid in water) to obtain compound A1.7-E (15 mg) as a brown solid.

LCMS (ESI) [M+H]⁺: 450.0.

### Step 5: Preparation of compound (S)-2-((2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)(methyl)amino)-2-oxoethyl acetate (A1.7-F)

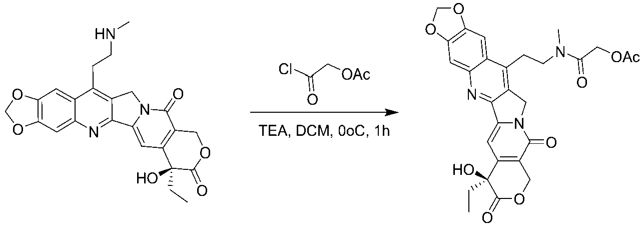

Compound (*S*)-7-ethyl-7-hydroxy-14-(2-(methylamino)ethyl)-10,13-dihydro-11*H-*[1,3]dioxolo[4,5-*g*]pyrano[3',4':6,7]indolizino[1,2-*b*]quinoline-8,11(7*H*)-dione (A1.7-E) (15 mg) and triethylamine (15 mg) were dissolved in a dichloromethane (5 mL) solution, then acetoxyacetyl chloride (20 mg) was added thereto at 0°C, and the reaction mixture was reacted for 1 hour. The reaction mixture was concentrated to remove the solvent to obtain a crude product, and the resulting solid was used directly in the next step without further purification.

LCMS (ESI) [M5+H]⁺ = 550.1.

### Step 6: Preparation of compound (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-methylacetamide (A1.7)

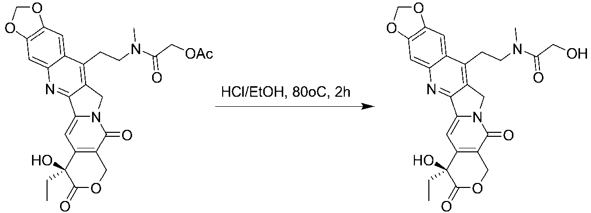

Compound (*S*)-2-((2-(7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)ethyl)(methyl)amino)-2-oxoethyl acetate (A1.7-F) (15 mg) was dissolved in an ethanol (5 mL) solution, then concentrated hydrochloric acid (1.5 mL) was added thereto, and the reaction mixture was reacted at 80°C for 2 hours. The reaction mixture was concentrated to remove the solvent, and the residue was purified by preparative high-performance liquid chromatography (acetonitrile/0.05% formic acid in water) to obtain compound A1.7 (1.6 mg) as a white solid.

LCMS (ESI) [M+H]⁺: 508.2.

### Example A1.8: Synthesis of (S)-N-isopropyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxyacetamide (A1.8)

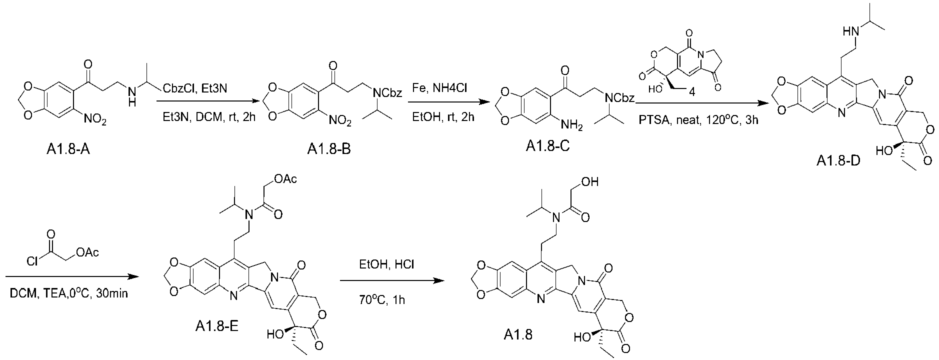

### Step 1: Preparation of compound benzyl isopropyl(3-(6-nitrobenzo[d][1,3]dioxin-5-yl)-3-oxopropyl)carbamate (A1.8-B)

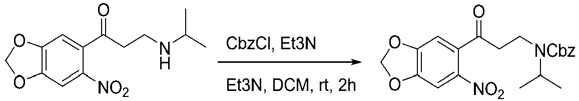

Triethylamine (1.8 g) and benzyloxycarbonyl chloride (734 mg, 4.3 mmol) were sequentially added to a dichloromethane solution (10 mL) of compound 3-(isopropylamino)-1-(6-nitrobenzo[*d*][1,3]dioxin-5-yl)propan-1-one (A1.8-A) (900 mg), and the reaction mixture was reacted at room temperature for 2 hours. The reaction mixture was diluted with water (50 mL) and extracted with dichloromethane (50 mL × 3). The organic phase was washed with saturated brine, dried over anhydrous sodium sulfate, and concentrated to obtain a crude product. The crude product was purified by column chromatography (petroleum ether: ethyl acetate = 3/1) to obtain the target compound (A1.8-B) (600 mg).
LCMS (ESI) [M+H]⁺: 414.9;
¹H NMR (400 MHz, DMSO-d6) δ 7.72 (s, 1H), 7.34 (br s, 6H), 6.31 (s, 2H), 5.08 (s, 2H), 4.14 - 4.10 (m, 1H), 3.48 (d, *J =* 7.9 Hz, 2H), 3.03 (s, 2H), 1.13 - 1.11 (m, 6H).

### Step 2: Preparation of compound benzyl (3-(6-aminobenzo[d][1,3]dioxol -5-yl)-3-oxopropyl)(isopropyl)carbamate (A1.8-C)

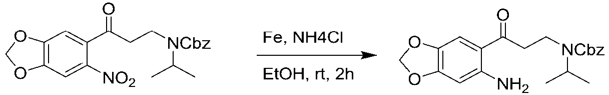

Compound benzyl isopropyl(3-(6-nitrobenzo[d][1,3]dioxol -5-yl)-3-oxopropyl)carbamate (A1.8-B) (200 mg, 0.48 mmol) was dissolved in a mixed solution of saturated ammonium chloride (3 mL) and ethanol (3 mL), then iron powder (135 mg) was added thereto, and the reaction mixture was stirred at room temperature for 2 hours. The reaction mixture was filtered, and the filtrate was concentrated under reduced pressure to a solid. The crude product was purified by column chromatography (petroleum ether: ethyl acetate = 3/1) to obtain the target compound (A1.8-C) (65 mg).

LCMS (ESI) [M+H]⁺: 395.2.

### Step 3: Preparation of (S)-7-ethyl-7-hydroxy-14-(2-(isopropylamino)ethyl)-10,13-dihydro-11H[1,3]dioxolo[4,5-g]pyrano[3',4':6,7]indolizino[1,2-b]quinoline-8,11(7H)-dione (A1.8-D)

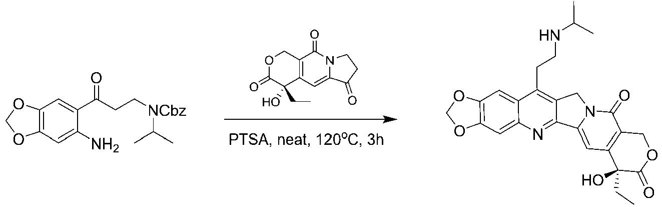

Compound benzyl (3-(6-aminobenzo[*d*][1,3]dioxin-5-yl)-3-oxopropyl)(isopropyl)carbamate (A1.8-C) (65 mg), compound (S)-4-ethyl-4-hydroxy-7,8-dihydro-1*H*-pyrano[3,4-*f*]indolizine-3,6,10(4*H*)-trione (45 mg), and *p*-toluenesulfonic acid (33 mg, 0.17 mmol) were dissolved in a dichloromethane (10 mL) solution at room temperature. The resulting solution was clarified and mixed homogeneously, then concentrated under reduced pressure, and evacuated to vacuum with an oil pump. The reaction mixture was reacted at 120°C under vacuum for 2 hours. The reaction mixture was cooled to room temperature, added with water (50 mL), and extracted with dichloromethane (30 mL × 3). The combined organic phases were sequentially dried over anhydrous sodium sulfate and filtered. The filtrate was concentrated under reduced pressure. The crude product was purified by column chromatography (dichloromethane/methanol = 10/1) to obtain the target compound (A1.8-D) (40 mg).
LCMS (ESI) [M+H]⁺: 478.0;
¹H NMR (400 MHz, DMSO-d6) δ 8.46 (s, 1H), 7.67 (s, 1H), 7.57 (s, 1H), 7.27 (s, 1H), 6.52 (s, 1H), 6.33 (s, 2H), 5.44 (s, 2H), 5.35 (s, 2H), 3.41 (br s, 2H), 3.23 (br s, 3H), 1.94 - 1.78 (m, 2H), 1.25 (d, *J* = 6.4 Hz, 6H), 0.88 (t, *J* = 7.3 Hz, 3H).

### Step 4: Preparation of compound (S)-2-((2-(7-ethyl-7-hydroxy-8,11-dioxo-7,8,11,13-tetrahydro-10H-[1,3]dioxolo[4,5-g]pyrano[3',4':6,7]indolizino[1,2-ib]quinolin-14-yl)ethyl)(isopropyl)amino)-2-oxoethyl acetate (A1.8-E)

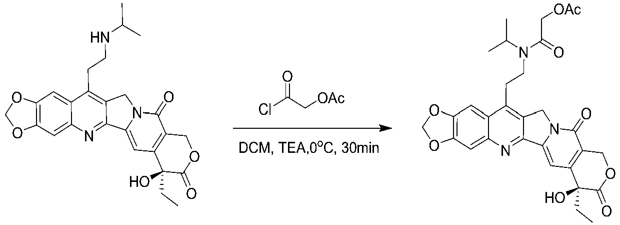

Under an ice bath, triethylamine (35 mg) and 2-chloro-2-oxoethyl acetate (57 mg) were added to a dichloromethane solution (2 mL) of compound (*S*)-7-ethyl-7-hydroxy-14-(2-(isopropylamino)ethyl)-10,13-dihydro-11*H*-[1,3]dioxolo[4,5-*g*]pyrano[3',4':6,7]indolizino[1,2-*b*]quinoline-8,11(7*H*)-dione (A1.8-D) (40 mg), and the reaction mixture was reacted at 0°C for 30 minutes. The reaction mixture was concentrated under reduced pressure to obtain compound A1.8-E, which was directly used in the next step.

LCMS (ESI) [M+H]⁺: 578.0.

### Step 5: Preparation of compound (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-isopropylacetamide (A1.8)

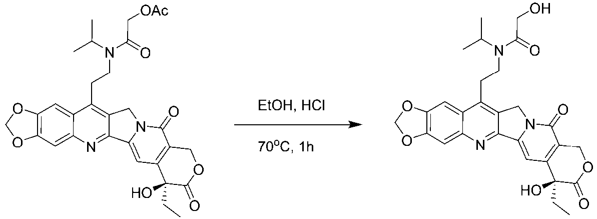

Concentrated hydrochloric acid (0.5 mL) was added to an ethanol solution (3 mL) of compound (*S*)-2-((2-(7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)ethyl)(isopropyl)amino)-2-oxoethyl acetate (A1.8-E, 50 mg), and the reaction mixture was reacted at 70°C for 1 hour. The reaction mixture was concentrated to obtain a crude product. The crude product was then purified by preparative high-performance liquid chromatography (acetonitrile/0.05% formic acid in water) to obtain the target compound (A1.8, 3 mg).
LCMS (ESI) [M+H]⁺: 464.0;
¹H NMR (400 MHz, DMSO-d6) δ 7.99 (s, 1H), 7.53 (s, 1H), 7.26 (s, 1H), 6.31 (s, 2H), 5.43 (s, 2H), 5.36 (s, 2H), 4.22 (s, 2H), 3.99 - 3.94 (m, 1H), 3.44 (dd, *J* = 16.7, 7.8 Hz, 2H), 3.33 - 3.21 (m, 2H), 1.95 - 1.79 (m, 2H), 1.19 (dd, *J* = 16.4, 5.8 Hz, 6H), 0.88 (t, *J =* 7.2 Hz, 3H).

### Example A1.9: Synthesis of (S)-7-ethyl-7-hydroxy-14-(3-hydroxypropyl)-10,13-dihydro-11H-[1,3]dioxolo[4,5-g]pyrano[3',4":6,7]indolizino[1,2-b]quinoline-8,1 1(7H)-dione (A1.9)

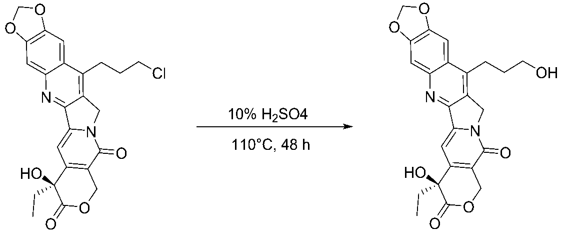

Compound (*S*)-7-ethyl-7-hydroxy-14-(3-chloropropyl)-10,13-dihydro-11*H-*[1,3]dioxolo[4,5-*g*]pyrano[3',4':6,7]indolizino[1,2-*b*]quinoline-8,11(7*R*)-dione (100 mg, 0.213 mmol) was dissolved in 10% sulfuric acid (5 mL) solution, and the reaction mixture was reacted at 110°C for 48 hours. The reaction mixture was added with saturated sodium bicarbonate (30 mL) solution, extracted with dichloromethane (10 mL × 5), dried over anhydrous sodium sulfate, subjected to suction filtration, and concentrated under reduced pressure to obtain a crude product. The crude product was purified by preparative high-performance liquid chromatography (acetonitrile/0.05% formic acid in water) to obtain (S)-7-ethyl-7-hydroxy-14-(3-hydroxypropyl)-10,13-dihydro-11*H*-[1,3]dioxolo[4,5-*g*]pyrano[3',4':6,7]indolizino[1,2-*b*]quinoline-8,11(7H)-dione (A1.9, 1.78 mg).
LCMS (ESI) [M+H]⁺: 451.0;
¹H NMR (400 MHz, DMSO-*d6*) δ 7.63 (s, 1H), 7.50 (s, 1H), 7.24 (s, 1H), 6.48 (s, 1H), 6.28 (s, 2H), 5.47 - 5.37 (m, 2H), 5.32 - 5.19 (m, 2H), 3.51 - 3.46 (m, 2H), 3.17 - 3.13 (m, 2H), 1.92 - 1.76 (m, 4H), 0.90 - 0.84 (m, 3H).

### Example A1.10: Synthesis of (S)-4-ethyl-8-fluoro-4-hydroxy-11-(3-hydroxypropyl)-9-methyl-1,12-dihydro-14H-pyrano[3',4':6,7]indolizino[1,2-b]quinoline-3,14(4H)-dione (A1.10)

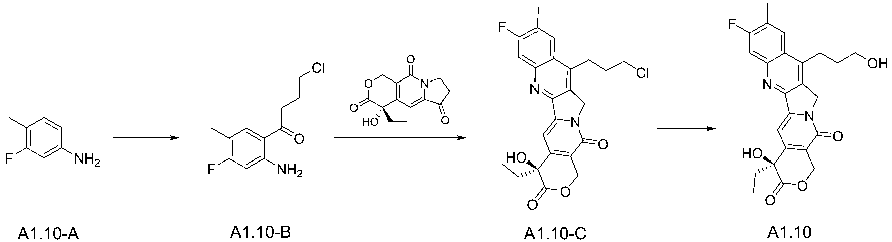

### Step 1:

At 0°C, 1 mol/L boron trichloride (96 mL) and 4-chlorobutyronitrile (9.9 g) were added dropwise to a solution of compound A1.10-A (10 g) in 1,2-dichloroethane (200 mL). The reaction mixture was stirred and reacted at 80°C for 2 hours. The reaction mixture was cooled to room temperature, then added with 2 mol/L hydrochloric acid (90 mL), and refluxed with stirring at 80°C for 0.5 hours. The reaction mixture was cooled to room temperature, diluted with a small amount of water, and extracted with dichloromethane (200 mL × 3). The organic phase was dried over anhydrous sodium sulfate, subjected to suction filtration, and concentrated to obtain a crude product. The crude product was purified by column chromatography (petroleum ether: ethyl acetate = 10/1) to obtain the target compound A1.10-B (4 g).

LCMS (ESI) [M+H]⁺: 230.0.

### Step 2:

(*S*)-4-Ethyl-4-hydroxy-7,8-dihydro-1*H*-pyrano[3,4-*f*]indolizine-3,6,10(4*H*)-trione (35 mg) and *p*-toluenesulfonic acid monohydrate (41.4 mg) were added to compound A1.10-B (50 mg), and the mixture was dissolved in a dichloromethane (30 mL) solution. The resulting solution was clarified and mixed homogeneously, then concentrated under reduced pressure, and evacuated to vacuum with an oil pump. The reaction mixture was reacted at 120°C under vacuum for 3 hours. LCMS showed the completion of the reaction. The reaction mixture was cooled to room temperature, added with water (20 mL), and extracted with dichloromethane (20 mL × 3). The combined organic phases were sequentially dried over anhydrous sodium sulfate, filtered, and the filtrate was concentrated under reduced pressure to obtain a crude product. The crude product was purified by column chromatography (dichloromethane to methanol = 20 to 1) to obtain the target compound A1.10-C (80 mg) as a white solid.

LCMS (ESI) [M+H]⁺: 457.0.

### Step 3:

Compound A1.10-C (75 mg) was dissolved in hexamethylphosphoramide, then pure water (0.8 mL) was added thereto, and the reaction mixture was stirred at 100°C for 72 hours. The completion of the reaction was detected by LCMS. The reaction mixture was purified by preparative chromatography (0.01% TFA in water, MeCN) to obtain the target compound (10 mg).
LCMS (ESI) [M+H]⁺: 439.2;
¹H NMR (400 MHz, DMSO-d6) δ 8.22 (d, *J* = 8.4 Hz, 1H), 7.87 (d, *J* = 10.9 Hz, 1H), 7.31 (s, 1H), 6.50 (s, 1H), 5.43 (s, 2H), 5.30 (s, 2H), 4.67 (t, *J* = 4.9 Hz, 1H), 3.55 - 3.47 (m, 2H), 3.28 - 3.20 (m, 2H), 2.51 (s, 3H), 1.93 - 1.81 (m, 4H), 0.88 (t, *J =* 7.3 Hz, 3H).

### Example A1.11: Synthesis of (S)-4-ethyl-8-fluoro-4-hydroxy-11-(3-aminopropyl)-9-methyl-1,12-dihydro-14H-pyrano[3',4':6,7]indolizino[1,2-b]quinoline-3,14(4H)-dione (A1.11)

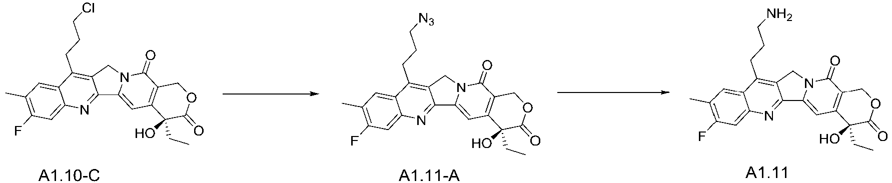

### Step 1:

Sodium azide (432 mg) was added to a solution of A1.10-C (395 mg) in *N'N-*dimethylformamide (10 mL). The reaction mixture was reacted at 80°C for 16 hours, then cooled to room temperature, diluted with water (50 mL), and extracted with ethyl acetate (80 mL × 3). The organic phases were combined, dried over anhydrous sodium sulfate, subjected to suction filtration, and concentrated to obtain the target product A1.11-A (290 mg).

LCMS (ESI) [M+H]⁺: 464.0.

### Step 2:

Compound A1.11-A (93 mg) was dissolved in tetrahydrofuran (5 mL), then triphenylphosphine (78 mg) was added thereto, and the reaction mixture was reacted at room temperature for 4 hours. Then hydrochloric acid (4 M, 1 mL) was added to the reaction mixture, and the reaction mixture was heated to 55°C and reacted for 16 hours. The completion of the reaction was detected by LCMS. The reaction mixture was directly concentrated, and the crude product was purified through a reversed-phase column (mobile phase A was 0.05% formic acid in water, and B was acetonitrile) to obtain the target product A1.11 (28 mg, yield: 36%) as a white solid.
LCMS (ESI) [M+H]⁺: 438.4;
¹H NMR (400 MHz, CD₃OD) δ 8.52 (s, 1H), 8.16 (d, *J* = 7.8 Hz, 1H), 7.76 (d,*J* = 10.7 Hz, 1H), 7.63 (s, 1H), 5.49 (ABq, *J* = 78.9, 16.3 Hz, 2H), 5.31 (br s, 2H), 3.35 - 3.32 (m, 2H), 3.22 - 3.10 (m, 2H), 2.55 (s, 3H), 2.13 - 2.11 (m, 2H), 1.96 - 1.94 (m, 2H), 1.01 (t, *J* = 7.4 Hz, 3H).

### Example A1.12: Synthesis of (S,E)-14-(3-amino-1-propen-1-yl)-7-ethyl-7-hydroxy-10,13-dihydro-11H-[1,3]dioxolo[4,5-g]pyrano[3',4":6,7]indolizino[1,2-b]quinoline-8,11(7H)-dione (A1.12)

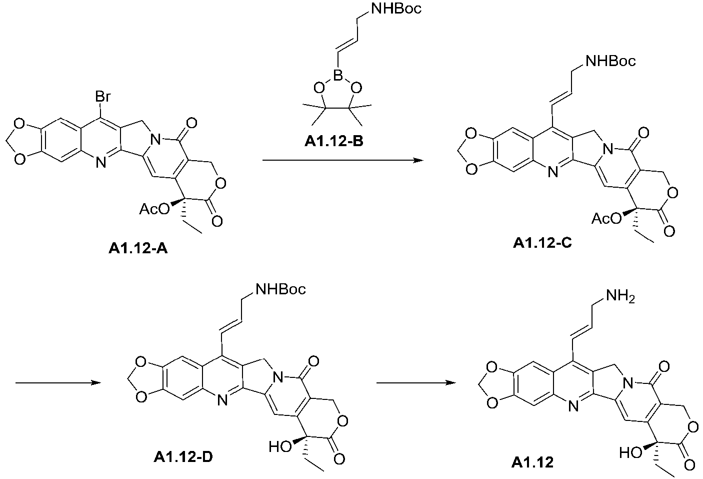

### Step 1:

Compound A1.12-A (200 mg, 0.39 mmol), compound A1.12-B (112 mg, 0.39 mmol), cesium fluoride (152 mg, 0.975 mmol), and tetrakis(triphenylphosphine)palladium (45 mg, 0.039 mmol) were added to 1,4-dioxane solution (8 mL). The reaction mixture was reacted under microwave irradiation at 120°C under nitrogen atmosphere for 0.5 hours. LCMS showed the completion of the reaction. The reaction mixture was diluted with a mixed solution of dichloromethane (20 mL) and methanol (10 mL) and filtered. The filtrate was concentrated, and the crude product was purified by preparative TLC (dichloromethane: methanol = 30:1) to obtain the target compound (*S,E*)-14-(3-((*tert*-butoxycarbonyl)amino)-1-propen-1-yl)-7-ethyl-8,11-dioxo-7,8,11,13-tetrahydro-10*H*-[1,3]dioxolo[4,5-*g*]pyrano[3',4':6,7]indolizino[1,2-*b*]quinolin-7-yl acetate (A1.12-C, 60 mg, yield: 26%) as a brown solid. LCMS (ESI) [M+H]⁺ = 590.3;
¹H NMR (400 MHz, DMSO-d6) δ 7.63 (s, 1H), 7.49 (s, 1H), 7.35 (s, 1H), 7.09 (d, *J* = 16.7 Hz, 1H), 6.93 (s, 1H), 6.45 (d, *J =* 16.6 Hz, 1H), 6.30 (s, 2H), 5.47 (s, 2H), 5.34 - 5.24 (m, 2H), 3.94 (s, 2H), 2.21 (br s, 3H), 2.03 - 1.96 (m, 2H), 1.45 (s, 9H), 0.91 (t, *J =* 6.7 Hz, 3H).

### Step 2:

Sodium methoxide (9.2 mg, 0.17 mmol) was added to a methanol solution (15 mL) of compound (*S*,*E*)-14-(3-((*tert*-butoxycarbonyl)amino)-1-propen-1-yl)-7-ethyl-8,11-dioxo-7,8,11,13-tetrahydro-10*H*-[1,3]dioxolo[4,5-*g*]pyrano[3',4':6,7]indolizino[1,2-*b*]quinolin-7-yl acetate (A1.12-C, 50 mg, 0.085 mmol), and the reaction mixture was stirred and reacted at 50°C for 2 hours. LCMS showed the completion of the reaction. The reaction mixture was concentrated to obtain the crude target compound (*S,E*)-14-(3-((*tert*-butoxycarbonyl)amino)-1-propen-1-yl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (A1.12-D, 50 mg) as a brown solid. LCMS (ESI) [M+H]⁺ = 548;

### Step 3:

Trifluoroacetic acid (1 mL) was added to a dichloromethane solution (2 mL) of compound (*S,E*)-14-(3-((*tert*-butoxycarbonyl)amino)-1-propen-1-yl)-7-ethyl-7-hydroxy-10,13-dihydro-11*H*-[1,3]dioxolo[4,5-*g*]pyrano[3',4':6,7]indolizino[1,2-*b*]quinoline-8,11(7iH)-dione (A1.12-D, 50 mg, 0.091 mmol), and the reaction mixture was stirred and reacted at room temperature for 30 minutes. LCMS showed the completion of the reaction. The reaction mixture was concentrated, and the crude product was purified by preparative high-performance liquid chromatography (acetonitrile/0.05% formic acid in water) to obtain the target compound (*S*,*E*)-14-(3-amino-1-propen-1-yl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (A1.12) (8.1 mg, yield: 21%) as a brown solid.
LCMS (ESI) [M+H]⁺ = 448.3;
¹H NMR (400 MHz, DMSO-d6) δ 8.20 (s, 2H), 7.72 (s, 1H), 7.54 (s, 1H), 7.40 (d, *J* = 16.5 Hz, 1H), 7.27 (s, 1H), 6.52 - 6.46 (m, 2H), 6.31 (s, 2H), 5.42 (s, 2H), 5.27 (s, 2H), 3.86 (br s, 2H), 1.90 - 1.83 (m, 2H), 0.88 (t, *J =* 7.1 Hz, 3H).

### Example A1.13: Synthesis of (S,E)-14-(3-hydroxy-1-propen-1-yl)-7-ethyl-7-hydroxy-10,13-dihydro-11H-[1,3]dioxolo[4,5-g]pyrano[3',4':6,7]indolizino[1,2-b]quinoline-8,11(7H)-dione (A1.13)

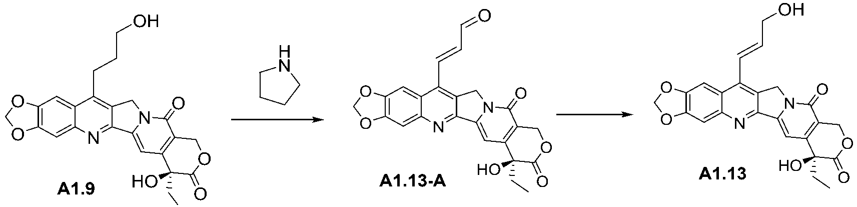

### Step 1:

Compound A1.9 (200 mg, 0.444 mmol) was dissolved in dimethyl sulfoxide (2 mL), and IBX (311 mg, 1.11 mmol) was added thereto. The mixture was stirred at room temperature for 2 hours, and then additional IBX (186 mg, 0.666 mmol) was added to the reaction mixture. Pyrrolidine (6.3 mg, 0.089 mmol) and acetonitrile (3 mL) were then added to the reaction mixture, and the reaction mixture was stirred at room temperature overnight. The completion of the reaction was detected by LCMS. The reaction mixture was extracted with ethyl acetate (20 mL × 3), and the organic phase was washed with saturated brine, dried over anhydrous sodium sulfate, subjected to suction filtration, and rotary evaporated to dryness to obtain a crude product. The crude product was purified by silica gel column chromatography (DCM: MeOH = 50: 1 to 10: 1) to obtain the target compound (*S,E*)-14-(3-oxo-1-propen-1-yl)-7-ethyl-7-hydroxy-10,13-dihydro-11*H*-[1,3]dioxolo[4,5-*g*]pyrano[3',4':6,7]indolizino[1,2-*b*]quinoline-8,11(7*H*)-dione (A1.13-A, 100 mg, purity: 50.0%, yield: 25.0%) as a yellow solid. LCMS (ESI) [M+H]⁺ = 447.1.

### Step 2:

Compound A1.13-A (40 mg, 0.09 mmol) was dissolved in tetrahydrofuran (1 mL), then sodium cyanoborohydride (28 mg, 0.448 mmol) was added thereto, and the reaction mixture was stirred at room temperature overnight. LCMS showed the completion of the reaction. The reaction mixture was concentrated to obtain a crude product, which was purified by prep-HPLC (HCl in water/MeCN) to obtain the target compound (3.56 mg, purity: 93.6%, yield: 9.0%) as a light yellow solid.
LCMS (ESI) [M+H]⁺ = 449.2;
1H NMR (400 MHz, DMSO-d6) δ 7.61 (s, 1H), 7.50 (s, 1H), 7.24 (s, 1H), 7.20 (d, *J* = 16.4 Hz, 1H), 6.69 - 6.59 (m, 1H), 6.49 (s, 1H), 6.30 (s, 2H), 5.42 (s, 2H), 5.26 (s, 2H), 5.16 (br s, 1H), 4.34 (br s, 2H), 1.93 - 1.81 (m, 2H), 0.88 (t, *J =* 7.3 Hz, 3H).

### Example A1.14: Synthesis of (S,E)-4-ethyl-8-fluoro-4-hydroxy-11-(3-hydroxy-1-propen-1-yl)-9-methyl-1,12-dihydro-14H-pyrano[3',4':6,7]indolizino[1,2-b]quinoline-3,14(4H)-dione (A1.14)

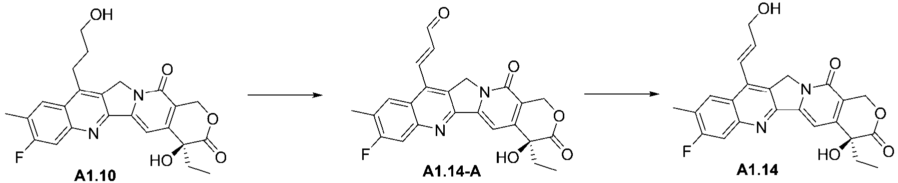

### Step 1:

Compound (*S*)-4-ethyl-8-fluoro-4-hydroxy-11-(3-hydroxypropyl)-9-methyl-1,12-dihydro-14*H*-pyrano[3',4':6,7]indolizino[1,2-*b*]quinoline-3,14(4*H*)-dione (A1.10, 100 mg, 0.228 mmol), DMSO (0.5 mL), and acetonitrile (0.75 mL) were added to a 25 mL single-neck flask, and then IBX (160 mg, 0.571 mmol) and (*R*)-diphenyl(pyrrolidin-2-yl)methanol (12 mg, 0.047 mmol) were sequentially added thereto. The reaction mixture was stirred and reacted at room temperature for 16 hours, and then the reaction mixture was added with a mixed solvent of methanol and dichloromethane (200 mL) (DCM: MeOH = 10:1) and extracted with saturated brine (300 mL). The organic phases were combined, washed with water, dried, and filtered. The filtrate was evaporated to dryness under reduced pressure to remove the solvent to obtain a crude product, which was purified by flash chromatography (DCM: MeOH = 10:1) to obtain the target compound (*S*)-4-ethyl-8-fluoro-4-hydroxy-11-(3-oxopropyl)-9-methyl-1,12-dihydro-14*H*-pyrano[3',4':6,7]indolo[1,2-*b*]quinoline-3,14(4*H*)-dione (A1.14-A, 40 mg, yield: 40%).

LCMS (ESI) [M+H]⁺ = 435.1.

### Step 2:

Compound (*S*)-4-ethyl-8-fluoro-4-hydroxy-11-(3-oxopropyl)-9-methyl-1,12-dihydro-14*H*-pyrano[3',4':6,7]indolo[1,2-*b*]quinoline-3,14(4*H*)-dione (A1.14-A, 40 mg, 0.228 mmol) and anhydrous THF (5 mL) were added to a 50 mL three-necked flask, and the mixture was cooled to -78°C under N₂ atmosphere. A THF solution of lithium tri-sec-butylborohydride (0.11 mL, 1 N) was slowly added dropwise thereto, and the reaction mixture was kept at -78°C and stirred for 1 hour. After the reaction was completed, saturated ammonium chloride aqueous solution was added to quench the reaction. The resulting mixture was warmed to room temperature, diluted with saturated brine (50 mL), and extracted with ethyl acetate (20 mL × 3). The combined organic phases were washed with water, dried, filtered, and evaporated to dryness under reduced pressure to remove the solvent to obtain (*S*,*E*)-4-ethyl-8-fluoro-4-hydroxy-11-(3-hydroxy-1-propen-1-yl)-9-methyl-1,12-dihydro-14*H-*pyrano[3',4':6,7]indolizino[1,2-*b*]quinoline-3,14(4*H*)-dione (A1.14, 28 mg) as an off-white solid.

LCMS (ESI) [M+H]⁺ = 437.1.

### Example A1.15: Synthesis of (S)-7-ethyl-7-hydroxy-14-(2-(n-propylamino)ethyl)-10,13-dihydro-11H-[1,3]dioxolo[4,5-g]pyrano[3',4':6,7]indolizino[1,2-b]quinoline-8,11(7H)-dione (A1.15a) and (S)-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)-2-hydroxy-N-propylacetamide (A1.15b)

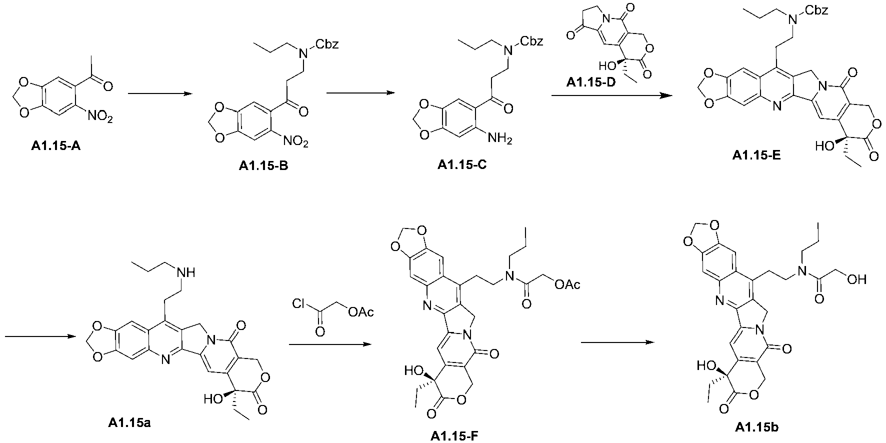

### Step 1:

Compound A1.15-A (5.0 g, 23.9 mmol), propylamine hydrochloride (22.8 g, 239 mmol), and paraformaldehyde (7.18 g, 239 mmol) were dissolved in ethanol (100 mL), and the reaction mixture was reacted in a sealed vessel at 110°C for 12 hours. The completion of the reaction was detected by LCMS. The reaction mixture was cooled to room temperature and concentrated under reduced pressure. Dichloromethane (100 mL) was added to the reaction solid, washed with water (80 mL × 3), and the pH of the aqueous phase was adjusted to 9 with sodium bicarbonate. Benzyloxycarbonyl chloride (3.7 g, 21.8 mmol) was added to the aqueous solution and reacted at room temperature for 2 hours. The completion of the reaction was detected by LCMS. The reaction mixture was extracted with dichloromethane (100 mL × 3). The organic phase was washed with saturated brine, dried over anhydrous sodium sulfate, subjected to suction filtration, and concentrated to obtain a crude product. The crude product was purified by column chromatography (petroleum ether: ethyl acetate = 10/1) to obtain the target compound A1.15-B (1.5 g, yield: 20.1%) as a yellow oily liquid.
LCMS (ESI) [M+H]⁺ = 415.1;
1H NMR (400 MHz, CDCl₃) δ 7.56 (s, 1H), 7.35 (m, 6H), 6.18 (s, 2H), 5.17 - 5.12 (m, 2H), 3.69 - 3.67 (m, 2H), 3.32 - 3.30 (m, 2H), 3.09 - 2.90 (m, 2H), 1.59 (m, 2H), 0.90 (m, 3H).

### Step 2:

Compound A1.15-B (1.5 g, 3.63 mmol) was dissolved in a mixed solution of saturated ammonium chloride (20 mL) and ethanol (20 mL), then iron powder (1.02 g, 18.1 mmol) was added thereto, and the reaction mixture was stirred at 80°C for 1 hour. LCMS showed the completion of the reaction. The reaction mixture was filtered, then the filtrate was concentrated under reduced pressure to a solid, and the crude product was purified by reversed-phase C18 column chromatography (acetonitrile/0.05% FA in water: 5% to 55%) to obtain the target compound A1.15-C (600 mg, yield: 43.0%) as a yellow solid.

LCMS (ESI) [M+H]⁺ = 385.2, t_{R} = 1.329 min.

### Step 3:

Compound A1.15-C (350 mg, 0.91 mmol), compound (S)-4-ethyl-4-hydroxy-7,8-dihydro-1*H*-pyrano[3,4-*f*]indolizine-3,6,10(4*H*)-trione (A1.15-D, 218 mg, 0.83 mmol), and*p-*toluenesulfonic acid (170 mg, 0.87 mmol) were dissolved in a dichloromethane (5 mL) solution at room temperature. The resulting solution was clarified and mixed homogeneously, then concentrated under reduced pressure, and evacuated to vacuum with an oil pump. The reaction mixture was reacted at 120°C under vacuum for 2 hours. LCMS showed the completion of the reaction. The reaction mixture was cooled to room temperature, added with water (50 mL), and extracted with dichloromethane (30 mL × 3). The combined organic phases were sequentially dried over anhydrous sodium sulfate and filtered. The filtrate was concentrated under reduced pressure to obtain compound A1.15-E (390 mg, yield: 76%) as a brown solid.

LCMS (ESI) [M+H]⁺ = 612.0.

### Step 4:

Compound A1.15-E (390 mg, 0.638 mmol) was dissolved in a dichloromethane (5 mL) solution at room temperature, and trimethylsilyl iodide (510 mg, 2.55 mmol) was added dropwise thereto under nitrogen atmosphere at 0°C. The reaction system was stirred at room temperature for 2 hours. Diethyl ether (2 mL) and concentrated hydrochloric acid (4 mL) were added to the reaction mixture, stirred for 30 minutes, and the pH of the reaction system was adjusted to 9 with saturated sodium bicarbonate. The resulting mixture was extracted with dichloromethane (50 mL × 5), then the combined organic phases were dried over anhydrous sodium sulfate, filtered, and the filtrate was concentrated under reduced pressure to obtain a crude product. The crude product was purified by preparative high-performance liquid chromatography (acetonitrile/0.05% formic acid in water) to obtain compound A1.15a (200 mg, yield: 66.1%) as an off-white solid.
LCMS (ESI) [M+H]⁺ = 478.2;
1H NMR (400 MHz, DMSO-d6) δ 8.30 (s, 1H, HCO₂H), 7.67 (s, 1H), 7.51 (s, 1H), 7.23 (s, 1H), 6.62 - 6.41 (m, 1H), 6.29 (s, 2H), 5.42 (s, 2H), 5.28 (s, 2H), 3.27 - 3.25 (m, 2H), 2.91 - 2.79 (m, 2H), 2.58 - 2.56 (m, 2H), 1.91 - 1.79 (m, 2H), 1.44 - 1.42 (m, 2H), 0.90 - 0.84 (m, 6H).

### Step 5:

Compound A1.15a (120 mg, 0.251 mmol) was dissolved in dichloromethane (5 mL) at room temperature, and compound acetoxyacetyl chloride (167 mg, 1.25 mmol) and triethylamine (133 mg, 1.25 mmol) were added dropwise thereto at 0°C, and the reaction mixture was stirred and reacted at 0°C for 30 minutes. The completion of the reaction was detected by LCMS. The reaction mixture was concentrated under reduced pressure to obtain crude compound A1.15-F (120 mg, yield: 82.8%) as a yellow solid. LCMS (ESI) [M+H]⁺ = 578.3.

### Step 6:

Compound A1.15-F (120 mg, 0.207 mmol) was dissolved in ethanol (4 mL) at room temperature, then concentrated hydrochloric acid (2 mL) was added thereto, and the reaction mixture was stirred and reacted at 70°C for 1 hour. The completion of the reaction was detected by LCMS. The reaction mixture was added with water (20 mL) and extracted with dichloromethane (30 mL × 5). The combined organic phases were sequentially dried over anhydrous sodium sulfate and filtered. The filtrate was concentrated under reduced pressure to obtain a crude product. The crude product was purified by preparative high-performance liquid chromatography (acetonitrile/0.05% formic acid in water) to obtain compound A1.15b (20 mg, yield: 18.1%) as an off-white solid.
LCMS (ESI) [M+H]⁺ = 536.2;
1H NMR (400 MHz, DMSO-d6) δ 7.93 (s, 0.7H), 7.69 (s, 0.3H), 7.51 (s, 1H), 7.24 (s, 1H), 6.49 (s, 1H), 6.30 (s, 2H), 5.42 (s, 2H), 5.35 (s, 1.5H), 5.29 (s, 0.5H), 4.65 - 4.62 (m, 1H), 4.13 - 3.97 (m, 2H), 3.51 - 3.49 (m, 2H), 3.33 - 3.30 (m, 2H), 3.24 - 3.20 (m, 2H), 1.86 - 1.84 (m, 2H), 1.60 - 1.52 (m, 2H), 0.89 - 0.86 (m, 6H).

### Example A1.16: Synthesis of (S)-4-ethyl-8-fluoro-4-hydroxy-11-(3-isopropylaminopropyl)-9-methyl-1,12-dihydro-14H-pyrano[3',4':6,7]indolizino[1,2-b]quinoline-3,14(4H)-dione (A1.16)

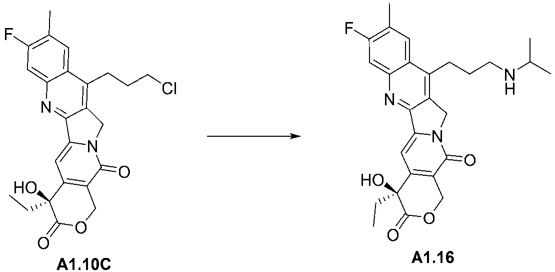

Diisopropylethylamine (170 mg, 1.32 mmol) and sodium iodide (99 mg, 0.659 mmol) were added to a DMF solution (10 mL) of A1.10-C (200 mg, 0.439 mmol) and isopropylamine (50 mg, 0.877 mmol), and the reaction mixture was reacted at 50°C in a sealed tube for 2 hours. The reaction mixture was added with ethyl acetate. The organic phase was washed with saturated brine, dried over anhydrous sodium sulfate, subjected to suction filtration, and the filtrate was concentrated under reduced pressure to obtain a crude product. The crude product was purified by pre-TLC (dichloromethane: methanol = 10:1) to obtain a yellow solid. The compound was further purified by preparative high-performance liquid chromatography (acetonitrile/0.05% formic acid in water) to obtain the target compound (*S*)-4-ethyl-8-fluoro-4-hydroxy-11-(3-isopropylaminopropyl)-9-methyl-1,12-dihydro-14*H-*pyrano[3',4':6,7]indolizino[1,2-b]quinoline-3,14(4*H*)-dione (A1.16, 2.03 mg).
LCMS (ESI) [M+H]⁺ = 480.2;
¹H NMR (400 MHz, DMSO-d6) δ 8.28 (d, *J* = 8.0 Hz, 1H), 8.27 (s, 1H, HCO2H) 7.92 (d, *J* = 11.0 Hz, 1H), 7.33 (s, 1H), 6.53 (s, 1H), 5.45 (s, 2H), 5.30 (s, 2H), 33.30 - 3.22 (m, 3H), 3.18 - 3.10 (m, 2H), 2.54 (s, 3H), 2.06 - 1.94 (m, 2H), 1.93 - 1.80 (m, 2H), 1.26 - 1.20 (m, 6H), 0.88 (t, *J =* 7.3 Hz, 3H).

### Example A1.17: Synthesis of (S)-4-ethyl-8-fluoro-4-hydroxy-11-(3-cyclopropylaminopropyl)-9-methyl-1,12-dihydro-14H-pyrano[3',4':6,7]indolizino[1,2-b]quinoline-3,14(4H)-dione (A1.17)

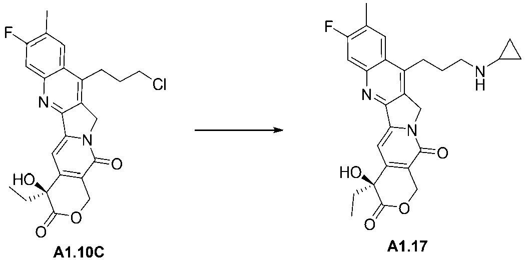

Diisopropylethylamine (165 mg, 1.28 mmol) and sodium iodide (96 mg, 0.640 mmol) were added to a DMF solution (10 mL) of A1.10C (200 mg, 0.439 mmol) and cyclopropylamine (52 mg, 0.881 mmol), and the reaction mixture was reacted at 50°C in a sealed tube for 2 hours. The completion of the reaction was detected by LCMS. The reaction mixture was added with ethyl acetate. The organic phase was washed with saturated brine, dried over anhydrous sodium sulfate, subjected to suction filtration, and the filtrate was concentrated under reduced pressure to obtain a crude product. The crude product was purified by TLC (dichloromethane: methanol = 10:1) and then further purified by preparative high-performance liquid chromatography (acetonitrile/0.05% formic acid in water) to obtain the target compound (*S*)-4-ethyl-8-fluoro-4-hydroxy-11-(3-cyclopropylaminopropyl)-9-methyl-1,12-dihydro-14*H*-pyrano[3',4':6,7]indolizino[1,2-*b*]quinoline-3,14(4*H*)-dione (A1.17, 5.12 mg).
LCMS (ESI) [M+H]⁺ = 478.2;
¹H NMR (400 MHz, DMSO-d6) δ 8.22 (br s, 2H, one is HCO2H), 7.86 (br s, 1H), 7.30 (s, 1H), 6.51 (s, 1H), 5.43 (s, 2H), 5.28 (s, 2H), 3.24 - 3.19 (m, 2H), 2.75 - 2.68 (m, 2H), 2.49 (s, 3H), 2.16 - 2.09 (m, 1H), 1.95 - 1.76 (m, 4H), 0.91 - 0.83(t, *J* = 7.3 Hz, 3H), 0.43 - 0.33 (m, 2H), 0.31 - 0.21 (m, 2H).

### Example A1.18: Synthesis of (S)-4-ethyl-8-fluoro-4-hydroxy-11-(4-aminophenyl)-9-methyl-1,12-dihydro-14H-pyrano[3',4':6,7]indolizino[1,2-b]quinoline-3,14(4H)-dione (A1.18)

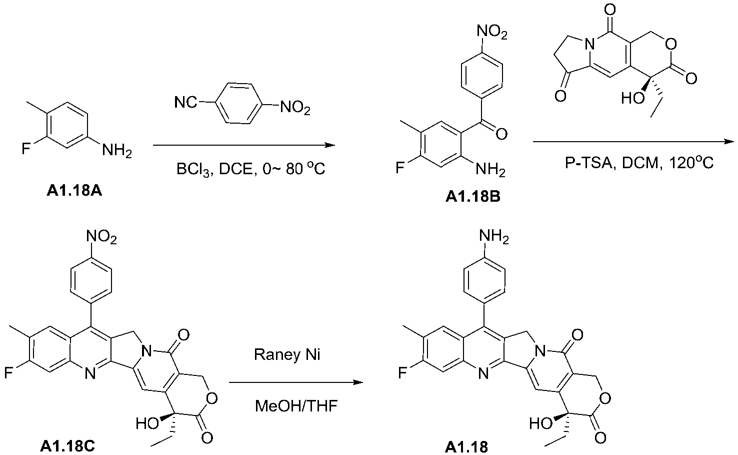

### Step 1:

Under nitrogen atmosphere, 3-fluoro-4-methylaniline (A1.18A, 2.0 g, 16.3 mmol) was dissolved in 1,2-dichloroethane (40 mL). Boron trichloride (19.2 mL, 19.2 mmol) was added dropwise thereto at 0°C, and then p-nitrobenzonitrile (2.8 g, 19.2 mmol) was slowly added thereto. After the addition, the reaction mixture was heated to 80°C and stirred overnight. The reaction mixture was cooled to room temperature, added with hydrochloric acid (40 mL, 2 M), then heated to 80°C and stirred for another 30 minutes. After the completion of the reaction was detected by LCMS, the reaction mixture was added with water (80 mL) and extracted with dichloromethane (100 mL × 3). The combined organic phases were dried over anhydrous sodium sulfate, filtered, and rotary evaporated to dryness. The crude product was purified by silica gel column chromatography (PE: EA = 10:1) to obtain the target compound (A1.18B, 2.0 g, yield: 46.5%).
LCMS (ESI) [M+H]⁺ = 275.1;
¹H NMR (400 MHz, DMSO) δ 8.34 (d, *J =* 8.6 Hz, 2H), 7.77 (d, *J* = 8.6 Hz, 2H), 7.39 (s, 2H), 7.11 (d, *J* = 8.8 Hz, 1H), 6.63 (d, *J =* 12.4 Hz, 1H), 2.00 (s, 3H).

### Step 2:

Compound A1.18B (620 mg, 2.28 mmol), (S)-4-ethyl-4-hydroxy-7,8-dihydro-1H-pyrano[3,4-*f*]indolizine-3,6,10(4*H*)-trione (600 mg, 2.28 mmol), and *p*-toluenesulfonic acid (560 mg, 2.96 mmol) were added to a reaction flask, and the mixture was evenly dissolved in dichloromethane. The dichloromethane was rotary evaporated to dryness, then the residue was evacuated to vacuum, heated to 120°C under vacuum, and maintained at this temperature for six hours. After the completion of the reaction was detected by LCMS, methanol (10 mL) was added to the system, and then water (80 mL) was added thereto, resulting in a large amount of precipitation. The precipitate was filtered and dried to obtain the target compound (A1.18C, 800 mg, yield: 80.0%) as a yellow solid.
LCMS (ESI) [M+H]⁺ = 502.2;
¹H NMR (400 MHz, DMSO) δ 8.52 (d, *J* = 8.2 Hz, 2H), 8.13 - 7.89 (m, 3H), 7.64 (d, *J =* 8.4 Hz, 1H), 7.37 (d, *J =* 13.4 Hz, 1H), 6.54 (s, 1H), 5.41 (s, 2H), 5.06 (m, 2H), 2.40 (s, 3H), 1.94 - 1.84 (m, 2H), 0.87 (m, 3H).

### Step 3:

Raney nickel (609 mg, 10.5 mmol) was added to a mixed solution of compound A1.18C (1.0 g, 2.1 mmol) in methanol (25 mL) and tetrahydrofuran (50 mL), and the reaction mixture was stirred under hydrogen atmosphere at room temperature for 5 hours. The completion of the reaction was detected by LCMS. The reaction mixture was filtered and the filtrate was rotary evaporated to dryness to obtain the target compound (*S*)-4-ethyl-8-fluoro-4-hydroxy-11-(4-aminophenyl)-9-methyl-1,12-dihydro-14*H*-pyrano[3',4':6,7]indolizino[1,2-b]quinoline-3,14(4*H*)-dione (A1.18, 650 mg, yield: 67%) as a yellow solid.
LCMS (ESI) [M+H]⁺ = 472.3;
¹H NMR (400 MHz, DMSO) δ 7.93 - 7.81 (m, 2H), 7.40 - 7.25 (m, 3H), 6.81 (d,*J* = 8.2 Hz, 2H), 6.51 (s, 1H), 5.62 (s, 2H), 5.41 (s, 2H), 5.10 (s, 2H), 2.41 (s, 3H), 1.93 - 1.80 (m, 2H), 0.88 (t, *J =* 7.2 Hz, 3H).

### B. Synthesis of intermediates containing bioactive molecule fragments

### Example B1.1: Synthesis of (S)-2-amino-N-((4-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)butoxy)methyl)acetamide (B1.1)

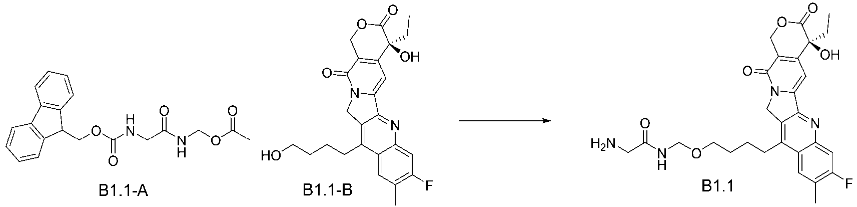

Compound (B1.1-A) (368 mg), compound (B1.1-B) (452 mg), and pyridinium p-toluenesulfonate (PPTS) (25 mg) were refluxed in dichloromethane (20 mL) for 20 hours, then washed with sodium bicarbonate aqueous solution and hydrochloric acid aqueous solution, respectively. The organic solvent was removed under reduced pressure, and the residue was dissolved in DMF (5 mL). Piperidine (1 mL) was added thereto, and the compound was stirred for 20 minutes and concentrated under reduced pressure to remove most of the low-boiling components. The residue was separated by preparative HPLC to obtain the target product (*S*)-2-amino-*N*-((4-(4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)butoxy)methyl)acetamide (B1.1).

ESI-MS (m/z): 539 [M+H]⁺.

### Example B1.2: Synthesis of (S)-2-((2-aminoacetamido)methoxy)-N-ethyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide (B1.2)

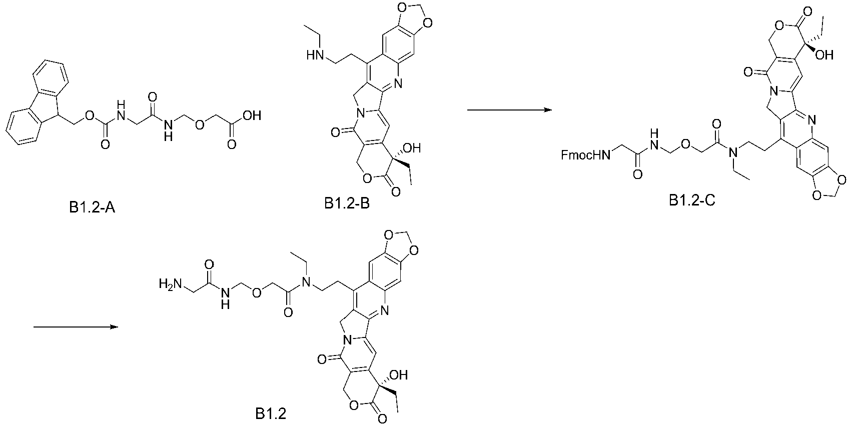

### Step 1:

Compound B1.2-A (274 mg), diisopropylethylamine (334 mg), and HBTU (369 mg) were sequentially added to *N,N-*dimethylformamide (10 mL), followed by addition of compound B1.2-B (300 mg). The reaction mixture was stirred at room temperature for 2 hours. The reaction mixture was added with ethyl acetate (50 mL), washed with saturated brine (30 mL × 3), dried over anhydrous sodium sulfate, subjected to suction filtration, and the filtrate was concentrated under reduced pressure to obtain a solid. The crude product was purified by column chromatography (dichloromethane: methanol = 10/1) to obtain the target compound B1.2-C (300 mg).
LCMS (ESI) [M+H]⁺: 830.2;
¹H NMR (400 MHz, DMSO-d6) δ 8.74 (s, 1H), 7.91 - 7.84 (m, 2H), 7.80 - 7.67 (m, 2H), 7.65 - 7.54 (m, 2H), 7.53 - 7.46 (m, 1H), 7.45 - 7.37 (m, 2H), 7.36 - 7.27 (m, 2H), 7.25 - 7.23 (m, 1H), 6.57 - 6.44 (m, 1H), 6.29 (s, 2H), 5.50 - 5.19 (m, 4H), 4.71 - 4.57 (m, 2H), 4.32 - 3.97 (m, 7H), 3.80 - 3.53 (m, 4H), 3.20 - 3.14 (m, 2H), 1.92 - 1.80 (m, 2H), 1.28 - 1.22 (m, 3H), 0.87 - 0.82 (m, 3H).

### Step 2:

Piperidine (1 mL) was added to a solution of compound B1.2-C (300 mg) in *N,N-*dimethylformamide (4 mL), and the reaction mixture was stirred at room temperature for 20 minutes. The target product was obtained by removing the low-boiling components from the reaction mixture and was used directly in the next step of synthesis.

LCMS (ESI) [M+H]⁺ = 608.0.

### Example B1.3: Synthesis of (S)-2-amino-N-((2-(((4-ethyl-8-fluoro-4-hydroxy-9-methyl-3,14-dioxo-3,4,12,14-tetrahydro-1H-pyrano[3',4':6,7]indolizino[1,2-b]quinolin-11-yl)methyl)amino)-2-oxoethoxy)methyl)acetamide (B1.3)

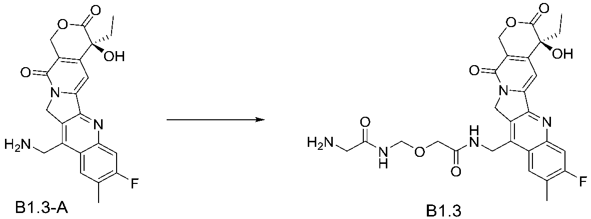

Using the same method and reaction conditions as in Example B1.1, compound (B1.3-A) was used instead of compound (B1.2-B) to obtain the target product (*S*)-2-amino-*N*-((2-(((4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)methyl)amino)-2-oxoethoxy)methyl)acetamide (B1.3).

ESI-MS (m/z): 554 [M+H]⁺.

### Example B1.4: Synthesis of (S)-2-amino-N-((4-ethyl-8-fluoro-4-hydroxy-9-methyl-3,14-dioxo-3,4,12,14-tetrahydro-1H-pyrano[3',4':6,7]indolizino[1,2-b]quinolin-11-yl)methyl)acetamide (B1.4)

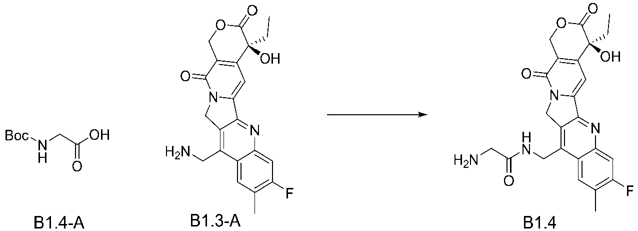

DIPEA (200 µL) and HBTU (420 mg) were added to a DMF (5 mL) solution of compound (B1.4-A) (175 mg) and compound (B1.3-A) (409 mg). The compounds were stirred and reacted at room temperature for 20 hours. The mixture was added with ethyl acetate (100 mL), washed with water (100 mL × 3), and the organic solvent was removed under reduced pressure. Then a mixture of DCM and TFA at a ratio of 1:1 (10 mL) was added to the residue and the mixture was left at room temperature for 20 minutes. The low-boiling components were removed under reduced pressure, and the residue was separated by preparative HPLC to obtain the target product (*S*)-2-amino-*N*-((4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)methyl)acetamide (B1.4).

ESI-MS (m/z): 467 [M+H]⁺.

### Example B1.5: Synthesis of (S)-2-amino-N-((7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)acetamide (B1.4)

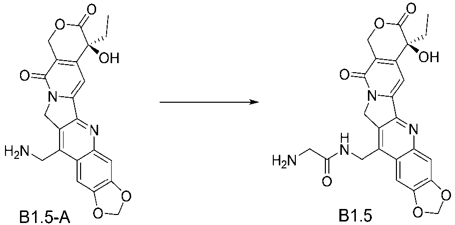

B1.5-A (200 mg) was dissolved in *N,N*-dimethylformamide (5 mL), and *N,N-*diisopropylethylamine (154.80 mg) and 2,5-dioxopyrrolidin-1-yl (*tert-*butyloxycarbonyl)glycine (157 mg) were sequentially added to the reaction mixture. The reaction mixture was stirred at 25°C for 60 minutes. The reaction mixture was added with water (30 mL), subjected to suction filtration, and the filter cake was dried to obtain a white solid product (ESI-MS (m/z): 579.4 [M+H]⁺). The above white solid product was dissolved in a mixed solution of trifluoroacetic acid and dichloromethane (volume ratio of 1:3; 4 mL), and the reaction mixture was stirred at room temperature for 1 hour. The reaction mixture was then concentrated to obtain a crude product, which was purified by reversed-phase C18 column chromatography (acetonitrile/0.05% formic acid in water: 5% to 50%) to obtain the target product (*S*)-2-amino-*N*-((7-ethyl-7-hydroxy-8,11-dioxo-7,8,11,13-tetrahydro-10*H-*[1,3]dioxolo[4,5-g]pyrano[3',4':6,7]indolizino[1,2-b]quinolin-14-yl)methyl)acetamide (B1.5, 110 mg).

ESI-MS (m/z): 479.3 [M+H]⁺.

### Example B1.6: Synthesis of (S)-2-amino-N-((4-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-1H-pyrazol-1-yl)methyl)acetamide (B1.6)

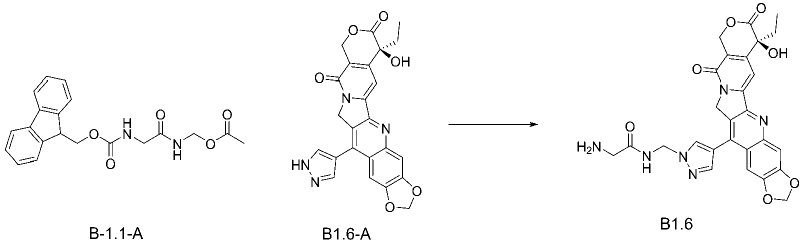

Using the same method and reaction conditions as in Example B1.1, compound (B1.6-A) was used instead of compound (B1.1-B) to obtain the target product (*S*)-2-amino-*N*-((4-(7-ethyl-7-hydroxy-8,11-dioxo-7,8,11,13-tetrahydro-10*H*-[1,3]dioxolo[4,5-g]pyrano[3',4':6,7]indolizino[1,2-b]quinolin-14-yl)-1H-pyrazol-1-yl)methyl)acetamide (B1.6).

ESI-MS (m/z): 545 [M+H]⁺.

### Example B1.7: Synthesis of (S)-2-amino-N-((2-(((7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethoxy)methyl)acetamide (B1.7)

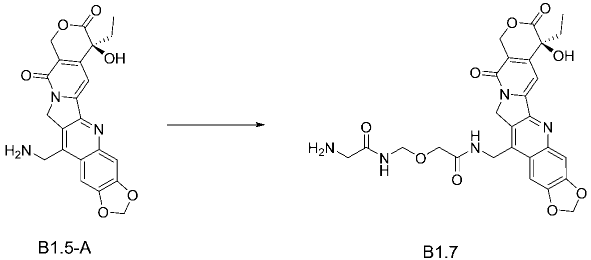

Using the same method and reaction conditions as in Example B1.2, compound (B1.5-A) was used instead of compound (B1.2-B) to obtain a mixture containing the target product (*S*)-2-amino-*N*-((2-(((7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)methyl)amino)-2-oxoethoxy)methyl)acetamide (B1.7), and the above mixture was directly used in the following synthesis reaction.

ESI-MS (m/z): 566 [M+H]⁺.

### Example B1.8: Synthesis of (S)-2-amino-N-(3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)phenyl)acetamide (B1.8)

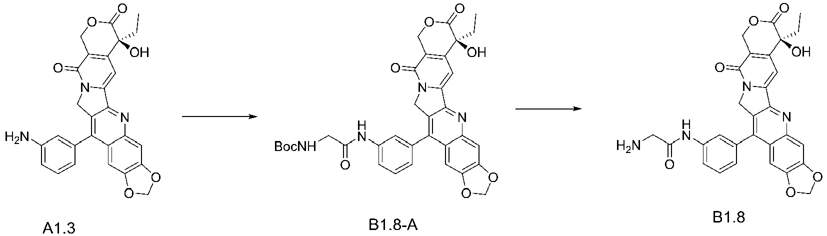

Using the same method and reaction conditions as in Example B1.4, compound (A1.3) was used instead of compound (B1.3-A) to obtain the target product (*S*)-2-amino-*N*-(3-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)phenyl)acetamide (B1.8).

ESI-MS (m/z): 541 [M+H]⁺.

### Example B1.9: Synthesis of (S)-2-amino-N-(4-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)phenyl)acetamide (B1.9)

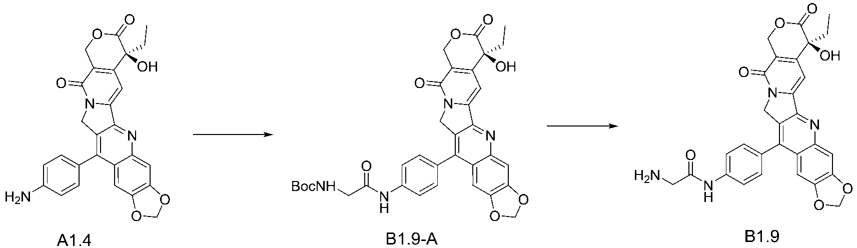

Using the same method and reaction conditions as in Example B1.4, compound (A1.4) was used instead of compound (B1.3-A) to obtain the target product (*S*)-2-amino-*N*-(4-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)phenyl)acetamide (B1.9). Alternatively, the target product was obtained using the following reaction conditions.

Compound A1.4 (60 mg, 0.12 mmol) was dissolved in *N,N*-dimethylformamide (3 mL), then (*tert*-butyloxycarbonyl)glycine (26 mg, 0.15 mmol), HATU (56 mg, 0.15 mmol), and *N,N*-diisopropylethylamine (48 mg, 0.37 mmol) were sequentially added thereto, and the reaction mixture was stirred at room temperature for 1 hour. The completion of the reaction was detected by TLC. TFA (1.0 mL) was then added directly to the above reaction mixture. The reaction mixture was stirred at room temperature for another 1 hour, and the completion of the reaction was detected by LCMS. The reaction mixture was concentrated to remove trifluoroacetic acid to obtain a crude product, which was purified by preparative chromatography (0.01% trifluoroacetic acid in water, acetonitrile) to obtain the target product (*S*)-2-amino-*N*-(4-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)phenyl)acetamide (B1.9) (26.3 mg, yield: 38%) as a yellow solid.
ESI-MS (m/z): 541 [M+H]⁺;
¹H NMR (400 MHz, DMSO) δ 10.71 (s, 1H), 8.16 (s, 2H), 7.86 (d, *J* = 8.6 Hz, 2H), 7.67-7.58 (m, 3H), 7.29 (s, 1H), 7.04 (s, 1H), 6.50 (s, 1H), 6.28 (s, 2H), 5.40 (s, 2H), 5.05 (s, 2H), 3.87 (s, 2H), 1.93-1.81 (m, 2H), 0.88 (t, *J* = 7.3 Hz, 3H).

### Example B1.10: Synthesis of (S)-2-amino-N-(3-(7-ethyl-7-hydroxy-8,11-dioxo-7,8,11,13-tetrahydro-10H-[1,3]-dioxolo[4,5-g]pyrano[3',4':6,7]indolizino[1,2-b]quinolin-14-yl)propyl)acetamide (B1.10)

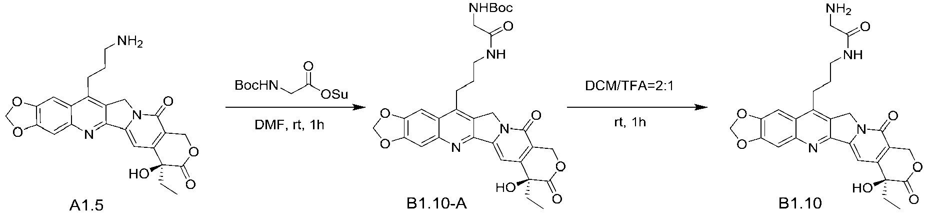

### Step 1:

Triethylamine (67 mg) and 2,5-dioxopyrrolidin-1-yl (*tert*-butoxycarbonyl)glycinate (182 mg) were added to a solution of compound A1.5 (200 mg) in *N,N*-dimethylformamide (5 mL) at room temperature, and the reaction mixture was reacted at room temperature for 1 hour. The reaction mixture was diluted with water (30 mL), extracted with ethyl acetate (30 mL × 2), and the organic phase was washed with saturated brine, dried over anhydrous sodium sulfate, and concentrated to obtain a crude product. The crude product was further purified by C18 column chromatography (acetonitrile/0.05% formic acid in water: 5% to 60%) to obtain the target compound (B1.10-A, 100 mg).

LCMS (ESI) [M+H]⁺: 607.

### Step 2:

Trifluoroacetic acid (2 mL) was added to a dichloromethane solution (4 mL) of compound B1.10-A (100 mg), and the mixture was stirred and reacted at room temperature for 1 hour. The reaction mixture was concentrated, and the crude product was added with *N,N-*dimethylformamide solution (3 mL) and further purified by C18 column chromatography (acetonitrile/0.05% formic acid in water: 5% to 60%) to obtain the target compound (*S*)-2-amino-*N*-(3-(7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)propyl)acetamide (B1.10, 80 mg).
LCMS (ESI) [M+H]⁺: 507;
¹H NMR (400 MHz, DMSO-d6) δ 8.26 (s, 1H), 7.64 (s, 1H), 7.51 (s, 1H), 7.24 (s, 1H), 6.51 (s, 1H), 6.29 (s, 2H), 5.42 (s, 2H), 5.23 (s, 2H), 3.31 (s, 2H), 3.30 - 3.27 (m, 2H), 3.13 - 3.07 (m, 2H), 2.04 - 1.76 (m, 4H), 0.87 (t, *J =* 7.3 Hz, 3H).

### Example B1.11: Synthesis of (S)-2-((2-aminoacetamido)methoxy)-N-isopropyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide (B1.11)

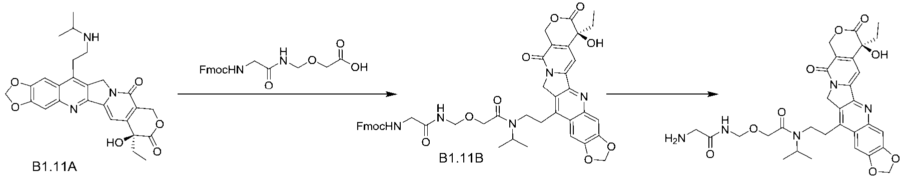

### Step 1:

Compound 1-(9*H*-fluoren-9-yl)-3,6-dioxo-2,9-dioxo-4,7-diazacyclo-11-carboxylic acid (242 mg), diisopropylethylamine (330 mg), and *N,N,N',N'*-tetramethyluronium hexafluorophosphate (359 mg) were dissolved in N,N-dimethylformamide (10 mL) solution. Compound (*S*)-7-ethyl-7-hydroxy-14-(2-(isopropylamino)ethyl)-10,13-dihydro-11*H-*[1,3]dioxolo[4,5-*g*]pyrano[3',4':6,7]indolizino[1,2-*b*]quinoline-8,11(7*H*)-dione (300 mg) was then added thereto, and the reaction mixture was reacted at room temperature for 2 hours. The reaction mixture was added with ethyl acetate (50 mL), washed with saturated brine (30 mL × 3), dried over anhydrous sodium sulfate, subjected to suction filtration, and the filtrate was concentrated under reduced pressure to obtain a solid. The crude product was purified by column chromatography (dichloromethane: methanol = 10/1) to obtain the target compound B1.11B (220 mg).

LCMS (ESI) [M+H]⁺: 844.0.

### Step 2: Preparation of (S)-2-((2-aminoacetamido)methoxy)-N-isopropyl-N-(2-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)acetamide

Piperidine (1 mL) was added to a solution of compound B1.11B (220 mg, 0.261 mmol) in *N,N*-dimethylformamide (4 mL), and the reaction mixture was stirred at room temperature for 20 minutes. The completion of the reaction was detected by LCMS. The reaction mixture was concentrated under reduced pressure to obtain the target compound (220 mg) as a brown solid, and the above product was used directly in the next step of synthesis without purification.

LCMS (ESI) [M+H]⁺: 622.1.

### Example B1.12: Synthesis of (S)-2-amino-N-((4-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)acetamide (B1.12)

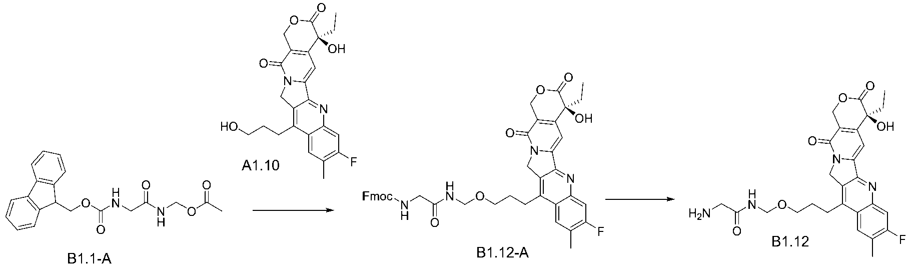

### Step 1:

Compound A1.10 (160 mg, 0.365 mmol) was dissolved in *N,N*-dimethylformamide (3 mL). (2-((((9*H*-Fluoren-9-yl)methoxy)carbonyl)amino)acetamido)methyl acetate (B1.1-A, 672 mg, 1.83 mmol) was added thereto, and then ethyl acetate hydrochloride (0.073 mL, 3 M) was added to the reaction mixture, and the reaction mixture was stirred at room temperature overnight. The completion of the reaction was detected by LCMS. The reaction mixture was directly purified by reversed-phase chromatography (acetonitrile/0.05% FA in water: 5% to 50%) to obtain the target compound (80 mg, yield: 29.0%) as a white solid.
LCMS (ESI) [M+H]⁺ = 747.4;
¹H NMR (400 MHz, DMSO-d6) δ 8.74 (t, *J* = 6.4 Hz, 1H), 8.28 - 8.17 (m, 1H), 7.99 - 7.88 (m, 3H), 7.73 (d, *J =* 7.3 Hz, 2H), 7.63 (t, *J =* 5.7 Hz, 1H), 7.44 (t, *J =* 7.4 Hz, 2H), 7.35 (t, *J* = 7.2 Hz, 3H), 6.58 (s, 1H), 5.48 (s, 2H), 5.29 (s, 2H), 4.66 (d, *J* = 6.3 Hz, 2H), 4.32 (d, *J* = 6.9 Hz, 2H), 4.26 (d, *J =* 6.1 Hz, 1H), 3.71 (d, *J* = 5.8 Hz, 2H), 3.57 (t, *J* = 5.7 Hz, 2H), 3.29 - 3.20 (m, 2H), 2.55 (s, 3H), 2.00 - 1.86 (m, 4H), 0.93 (t, *J=* 7.2 Hz, 3H).

### Step 2: B1.12-A (240 mg) was dissolved in DMF (5 mL), and piperidine (1 mL) was added thereto. The compound was stirred for 20 minutes, dissolved under reduced pressure to remove the low-boiling components, and the residue was used directly in the next step of synthesis.

### ESI-MS (m/z): 525.2 [M+H]⁺.

A small amount of the crude product was purified by reversed-phase chromatography (acetonitrile/0.05% FA in water: 5% to 50%) to obtain the target compound.
ESI-MS (m/z): 525.1 [M+H]⁺;
¹H NMR (400 MHz, DMSO) δ 9.13 (t, *J* = 6.6 Hz, 1H), 8.21 (d, *J* = 8.1 Hz, 1H), 8.02 (brs, 2H), 7.89 (d, *J* = 10.8 Hz, 1H), 7.32 (s, 1H), 6.54 (s, 1H), 5.44 (s, 2H), 5.28 (s, 2H), 4.66 (d, *J =* 6.5 Hz, 2H), 3.64 (s, 2H), 3.53 (t, *J =* 6.1 Hz, 2H), 3.25-3.18 (m, 2H), 2.52 (s, 3H), 1.98-1.84 (m, 4H), 0.88 (t, *J* = 7.3 Hz, 3H).

### Example B1.13: Synthesis of (S)-2-amino-N-(3-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)acetamide (B1.13)

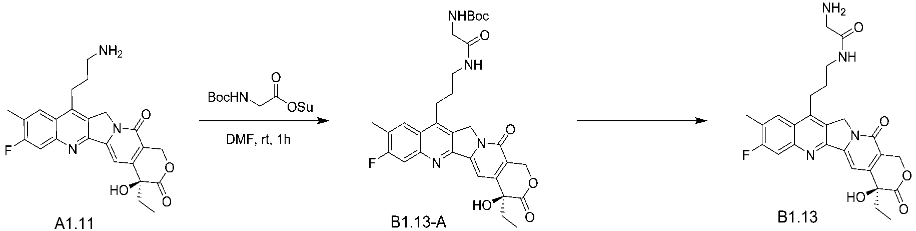

### Step 1:

Triethylamine (67 mg) and 2,5-dioxopyrrolidin-1-yl (tert-butoxycarbonyl)glycinate (182 mg) were added to a solution of compound A1.11 (200 mg) in *N,N*-dimethylformamide (5 mL) at room temperature, and the reaction mixture was reacted at room temperature for 1 hour. The reaction mixture was diluted with water (30 mL), extracted with ethyl acetate (30 mL × 2), and the organic phase was washed with saturated brine, dried over anhydrous sodium sulfate, and concentrated to obtain a crude product. The crude product was further purified by C18 column chromatography (acetonitrile/0.05% formic acid in water: 5% to 60%) to obtain the target compound (B1.13-A, 104 mg).

LCMS (ESI) [M+H]⁺: 595.

### Step 2:

Trifluoroacetic acid (2 mL) was added to a dichloromethane solution (4 mL) of compound B1.13-A (100 mg), and the mixture was stirred and reacted at room temperature for 1 hour. The reaction mixture was concentrated, and the crude product was added with *N,N-*dimethylformamide solution (3 mL) and further purified by C18 column chromatography (acetonitrile/0.05% formic acid in water: 5% to 60%) to obtain the target compound (*S*)-2-amino-*N*-(3-(4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)propyl)acetamide (B1.13, 88 mg).

LCMS (ESI) [M+H]⁺: 495.

### Example B1.14: Synthesis of (S)-2-amino-N-((3-(7-ethyl-7-hydroxy-8,11-dioxo-7,8,11,13-tetrahydro-10H-[1,3]-dioxolo[4,5-g]pyrano[3',4':6,7]indolizino[1,2-b]quinolin-14-yl)propoxy)methyl)acetamide (B1.14)

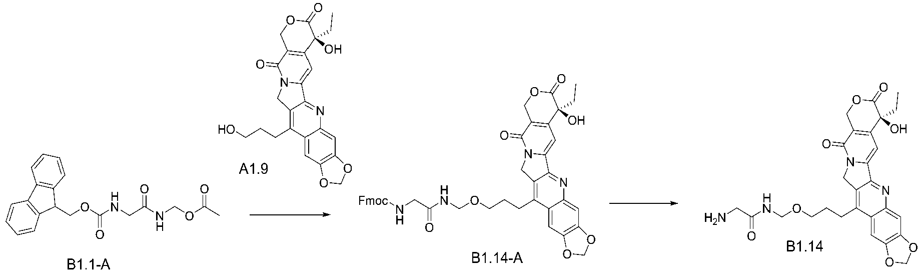

Step 1: Compound (B1.1-A) (368 mg), compound (A1.9) (440 mg), and pyridinium *p*-toluenesulfonate (PPTS) (25 mg) were refluxed in dichloromethane (20 mL) for 20 hours, then washed with sodium bicarbonate aqueous solution and hydrochloric acid aqueous solution, respectively. The organic solvent was removed under reduced pressure to obtain a crude product. The crude product was purified by column chromatography (dichloromethane: methanol = 10/1) to obtain the target compound B1.14A (240 mg).

LCMS (ESI) [M+H]⁺: 759.5.

Step 2: B1.14-A (240 mg) was dissolved in DMF (5 mL), and piperidine (1 mL) was added thereto. The compound was stirred for 20 minutes, dissolved under reduced pressure to remove the low-boiling components, and the residue was used directly in the next step of synthesis. A small amount of the crude product was purified by reversed-phase chromatography (acetonitrile/0.05% FA in water: 5% to 50%) to obtain the target compound.
ESI-MS (m/z): 537.4 [M+H]⁺;
¹H NMR (400 MHz, DMSO-d6) δ 9.13 (t, 1H), 8.04 (br, 2H), 7.58 (s, 1H), 7.51 (s, 1H), 7.25 (s, 1H), 6.29 (s, 2H), 5.43 (S, 2H), 5.21 (s, 2H), 4.65 (d, 2H), 3.63 (m, 2H), 3.53 (m, 2H), 3.11 (m, 2H), 1.87 (m, 4H), 0.88 (t, 3H).

### Example B1.15: (S,E)-2-Amino-N-(3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)acetamide

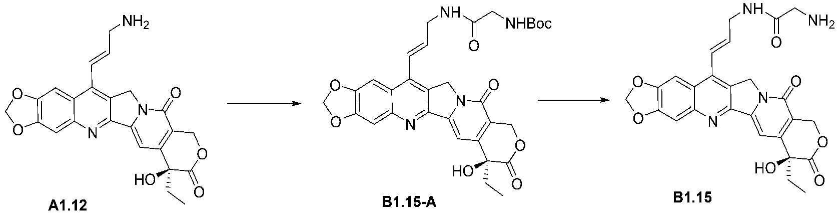

### Step 1:

Triethylamine (135 mg, 1.341 mmol) and *N*-hydroxy-2,5-dioxopyrrolidine *tert-*butoxycarbonylglycinate (183 mg, 0.671 mmol) were added to a solution of compound A1.12 (200 mg, 0.447 mmol) in *N,N*-dimethylformamide (5 mL) at room temperature, and the reaction mixture was reacted at room temperature for 1 hour. LCMS showed the completion of the reaction. The reaction mixture was added with water (20 mL) and extracted with ethyl acetate (20 mL × 3). The organic phase was washed with saturated brine, dried over anhydrous sodium sulfate, and concentrated to obtain the target compound B1.15A (130 mg, yield: 48%) as a brown solid.

LCMS (ESI) [M+H]⁺ = 605.6.

### Step 2:

Compound B1.15-A (130 mg, 0.215 mmol) was dissolved in hydrochloric acid/1,4-dioxane solution (5 mL) at room temperature and reacted for 30 minutes at room temperature. LCMS showed the completion of the reaction. The reaction mixture was purified by reversed-phase chromatography to obtain the target compound (60 mg, yield: 52%) as a brown solid.
LCMS (ESI) [M+H]⁺ =505.2;
¹H NMR (400 MHz, DMSO-d6) δ 8.85 (s, 1H), 8.15 (s, 2H), 7.70 (s, 1H), 7.54 (s, 1H), 7.26 (s, 1H), 7.21 (d, *J =* 16.2 Hz, 1H), 6.51 (d, *J =* 16.2 Hz, 1H), 6.31 (s, 2H), 5.42 (s, 2H), 5.28 (s, 2H), 4.19 (s, 2H), 1.86 - 1.82 (m, 4H), 0.87 (t, *J=* 7.3 Hz, 3H).

### Example B1.16: (S,E)-2-Amino-N-(((3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)acetamide

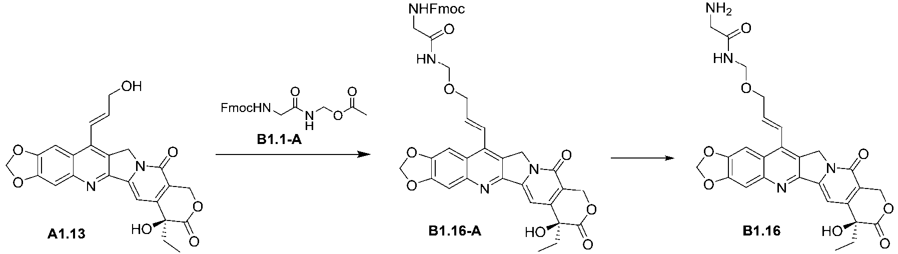

### Step 1:

Raw material A1.13 (150 mg, 0.335 mmol) was dissolved in toluene (3 mL) under nitrogen atmosphere. (2-((((9*H*-Fluoren-9-yl)methoxy)carbonyl)amino)acetamido)methyl acetate (B1.1-A, 308 mg, 0.84 mmol) was added thereto, and then zinc acetate (123 mg, 0.67 mmol) was added to the reaction mixture, and the reaction mixture was stirred at 100°C overnight. The completion of the reaction was detected by LCMS. The reaction mixture was directly purified by reversed-phase chromatography (acetonitrile/0.05% formic acid in water: 5% to 50%) to obtain the target compound (9*H*-fluoren-9-yl)methyl (S,E)-(2-((((3-(7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)allyl)oxy)methyl)amino)-2-oxoethyl)carbamate (B1.16-A, 80 mg, yield: 31%) as a white solid.

LCMS (ESI) [M+H]⁺ = 757.4.

### Step 2:

Compound B1.16-A (80 mg, 0.11 mmol) was dissolved in *N,N-*dimethylformamide (2 mL), then piperidine (0.05 mL) was added thereto, and the reaction mixture was stirred at room temperature for 1 hour. LCMS showed the completion of the reaction. The reaction mixture was concentrated to obtain a crude product, which was purified by reversed-phase C18 column chromatography (acetonitrile/0.05% ammonia in water: 5% to 50%) to obtain the target compound (35 mg, yield: 62%) as a brown solid.

LCMS (ESI) [M+H]⁺ = 535.2, t_{R} = 1.070 min.

### Example B1.17: (S,E)-2-Amino-N-(((3-(4-ethyl-8-fluoro-4-hydroxy-9-methyl-3,14-oxo-3,4,12,14-tetrahydro-1H-pyrano[3',4':6,7]indolizino[1,2-b]quinolin-11-yl)allyl)oxy)methyl)acetamide

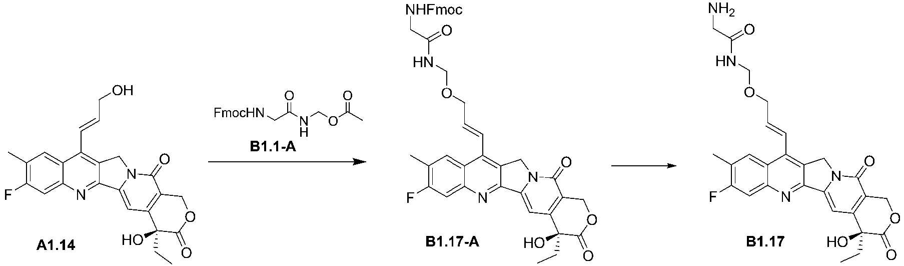

### Step 1:

Compound A1.14 (80 mg, 0.183 mmol) and compound (2-((((9*H*-fluoren-9-yl)methoxy)carbonyl)amino)acetamido)methyl acetate (B1.1-A, 135 mg, 0.367 mmol) were dissolved in toluene (4 mL), then zinc acetate (190 mg, 1.03 mmol) was added thereto, and the reaction mixture was stirred at 100°C for 48 hours. The reaction mixture was cooled to room temperature, added with water (10 mL), and extracted with ethyl acetate (10 mL × 3). The combined organic phases were dried over anhydrous sodium sulfate, filtered, and concentrated to obtain a crude product, which was purified by reversed-phase chromatography (0.05% FA in water/acetonitrile: 5% to 100%) to obtain the target compound (*S,E*)-2-(Fmocamino)-*N*-(((3-(4-ethyl-8-fluoro-4-hydroxy-9-methyl-3,14-oxo-3,4,12,14-tetrahydro-1*H-*pyrano[3',4':6,7]indolizino[1,2-*b*]quinolin-11-yl)allyl)oxy)methyl)acetamide (B1.17-A, 30 mg) as a white solid.
LCMS (ESI) [M+H]⁺ = 745.2;
¹H NMR (400 MHz, DMSO) δ 8.82 (m 1H), 8.21 (m, 1H), 7.89 (m, 2H), 7.84 (d,*J* = 7.8 Hz, 2H), 7.68 (m, 1H), 7.61 (m, 1H), 7.42 (m, 1H), 7.37 (m, 1H), 7.33 - 7.28 (m, 4H), 6.72 - 6.55 (m, 1H), 6.53 (s, 1H), 5.42 (s, 2H), 5.28 (s, 2H), 4.75 (m, 2H), 4.34 (m, 2H), 4.28 - 4.24 (m, 2H), 4.20 (m, 1H), 3.70 (d, *J =* 6.1 Hz, 2H), 2.47 (s, 3H), 1.90 - 1.82 (m, 2H), 0.88 (t, *J =* 7.3 Hz, 3H).

### Step 2:

Compound 4 (25 mg, 0.034 mmol) was dissolved in *N*,*N*-dimethylformamide (2 mL), and then diethylamine (0.2 mL) was added thereto. After the addition, the reaction mixture was stirred at room temperature for 1 hour. After the completion of the reaction was detected by LCMS, the reaction mixture was directly evaporated to dryness under reduced pressure to remove the solvent to obtain the crude target compound (S,E)-2-amino-*N*-(((3-(4-ethyl-8-fluoro-4-hydroxy-9-methyl-3,14-oxo-3,4,12,14-tetrahydro-1*H-*pyrano[3',4':6,7]indolizino[1,2-*b*]quinolin-11-yl)allyl)oxy)methyl)acetamide as a yellow viscous product.
LCMS (ESI) [M+H]+ = 523.2;
LCMS (ESI) [M+H]⁺ = 523.1, t_{R} = 0.446 min, 1.311 min.
¹H NMR (400 MHz, DMSO) δ 9.26 (t, *J =* 6.5 Hz, 1H), 8.23 (d, *J* = 8.2 Hz, 1H), 8.06 (s, 2H), 7.91 (m, 1H), 7.40 (d, *J =* 16.3 Hz, 1H), 7.34 (s, 1H), 6.70 - 6.60 (m, 1H), 6.54 (s, 1H), 5.44 (s, 2H), 5.35 (s, 2H), 4.80 (d, *J =* 6.6 Hz, 2H), 4.38 (d, *J =* 3.9 Hz, 2H), 3.68 (d, 2H), 2.52 (s, 3H), 1.90 - 1.84 (m, 2H), 0.88 (m, 3H).

### C. Synthesis of molecular fragment intermediates containing conjugation linkers

### Example C1.1: N⁶-(tert-Butoxycarbonyl)-N²-((1-(2-(methylthio)pyrimidin-5-yl)-1-oxo-5,8,11,14,17,20,23,26,29-nonaoxa-2-azahentriacontan-31-oyl)-L-valyl)-L-lysine (compound C1.1)

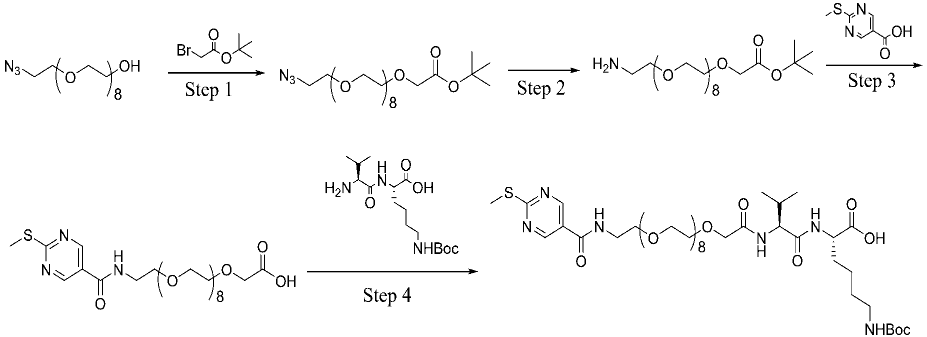

### Step 1: tert-Butyl 29-azido-3,6,9,12,15,18,21,24,27-nonaoxanonacosanoate

NaH (0.6 g, 60%) was added to a solution of 26-azido-3,6,9,12,15,18,21,24-octaoxahexacosan-1-ol (4.39 g) in DMF (40 mL). After the above mixture was stirred for 30 minutes, *tert-*butyl bromoacetate (2.4g) was added thereto. The mixture was stirred under dry conditions for 20 hours. Ethyl acetate (200 mL) and water (200 mL, slowly added at the beginning) were then added to the mixture. The organic phase was washed with water (100 mL × 3), dried over anhydrous sodium sulfate, and the solvent was removed under reduced pressure. The residue was separated by silica gel column chromatography to obtain the target product *tert-*butyl 29-azido-3,6,9,12,15,18,21,24,27-nonaoxanonacosanoate.

ESI-MS (*m*/*z*): 554 [M + H]⁺.

### Step 2: tert-Butyl 29-amino-3,6,9,12,15,18,21,24,27-nonaoxanonacosanoate

Palladium on carbon catalyst (Pd/C, 10%, 100 mg) was added to a solution of *tert-*butyl 29-azido-3,6,9,12,15,18,21,24,27-nonaoxanonacosanoate (1.16 g) in ethyl acetate (20 mL). The above mixture was stirred under hydrogen atmosphere for 5 hours, and then the palladium on carbon was filtered off, and the solvent was removed under reduced pressure to obtain the target product.

ESI-MS (*m*/*z*): 528 [M + H]⁺.

### Step 3: 1-(2-(Methylthio)pyrimidin-5-yl)-1-oxo-5,8,11,14,17,20,23,26,29-nonaoxa-2-azahentriacontan-31-oic acid

*tert*-Butyl 29-amino-3,6,9,12,15,18,21,24,27-nonaoxanonacosanoate (527 mg) and 2-methylthio-pyrimidine-5-carboxylic acid (170 mg) were added to dry DMF (10 mL), and then DIPEA (0.2 mL) and HBTU (420 mg) were sequentially added to the above mixture under cooling in an ice bath. The above mixture was stirred at room temperature for 20 hours. The mixture was then diluted with ethyl acetate (100 mL) and washed with water (100 mL × 4). After the organic phase was dried over anhydrous sodium sulfate, the organic solvent was removed under reduced pressure. The residue was dissolved in DCM (10 mL), and then TFA (10 mL) was added thereto. The mixture was stirred at room temperature for 1 hour, and then the low-boiling components were removed under reduced pressure. The residue was separated by preparative HPLC to obtain 1-(2-(methylthio)pyrimidin-5-yl)-1-oxo-5,8,11,14,17,20,23,26,29-nonaoxa-2-azahentriacontan-31-oic acid.

ESI-MS (*m*/*z*): 624 [M + H]⁺.

### Step 4: N⁶-(tert-Butoxycarbonyl)-N²-((1-(2-(methylthio)pyrimidin-5-yl)-1-oxo-5,8,11,14,17,20,23,26,29-nonaoxa-2-azahentriacontan-31-oyl)-L-valyl)-L-lysine

1-(2-(Methylthio)pyrimidin-5-yl)-1-oxo-5,8,11,14,17,20,23,26,29-nonaoxa-2-azahentriacontan-31-oic acid (623 mg) was dissolved in DMF (10 mL), and DIPEA (300 µL) and HBTU (420 mg) were added thereto at 0°C. The mixture was stirred for 30 minutes, then valine-(Boc)lysine dipeptide compound (345 mg) was added thereto. The reaction mixture was stirred for 20 hours, then added with ethyl acetate (100 mL) and washed with dilute hydrochloric acid (20 mL × 3). The organic phase was dried and the organic solvent was removed under reduced pressure. The crude product was separated by preparative HPLC to obtain the target compound *N*⁶-(*tert*-butoxycarbonyl)-*N*²-((1-(2-(methylthio)pyrimidin-5-yl)-1-oxo-5,8,11,14,17,20,23,26,29-nonaoxa-2-azahentriacontan-31-oyl)-*L*-valyl)-*L*-lysine (compound C1.1).

ESI-MS (*m*/*z*): 951 [M + H]⁺.

### Example C1.2: N⁶-(tert-Butoxycarbonyl)-N²-((29-(4-(2-(methylthio)pyrimidin-5-yl)-1H-1,2,3-triazol-1-yl)-3,6,9,12,15,18,21,24,27-nonaoxanonacosanoyl)-L-valyl)-L-lysine (compound C1.2)

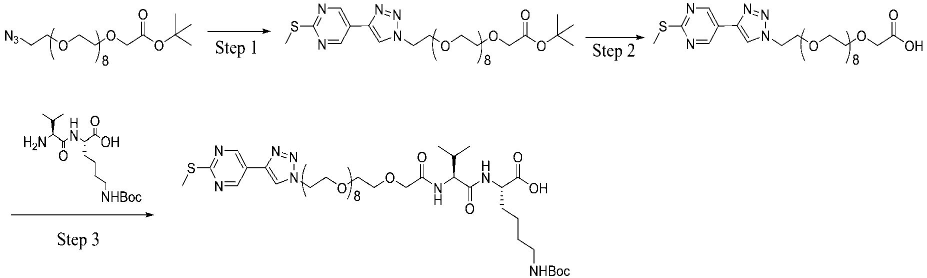

### Step 1: tert-Butyl 29-(4-(2-(methylthio)pyrimidin-5-yl)-1H-1,2,3-triazol-1-yl)-3,6,9,12,15,18,21,24,27-nonaoxanonacosanoate

tert-Butyl 29-azido-3,6,9,12,15,18,21,24,27-nonaoxanonacosanoate (1.7 g) and 2-methylthio-5-ethynylpyrimidine (450 mg) were dissolved in DMSO-water (20 mL, 4:1), and cuprous bromide (50 mg) was added to the mixture. The reaction mixture was stirred at room temperature for 2 hours, then added with ethyl acetate (100 mL), washed with water (100 mL × 3), dried, and then the organic solvent was removed under reduced pressure. The crude product was separated by silica gel column chromatography to obtain the target product *tert-*butyl 29-(4-(2-(methylthio)pyrimidin-5-yl)-1*H*-1,2,3-triazol-1-yl)-3,6,9,12,15,18,21,24,27-nonaoxanonacosanoate.

MS *(m*/*z):* 704 [M + H]⁺.

### Step 2: 29-(4-(2-(Methylthio)pyrimidin-5-yl)-1H-1,2,3-triazol-1-yl)-3,6,9,12,15,18,21,24,27-nonaoxanonacosanoic acid

*tert*-Butyl 29-(4-(2-(methylthio)pyrimidin-5-yl)-1*H*-1,2,3-triazol-1-yl)-3,6,9,12,15,18,21,24,27-nonaoxanonacosanoate (1 g) was dissolved in dichloromethane (10 mL), and then TFA (5 mL) was added thereto. The mixture was allowed to stand at room temperature for 1 hour, and then the low-boiling components were removed under reduced pressure to obtain the target product 29-(4-(2-(methylthio)pyrimidin-5-yl)-1*H*-1,2,3-triazol-1-yl)-3,6,9,12,15,18,21,24,27-nonaoxanonacosanoic acid.

MS *(m*/*z):* 648 [M + H]⁺.

### Step 3: N⁶-(tert-Butoxycarbonyl)-N²-((29-(4-(2-(methylthio)pyrimidin-5-yl)-1H-1,2,3-triazol-1-yl)-3,6,9,12,15,18,21,24,27-nonaoxanonacosanoyl)-L-valyl)-L-lysine

29-(4-(2-(Methylthio)pyrimidin-5-yl)-1*H*-1,2,3-triazol-1-yl)-3,6,9,12,15,18,21,24,27-nonaoxanonacosanoic acid (647 mg) was dissolved in DMF (10 mL), and DIPEA (300 µL) and HBTU (420 mg) were added thereto at 0°C. The mixture was stirred for 30 minutes, then valine-lysine dipeptide compound (345 mg) was added thereto. The reaction mixture was stirred for 20 hours, then added with ethyl acetate (100 mL) and washed with dilute hydrochloric acid (20 mL × 3). The organic phase was dried and the organic solvent was removed under reduced pressure. The crude product was separated by preparative HPLC to obtain the target compound *N*⁶-(*tert*-butoxycarbonyl)-*N*²-((29-(4-(2-(methylthio)pyrimidin-5-yl)-1*H*-1,2,3-triazol-1-yl)-3,6,9,12,15,18,21,24,27-nonaoxanonacosanoyl)-*L*-valyl)-*L*-lysine (compound C1.2).

ESI-MS (*m*/*z*): 975 [M + H]⁺.

### Example C1.3: (1-(2-(Methylthio)pyrimidin-5-yl)-1-oxo-5,8,11,14,17,20,23,26,29-nonaoxa-2-azahentriacontan-31-oyl)-glycyl-glycyl-L-phenylalanine (compound C1.3)

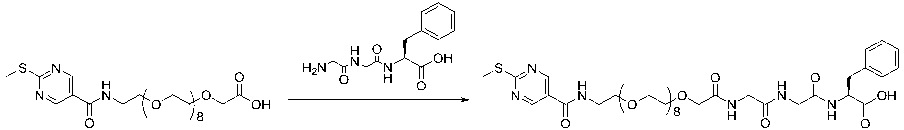

Using the same method and reaction conditions as in step 4 of Example C1.1, and using corresponding raw materials, the target product (1-(2-(methylthio)pyrimidin-5-yl)-1-oxo-5,8,11,14,17,20,23,26,29-nonaoxa-2-azahentriacontan-31-oyl)-glycyl-glycyl-*L-*phenylalanine (compound C1.3) was obtained.

ESI-MS (m/z): 885 [M+H]⁺.

### Example C1.4: (36-(2-(Methylthio)pyrimidin-5-yl)-31-oxo-3,6,9,12,15,18,21,24,27-nonaoxa-30-azahexatriacont-35-ynoyl)glycylglycyl-L-phenylalanine (compound C1.4)

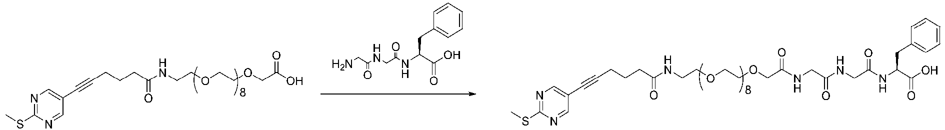

Using the same method and reaction conditions as in step 4 of Example C1.1, and using the raw materials shown in the reaction scheme, the target product (36-(2-(methylthio)pyrimidin-5-yl)-31-oxo-3,6,9,12,15,18,21,24,27-nonaoxa-30-azahexatriacont-35-ynoyl)glycylglycyl-*L*-phenylalanine (compound C1.4) was obtained.

ESI-MS (m/z): 951 [M+H]⁺.

### Example C1.5: N⁶-(tert-Butoxycarbonyl)-N²-((36-(2-(methylthio)pyrimidin-5-yl)-31-oxo-3,6,9,12,15,18,21,24,27-nonaoxa-30-azahexatriacont-35-ynoyl)-L-valyl)-L-lysine (C1.5)

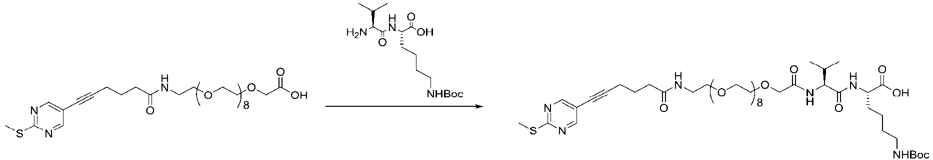

Using the same method and reaction conditions as in step 4 of Example C1.1, and using the compound shown in the reaction scheme as a raw material, the target product *N*⁶-(*tert*-butoxycarbonyl)-*N*²-((36-(2-(methylthio)pyrimidin-5-yl)-31-oxo-3,6,9,12,15,18,21,24,27-nonaoxa-30-azahexatriacont-35-ynoyl)-*L*-valyl)-*L*-lysine (compound C1.5) was obtained.

ESI-MS (m/z): 1017 [M+H]⁺.

### Example C1.6: N⁶-(tert-Butoxycarbonyl)-N²-((6-(2-(methylthio)pyrimidin-5-yl)hex-5-ynoyl)-L-valyl)-L-lysine (compound C1.6)

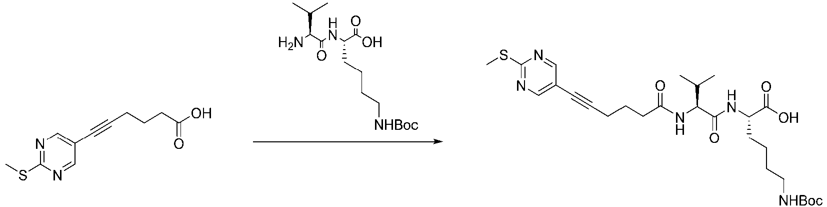

6-(2-(Methylthio)pyrimidin-5-yl)hex-5-ynoic acid (920 mg) and *N-*hydroxysuccinimide (537 mg) were dissolved in dichloromethane (50 mL), followed by addition of dicyclohexylcarbodiimide (963 mg). The reaction mixture was stirred at room temperature for 1 hour, and then concentrated under reduced pressure to remove dichloromethane. The residue was dissolved in *N,N*-dimethylformamide (50 mL), followed by addition of *N*²-*L*-valyl-*N*⁶-(Boc)-*L*-lysine (1.5 g), and the reaction mixture was stirred and reacted at room temperature for 16 hours. The reaction mixture was poured into 200 mL of water, and added with saturated sodium bicarbonate solution to adjust the pH to 11. The above aqueous solution was extracted twice with ethyl acetate, and the organic phase was discarded. The aqueous phase was added with citric acid to adjust the pH to 4 to 5, and extracted three times with ethyl acetate (100 mL × 3). The organic phase was washed with saturated brine (100 mL), dried over anhydrous sodium sulfate, subjected to suction filtration, and subjected to rotary evaporation to obtain 2.8 g of crude product, which was purified by flash chromatography (DCM: MeOH = 30:1) to obtain the target compound *N*⁶-(*tert-*butoxycarbonyl)-*N*²-((6-(2-(methylthio)pyrimidin-5-yl)hex-5-ynoyl)-*L*-valyl)-*L*-lysine (1.0 g) (compound C1.6).

ESI-MS (m/z): 564.3 [M+H]⁺.

¹H NMR (400 MHz, DMSO-*d*₆) δ 12.45 (s, 1H), 8.68 (s, 2H), 8.12 (d, *J* = 7.3 Hz, 1H), 7.88 (d, *J =* 8.9 Hz, 1H), 6.76 (s, 1H), 4.27 - 4.19 (m, 1H), 4.11 (d, *J* = 4.9 Hz, 1H), 2.88 (d, *J* = 7.9 Hz, 3H), 2.73 (s, 1H), 2.59 (s, 1H), 2.52 (s, 4H), 2.40 - 2.24 (m, 3H), 2.03 - 1.88 (m, 2H), 1.77 (d, *J =* 3.2 Hz, 2H), 1.68 (d, *J =* 7.0 Hz, 1H), 1.60 - 1.49 (m, 1H), 1.36 (s, 15H), 0.85 (dd, *J =* 14.8, 6.7 Hz, 7H).

### Example C1.7: N⁶-(tert-Butoxycarbonyl)-N²-((6-(2-(methylsulfonyl)pyrimidin-5-yl)hex-5-ynoyl)-L-valyl)-L-lysine (compound C1.7)

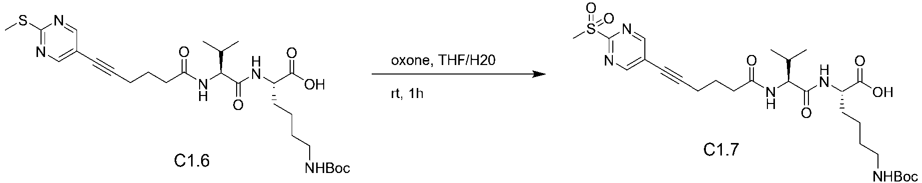

Potassium peroxymonosulfate (1.414 g) was added to a mixed solution of compound C1.6 (260 mg) in tetrahydrofuran (3 mL) and water (3 mL), and the reaction mixture was stirred and reacted at room temperature for 1 hour. The reaction mixture was filtered, and the filtrate was purified by C18 column chromatography (acetonitrile/0.05% formic acid in water: 5% to 60%) to obtain the target compound *N*⁶-(*tert*-butoxycarbonyl)-*N*²-((6-(2-(methylsulfonyl)pyrimidin-5-yl)hex-5-ynoyl)-*L*-valyl)-*L*-lysine (compound C1.7) (120 mg).
LCMS (ESI) [M+H]⁺: 596;
¹H NMR (400 MHz, DMSO-d6) δ 12.48 (s, 1H), 9.13 (s, 2H), 8.14 (d, *J =* 7.0 Hz, 1H), 7.90 (d, *J =* 9.1 Hz, 1H), 6.77 (s, 1H), 4.24 (t, *J =* 7.7 Hz, 1H), 4.12 (br s, 1H), 3.41 (s, 3H), 2.89 (d, *J =* 6.0 Hz, 2H), 2.43 - 2.29 (m, 2H), 2.03 - 1.93 (m, 2H), 1.82 (br s, 2H), 1.63 - 1.60 (m, 4H), 1.37 (s, 12H), 0.88 - 0.83 (m, 6H).

### Example C1.8: N⁶-(tert-Butoxycarbonyl)-N²-((6-(2-(methylthio)pyrimidine-5-carboxamido)hexanoyl)-L-valyl)-L-lysine (compound C1.8)

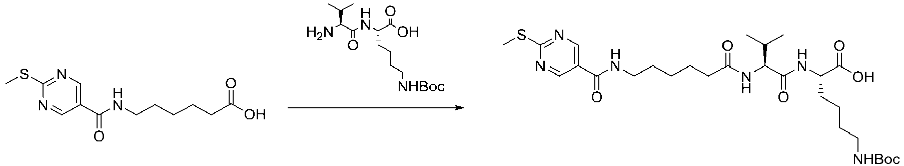

Using the same method and reaction conditions as in example C1.6, and using the known compounds shown in the reaction scheme as raw materials, the target product *N*⁶-(*tert-*butoxycarbonyl)-*N*²-((6-(2-(methylthio)pyrimidine-5-carboxamido)hexanoyl)-*L*-valyl)-*L-*lysine (compound C1.8) was obtained.

ESI-MS (m/z): 611 [M+H]⁺.

### Example C1.9: N⁶-(tert-Butoxycarbonyl)-N²-((6-(4-(2-(methylthio)pyrimidin-5-yl)-1H-1,2,3-triazol-1-yl)hexanoyl)-L-valyl)-L-lysine (compound C1.9)

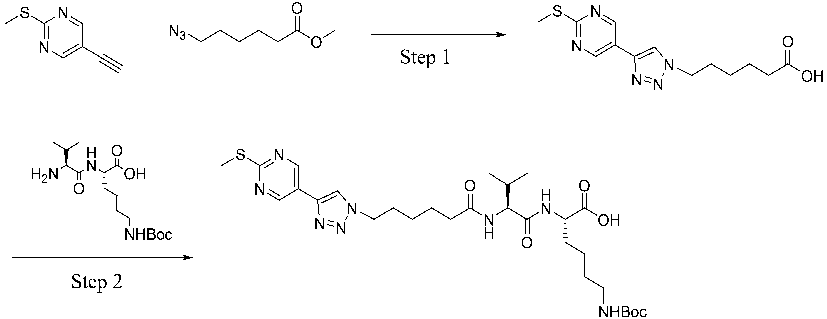

### Step 1: 6-(4-(2-(Methylthio)pyrimidin-5-yl)-1H-1,2,3-triazol-1-yl)hexanoic acid

2-Methylthio-5-ethynylpyrimidine (1.5 g) and methyl 6-azidohexanoate (1.71 g) were dissolved in a mixed solvent of *tert*-butanol and water (20 mL/25 mL) at room temperature, then sodium vitamin C (5.7 g) and cuprous bromide (2.9 g) were added thereto, and the mixture was stirred and reacted for 10 hours. The reaction mixture was added with ethyl acetate (200 mL), washed with water (100 mL × 5), dried, and the organic solvent was removed. The residue was subjected to silica gel column chromatography to obtain methyl 6-(4-(2-(methylthio)pyrimidin-5-yl)-1*H*-1,2,3-triazol-1-yl)hexanoate (2.8 g). ESI-MS (m/z): 321.9 [M+H]⁺. The above product was dissolved in tetrahydrofuran (100 mL), then lithium hydroxide (1 M, 20 mL) was added thereto, and the mixture was stirred for 3 hours. The reaction mixture was added with hydrochloric acid to adjust the pH to 2, then added with ethyl acetate (200 mL), washed with water (100 mL × 3), and dried. The organic solvent was removed to obtain the target product 6-(4-(2-(methylthio)pyrimidin-5-yl)-1*H*-1,2,3-triazol-1-yl)hexanoic acid. ESI-MS (m/z): 308.4 [M+H]⁺.

### Step 2: N⁶-(tert-Butoxycarbonyl)-N²-((6-(4-(2-(methylthio)pyrimidin-5-yl)-1H-1,2,3-triazol-1-yl)hexanoyl)-L-valyl)-L-lysine

Compound 6-(4-(2-(methylthio)pyrimidin-5-yl)-1*H*-1,2,3-triazol-1-yl)hexanoic acid (370 mg) and *N*-hydroxysuccinimide (152 mg) were dissolved in dichloromethane (5 mL) with stirring, followed by addition of dicycloethylcarbodiimide (273 mg), and the reaction mixture was stirred at room temperature for 1 hour. The reaction mixture was added with water (10 mL) and extracted with ethyl acetate (20 mL × 2). The organic phase was washed with saturated brine, dried over anhydrous sodium sulfate, filtered, and the filtrate was concentrated under reduced pressure to obtain a crude product. The crude product was dissolved in *N,N-*dimethylformamide (10 mL) with stirring, then compound *N*²-*L*-valyl-*N*⁶-(Boc)-*L*-lysine was added thereto, and the reaction mixture was reacted at room temperature for 16 hours. The reaction mixture was slowly added with citric acid to adjust the pH to approximately 5, then added with water, and extracted with ethyl acetate (10 mL × 3). The organic phase was washed with saturated brine, dried over anhydrous sodium sulfate, concentrated to remove ethyl acetate to obtain the target product *N*⁶-(*tert*-butoxycarbonyl)-*N*²-((6-(4-(2-(methylthio)pyrimidin-5-yl)-1*H*-1,2,3-triazol-1-yl)hexanoyl)-*L*-valyl)-*L*-lysine (compound C1.9, 400 mg).
ESI-MS (m/z): 635.3 [M+H]⁺;
¹H NMR (400 MHz, DMSO-d6) δ 12.46 (s, 1H), 9.06 (s, 2H), 9.06 (s, 1H), 8.70 (s, 1H), 8.07 (d, *J =* 7.4 Hz, 1H), 7.77 (d, *J* = 9.0 Hz, 1H), 6.76 (t, *J =* 5.6 Hz, 1H), 4.41 (t, *J =* 7.0 Hz, 2H), 4.19 (dd, *J =* 8.9*,* 7.0 Hz, 1H), 4.14 - 4.05 (m, 1H), 2.92 - 2.85 (m, 2H), 2.56 (s, 3H), 2.24 - 2.10 (m, 2H), 1.97 - 1.81 (m, 3H), 1.71 - 1.64 (m, 1H), 1.61 - 1.50 (m, 3H), 1.36 (s, 9H), 1.28 - 1.22 (m, 6H), 0.88 - 0.76 (m, 6H).

### Example C1.10: (6-(2-(Methylthio)pyrimidin-5-yl)hex-5-ynoyl)glycylglycyl-L-phenylalanine (compound C1.10)

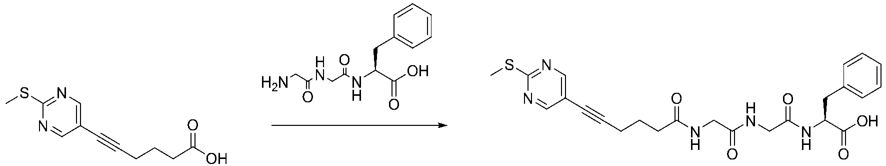

Using the same method and reaction conditions as in Example C1.6, and using the known raw materials shown in the above reaction scheme, the target product (6-(2-(methylthio)pyrimidin-5-yl)hex-5-ynoyl)glycylglycyl-*L*-phenylalanine (compound C1.10) was obtained. ESI-MS (m/z): 498 [M+H]⁺.

Using the same method and reaction conditions as in Example C1.6 and using different reaction raw materials, the target products in the table below were obtained.

| Compound | Chemical structure | Chemical name | ESI-MS (*m*/*z*): [M + H]⁺ |
|---|---|---|---|
| C1.11 | 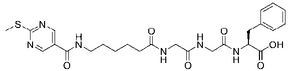 | (6-(2-(Methylthio)pyrimidine-5-carboxamido)hexanoyl)gl ycylglycyl-*L-*phenylalanine | 545 |
| C1.12 | 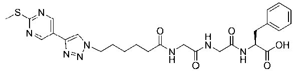 | (6-(4-(2-(Methylthio)pyrimidin-5-yl)-1*H*-1,2,3-triazol-1-yl)hexanoyl)glycylglycyl-*L*-phenylalanine | 569 |

### Example C1.13: (29-(4-(2-(Methylthio)pyrimidin-5-yl)-1H-1,2,3-triazol-1-yl)-3,6,9,12,15,18,21,24,27-nonaoxanonacosanoyl)glycylglycyl-L-phenylalanine (compound C1.13)

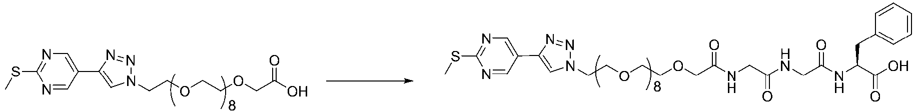

Using the same method and reaction conditions as in step 4 of Example C1.1, and using known raw materials, the target product (29-(4-(2-(methylthio)pyrimidin-5-yl)-1*H*-1,2,3-triazol-1-yl)-3,6,9,12,15,18,21,24,27-nonaoxanonacosanoyl)glycylglycyl-*L*-phenylalanine (compound C1.13) was obtained.

ESI-MS (m/z): 909 [M+H]⁺.

### Example C1.14: N⁶-(tert-Butoxycarbonyl)-N²-((39-(2-(methylthio)pyrimidin-5-yl)-5,34-dioxo-3,9,12,15,18,21,24,27,30-nonaoxa-6,33-diazanonatriacont-38-ynoyl)-L-valyl)-L-lysine (C1.14)

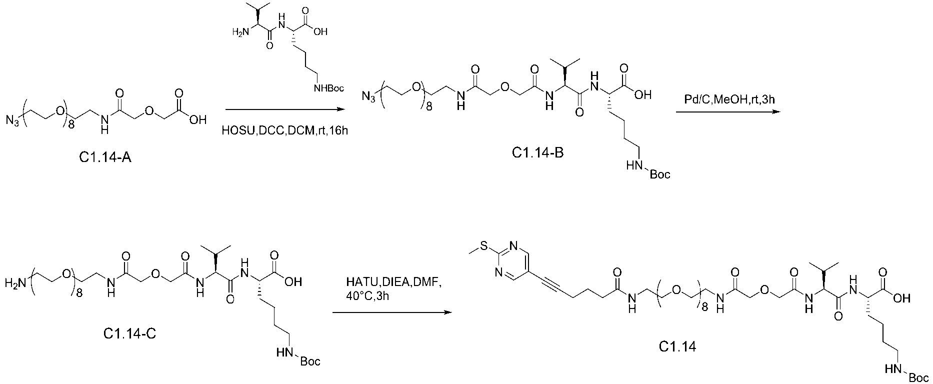

### Step 1:

Compound C1.14-A (336 mg) was dissolved in dichloromethane (5 mL), then N-hydroxysuccinimide (98 mg, 0.85 mmol) and dicyclohexylcarbodiimide (175 mg, 0.85 mmol) were added thereto, and the reaction mixture was stirred and reacted at room temperature for 1 hour. Compound *N*²-*L*-valyl-*N*⁶-(Boc)-*L*-lysine (230 mg) was then added thereto, and the reaction mixture was stirred and reacted at room temperature overnight. The reaction mixture was concentrated to remove the solvent to obtain a crude product, which was purified by reversed-phase C18 column chromatography (acetonitrile/0.01% FA in water: 5% to 50%) to obtain the target compound (C1.14B, 290 mg) as a colorless oil.

LCMS (ESI) [M+H]⁺ = 882.3.

### Step 2:

Compound C1.14-B (300 mg) was dissolved in anhydrous methanol (5 mL), then Pd/C (10%, 60 mg) was added thereto, and the reaction system was replaced with hydrogen three times. The reaction mixture was stirred and reacted at room temperature overnight, subjected to suction filtration, and concentrated to obtain the target compound C1.14-C (293 mg) as a colorless oil.

LCMS (ESI) [M+H]⁺: 856.1.

### Step 3:

6-(2-(Methylthio)pyrimidin-5-yl)hex-5-ynoic acid (84 mg) was dissolved in *N,N-*dimethylformamide (3 mL), and then *N*,*N*-diisopropylethylamine (61 mg) and HATU (108 mg) were sequentially added thereto. The reaction mixture was stirred at room temperature for 20 minutes, then added with compound C1.14-C (196 mg), and stirred and reacted at 40°C for 3 hours. The crude product was purified by reversed-phase C18 column chromatography (acetonitrile/0.01% FA in water: 5% to 65%) to obtain the target compound *N*⁶-(*tert-*butoxycarbonyl)-*N*²-((39-(2-(methylthio)pyrimidin-5-yl)-5,34-dioxo-3,9,12,15,18,21,24,27,30-nonaoxa-6,33-diazanonatriacont-38-ynoyl)-*L*-valyl)-*L*-lysine (C1.14) (170 mg).

LCMS (ESI) [M+H]⁺: 1074.0;

¹H NMR (400 MHz, DMSO-d6) δ 8.68 (s, 2H), 8.23 (t, *J* = 5.7 Hz, 1H), 8.13 (d*, J =* 8.9 Hz, 1H), 7.99 (t, *J =* 5.6 Hz, 1H), 7.43 (d, *J =* 6.5 Hz, 1H), 6.69 (s, 1H), 4.09 (dd, *J =* 8.8, 6.5 Hz, 1H), 4.02 (s, 2H), 3.98 (d, *J =* 2.9 Hz, 2H), 3.74 - 3.70 (m, 1H), 3.50 (s, 30H), 3.45 (d, *J* = 6.2 Hz, 2H), 3.39 (d, *J =* 5.8 Hz, 2H), 3.26 (d, *J =* 6.1 Hz, 2H), 3.20 (q, *J =* 5.9 Hz, 2H), 2.81 (t, *J =* 6.6 Hz, 2H), 2.52 (s, 3H), 2.24 (t, *J =* 7.4 Hz, 2H), 2.12 - 2.06 (m, 1H), 1.79 - 1.74 (m, 2H), 1.67 - 1.59 (m, 1H), 1.50 (d, *J =* 5.3 Hz, 1H), 1.36 (s, 9H), 1.24 (s, 2H), 1.20 - 1.09 (m, 2H), 0.85 (t, *J =* 6.3 Hz, 6H).

### Example C1.15: N⁶-(tert-Butoxycarbonyl)-N²-((32-(4-(2-(methylthio)pyrimidin-5-yl)-1H-1,2,3-triazol-1-yl)-5-oxo-3,9,12,15,18,21,24,27,30-nonaoxa-6-azadotriacontanoyl)-L-valyl)-L-lysine (C1.15)

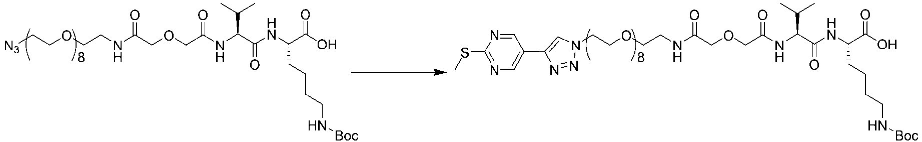

Compound C1.14-B (130 mg), 5-ethynyl-2-methylthiopyrimidine (44 mg), sodium vitamin C (3 mg), and copper sulfate (5 mg) were added to a mixed solution of *tert*-butanol (2 mL) and water (2 mL), and the reaction mixture was reacted at room temperature under nitrogen atmosphere for 3 hours. The reaction mixture was added with water (10 mL) and extracted with dichloromethane (30 mL × 3). The organic phase was washed with saturated brine, dried over anhydrous sodium sulfate, subjected to suction filtration, and concentrated to obtain a crude product. The crude product was purified by TLC (dichloromethane: methanol = 10:1) to obtain the target compound *N*⁶-(*tert*-butoxycarbonyl)-*N*²-((32-(4-(2-(methylthio)pyrimidin-5-yl)-1*H*-1,2,3-triazol-1-yl)-5-oxo-3,9,12,15,18,21,24,27,30-nonaoxa-6-azadotriacontanoyl)-*L*-valyl)-*L*-lysine (C1.15) (90 mg).

LCMS (ESI) [M+H]⁺ = 1032.3.

### Example C1.16: N⁶,N⁶-Dimethyl-N²-((6-(2-(methylthio)pyrimidin-5-yl)hex-5-ynoyl)-L-valyl)-L-lysine

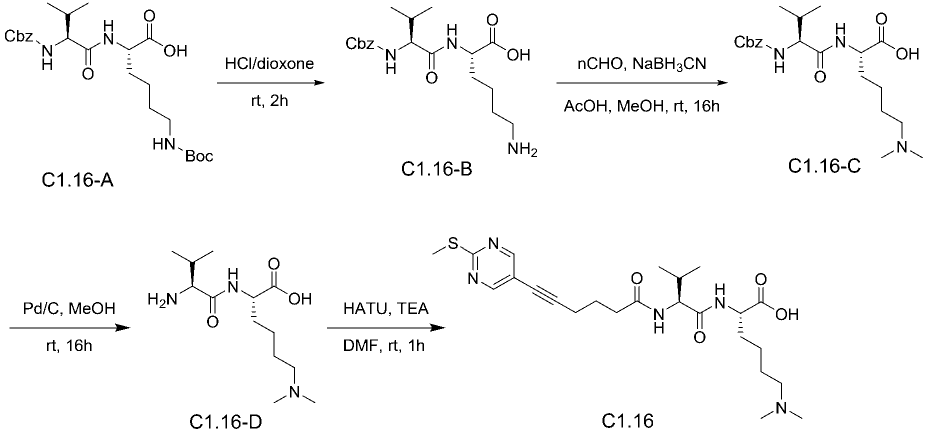

### Step 1:

A solution of HCl-dioxane (100 mL) was added to compound C1.16-A (10.0 g), and the mixture was reacted at room temperature for 2 hours. The reaction mixture was concentrated under reduced pressure to obtain the target compound C1.16-B (8.0 g) as a white solid.

LCMS (ESI) [M+H]⁺: 380.1.

¹H NMR (400 MHz, DMSO) δ 8.18 (d, *J* = 7.4 Hz, 1H), 7.40 - 7.30 (m, 5H), 7.26 (d, *J* = 8.8 Hz, 1H), 5.08 - 4.99 (m, 2H), 4.23 - 4.11 (m, 1H), 3.94 - 3.88 (m, 1H), 2.78 - 2.73 (m, 2H), 2.03 - 1.94 (m, 1H), 1.77 - 1.51 (m, 4H), 1.44 - 1.31 (m, 2H), 0.87 (dd, *J =* 17.3, 6.6 Hz, 6H).

### Step 2:

Compound C1.16-B (3.0 g) and sodium acetate (1.90 g) were dissolved in a solution of methanol (100 mL), and the reaction mixture was reacted at room temperature for 10 minutes. Paraformaldehyde (2.8 g) was added thereto, and the reaction mixture was stirred and reacted at room temperature for 30 minutes. Sodium cyanoborohydride (1.0 g) was then added thereto, and the reaction mixture was stirred and reacted at room temperature for 16 hours. The reaction mixture was filtered, and the filtrate was purified by reversed-phase C18 column chromatography (acetonitrile/0.05% formic acid in water: 5% to 55%) to obtain the target compound C1.16-C (1.70 g).
LCMS (ESI) [M+H]⁺: 408.1;
¹H NMR (400 MHz, DMSO) δ 7.86 (d, *J =* 7.3 Hz, 1H), 7.40 - 7.26 (m, 6H), 5.03 (s, 2H), 4.08 - 4.06 (m, 1H), 3.90 - 3.85 (m, 1H), 2.50 - 2.45 (m, 2H), 2.35 (s, 6H), 2.05 - 1.93 (m, 1H), 1.73 - 1.55 (m, 2H), 1.51 - 1.40 (m, 2H), 1.33 - 1.22 (m, 2H), 0.85 (dd, *J =* 16.5, 6.8 Hz, 6H).

### Step 3:

Compound C1.16-C (1.6 g) was dissolved in methanol (80 mL) at room temperature, then Pd/C (10%, 0.16 g) was added thereto, and the reaction mixture was stirred and reacted at room temperature under hydrogen atmosphere for 12 hours. The reaction mixture was filtered, and the filtrate was concentrated under reduced pressure to obtain the target compound C1.16-D (680 mg).

LCMS (ESI) [M+H]⁺: 274.2.

### Step 4:

6-(2-(Methylthio)pyrimidin-5-yl)hex-5-ynoic acid (944 mg) was dissolved in *N,N-*dimethylformamide (30 mL), and then *N,N*-diisopropylethylamine (1.3 g) and 2-(7-azabenzotriazol-1-yl)-*N,N,N',N*'-tetramethyluronium hexafluorophosphate (HBTU, 1.8 g) were sequentially added thereto. The reaction mixture was stirred at room temperature for 20 minutes, then added with compound C1.16-D (1.1 g), and stirred and reacted at 40°C for 3 hours. The crude product was purified by reversed-phase C18 column chromatography (acetonitrile/0.01% formic acid in water: 5% to 65%) to obtain the target compound *N*⁶,*N*⁶-dimethyl-*N*²-((6-(2-(methylthio)pyrimidin-5-yl)hex-5-ynoyl)-*L*-valyl)-*L*-lysine (C1.16) (1.2 g) as a white solid.
LCMS (ESI) [M+H]⁺: 492.1;
¹H NMR (400 MHz, DMSO) δ 8.68 (s, 2H), 8.00 (d, *J* = 7.6 Hz, 1H), 7.91 (d, *J* = 9.0 Hz, 1H), 4.20 (t, 1H), 4.08 (dd, *J* = 12.7, 7.7 Hz, 1H), 2.47 - 2.40 (m, 4H), 2.37 - 2.31 (m, 2H), 2.30 (s, 6H), 2.01 - 1.92 (m, 1H), 1.82 - 1.73 (m, 2H), 1.71 - 1.56 (m, 2H), 1.49 - 1.37 (m, 2H), 1.33 - 1.23 (m, 2H), 0.88 - 0.82 (m, 6H).

### Example C1.17: N⁶,N⁶-Dimethyl-N²-((6-(2-(methylsulfonyl)pyrimidin-5-yl)hex-5-ynoyl)-L-valyl)-L-lysine (C1.17)

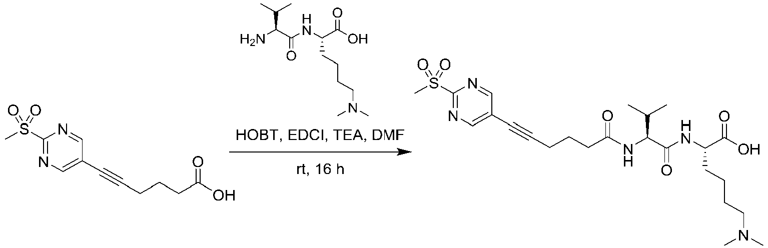

6-(2-(Methylsulfonyl)pyrimidin-5-yl)hex-5-ynoic acid (268 mg), compound C1.16-D (328 mg), and triethylamine (322 mg) were dissolved in *N,N*-dimethylformamide (5 mL). 1-Hydroxybenzotriazole (HOBT, 162 mg) and 1-(3-dimethylaminopropyl)-3-ethylcarbodiimide hydrochloride (EDCI, 229 mg) were then added thereto, and the reaction mixture was stirred at room temperature for 16 hours. The reaction mixture was directly purified by reversed-phase C18 column chromatography (acetonitrile/0.05% formic acid in water system) to obtain the target compound *N*⁶,*N*⁶-dimethyl-*N*²-((6-(2-(methylsulfonyl)pyrimidin-5-yl)hex-5-ynoyl)-*L*-valyl)-*L*-lysine (C1.17, white solid, 327 mg).

LCMS (ESI) [M+H]⁺: 524.4.

¹H NMR (400 MHz, ) δ 9.13 (s, 2H), 7.95 (t, *J =* 8.8 Hz, 2H), 4.21 (dd, *J =* 8.8, 6.9 Hz, 1H), 4.08 - 4.03 (m, 1H), 3.41 (s, 3H), 2.55 (t, *J =* 7.0 Hz, 2H), 2.42 - 2.32 (m, 4H), 2.27 (s, 6H), 1.98 (dd, *J =* 13.6, 6.8 Hz, 1H), 1.86 - 1.77 (m, 2H), 1.74 - 1.55 (m, 2H), 1.47 - 1.37 (m, 2H), 1.31 - 1.23 (m, 2H), 0.85 (dd, *J =* 12.8, 6.8 Hz, 6H).

### Example C1.18: N²-(tert-Butoxycarbonyl)-N⁶-((6-(2-(methylsulfonyl)pyrimidin-5-yl)hex-5-ynoyl)-L-valyl)-L-lysine

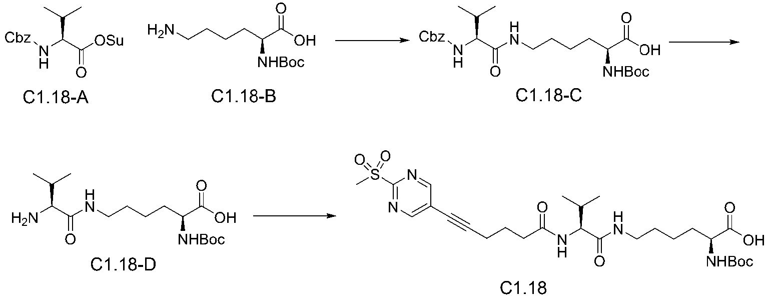

### Step 1:

Compound C1.18-B (3 g) was dissolved in dichloromethane (30 mL), then DIPEA (4 mL) was added thereto, followed by addition of compound C1.18-A (3.48 g). The mixture was stirred and reacted at room temperature for 20 hours. The reaction mixture was added with ethyl acetate (200 mL), sequentially washed with hydrochloric acid (0.1 M, 30 mL × 3) and water (30 mL × 3), dried over anhydrous sodium sulfate, and concentrated under reduced pressure to obtain the target mixture C1.18-C (4.7 g).

### Step 2:

Compound C1.18-C (4.7 g) was dissolved in methanol (80 mL) at room temperature, then Pd/C (10%, 0.6 g) was added thereto, and the reaction mixture was stirred and reacted at room temperature under hydrogen atmosphere for 12 hours. The reaction mixture was filtered, and the filtrate was concentrated under reduced pressure to obtain the target compound C1.18-D (3.4 g). LCMS (ESI) [M+H]⁺: 346.2.

### Step 3:

6-(2-(Methylsulfonyl)pyrimidin-5-yl)hex-5-ynoic acid (2.68 g) was dissolved in *N,N-*dimethylformamide (50 mL), then triethylamine (3 mL) and HBTU (3.8 g) were added thereto, and the mixture was stirred at room temperature for 10 minutes. The reaction mixture was then added with ethyl acetate (200 mL), washed with saturated sodium bicarbonate (20 mL × 2), hydrochloric acid (0.1 M, 50 mL × 2), and water (50 mL × 2), dried over anhydrous sodium sulfate, and concentrated under reduced pressure to obtain an intermediate crude product. The above crude product was dissolved in DMF (30 mL), then DIPEA (1.6 mL) was added thereto, followed by addition of C1.18-D (3.4 g). The reaction mixture was stirred at room temperature for 3 hours, then added with ethyl acetate (200 mL), washed with hydrochloric acid (0.1 M, 50 mL × 2) and water (50 mL × 2), dried over anhydrous sodium sulfate, and concentrated under reduced pressure to obtain a crude product, which was then purified by silica gel column chromatography to obtain the target compound *N*²-(*tert*-butoxycarbonyl)-*N*⁶-((6-(2-(methylsulfonyl)pyrimidin-5-yl)hex-5-ynoyl)-*L*-valyl)-*L*-lysine (C1.18, white solid, 3.8 g).

LCMS (ESI) [M+H]⁺: 596.4.

### Example C1.19: N⁶,N⁶-Dimethyl-N²-((6-(4-(2-(methylsulfonyl)pyrimidin-5-yl)-1H-1,2,3-triazol-1-yl)hexanoyl)-L-valyl)-L-lysine

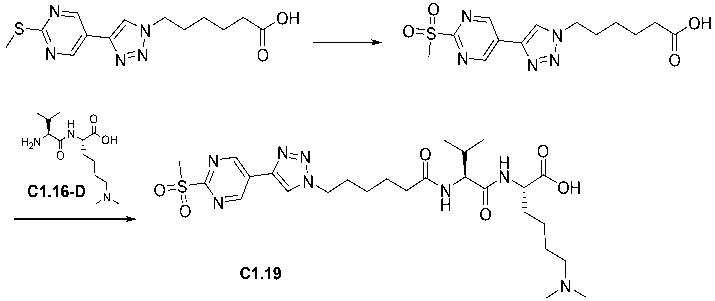

### Step 1:

Compound 6-(4-(2-(methylthio)pyrimidin-5-yl)-1*H*-1,2,3-triazol-1-yl)hexanoic acid (1.0 g, 3.3 mmol) was dissolved in a mixed solution of tetrahydrofuran and water (40 mL, 3:1), and potassium peroxymonosulfate (10.0 g, 16.3 mmol) was added thereto. The reaction mixture was stirred and reacted at room temperature for 3 hours. The completion of the reaction was detected by LCMS. The reaction mixture was filtered, and the filter cake was washed with DMSO. The filtrates were combined and purified by reversed-phase chromatography (C18, acetonitrile: 0.1% formic acid = 5% to 55%) to obtain 6-(4-(2-(methylsulfonyl)pyrimidin-5-yl)-1*H*-1,2,3-triazol-1-yl)hexanoic acid (750 mg, yield: 67.9%) as a white solid.
LCMS (ESI) [M+H]⁺ = 340.1;
¹H NMR (400 MHz, DMSO-*d*₆) δ 9.48 (s, 2H), 8.95 (s, 1H), 4.49 (t, *J =* 7.0 Hz, 2H), 3.45 (s, 3H), 2.22 (t, *J =* 7.3 Hz, 2H), 1.95 - 1.84 (m, 2H), 1.61 - 1.50 (m, 2H), 1.36 - 1.26 (m, 2H).

### Step 2:

Compound 6-(4-(2-(methylsulfonyl)pyrimidin-5-yl)-1*H*-1,2,3-triazol-1-yl)hexanoic acid (300 mg, 0.88 mmol) was dissolved in *N,N*-dimethylformamide (6 mL), then HATU (337 mg, 0.88 mmol) and DIPEA (286 mg, 2.21 mmol) were added thereto, and the mixture was stirred and reacted at room temperature for 30 minutes. Dipeptide C1.16-D (243 mg, 0.88 mmol) was then added thereto, and the mixture was stirred and reacted at room temperature for 2 hours. The completion of the reaction was detected by LCMS. The reaction mixture was purified by C18 column chromatography (acetonitrile/0.01% FA in water: 5% to 50%) to obtain the target compound *N*⁶*,N*⁶-dimethyl-*N*²-((6-(4-(2-(methylsulfonyl)pyrimidin-5-yl)-1*H-*1,2,3-triazol-1-yl)hexanoyl)-*L*-valyl)-*L*-lysine (300 mg, yield: 57 %) as a white solid.

LCMS (ESI) [M+H]⁺ = 595.5, t_{R} = 2.024 min.

¹H NMR (400 MHz, DMSO-d6) δ 9.49 (s, 2H), 9.01 (s, 1H), 7.85 (br s, 1H), 7.84 (d, *J =* 8.9 Hz, 1H), 4.48 (t, *J =* 6.8 Hz, 2H), 4.17 - 4.15 (m, 1H), 4.02 (br s, 1H), 3.44 (s, 3H), 2.42 (br s, 2H), 2.30 (s, 6H), 2.18 - 2.14 (m, 2H), 1.99 - 1.96 (m, 1H), 1.88 (dd, *J =* 14.6, 7.1 Hz, 2H), 1.67 (br s, 1H), 1.59 - 1.53 (m, 2H), 1.43 (br s, 2H), 1.28 - 1.26 (m, 5H), 0.83 - 0.89 (m, 6H).

### Example C1.20: N⁶,N⁶-Diethyl-N²-((6-(2-(methylsulfonyl)pyrimidin-5-yl)hex-5-ynoyl)-L-valyl)-L-lysine

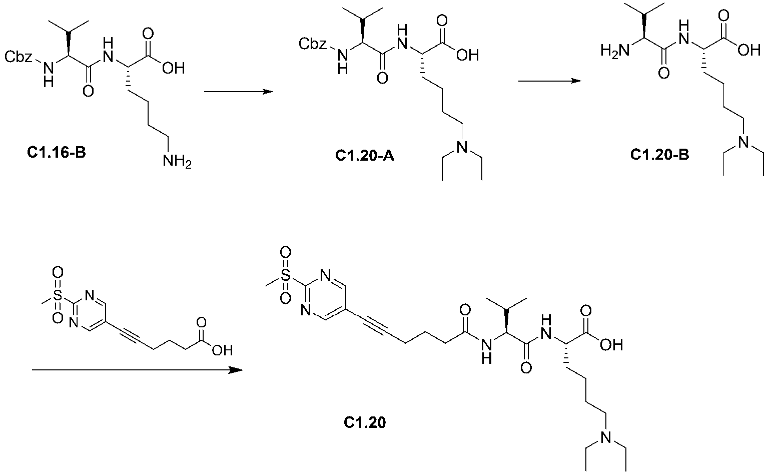

### Step 1:

Compound C1.16-B (5.0 g, 12.05 mmol) was dissolved in dichloromethane (100 mL), then acetaldehyde (3.2 g, 72.3 mmol) was added thereto, and the reaction mixture was stirred and reacted at room temperature for 10 minutes. Sodium triacetoxyborohydride (12.8 g, 60.25 mmol) was then added thereto, and the reaction mixture was stirred and reacted at room temperature for 1 hour. LCMS showed the completion of the reaction. The reaction mixture was added with saturated ammonium chloride aqueous solution, stirred for 1 hour, rotary evaporated to dryness, filtered, and the filtrate was purified by reversed-phase C18 column chromatography (acetonitrile/0.05% formic acid in water: 5% to 55%) to obtain the target compound C1.20-A (4.57 g, yield: 82.0%) as a white solid.
LCMS (ESI) [M+H]⁺ = 436.4;
¹H NMR (400 MHz, DMSO-d6) δ 7.70 (d, *J* = 7.0 Hz, 1H), 7.41 (d, *J* = 9.0 Hz, 1H), 7.38 - 7.26 (m, 5H), 5.08 - 4.99 (m, 2H), 4.00 (dd, *J =* 12.6, 6.5 Hz, 1H), 3.86 (dd, *J* = 8.6, 6.8 Hz, 1H), 2.74 (dd, *J =* 14.0, 6.9 Hz, 4H), 2.64 - 2.54 (m, 2H), 2.05 - 1.94 (m, 1H), 1.72 - 1.52 (m, 2H), 1.52 - 1.38 (m, 2H), 1.38 - 1.18 (m, 2H), 1.04 (t, *J =* 7.1 Hz, 6H), 0.87 - 0.81 (m, 6H).

### Step 2:

Compound C1.20-A (1.6 g, 3.68 mmol) was dissolved in methanol (80 mL) at room temperature, then Pd/C (0.16 g) was added thereto, and the reaction mixture was stirred and reacted at room temperature under hydrogen atmosphere for 12 hours. LCMS showed the completion of the reaction. The reaction mixture was filtered, and the filtrate was concentrated under reduced pressure to obtain the target compound C1.20-B (900 mg, yield: 82%) as an off-white solid.

¹H NMR (400 MHz, DMSO-d6) δ 8.04 (br s, 1H), 4.02 - 3.99 (m, 1H), 3.10 *(d, J=* 4.5 Hz, 1H), 2.65 (q, *J =* 7.1 Hz, 4H), 2.55 - 2.51 (m, 2H), 2.06 - 1.93 (m, 1H), 1.73 - 1.54 (m, 2H), 1.47 - 1.38 (m, 2H), 1.30 - 1.21 (m, 2H), 1.01 (t*, J =* 7.1 Hz, 6H), 0.89 (d, *J =* 6.9 Hz, 3H), 0.79 (d, *J=* 6.8 Hz, 3H).

### Step 3:

Compound 6-(2-(methylsulfonyl)pyrimidin-5-yl)hex-5-ynoic acid (268 mg, 1 mmol) was dissolved in DMF (8 mL), then HATU (380 mg, 1 mmol) and triethylamine (322 mg, 2.5 mmol) were sequentially added thereto, and the reaction mixture was stirred at room temperature for 20 minutes. Compound C1.20-B (301 mg, 1 mmol) was then added thereto, and the reaction mixture was stirred at room temperature for another 30 minutes. After the completion of the reaction was detected by LCMS, the reaction mixture was directly purified by reversed-phase C18 column chromatography (acetonitrile/0.05% formic acid in water system) to obtain the target compound (280 mg, yield: 51%) as a white solid.
LCMS (ESI) [M+H]⁺ = 552.3;
¹H NMR (400 MHz, DMSO-d6) δ 9.13 (s, 2H), 7.95 (d, *J* = 8.9 Hz, 1H), 7.86 (d,*J* = 7.2 Hz, 1H), 4.18 (dd, *J =* 8.8, 6.8 Hz, 1H), 4.02 (dd, *J =* 12.8, 7.2 Hz, 1H), 3.41 (s, 3H), 2.74 - 2.69 (m, 4H), 2.62 - 2.52 (m, 4H), 2.44 - 2.29 (m, 2H), 2.04 - 1.94 (m, 1H), 1.86 - 1.77 (m, 2H), 1.72 - 1.54 (m, 2H), 1.51 - 1.39 (m, 2H), 1.33 - 1.23 (m, 2H), 1.02 (t, *J =* 7.2 Hz, 6H), 0.87 - 0.82 (m, 6H).

### Example C1.21: N⁶,N⁶-Diethyl-N²-((6-(4-(2-(methylsulfonyl)pyrimidin-5-yl)-1H-1,2,3-triazol-1-yl)hexanoyl)-L-valyl)-L-lysine

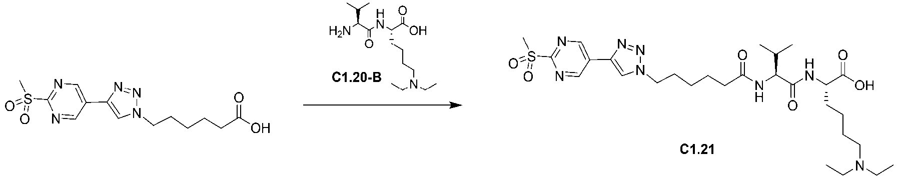

Compound 6-(4-(2-(methylsulfonyl)pyrimidin-5-yl)-1*H*-1,2,3-triazol-1-yl)hexanoic acid (500 mg, 1.475 mmol) was dissolved in *N,N*-dimethylformamide (10 mL), then HATU (560 mg, 1.475 mmol) and *N,N*-diisopropylethylamine (476 mg, 3.688 mmol) were added thereto, and the reaction mixture was stirred for 30 minutes. Compound C1.20-B (444 mg, 1.475 mmol) was then added thereto, and the reaction mixture was stirred at room temperature for 1 hour. After the completion of the reaction was detected by LCMS, the reaction mixture was directly purified by reversed-phase C18 column chromatography (acetonitrile/0.05% formic acid in water system) to obtain the target compound *N*⁶,*N*⁶-diethyl-*N*²-((6-(4-(2-(methylsulfonyl)pyrimidin-5-yl)-1*H*-1,2,3-triazol-1-yl)hexanoyl)-L-valyl)-L-lysine (330 mg, yield: 34%) as a white solid.
LCMS (ESI) [M+H]⁺ = 623.4;
¹H NMR (400 MHz, DMSO) δ 9.49 (s, 2H), 9.04 (s, 1H), 7.84 (d, *J =* 8.9 Hz, 1H), 7.80 (d, *J* = 7.1 Hz, 1H), 4.47 (t, *J =* 7.0 Hz, 2H), 4.14 (dd, *J =* 8.8, 6.6 Hz, 1H), 4.06 - 3.95 (m, 1H), 3.44 (s, 3H), 2.67 - 2.64 (m, 4H), 2.55 (t, *J =* 7.4 Hz, 2H), 2.21 - 2.10 (m, 2H), 2.02 - 1.94 (m, 1H), 1.92 - 1.84 (m, 2H), 1.72 - 1.63 (m, 1H), 1.72 - 1.63 (m, 3H), 1.59 - 1.54 (m, 2H), 1.32 - 1.21 (m, 4H), 1.01 (t, *J =* 7.1 Hz, 6H), 0.81 (dd, *J =* 9.6, 6.8 Hz, 6H).

### Example C1.22: N⁶,N⁶-Dipropyl-N²-((6-(2-(methylsulfonyl)pyrimidin-5-yl)hex-5-ynoyl)-L-valyl)-L-lysine

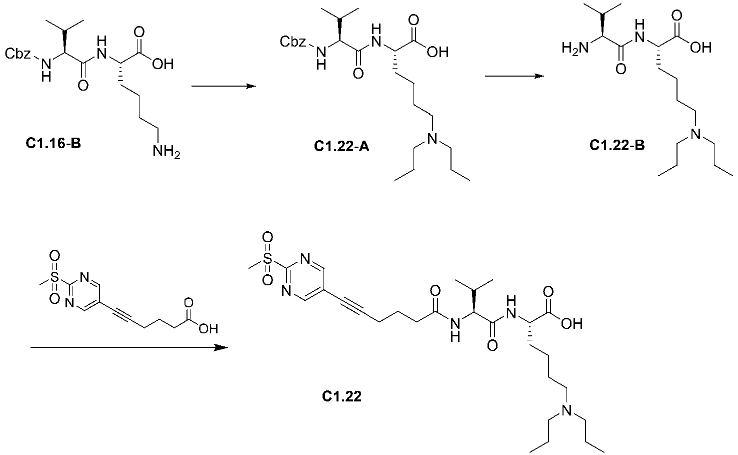

### Step 1:

Compound C1.16-B (5.0 g, 12 mmol) was dissolved in dichloromethane (100 mL), then *n*-propionaldehyde (4.2 g, 72.3 mmol) was added thereto, and the reaction mixture was stirred and reacted at room temperature for 10 minutes. Sodium triacetoxyborohydride (12.8 g, 60.25 mmol) was then added thereto, and the reaction mixture was stirred and reacted at room temperature for 1 hour. LCMS showed the completion of the reaction. The reaction mixture was added with saturated ammonium chloride aqueous solution, stirred for 1 hour, rotary evaporated to dryness, filtered, and the filtrate was purified by reversed-phase C18 column chromatography (acetonitrile/0.05% formic acid in water: 5% to 55%) to obtain the target compound C1.22-A (4.57 g, yield: 82.0%) as a white solid.
LCMS (ESI) [M+H]⁺ = 464.0;
¹H NMR (400 MHz, DMSO-*d*₆) δ 7.81 (d, *J* = 7.3 Hz, 1H), 7.38 - 7.28 (m, 5H), 5.04 (d, *J =* 1.7 Hz, 2H), 4.12 - 4.02 (m, 1H), 3.94 - 3.82 (m, 1H), 2.65 - 2.52 (m, 6H), 2.06 - 1.94 (m, 1H), 1.76 - 1.64 (m, 1H), 1.64 - 1.53 (m, 1H), 1.52 - 1.40 (m, 6H), 1.34 - 1.18 (m, 2H), 0.92 - 0.80 (m, 12H).

### Step 2:

Compound C1.22-A (2.0 g, 4.32 mmol) was dissolved in methanol (80 mL) at room temperature, then Pd/C (0.16 g) was added thereto, and the reaction mixture was stirred and reacted at room temperature under hydrogen atmosphere for 12 hours. LCMS showed the completion of the reaction. The reaction mixture was filtered, and the filtrate was concentrated under reduced pressure to obtain the target compound C1.22-B (1.2 g, yield: 85.5%) as a white solid.

### Step 3:

Compound 6-(2-(methylsulfonyl)pyrimidin-5-yl)hex-5-ynoic acid (100 mg, 0.373 mmol) was dissolved in *N,N*-dimethylformamide (1 mL), then HATU (142 mg, 0.373 mmol) and *N*,*N*-diisopropylethylamine (120 mg, 0.93 mmol) were added thereto, and the system was stirred for 30 minutes. Compound C1.22-B (122 mg, 0.371 mmol) was then added thereto, and the reaction mixture was stirred at room temperature for 1 hour. After the completion of the reaction was detected by LCMS, the reaction mixture was directly purified by reversed-phase C18 column chromatography (acetonitrile/0.05% formic acid in water system) to obtain the target compound *N*⁶,*N*⁶-dipropyl-*N*²-((6-(2-(methylsulfonyl)pyrimidin-5-yl)hex-5-ynoyl)-*L*-valyl)-*L*-lysine (50 mg, yield: 28%) as a light yellow solid.
LCMS (ESI) [M+H]⁺ = 580.0;
¹H NMR (400 MHz, DMSO-d6) δ 8.24 (s, 2H), 7.98 - 7.93 (m, 2H), 4.24 - 4.16 (m, 1H), 4.10 (d, *J =* 5.2 Hz, 1H), 3.41 (s, 3H), 2.79 - 2.64 (m, 6H), 2.55 (t, *J=* 7.1 Hz, 2H), 2.45 - 2.26 (m, 2H), 2.06 - 1.91 (m, 1H), 1.89 - 1.78 (m, 2H), 1.76 - 1.66 (m, 1H), 1.64 - 1.57 (m, 1H), 1.57 - 1.42 (m, 6H), 1.37 - 1.24 (m, 2H), 0.93 - 0.78 (m, 12H).

### Example C1.23: N⁶,N⁶-Dipropyl-N²-((6-(4-(2-(methylsulfonyl)pyrimidin-5-yl)-1H-1,2,3-triazol-1-yl)hexanoyl)-L-valyl)-L-lysine

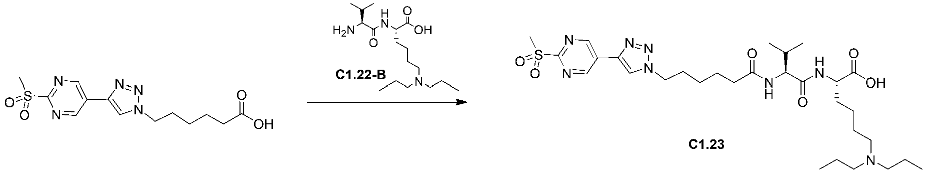

Compound 6-(4-(2-(methylsulfonyl)pyrimidin-5-yl)-1*H*-1,2,3-triazol-1-yl)hexanoic acid (500 mg, 1.475 mmol) was dissolved in *N,N*-dimethylformamide (10 mL), then HATU (560 mg, 1.475 mmol) and *N,N*-diisopropylethylamine (476 mg, 3.688 mmol) were added thereto, and the system was stirred for 30 minutes. Compound C1.22-B (485 mg, 1.475 mmol) was then added thereto, and the reaction mixture was stirred at room temperature for 1 hour. After the completion of the reaction was detected by LCMS, the reaction mixture was directly purified by reversed-phase C18 column chromatography (acetonitrile/0.05% formic acid in water system) to obtain the target compound *N*⁶,*N*⁶-dipropyl-*N*²-((6-(4-(2-(methylsulfonyl)pyrimidin-5-yl)-1*H*-1,2,3-triazol-1-yl)hexanoyl)-*L*-valyl)-*L*-lysine (320 mg, yield: 33%) as a white solid.
LCMS (ESI) [M+H]⁺ = 651.5;
¹H NMR (400 MHz, DMSO) δ 9.49 (s, 2H), 9.00 (s, 1H), 7.95 (d, *J =* 7.2 Hz, 1H), 7.79 (d, *J =* 8.9 Hz, 1H), 4.47 (t, *J =* 6.9 Hz, 2H), 4.21 - 4.11 (m, 1H), 4.09 - 4.01 (m, 1H), 3.44 (s, 3H), 2.45 - 2.35 (m, 6H), 2.23 - 2.09 (m, 2H), 1.97 - 1.86 (m, 3H), 1.72 - 1.64 (m, 1H), 1.61 - 1.50 (m, 3H), 1.43 - 1.34 (m, 6H), 1.32 - 1.22 (m, 4H), 0.86 - 0.77 (m, 12H).

### Example C1.24: tert-Butyl (S)-(6-((4-(hydroxymethyl)phenyl)amino)-5-(6-(2-(methylthio)pyrimidin-5-yl)hex-5-ynamido)-6-oxohexyl)carbamate

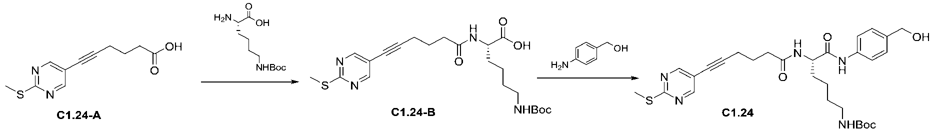

### Step 1:

Compound C1.24-A (3.70 g, 15.7 mmol), diisopropylethylamine (11.0 mL, 62.8 mmol), and HBTU (8.90 g, 23.6 mmol) were dissolved in N,N-dimethylformamide (30 mL), and the reaction mixture was reacted at room temperature for 30 minutes. Compound Boc-protected lysine (3.86 g, 15.7 mmol) was then added thereto, and the reaction mixture was reacted at room temperature for 2 hours. The completion of the reaction was detected by LCMS. The reaction mixture was added with citric acid aqueous solution (30 mL) to adjust the pH to 5, and the aqueous solution was extracted with dichloromethane (50 mL × 3). The organic phases were combined, dried over anhydrous sodium sulfate, subjected to suction filtration, and the filtrate was concentrated under reduced pressure to obtain the target compound C1.24-B (7.0 g, crude product) as a yellow oil. LCMS (ESI) [M+H]⁺ = 465.1, t_{R} = 1.751 min.

### Step 2:

Compound C1.24-B (7.00 g, 5.60 mmol), diisopropylethylamine (10.7 mL, 60.4 mmol), and HBTU (8.60 g, 22.7 mmol) were dissolved in *N,N*-dimethylformamide (100 mL), followed by addition of *p*-aminobenzyl alcohol (3.71 g, 30.2 mmol), and the reaction mixture was reacted at room temperature for 2 hours. The completion of the reaction was detected by LCMS. The reaction mixture was added with ethyl acetate (300 mL), washed with saturated brine (100 mL × 3), dried over anhydrous sodium sulfate, subjected to suction filtration, and the filtrate was concentrated under reduced pressure to obtain a solid. The crude product was purified by column chromatography (dichloromethane: methanol = 10/1) to obtain the target compound *tert-*butyl (*S*)-(6-((4-(hydroxymethyl)phenyl)amino)-5-(6-(2-(methylthio)pyrimidin-5-yl)hex-5-ynamido)-6-oxohexyl)carbamate (6.00 g, yield: 69.9%) as a yellow oil.
LCMS (ESI) [M+H]⁺ = 570.1;
¹H NMR (400 MHz, CDCl₃) δ 9.24 (s, 1H), 8.46 (s, 2H), 7.51 - 7.47 (m, 2H), 7.31 - 7.23 (m, 3H), 7.02 - 6.95 (m, 1H), 4.83 (br s, 1H), 4.63 - 4.60 (m, 2H), 3.19 - 2.96 (m, 4H), 2.57 (s, 3H), 2.52 - 2.48 (m, 2H), 2.47 - 2.41 (m, 2H), 1.99 - 1.92 (m, 2H), 1.87 - 1.77 (m, 2H), 1.76 - 1.63 (m, 2H), 1.43 (s, 9H).

### Example C1.25: tert-Butyl (S)-(6-((4-(hydroxymethyl)phenyl)amino)-5-(6-(2-(methylthio)pyrimidin-5-yl)-1H-1,2,3-triazol-1-yl)hexanamido)-6-oxohexyl)carbamate

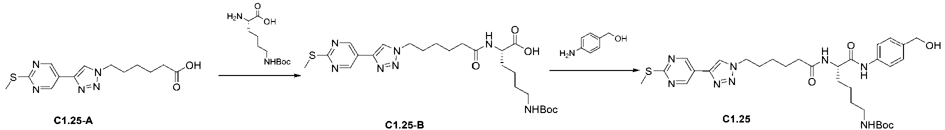

### Step 1:

Compound C1.25-A (1.60 g, 5.21 mmol), diisopropylethylamine (2.68 mL, 20.8 mmol), and HBTU (2.97 g, 7.82 mmol) were dissolved in *N,N*-dimethylformamide (30 mL), and the reaction mixture was reacted at room temperature for 30 minutes. Boc-protected lysine (1.28 g, 5.21 mmol) was then added thereto, and the reaction mixture was reacted at room temperature for 2 hours. The completion of the reaction was detected by LCMS. The reaction mixture was added with citric acid aqueous solution (30 mL) to adjust the pH to 5, and the aqueous solution was extracted with dichloromethane (50 mL × 3). The organic phases were combined, dried over anhydrous sodium sulfate, subjected to suction filtration, and the filtrate was concentrated under reduced pressure to obtain the target compound C1.25-B (3.0 g, crude product) as a yellow oil.
LCMS (ESI) [M+H]⁺ = 536.0;

### Step 2:

Compound C1.25-B (3.00 g, 5.60 mmol), diisopropylethylamine (2.89 g, 22.4 mmol), and HBTU (3.19 g, 8.40 mmol) were dissolved in *N*,*N*-dimethylformamide (30 mL), followed by addition of *p*-aminobenzyl alcohol (1.38 g, 11.2 mmol), and the reaction mixture was reacted at room temperature for 2 hours. The completion of the reaction was detected by LCMS. The reaction mixture was added with ethyl acetate (200 mL), washed with saturated brine (80 mL × 3), dried over anhydrous sodium sulfate, subjected to suction filtration, and the filtrate was concentrated under reduced pressure to obtain a solid. The crude product was purified by column chromatography (dichloromethane: methanol = 10/1) to obtain the target compound *tert*-butyl (*S*)-(6-((4-(hydroxymethyl)phenyl)amino)-5-(6-(2-(methylthio)pyrimidin-5-yl)-1*H*-1,2,3-triazol-1-yl)hexanamido)-6-oxohexyl)carbamate (2.90 g) as a yellow oil.
LCMS (ESI) [M+H]⁺ = 641.1;
¹H NMR (400 MHz, CDCl₃) δ 9.13 (s, 1H), 8.95 (s, 2H), 7.94 (s, 1H), 7.47 (d, *J* = 8.4 Hz, 2H), 7.24 (d, *J =* 8.4 Hz, 2H), 6.83 - 6.77 (m, 1H), 4.93 - 4.80 (m, 1H), 4.64 - 4.58 (m, 2H), 4.40 - 4.29 (m, 2H), 3.12 - 2.97 (m, 4H), 2.60 (s, 3H), 2.31 - 2.23 (m, 2H), 1.96 - 1.83 (m, 4H), 1.76 - 1.61 (m, 4H), 1.41 (s, 9H), 1.37 - 1.29 (m, 4H).

### Example C1.26: N⁶,N⁶-Dibutyl-N²-((6-(2-(methylsulfonyl)pyrimidin-5-yl)hex-5-ynoyl)-L-valyl)-L-lysine

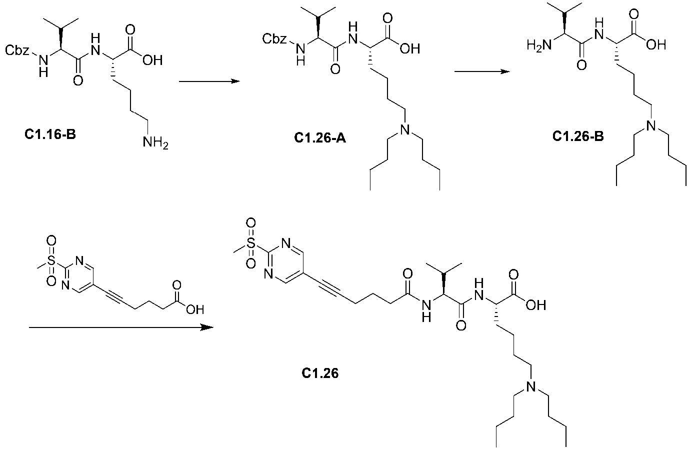

### Step 1:

Compound C1.16-B (10.0 g) was dissolved in a solution of dichloromethane (200 mL), then n-butyraldehyde (10.4 g) was added thereto, and the reaction mixture was stirred and reacted at room temperature for 10 minutes. Sodium triacetoxyborohydride (25.6 g) was then added thereto in batches, and the reaction mixture was stirred and reacted at room temperature for 1 hour. LCMS showed the completion of the reaction. The reaction mixture was added with saturated ammonium chloride aqueous solution, stirred for 1 hour, rotary evaporated to dryness, filtered, and the filtrate was purified by reversed-phase C18 column chromatography (acetonitrile/0.05% formic acid in water: 5% to 55%) to obtain the target compound C1.26-A (4.9 g) as a white solid.
LCMS (ESI) [M+H]⁺ = 492.7;
¹H NMR (400 MHz, CDCl3) δ 7.35 - 7.30 (m, 5H), 5.14 - 5.08 (m, 2H), 4.33 - 4.30 (m, 1H), 4.14 - 4.12 (m, 1H), 2.94 - 2.80 (m, 6H), 2.13 - 2.11 (m, 1H), 1.85 - 1.82 (m, 2H), 1.58 - 1.55 (m, 4H), 1.40 - 1.22 (m, 8H), 0.98 - 0.87 (m, 12H).

### Step 2:

Compound C1.26-A (5.0 g) was dissolved in a solution of methanol (100 mL) at room temperature, then Pd/C (10%, 1 g) was added thereto, and the reaction mixture was stirred and reacted under hydrogen atmosphere for 12 hours. LCMS showed the completion of the reaction. The reaction mixture was filtered, and the filtrate was concentrated under reduced pressure to obtain the target compound C1.26-B (3.68 g) as a white solid.

LCMS (ESI) [M+H]⁺ = 358.3.

### Step 3:

Compound 6-(2-(methylsulfonyl)pyrimidin-5-yl)hex-5-ynoic acid (1.0 g) was dissolved in *N,N*-dimethylformamide (15 mL), then *N,N*-diisopropylethylamine (1.2 g) and HATU (1.4 g) were sequentially added thereto, and the reaction mixture was stirred for 30 minutes. Compound C1.26-B (1.3 g) was then added thereto, and the reaction mixture was stirred at room temperature for 1 hour. After the completion of the reaction was detected by LCMS, the reaction mixture was directly purified by preparative chromatography (0.01% trifluoroacetic acid in water, acetonitrile) to obtain the target compound *N*⁶,*N*⁶-dibutyl-*N*²-((6-(2-(methylsulfonyl)pyrimidin-5-yl)hex-5-ynoyl)-*L*-valyl)-*L*-lysine (C1.26, 500 mg) as a yellow solid.
LCMS (ESI) [M+H]⁺ = 608.7;
¹H NMR (400 MHz, DMSO) δ 9.37 (s, 1H), 9.13 (s, 2H), 8.19 (d, *J* = 7.3 Hz, 1H), 7.92 (d, *J =* 8.8 Hz, 1H), 4.25 - 4.20 (m, 1H), 4.20 - 4.13 (m, 1H), 3.42 (s, 3H), 3.06 - 2.99 (m, 6H), 2.57 - 2.53 (m, 2H), 2.41 - 2.30 (m, 2H), 1.99 - 1.95 (m, 2H), 1.87 - 1.79 (m, 2H), 1.78 - 1.71 (m, 1H), 1.62 - 1.56 (m, 4H), 1.34 - 1.32 (m, 8H), 0.94 - 0.85 (m, 12H).

### Example C1.27: (S)-1-Ethyl-4-(3-methyl-2-(6-(2-(methylsulfonyl)pyrimidin-5-yl)hex-5-ynamido)butanamido)piperidine-4-carboxylic acid

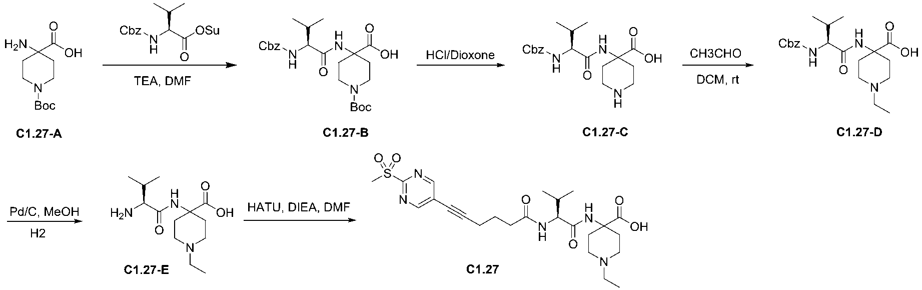

### Step 1:

Compound C1.27-A (5.0 g, 20.49 mmol) was dissolved in DMF (100 mL), then 2,5-dioxopyrrolidin-1-yl ((benzyloxy)carbonyl)-L-valinate (7.13 g, 20.49 mmol) was added thereto, and the reaction mixture was stirred and reacted at 100°C for 48 hours. LCMS showed the completion of the reaction. The reaction mixture was purified by reversed-phase C18 column chromatography (acetonitrile/0.05% formic acid in water: 5% to 55%) to obtain the target compound C1.27-B (1.3 g, yield: 13.3%) as a yellow solid.
LCMS (ESI) [M-boc]⁺ = 378.2;
¹H NMR (400 MHz, DMSO) δ 7.38 - 7.33 (m, 5H), 7.32 - 7.27 (m, 1H), 7.20 (d,*J* = 9.0 Hz, 1H), 5.14 - 5.02 (m, 2H), 3.97 - 3.87 (m, 1H), 3.70 - 3.59 (m, 2H), 3.08 - 2.95 (m, 2H), 2.04 - 1.97 (m, 1H), 1.95 - 1.87 (m, 2H), 1.76 - 1.69 (m, 2H), 1.40 (s, 9H), 0.91 - 0.84 (m, 6H).

### Step 2:

Compound C1.27-B (1.3 g, 2.71 mmol) was dissolved in a solution of ethyl acetate (10 mL) at room temperature, then dioxane-HCl (3 N, 10 mL) was added thereto, and the reaction mixture was stirred and reacted at room temperature for 1 hour. LCMS showed the completion of the reaction. The reaction mixture was concentrated under reduced pressure to obtain the target compound C1.27-C (1.06 g, yield: 94.4%) as a yellow solid.

LCMS (ESI) [M+H]⁺ = 378.4.

### Step 3:

Compound C1.27-C (1.06 g, 2.81 mmol) was dissolved in dichloromethane (10 mL), then acetaldehyde (0.75 g, 16.87 mmol) was added thereto, and the reaction mixture was stirred and reacted at room temperature for 10 minutes. Sodium triacetoxyborohydride (3 g, 14.05 mmol) was then added thereto in batches, and the reaction mixture was stirred and reacted at room temperature for 1 hour. LCMS showed the completion of the reaction. The reaction mixture was added with saturated ammonium chloride aqueous solution, stirred for 1 hour, and evaporated under reduced pressure to remove dichloromethane. The residual aqueous phase was separated by reversed-phase chromatography (acetonitrile/0.05% formic acid in water: 5% to 55%) to obtain the target compound C1.27-D (1.13 g, yield: 99.23%) as a white solid.
LCMS (ESI) [M+H]⁺ = 406.1;
1H NMR (400 MHz, MeOD) δ 7.42 - 7.25 (m, 5H), 5.22 - 5.06 (m, 2H), 3.91 (d,*J* = 7.0 Hz, 1H), 3.52 - 3.36 (m, 2H), 3.25 - 2.93 (m, 4H), 2.66 - 2.54 (m, 1H), 2.47 - 2.36 (m, 1H), 2.27 - 2.14 (m, 2H), 2.13 - 2.06 (m, 1H), 1.35 - 1.29 (m, 4H), 0.98 - 0.90 (m, 6H).

### Step 4:

Compound C1.27-D (1.13 g, 2.78 mmol) was dissolved in a solution of methanol (20 mL) at room temperature, then Pd/C (0.5 g) was added thereto, and the reaction mixture was stirred and reacted under hydrogen atmosphere for 12 hours. LCMS showed the completion of the reaction. The reaction mixture was filtered, and the filtrate was concentrated under reduced pressure to obtain the target compound C1.27-E (0.58 g, yield: 76.18%) as a white solid. LCMS (ESI) [M+H]⁺ = 272.4.

### Step 5:

Compound 6-(2-(methylsulfonyl)pyrimidin-5-yl)hex-5-ynoic acid (1 g, 3.73 mmol) was dissolved in *N,N-*dimethylformamide (5 mL), then *N,N*-diisopropylethylamine (1.2 g, 9.32 mmol) and HATU (1.42 g, 3.73 mmol) were sequentially added thereto, and the reaction mixture was stirred for 30 minutes. Compound C1.27-E (1.01 g, 3.73 mmol) was then added thereto, and the reaction mixture was stirred at room temperature for 1 hour. After the completion of the reaction was detected by LCMS, the reaction mixture was directly purified by preparative chromatography (0.01% trifluoroacetic acid in water, acetonitrile) to obtain the target compound (S)-1-ethyl-4-(3-methyl-2-(6-(2-(methylsulfonyl)pyrimidin-5-yl)hex-5-ynamido)butanamido)piperidine-4-carboxylic acid (C1.27, 500 mg, yield: 28.7%) as a yellow solid.
LCMS (ESI) [M+H]⁺ = 522.5;
¹H NMR (400 MHz, DMSO) δ 9.12 (s, 2H), 8.08 (s, 1H), 7.88 (d, *J =* 9.0 Hz, 1H), 4.28 - 4.25 (m, 1H), 3.41 (s, 3H), 2.89 - 2.78 (m, 2H), 2.57 - 2.52 (m, 4H), 2.45 - 2.30 (m, 4H), 2.07 - 2.01 (m, 2H), 1.99 - 1.96 (m, 1H), 1.95 - 1.88 (m, 2H), 1.85 - 1.79 (m, 2H), 1.04 (t, *J =* 7.1 Hz, 3H), 0.88 - 0.83 (m, 6H).

### II. Synthesis of drug-linker compounds

### Example 2.1: N-((S)-1-(((S)-6-Amino-1-((2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-001)

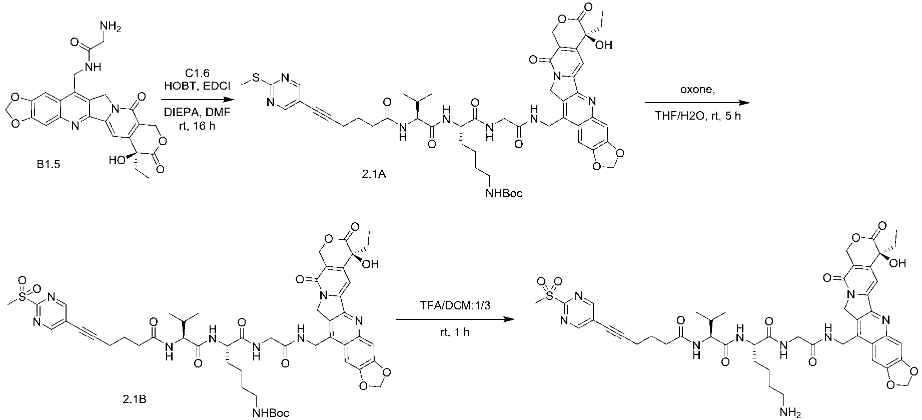

### Step 1:

Compound C1.6 (50 mg, 0.10 mol), compound B1.5 (71 mg, 0.12 mol), and *N,N-*diisopropylethylamine (39 mg, 0.30 mol) were dissolved in DMF (5 mL). Then, 1-hydroxybenzotriazole (16 mg, 0.12 mmol) and 1-(3-dimethylaminopropyl)-3-ethylcarbodiimide hydrochloride (23 mg, 0.12 mmol) were sequentially added thereto. The above reaction mixture was stirred at room temperature for 16 hours, diluted with 50 mL of ethyl acetate, and the organic phase was washed with water three times (25 mL × 3), dried over anhydrous sodium sulfate, and concentrated to remove ethyl acetate to obtain a crude product. The crude product was purified by flash silica gel column chromatography (DCM: MeOH = 100:0 to 90:10) to obtain the target compound 2.1A (62 mg, yellow solid, yield: 58%).

LCMS (ESI) [M+H]⁺: 1024.8.

### Step 2:

Compound 2.1A (40 mg, 0.04 mmol) was dissolved in a mixed solution of tetrahydrofuran and water (volume ratio of 1/1; 4 mL), and potassium peroxymonosulfate (246 mg, 0.4 mmol) was added thereto. The reaction mixture was stirred at room temperature for 5 hours, diluted with 30 mL of ethyl acetate, and the organic phase was washed with water three times (25 mL × 3), dried over anhydrous sodium sulfate, and concentrated to remove ethyl acetate to obtain the target compound 2.1B (30 mg, yellow solid, yield: 74%).

LCMS (ESI) [M+H]⁺ = 1055.9.

### Step 3:

Compound 2.1B (30 mg, 0.03 mmol) was dissolved in a mixed solution of trifluoroacetic acid and dichloromethane (volume ratio of 1:3; 4 mL), and the reaction mixture was stirred at room temperature for 1 hour. The reaction mixture was then concentrated under reduced pressure to obtain a crude product, which was purified by preparative chromatography (0.01% TFA in water, MeCN) to obtain the target compound *N*-((*S*)-1-(((*S*)-6-amino-1-((2-((((*S*)-7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)methyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (9 mg, white solid, yield: 33%).
LCMS (ESI) [M+H]⁺: 955.8;
¹H NMR (400 MHz, DMSO-*d6*) δ 9.09 (s, 2H), 8.54 (s, 1H), 8.15 (m, 2H), 7.84 (m, 1H), 7.80 (s, 1H), 7.64 (br s, 3H), 7.53 (s, 1H), 7.26 (s, 1H), 6.51 (s, 1H), 6.30 (d, *J =* 2.9 Hz, 2H), 5.59 - 5.46 (m, 2H), 5.43 (s, 2H), 4.87 - 4.78 (m, 1H), 4.70 - 4.60 (m, 1H), 4.17 - 4.03 (m, 2H), 3.82 - 3.58 (m, 2H), 3.41 (s, 3H), 2.80 - 2.70 (m, 2H), 2.40 - 2.22 (m, 2H), 1.93 - 1.68 (m, 6H), 1.65 - 1.57 (m, 1H), 1.56 - 1.45 (m, 3H), 1.36 - 1.22 (m, 2H), 0.87 (t, *J =* 7.3 Hz, 3H), 0.80 (d, *J =* 5.2 Hz, 6H).

Using the same method and reaction conditions as in Example 2.1, and using the different starting materials shown, the target products shown in Examples 2.2 to 2.8 in the table below were obtained.

| Example | Compound | Name | (m/z): [M+H]⁺ |
|---|---|---|---|
| Example 2.2 | 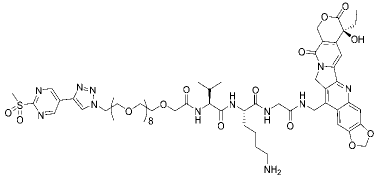 | N-((*S*)-1-(((*S*)-6-Amino-1-((2-(((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2 -*b*]quinolin-14-yl)methyl)amino)-2-oxoethyl)amino)-1-oxo-hex-2-yl)amino)-3-methyl-1-oxo-but-2-yl)-29-(4-(2-(methylsulfonyl)pyrimidin-5-yl)-1*H*-1,2,3-triazol-1-yl)-3,6,9,12,15,18,21,24,27-nonaoxanonacosanamide | 1366.9 |
| | DL-002 | | |
| | Raw materials: C1.2, B1.5 | | |
| Example 2.3 | 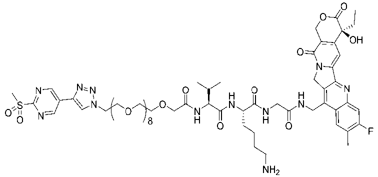 | *N*-((*S*)-1-(((*S*)-6-Amino-1-((2-(((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)methyl)amino)-2-oxoethyl)amino)-1-oxohexan-2-hexyl)amino)-3-methyl-1-oxobut-2-yl)-29-(4-(2-(methylsulfonyl)pyrimidin-5-yl)-1*H*-1,2,3-triazol-1-yl)-3,6,9,12,15,18,21,24,27-nonaoxanonacosanamide | 1354.9 |
| | DL-003 | | |
| | Raw materials: C1.2, B1.4 | | |
| Example 2.4 | 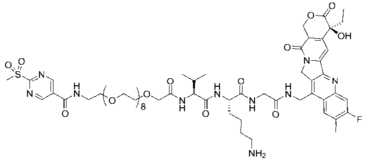 | *N-*((7*S*,10*S*)-7-(4-Aminobutyl)-1-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)-10-isopropyl-3,6,9,12-tetraoxo-14,17,20,23,26,29,32,35,38-nonaoxa-2,5,8,11-tetraazatetracontan-40-yl)-2-(methylsulfonyl)pyrimidine-5-carboxamide | 1330.9 |
| | DL-004 | | |
| | Raw materials: C1.1, B1.4 | | |
| Example 2.5 | 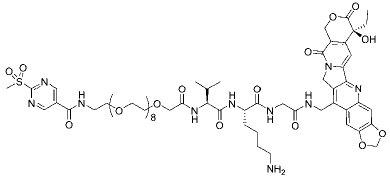 | *N-*((7*S*,10*S*)-7-(4-Aminobutyl)-1-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2 -*b*]quinolin-14-yl)-10-isopropyl-3,6,9,12-tetraoxo-14,17,20,23,26,29,32,35,38-nonaoxa-2,5,8,11-tetraazatetracontan-40-yl)-2-(methylsulfonyl)pyrimidine-5-carboxamide | 1342.8 |
| | DL-005 | | |
| | Raw materials: C1.1, B1.5 | | |
| Example 2.6 | 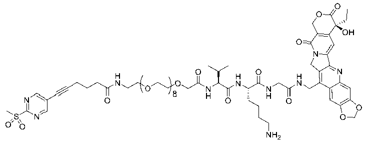 | *N*-((*S*)-1-(((*S*)-6-Amino-1-((2-(((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2 -*b*]quinolin-14-yl)methyl)amino)-2-oxoethyl)amino)-1-oxohex-2-yl)amino)-3-methyl-1-oxobut-2-yl)-29-(6-(2-(methylsulfonyl)pyrimidin-5-yl)hex-5-ynamido)-3,6,9,12,15,18,21,24,27-nonaoxanonacosanamide | 1408.5 |
| | DL-006 | | |
| | Raw materials: C1.5, B1.5 | | |
| Example 2.7 | 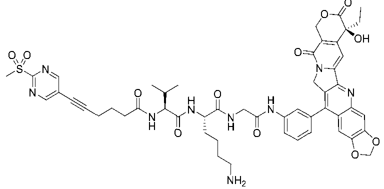 | *N-*((*S*)*-*1-(((*S*)-6-Amino-1-((2-((3-((S)-7-ethyl-7-hydroxy-8,11-dioxo-7,8,11,13-tetrahydro-10*H-*[1,3]dioxolo[4,5-*g*]pyrano[3',4':6,7]indolizino[1,2 -*b*]quinolin-14-yl)phenyl)amino)-2-oxoethyl)amino)-1-oxohexan-2-hexyl)amino)-3-methyl-1-oxobutan-2-yl)-6-(2-(methylsulfonyl)pyrimidin-5-yl)hex-5-ynamide | 1017.9 |
| | DL-007 | | |
| | Raw materials: C1.7, B1.8 | | |
| Example 2.8 | 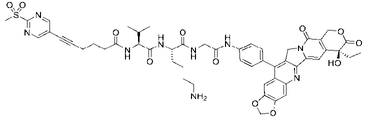 | *N*-((*S*)-1-(((*S*)-6-Amino-1-((2-((4-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2 -*b*]quinolin-14-yl)phenyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide | 1017.8 |
| | DL-008 | | |
| | Raw materials: C1.7, B1.9 | | |

### Example 2.9: (S)-6-Amino-N-(2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-009)

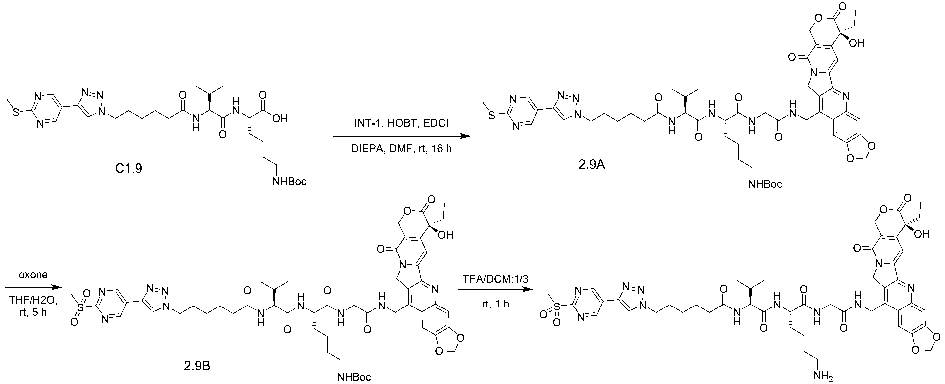

### Step 1:

Compound B1.5 (30 mg), compound C1.9 (48 mg), and *N,N*-diisopropylethylamine (23 mg) were dissolved in DMF (5 mL). 1-Hydroxybenzotriazole (9 mg) and 1-(3-dimethylaminopropyl)-3-ethylcarbodiimide hydrochloride (13 mg) were added to the above mixture. The above reaction mixture was stirred at room temperature for 16 hours, diluted with 50 mL of ethyl acetate, and the organic phase was washed with water three times (25 mL × 3), dried over anhydrous sodium sulfate, and concentrated to remove ethyl acetate to obtain a crude product. The crude product was purified by flash chromatography (DCM: MeOH = 100:0 to 90:10) to obtain the target compound 2.9A (35 mg, light yellow solid, yield: 43%).

LCMS (ESI) [M+H]⁺: 1095.8.

### Step 2:

Compound 2.9A (35 mg) was dissolved in a mixed solution of tetrahydrofuran and water (volume ratio of 1/1; 4 mL), and potassium peroxymonosulfate (184 mg) was added thereto. The reaction mixture was stirred at room temperature for 5 hours, diluted with 30 mL of ethyl acetate, and the organic phase was washed with water three times (25 mL × 3), dried over anhydrous sodium sulfate, and concentrated to remove ethyl acetate to obtain the target compound 2.9B (25 mg, light yellow solid, yield: 71%).

LCMS (ESI) [M+H]⁺: 1126.9.

### Step 3:

Compound 2.9B (25 mg) was dissolved in a mixed solution of trifluoroacetic acid and dichloromethane (volume ratio of 1:3; 4 mL), and the reaction mixture was stirred at room temperature for 1 hour. The reaction mixture was concentrated to obtain a crude product, which was purified by prep-HPLC (0.01% TFA in water, MeCN) to obtain the target compound (*S*)-6-amino-*N*-(2-((((*S*)-7-ethyl-7-hydroxy-8,11-dioxo-7*,*8,11*,*13-tetrahydro*-*10*H-*[1,3]dioxolo[4,5-*g*]pyrano[3',4':6,7]indolizino[1,2-*b*]quinolin-14-yl)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide trifluoroacetate (5.1 mg).
LCMS (ESI) [M+H]⁺: 1026.9;
¹H NMR (400 MHz, DMSO) δ 9.46 (s, 2H), 8.91 (s, 1H), 8.53 (s, 1H), 8.18 (s, 1H), 8.11 - 8.10 (m, 1H), 7.79 (s, 1H), 7.73 - 7.72 (m, 1H), 7.63 (s, 3H), 7.53 (s, 1H), 7.26 (s, 1H), 6.52 (s, 1H), 6.29 (m, 2H), 5.61 - 5.46 (m, 2H), 5.43 (s, 2H), 4.85 - 4.83 (m, 1H), 4.63 - 4.60 (m 1H), 4.44 (s, 2H), 4.06 - 4.03 (m, 3H), 2.75 (s, 2H), 2.12 - 2.10 (m, 2H), 1.89 - 1.85 (m, 6H), 1.56 - 1.53 (m, 8H), 1.24 (br s, 4H), 0.87 - 0.84 (m, 3H), 0.77 (d, *J =* 6.1 Hz, 6H).

### Example 2.10: N-((7S,10S)-7-(4-Aminobutyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-10-isopropyl-3,6,9,12,16-pentaoxo-14,20,23,26,29,32,35,38,41-nonaoxa-2,5,8,11,17-pentazatritetracontan-43-yl)-6-(2-(methylsulfonyl)pyrimidin-5-yl)hex-5-ynamide (DL-010)

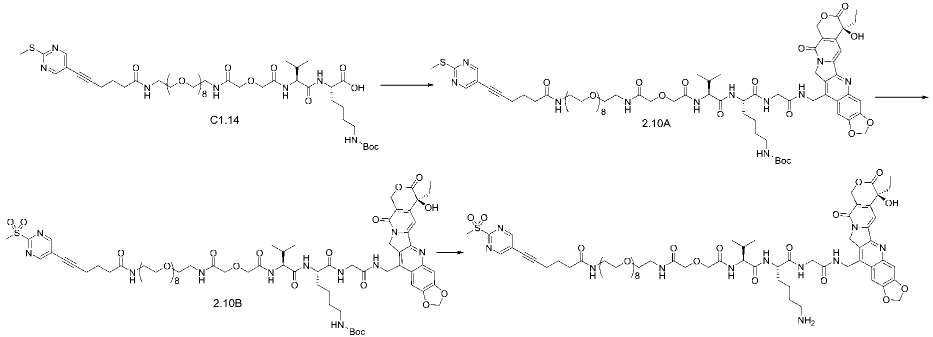

### Step 1:

Compound C1.14 (112 mg) was dissolved in *N,N*-dimethylformamide (2 mL), then triethylamine (32 mg, 0.31 mmol), 1-hydroxybenzotriazole (17 mg, 0.13 mmol), and 1-(3-dimethylaminopropyl)-3-ethylcarbodiimide hydrochloride (24 mg, 0.13 mmol) were sequentially added thereto. The mixture was stirred for 20 minutes, and then compound B1.5 (50 mg) was added thereto. The reaction mixture was stirred at 40°C overnight. The reaction mixture was added with 10 mL of water, extracted with dichloromethane, dried over anhydrous sodium sulfate, subjected to suction filtration, and rotary evaporated to dryness to obtain a crude product. The crude product was purified by reversed-phase C18 column chromatography (acetonitrile/0.01% FA in water: 0% to 55%) to obtain the target compound 2.10A (75 mg, yield: 46%) as a white solid.

LCMS (ESI) [M+H]⁺: 1534.0.

### Step 2:

Compound 2.10A (75 mg) was dissolved in a solution of tetrahydrofuran and water (8 mL, 1:1), then potassium peroxymonosulfate (211 mg) was added thereto, and the reaction mixture was stirred and reacted at room temperature for 10 hours. The reaction mixture was added with 10 mL of dichloromethane, extracted to separate the phases, dried over anhydrous sodium sulfate, subjected to suction filtration, and rotary evaporated to dryness to obtain the target product 2.10B (50 mg, yield: 65%).

LCMS (ESI) [M+H]⁺: 1566.2.

### Step 3:

Compound 2.10B (50 mg) was dissolved in a mixed solution of trifluoroacetic acid and dichloromethane (volume ratio of 1:2; 4 mL), and the reaction mixture was stirred at room temperature for 1 hour. The reaction mixture was concentrated to obtain a crude product, which was subjected to preparative chromatography (0.01% TFA in water, MeCN) to obtain the target compound *N*-((7*S*,10*S*)-7-(4-aminobutyl)-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-10-isopropyl-3,6,9,12,16-pentaoxo-14,20,23,26,29,32,35,38,41-nonaoxa-2,5,8,11,17-pentazatritetracontan-43-yl)-6-(2-(methylsulfonyl)pyrimidin-5-yl)hex-5-ynamide (10.5 mg, yield: 21%).
LCMS (ESI) [M+H]⁺: 1466.9;
¹H NMR (400 MHz, DMSO-d6) δ 9.12 (s, 2H), 8.62 (t, *J =* 5.7 Hz, 1H), 8.26 - 8.11 (m, 2H), 8.03 (t, *J =* 5.8 Hz, 1H), 7.93 (t, *J =* 5.3 Hz, 1H), 7.85 (d, *J* = 8.7 Hz, 1H), 7.79 (d, *J* = 5.6 Hz, 1H), 7.61 (s, 3H), 7.54 (s, 1H), 7.26 (s, 1H), 6.50 (s, 1H), 6.31 (d, *J =* 1.7 Hz, 2H), 5.46 (d, *J =* 17.2 Hz, 4H), 4.76 (dt, *J =* 14.8, 8.7 Hz, 2H), 4.20 - 4.15 (m, 4H), 4.00 (s, 3H), 3.95 (d, *J* = 5.5 Hz, 3H), 3.72 - 3.67 (m, 2H), 3.50 (d, *J =* 3.9 Hz, 30H), 3.41 (s, 3H), 3.25 - 3.19 (m, 4H), 2.76 (d, *J =* 5.6 Hz, 2H), 2.56 (t, *J =* 4.9 Hz, 2H), 2.27 (t, *J =* 7.3 Hz, 2H), 1.90 (dd, *J =* 16.1, 7.4 Hz, 2H), 1.84 - 1.78 (m, 2H), 1.63 (s, 1H), 1.53 - 1.48 (m, 2H), 1.30 (d, *J =* 7.7 Hz, 2H), 0.87 - 0.84 (m, 4H), 0.78 - 0.75 (m, 6H).

### Example 2.11: N-((S)-1-(((S)-6-Amino-1-((2-((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-011)

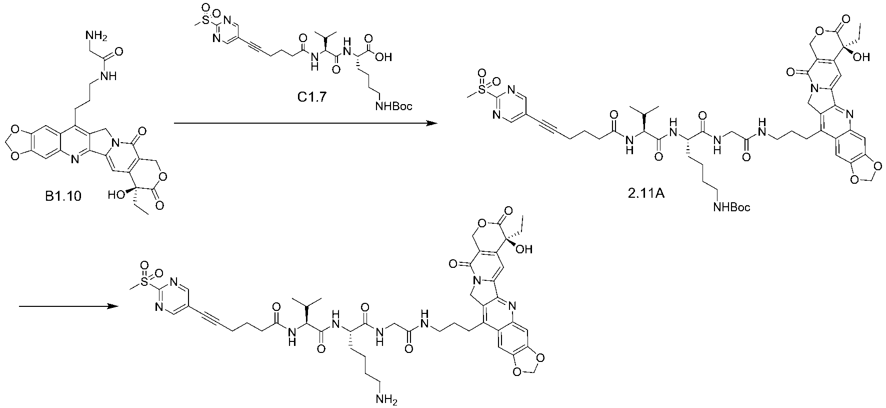

### Step 1:

Compound C1.7 (107 mg), triethylamine (45 mg), HOBT (25 mg), and EDCI (35 mg) were sequentially added to a solution of compound B 1.10 (75 mg) in *N*,*N-*dimethylformamide (3 mL), and the reaction mixture was reacted at 35°C for 4 hours. The reaction mixture was added with water (30 mL) and extracted with ethyl acetate (30 mL × 2). The organic phase was dried over anhydrous sodium sulfate and concentrated to obtain the target compound 2.11A (60 mg, yield: 37%).
LCMS (ESI) [M+H]⁺: 1084;

### Step 2:

A mixed solution of trifluoroacetic acid and dichloromethane (volume ratio of 1:2; 4 mL) was added to a dichloromethane solution (4 mL) of compound 2.11A (60 mg) and the reaction mixture was stirred and reacted at room temperature for 1 hour. The reaction mixture was concentrated and the crude product was purified by preparative high-performance liquid chromatography (acetonitrile/0.05% formic acid in water) to obtain the target compound *N-*((*S*)-1-(((*S*)-6-amino*-*1-((2-((3-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (9 mg, yield: 18%).
LCMS (ESI) [M+H]⁺: 983.9;
¹H NMR (400 MHz, DMSO-d6) δ 9.08 (s, 2H), 8.34 (s, 1H), 8.24 (s, 1H), 8.19 (d, *J* = 7.1 Hz, 1H), 8.00 (d, *J =* 8.3 Hz, 1H), 7.95 (s, 1H), 7.63 (s, 1H), 7.49 (d, *J =* 2.8 Hz, 1H), 7.24 (s, 1H), 6.50 (s, 1H), 6.28 (d, *J =* 1.9 Hz, 2H), 5.42 (s, 2H), 5.24 (s, 2H), 4.25 - 4.07 (m, 2H), 3.74 - 3.70 (m, 3H), 3.25 (d, *J =* 12.4 Hz, 3H), 3.08 (d, *J=* 7.6 Hz, 2H), 2.73 (s, 2H), 2.40 - 2.19 (m, 3H), 2.01 - 1.64 (m, 10H), 1.52 (br s, 4H), 1.34 (br s, 2H), 0.87 (t, *J =* 7.3 Hz, 3H), 0.83 - 0.75 (m, 6H).

### Example 2.12: (S)-6-Amino-N-(2-((((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)amino)-2-oxoethyl)-2-((S)-2-isopropyl-35-(4-(2-(methylsulfonyl)pyrimidin-5-yl)-1H-1,2,3-triazol-1-yl)-4,8-dioxo-6,12,15,18,21,24,27,30,33-nonaoxa-3,9-diazapentatriacontanamido)hexanamide (DL-012)

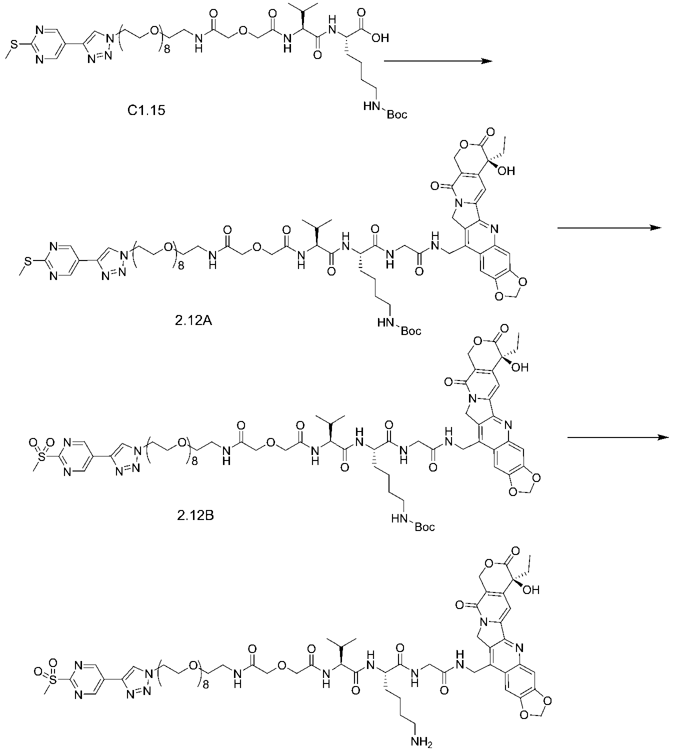

### Step 1:

Compound C1.15 (80 mg), compound B1.5 (37 mg), and triethylamine (23 mg, 0.231 mo,) were dissolved in DMF (5 mL). 1-Hydroxybenzotriazole (12 mg) and 1-(3-dimethylaminopropyl)-3-ethylcarbodiimide hydrochloride (17 mg) were added to the above mixture. The above reaction mixture was stirred at room temperature for 16 hours, diluted with 50 mL of ethyl acetate, and the organic phase was washed with water three times (25 mL × 3), dried over anhydrous sodium sulfate, and concentrated to remove ethyl acetate to obtain a crude product. The crude product was purified by flash chromatography (DCM: MeOH = 100:0 to 90:10) to obtain the target compound 2.12A (75 mg, yield: 65%).

LCMS (ESI) [M+H]⁺: 1492.0.

### Step 2:

Compound 2.12A (50 mg) was dissolved in a mixed solution of tetrahydrofuran and water (volume ratio of 1/1; 4 mL), and potassium peroxymonosulfate (202 mg) was added thereto. The reaction mixture was stirred at room temperature for 5 hours, diluted with 30 mL of ethyl acetate, and the organic phase was washed with water three times (25 mL × 3), dried over anhydrous sodium sulfate, and concentrated to remove ethyl acetate to obtain the target compound 2.12B (40 mg, yield: 80%).

LCMS (ESI) [M+H]⁺: 1524.1.

### Step 3:

Compound 2.12B (30 mg) was dissolved in a mixed solution of trifluoroacetic acid and dichloromethane (volume ratio of 1:3; 4 mL), and the reaction mixture was stirred at room temperature for 1 hour. The reaction mixture was concentrated to obtain a crude product, which was purified by prep-HPLC (0.01% trifluoroacetic acid in water, acetonitrile) to obtain the target compound (*S*)-6-amino-*N*-(2-((((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)amino)-2-oxoethyl)-2-((*S*)-2-isopropyl-35-(4-(2-(methylsulfonyl)pyrimidin-5-yl)-1*H*-1,2,3-triazol-1-yl)-4,8-dioxo-6,12,15,18,21,24,27,30,33-nonaoxa-3,9-diazapentatriacontanamido)hexanamide (9 mg, yield: 30%).

LCMS (ESI) [M+H]⁺: 1423.9.

### Example 2.13: N-((13S,16S)-13-(4-(Dimethylamino)butyl)-3-ethyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (DL-013)

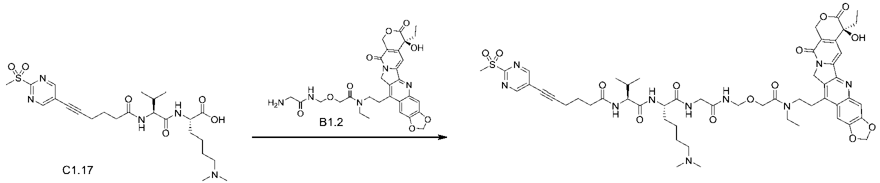

Compound C1.17 (50 mg), compound B1.2 (58 mg), and triethylamine (24 mg) were dissolved in *N,N*-dimethylformamide (2 mL). Then 1-hydroxybenzotriazole (16 mg) and 1-(3-dimethylaminopropyl)-3-ethylcarbodiimide hydrochloride (22 mg) were sequentially added thereto, and the reaction mixture was stirred at room temperature for 16 hours. The reaction mixture was directly purified by preparative chromatography (0.01% formic acid in water, acetonitrile) to obtain the target compound (21 mg, yield: 10%).
LCMS (ESI) [M+H]⁺: 1113.0;
¹H NMR (400 MHz, MeOD) δ 8.93 (s, 2H), 8.40 - 8.33 (m, 1H), 8.30 - 8.25 (m, 1H), 8.25 - 8.21 (m, 1H), 7.65 (s, 1H), 7.48 (s, 1H), 7.34 (s, 1H), 6.22 (s, 2H), 5.47 (dd, *J =* 78.6, 16.4 Hz, 2H), 5.28 - 5.13 (m, 2H), 4.73 - 4.59 (m, 2H), 4.30 - 4.05 (m, 4H), 3.94 - 3.82 (m, 2H), 3.70 - 3.57 (m, 2H), 3.54 - 3.44 (m, 1H), 3.34 (s, 6H), 3.16 - 3.08 (m, 2H), 2.90 (s, 6H), 2.58 - 2.40 (m, 4H), 2.04 - 1.83 (m, 6H), 1.80 - 1.68 (m, 3H), 1.54 - 1.36 (m, 2H), 1.24 - 1.15 (m, 3H), 1.03 - 0.86 (m, 9H).

### Example 2.14: N-((13S,16S)-13-(4-(N-oxo-N,N-dimethylamino)butyl)-3-ethyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (DL-014)

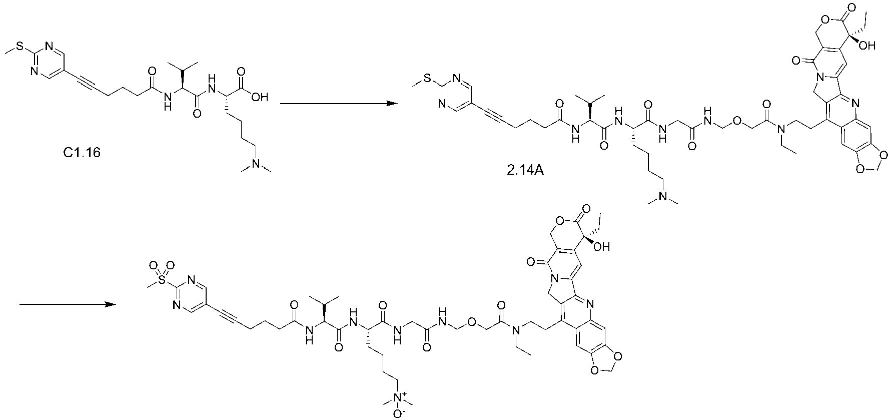

### Step 1:

Compound C1.16 (49 mg), compound B1.2 (71 mg), and*N,N*-diisopropylethylamine (39 mg) were dissolved in DMF (5 mL). Then, 1-hydroxybenzotriazole (16 mg) and 1-(3-dimethylaminopropyl)-3-ethylcarbodiimide hydrochloride (23 mg) were sequentially added thereto. After the addition, the above reaction mixture was stirred at room temperature for 16 hours, diluted with 50 mL of ethyl acetate, and the organic phase was washed with water three times (25 mL × 3), dried over anhydrous sodium sulfate, and concentrated to remove ethyl acetate to obtain a crude product. The crude product was purified by flash silica gel column chromatography (DCM: MeOH = 100:0 to 90:10) to obtain the target compound 2.14A (61 mg, yield: 60%).
LCMS (ESI) [M+H]⁺: 1081.6;

### Step 2:

Compound 2.14A (40 mg) was dissolved in a mixed solution of tetrahydrofuran and water (volume ratio of 1/1; 4 mL), and potassium peroxymonosulfate (227 mg, 0.37 mmol) was added thereto. The reaction mixture was stirred at room temperature for 5 hours, and LCMS showed the completion of the reaction. The reaction mixture was diluted with 30 mL of ethyl acetate, and the organic phase was washed with water three times (25 mL × 3), dried over anhydrous sodium sulfate, and concentrated to remove ethyl acetate to obtain a crude compound. The crude product was purified by preparative chromatography (0.01% formic acid in water, acetonitrile) to obtain the target compound *N*-((13*S,*16*S*)*-*13-(4-(*N-*oxo-*N,N-*dimethylamino)butyl)-3-ethyl-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (9 mg, yield: 33%).

LCMS (ESI) [M+H]⁺= 1129.0.

### Example 2.15: N-((13S,16S)-13-(4-(N,N-Dimethylamino)butyl)-3-isopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (DL-015)

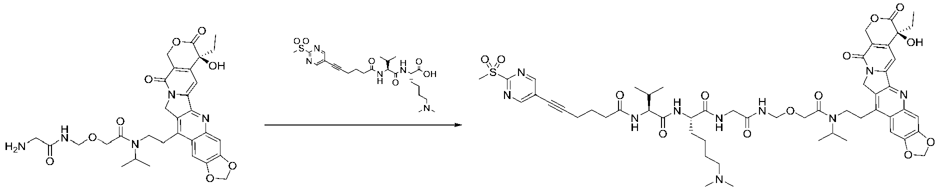

HBTU (31 mg) and diisopropylethylamine (21 mg) were sequentially added to a solution of compound *N*⁶,*N*⁶-dimethyl-*N*²-((6-(2-(methylsulfonyl)pyrimidin-5-yl)hex-5-ynoyl)-*L*-valyl)-*L*-lysine (42 mg, C1.17) and compound (*S*)-2-((2-aminoacetamido)methoxy)-*N-*isopropyl-*N-*(2-(7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)ethyl)acetamide (50 mg, B1.11) in *N,N-*dimethylformamide (10 mL). The reaction mixture was stirred at room temperature for 2 hours. The reaction mixture was filtered and then purified by preparative high-performance liquid chromatography (acetonitrile/0.05% trifluoroacetic acid in water) to obtain the target compound *N*-((13*S*,16*S*)-13-(4-(*N*,*N*-dimethylamino)butyl)-3-isopropyl-1-((*S*)-7-ethyl-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)-17-methyl-4,9,12,15-tetraoxo-6-oxa-3,8,11,14-tetraazaoctadecan-16-yl)-6-(2-(methylsulfonyl)pyrimidin-5-yl)hex-5-ynamide (15.2 mg).
LCMS (ESI) [M+H]⁺ = 1127.6;
¹H NMR (400 MHz, DMSO-d6) δ 9.10 (s, 2H), 8.73 (t, *J* = 8.7 Hz, 1H), 8.23 - 8.17 (m, 1H), 8.10 (d, *J* = 7.5 Hz, 1H), 7.97 - 7.93 (m, 2H), 7.52 (s, 1H), 7.25 (s, 1H), 6.50 (s, 1H), 6.30 (s, 2H), 5.42 (s, 2H), 5.36 (s, 2H), 4.66 (d, *J =* 6.6 Hz, 2H), 4.33 - 4.21 (m, 4H), 4.20 - 4.13 (m, 2H), 3.76 - 3.74 (m, 2H), 3.40 (s, 3H), 3.40 - 3.38 (m, 1H), 3.30 - 3.21 (m, 2H), 3.04 - 2.95 (m, 2H), 2.76 (s, 3H), 2.74 (s, 3H), 2.39 - 2.31 (m, 2H), 2.00 - 1.74 (m, 6H), 1.68 - 1.49 (m, 4H), 1.37 - 1.27 (m, 2H), 1.23 - 1.16 (m, 6H), 0.89 - 0.82 (m, 9H).

### Example 2.16: N-((10S,13S)-10-(4-(N,N-Dimethylamino)butyl)-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-14-methyl-1,6,9,12-tetraoxo-3-oxa-5,8,11-triazapentadecan-13-yl)-6-(2-(methylsulfonyl)pyrimidin-5-yl)hex-5-ynamide (DL-016)

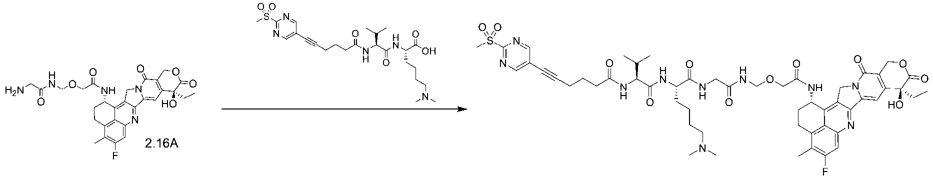

Compound *N*⁶,*N*⁶-dimethyl-*N*²-((6-(2-(methylsulfonyl)pyrimidin-5-yl)hexadecyl-5-ynoyl)-*L*-propionyl)-*L*-lysine (C1.17, 52.3 mg) was dissolved in *N,N*-dimethylformamide (2 mL), and then HBTU (38 mg, 0.10 mmol) and *N,N-*diisopropylethylamine (26 mg, 0.20 mmol) were sequentially added thereto. After the addition, the reaction mixture was stirred at room temperature for 30 minutes, and then compound 2-amino-*N*-((2-(((1*S*,9*S*)-9-ethyl-5-fluoro-9-hydroxy-4-methyl*-*10*,*13*-*dioxo-2,3,9,10*,*13,15-hexahydro-1*H,*12*H-*benzo[*de*]pyrano[3',4':6,7]indolizino[1,2-*b*]quinolin-1-yl)amino)-2-oxoethoxy)methyl)acetamide (2.16A, prepared according to the same method reported on pages 147 to 148 of CN104755494A, 63 mg) was added thereto. After the completion of the reaction was detected by LCMS, the reaction mixture was directly purified by preparative chromatography (0.01% trifluoroacetic acid in water, acetonitrile) to obtain the target compound *N*-((10S,13*S*)-10-(4-(*N,N*-dimethylamino)butyl)-1-(((1*S*,9*S*)-9-ethyl-5-fluoro-9-hydroxy-4-methyl-10,13-dioxo-2,3,9,10,13,15-hexahydro-1*H*,12*H-*benzo[*de*]pyrano[3',4':6,7]indolizino[1,2-*b*]quinolin-1-yl)amino)-14-methyl-1,6,9,12-tetraoxo-3-oxa-5,8,11-triazapentadecan-13-yl)-6-(2-(methylsulfonyl)pyrimidin-5-yl)hex-5-ynamide (8.1 mg, yield: 7%).
LCMS (ESI) [M+H]⁺ = 1085.5;
¹H NMR (400 MHz, DMSO-d6) δ 9.31 (s, 1H), 9.11 (s, 2H), 8.72 (t, *J =* 6.8 Hz, 1H), 8.51 (d, *J=* 8.8 Hz, 1H), 8.19 (t, *J =* 6.8 Hz, 1H), 8.10 (d*, J =* 7.1 Hz, 1H), 7.91 (d, *J =* 8.6 Hz, 1H), 7.79 (d, *J =* 10.9 Hz, 1H), 7.32 (s, 1H), 6.53 (s, 1H), 5.63 - 5.54 (m, 1H), 5.42 (s, 2H), 5.20 (s, 2H), 4.68 - 4.58 (m, 2H), 4.26 - 4.12 (m, 2H), 4.01 (s, 2H), 3.73 (d, *J =* 5.4 Hz, 2H), 3.20 - 3.10 (m, 2H), 3.05 - 2.95 (m, 2H), 2.76 (d*, J=* 4.7 Hz, 6H), 2.55 - 2.53 (m, 2H), 2.42 - 2.28 (m, 6H), 2.21 - 2.13 (m, 2H), 2.02 - 1.77 (m, 6H), 1.76 - 1.62 (m, 2H), 1.62 - 1.51 (m, 3H), 1.37 - 1.21 (m, 2H), 0.90 - 0.80 (m, 9H).

### Example 2.17: tert-Butyl ((S)-3-ethyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-13-((S)-3-methyl-2-(6-(2-(methylsulfonyl)pyrimidin-5-yl)hex-5-ynamido)butanamido)-4,9,12-trioxo-6-oxa-3,8,11-triazaheptadecan-17-yl)carbamate (DL-017)

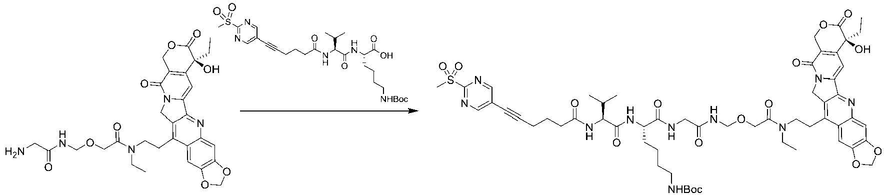

Compound C1.7 (90.0 mg), diisopropylethylamine (68.0 mg), and HATU (100 mg) were dissolved in *N,N*-dimethylformamide (5 mL). Compound B1.2 (80 mg) was then added thereto, and the reaction mixture was reacted at room temperature for 2 hours. The completion of the reaction was detected by LCMS. The reaction mixture was added with ethyl acetate (50 mL), washed with saturated brine (30 mL × 3), dried over anhydrous sodium sulfate, subjected to suction filtration, and the filtrate was concentrated under reduced pressure to a solid. The crude product was purified by preparative high-performance liquid chromatography (acetonitrile/0.05% formic acid in water) to obtain compound *tert-*butyl ((*S*)-3-ethyl-1-((S)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-13-((*S*)-3-methyl-2-(6-(2-(methylsulfonyl)pyrimidin-5-yl)hex-5-ynamido)butanamido)-4,9,12-trioxo-6-oxa-3,8,11-triazaheptadecan-17-yl)carbamate (2.06 mg, yield: 10.6%) as a yellow solid.

LCMS (ESI) [M+H]⁺: 1184.9.

### Example 2.18: N-((11S,14S)-11-(4-(Dimethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-018)

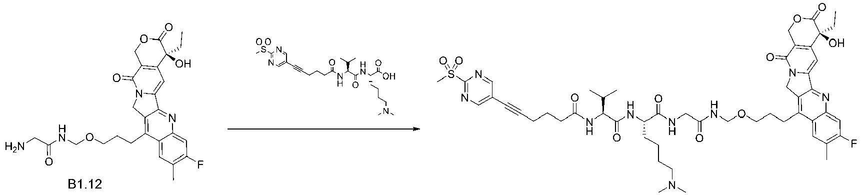

Compound *N*⁶,*N*⁶-dimethyl-*N*²-((6-(2-(methylsulfonyl)pyrimidin-5-yl)hex-5-ynoyl)-*L*-valyl)-*L*-lysine (126 mg) (C1.17), B1.12 (150 mg), 1-hydroxybenzotriazole (49 mg), 1-(3-dimethylaminopropyl)-3-ethylcarbodiimide hydrochloride (69 mg), and diisopropylethylamine (156 mg, 1.21 mmol) were dissolved in *N,N*-dimethyl formamide (10 mL) solution, and the reaction mixture was stirred at 40°C for 12 hours. LCMS showed the completion of the reaction. The reaction mixture was filtered and purified by preparative high-performance liquid chromatography (acetonitrile/0.05% formic acid in water) to obtain compound *N*-((11*S*,14*S*)-11-(4-(dimethylamino)butyl)-1-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-018) (12 mg, yield: 6.0%) as a yellow solid.
LCMS (ESI) [M+H]⁺ = 1030.6;
¹H NMR (400 MHz, DMSO-d6) δ 9.10 (s, 2H), 8.62 (t, *J* = 6.4 Hz, 1H), 8.23 - 8.13 (m, 2H), 8.07 (d, *J =* 7.2 Hz, 1H), 7.95 - 7.83 (m, 2H), 7.31 (s, 1H), 6.53 (s, 1H), 5.44 (s, 2H), 5.29 (s, 2H), 4.64 - 4.53 (m, 2H), 4.28 - 4.20 (m, 1H), 4.19 - 4.11 (m, 1H), 3.76 - 3.69 (m, 2H), 3.50 (t, *J =* 5.7 Hz, 2H), 3.41 (s, 3H), 3.23 - 3.19 (m, 2H), 2.67 - 2.62 (m, 2H), 2.57 - 2.54 (m, 2H), 2.54 (s, 3H), 2.42 - 2.26 (m, 3H), 2.02 - 1.77 (m, 6H), 1.68 - 1.66 (m, 1H), 1.58 - 1.45 (m, 3H), 1.36 - 1.25 (m, 2H), 0.88 (t, *J =* 7.4 Hz, 3H), 0.82 (t, *J =* 6.3 Hz, 6H).

### Example 2.19: N-((11S,14S)-11-(4-(Dimethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-019)

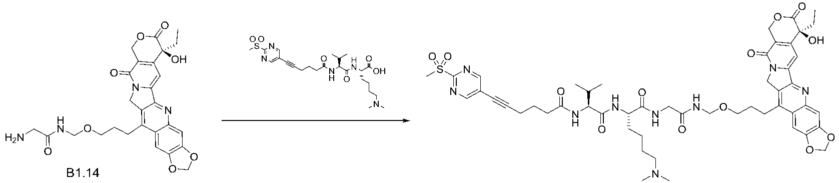

Compound B1.14 (100 mg, 0.186 mmol) and compound *N*⁶,*N*⁶-dimethyl-*N*²-((6-(2-(methylsulfonyl)pyrimidin-5-yl)hex-5-ynoyl)-*L*-valyl)-*L*-lysine (100 mg, 0.191 mmol) were dissolved in DMF (2 mL). Then, 1-hydroxybenzotriazole (38 mg, 0.280 mmol) and 1-(3-dimethylaminopropyl)-3-ethylcarbodiimide hydrochloride (53 mg, 0.280 mmol) were added thereto, and then triethylamine (56 mg, 0.559 mmol) was added thereto. After the addition, the reaction mixture was stirred at room temperature for 16 hours. After the completion of the reaction was detected by LCMS, the reaction mixture was purified by preparative chromatography (0.01% TFA in water, MeCN) to obtain the target compound *N*-((1*S,*14*S*)-11-(4-(dimethylamino)butyl)-1-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (20 mg, yield: 10%) as a yellow solid.
LCMS (ESI) [M+H]⁺ = 1042.5;
¹H NMR (400 MHz, DMSO-d6) δ 9.28 (s, 1H, TFA), 9.10 (s, 2H), 8.64 (br s, 1H), 8.19 (br s, 1H), 8.09 (d, *J =* 6.4 Hz, 1H), 7.92 (d, *J =* 7.8 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.67 - 4.52 (m, 2H), 4.30 - 4.10 (m, 2H), 3.74 (br s, 2H), 3.50 (br s, 2H), 3.41 (s, 3H), 3.17 - 3.07 (m, 2H), 3.04 - 2.91 (m, 2H), 2.75 (s, 6H), 2.46 - 2.24 (m, 3H), 2.04 - 1.66 (m, 9H), 1.63 - 1.46 (m, 3H), 1.32 - 1.29 (m, 2H), 0.87 - 0.82 (m, 9H).

### Example 2.20: N-((S)-1-(((S)-6-Amino-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-020)

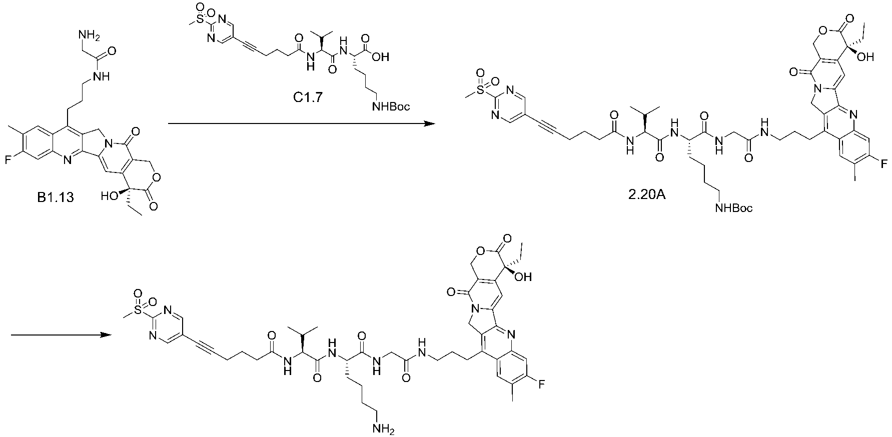

### Step 1:

Compound C1.7 (107 mg), triethylamine (45 mg), HOBT (25 mg), and EDCI (35 mg) were sequentially added to a solution of compound B1.13 (75 mg) in *N*,*N-*dimethylformamide (3 mL), and the reaction mixture was reacted at 35°C for 4 hours. The reaction mixture was added with water (30 mL) and extracted with ethyl acetate (30 mL × 2). The organic phase was dried over anhydrous sodium sulfate and concentrated to obtain the target compound 2.20A (76 mg).
LCMS (ESI) [M+H]⁺: 1072.5;

### Step 2:

Trifluoroacetic acid (1 mL) was added to a dichloromethane solution (2 mL) of compound 2.20A (60 mg, 0.06 mmol), and the mixture was stirred and reacted at room temperature for 1 hour. LCMS showed the completion of the reaction. The reaction mixture was concentrated and purified by preparative chromatography (0.01% TFA in water, MeCN) to obtain the target compound *N*-((*S*)-1-(((*S*)-6-amino-1-((2-((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (43 mg, yield: 80%) as a yellow solid.
LCMS (ESI) [M+H]⁺: 972.9;
¹H NMR (400 MHz, DMSO-d6) δ 9.09 (s, 2H), 8.24 - 8.17 (m, 2H), 8.12 (d, *J =* 7.0 Hz, 1H), 7.99 - 7.93 (m, 1H), 7.88 - 7.84 (m, 2H), 7.64 (s, 3H, NH2-TFA), 7.31 (s, 1H), 6.52 (s, 1H), 5.44 (s, 2H), 5.30 (s, 2H), 4.25 - 4.18 (m, 1H), 4.15 - 4.11 (m, 1H), 3.76 - 3.71 (m, 2H), 3.41 (s, 3H), 3.32 - 3.26 (m, 2H), 3.21 - 3.17 (m, 1H), 2.79 - 2.70 (m, 2H), 2.52 (s, 3H), 2.40 - 2.24 (m, 3H), 1.94 - 1.82 (m, 7H), 1.80 - 1.73 (m, 2H), 1.58 - 1.49 (m, 3H), 1.36 - 1.31 (m, 1H), 1.18 - 1.14 (m, 1H), 0.87 (t, *J =* 7.4 Hz, 3H), 0.80 - 0.76 (m, 6H).

### Example 2.21: N-((S)-1-(((S)-5-Amino-6-((2-((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propyl)amino)-2-oxoethyl)amino)-6-oxohexyl)amino)-3-methyl-1-oxobutan-2-yl)-6-(2-(methylsulfonyl)pyrimidin-5-yl)hex-5-ynamide

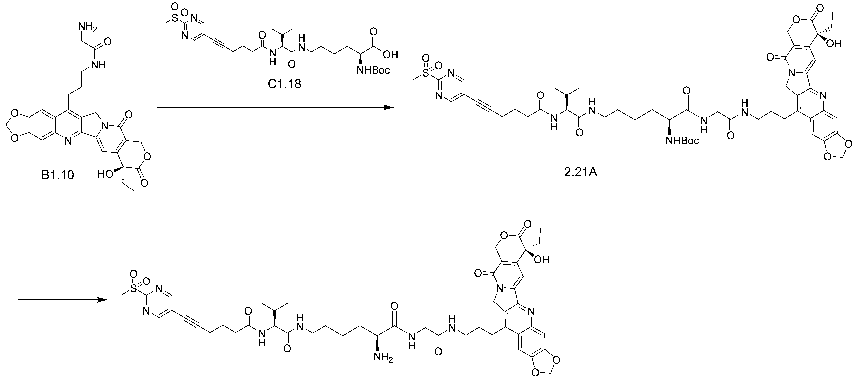

### Step 1:

Compound C1.18 (430 mg) was dissolved in N,N-dimethylformamide (5 mL), then EDCI (160 mg), HOBT (115 mg), and triethylamine (214 mg) were added thereto. After the addition, the reaction mixture was stirred at room temperature for 20 minutes, then added with compound B1.10 (314 mg), stirred, and reacted at room temperature for another 3 hours. The completion of the reaction was detected by LCMS. The reaction mixture was diluted with water, extracted with ethyl acetate, and the organic phase was concentrated to obtain a crude product 2.21A (350 mg).
LCMS (ESI) [M+H]⁺: 1084.3;

### Step 2:

A mixed solution of trifluoroacetic acid and dichloromethane (volume ratio of 1:2; 4 mL) was added to a dichloromethane solution (4 mL) of compound 2.21A (350 mg) and the reaction mixture was stirred and reacted at room temperature for 1 hour. The reaction mixture was concentrated and the crude product was purified by preparative high-performance liquid chromatography (acetonitrile/0.05% formic acid in water) to obtain the target compound N-((*S*)-1-(((*S*)-5-amino-6-((2-((3-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)propyl)amino)-2-oxoethyl)amino)-6-oxohexyl)amino)-3-methyl-1-oxobutan-2-yl)-6-(2-(methylsulfonyl)pyrimidin-5-yl)hex-5-ynamide (100 mg).
LCMS (ESI) [M+H]⁺: 984.4;
¹H NMR (400 MHz, DMSO-d6) δ 9.10 (s, 2H), 8.69 (t, *J* = 6.1 Hz, 1H), 8.14 (t,*J* = 5.4 Hz, 1H), 8.08 (s, 3H, NH2-TFA), 7.95 (t, *J* = 4.9 Hz, 1H), 7.89 (d, *J* = 8.9 Hz, 1H), 7.66 (s, 1H), 7.51 (s, 1H), 7.25 (s, 1H), 6.48 (s, 1H), 6.29 (s, 2H), 5.42 (s, 2H), 5.25 (s, 2H), 4.09 (t, *J* = 8.1 Hz, 1H), 3.83 (d, *J* = 5.7 Hz, 2H), 3.40 (s, 3H), 3.33 - 3.27 (m, 2H), 3.15 - 3.06 (m, 3H), 2.97 - 2.94 (m, 1H), 2.57 - 2.53 (m, 2H), 2.43 - 2.28 (m, 3H), 1.95 - 1.68 (m, 9H), 1.46 - 1.28 (m, 4H), 0.88 (t, *J =* 7.3 Hz, 3H), 0.82 (d, *J* = 6.7 Hz, 6H).

### Example 2.22: N-((S)-1-(((S)-5-Amino-6-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-6-oxohexyl)amino)-3-methyl-1-oxobutan-2-yl)-6-(2-(methylsulfonyl)pyrimidin-5-yl)hex-5-ynamide (DL-022)

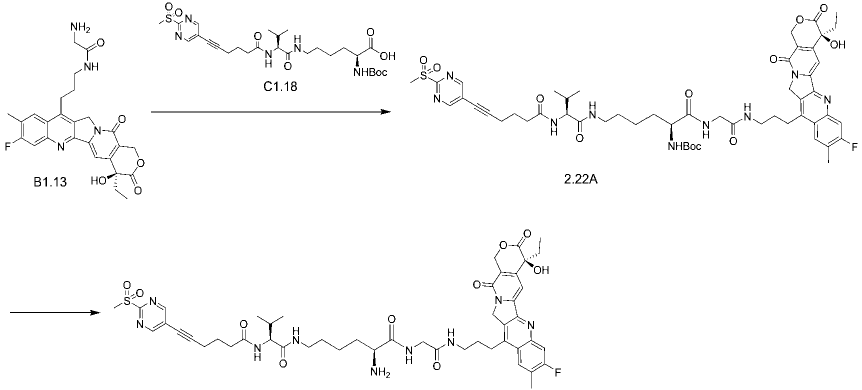

### Step 1:

Compound C1.18 (136 mg, 0.23 mmol) was dissolved in N,N-dimethylformamide (5 mL), then EDCI (48 mg, 0.25 mmol), HOBT (34 mg, 0.25 mmol), and triethylamine (64 mg, 0.63 mmol) were added thereto. After the addition, the reaction mixture was stirred at room temperature for 20 minutes, then added with compound B1.13 (3105 mg, 0.19 mmol), stirred, and reacted at room temperature for another 3 hours. The completion of the reaction was detected by LCMS. The reaction mixture was diluted with water, extracted with ethyl acetate, and the organic phase was concentrated to obtain a crude product. The obtained compound was dissolved in dichloromethane (4 mL), and then trifluoroacetic acid (2 mL) was added thereto. The reaction mixture was reacted for 1 hour and then concentrated. The crude product was purified by preparative chromatography to obtain the target compound *N*-((*S*)-1-(((*S*)-5-amino-6-((2-((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propyl)amino)-2-oxoethyl)amino)-6-oxohexyl)amino)-3-methyl-1-oxobutan-2-yl)-6-(2-(methylsulfonyl)pyrimidin-5-yl)hex-5-ynamide (DL-022) (88 mg).
LCMS (ESI) [M+H]⁺ = 972.9;
¹H NMR (400 MHz, DMSO-d6) δ 9.10 (s, 2H), 8.72 (t, *J =* 5.5 Hz, 1H), 8.23 (d,*J* = 8.4 Hz, 1H), 8.17 (t, *J* = 5.4 Hz, 1H), 8.11 (s, 3H, NH2-TFA), 7.97 (t, *J* = 5.6 Hz, 1H), 7.93 - 7.86 (m, 2H), 7.32 (s, 1H), 6.53 (s, 1H), 5.44 (s, 2H), 5.29 (s, 2H), 4.14 - 4.02 (m, 2H), 3.85 - 3.83 (m, 2H), 3.41 (s, 3H), 3.32 (d, *J =* 6.2 Hz, 2H), 3.24 - 3.16 (m, 2H), 3.11 - 2.94 (m, 2H), 2.55 (s, 3H), 2.35 - 2.30 (m, 3H), 1.92 - 1.69 (m, 10H), 1.43 - 1.29 (m, 4H), 0.88 (t, *J =* 7.3 Hz, 3H), 0.82 (d, *J* = 6.7 Hz, 6H).

### Example 2.23: (S)-6-Amino-N-(2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)-2-((S)-2-isopropyl-35-(4-(2-(methylsulfonyl)pyrimidin-5-yl)-1H-1,2,3-triazol-1-yl)-4,8-dioxo-6,12,15,18,21,24,27,30,33-nonaoxa-3,9-diazapentatriacontanamido)hexanamide (DL-023)

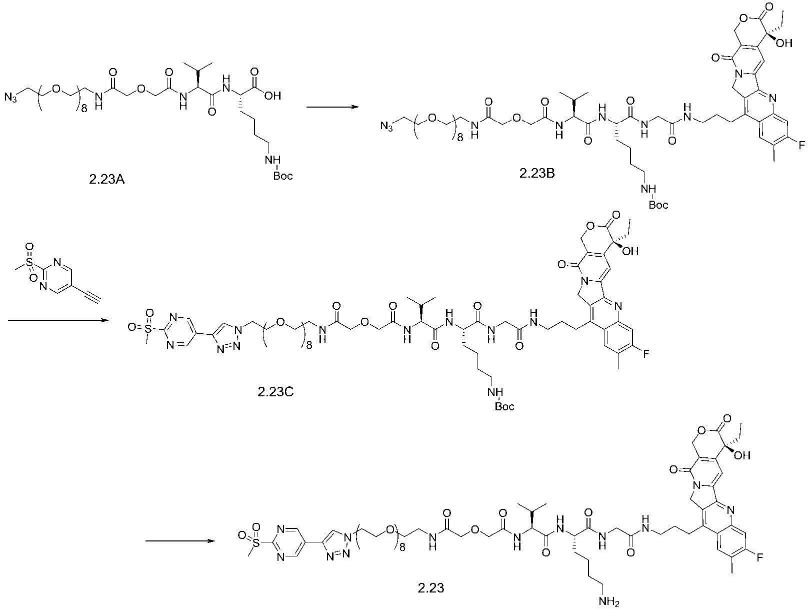

### Step 1:

Compound 2.23A (100 mg), triethylamine (50 µL), HOBT (16 mg), and EDCI (23 mg) were sequentially added to a solution of compound B1.13 (46 mg) in *N,N-*dimethylformamide (3 mL), and the reaction mixture was reacted at 35°C for 4 hours. The reaction mixture was added with water (30 mL) and extracted with ethyl acetate (30 mL × 2). The organic phase was dried over anhydrous sodium sulfate and concentrated to obtain the target compound 2.23B (105 mg).
LCMS (ESI) [M+H]⁺: 1358.4;

### Step 2:

Compound 2.23B (105 mg) and 2-methylsulfonyl-5-ethynylpyrimidine (45 mg) were dissolved in DMSO-water (2 mL, 4:1), and cuprous bromide (50 mg) was added to the mixture. The reaction mixture was stirred at room temperature for 2 hours, then added with ethyl acetate (100 mL), washed with water (100 mL × 3), dried, and then the organic solvent was removed under reduced pressure. The crude product was separated by silica gel column chromatography to obtain the target product 2.23C (84 m mg). MS (*m*/*z*): 1540.8 [M + H]⁺;

### Step 3:

Compound 2.23C was dissolved in a mixed solution of trifluoroacetic acid and dichloromethane (volume ratio of 1:2; 4 mL), and the reaction mixture was stirred at room temperature for 30 minutes. LCMS showed the completion of the reaction. The reaction mixture was concentrated to obtain a crude product, which was subjected to preparative chromatography (0.01% TFA in water, MeCN) to obtain the target compound (*S*)-6-amino-*N-*(2-((3-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)propyl)amino)-2-oxoethyl)-2-((*S*)-2-isopropyl-35-(4-(2-(methylsulfonyl)pyrimidin-5-yl)-1*H*-1,2,3-triazol-1-yl)-4,8-dioxo-6,12,15,18,21,24,27,30,33-nonaoxa-3,9-diazapentatriacontanamido)hexanamide (DL-023, 8 mg, trifluoroacetate, yield: 5%).
LCMS (ESI) [M+H]⁺ = 1440.8;
¹H NMR (400 MHz, DMSO-d6) δ 9.49 (s, 2H), 8.91 (s, 1H), 8.24 - 8.18 (m, 3H), 8.01 (t, *J* = 5.6 Hz, 1H), 7.96 (t, *J* = 5.5 Hz, 1H), 7.90 - 7.84 (m, 2H), 7.63 (s, 3H, TFA salt), 7.32 (s, 1H), 5.44 (s, 2H), 5.30 (s, 2H), 4.66 (t, *J =* 5.1 Hz, 2H), 4.21 (dd, *J* = 15.2, 7.9 Hz, 2H), 3.97 (s, 2H), 3.93 (s, 2H), 3.89 (t, *J =* 5.1 Hz, 2H), 3.75 - 3.72 (m, 2H), 3.56 - 3.54 (m, 2H), 3.54 - 3.38 (m, 30H), 3.33 - 3.13 (m, 5H), 2.75 (d, *J =* 7.1 Hz, 2H), 2.52 (s, 3H), 2.02 - 1.94 (m, 1H), 1.91 - 1.81 (m, 3H), 1.71 - 1.65 (m, 1H), 1.58 - 1.46 (m, 3H), 1.38 - 1.29 (m, 2H), 0.88 (t, *J* = 7.3 Hz, 3H), 0.81 - 0.75 (m, 6H).

### Example 2.24: N-((S)-1-(((S)-6-(Dimethylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-024)

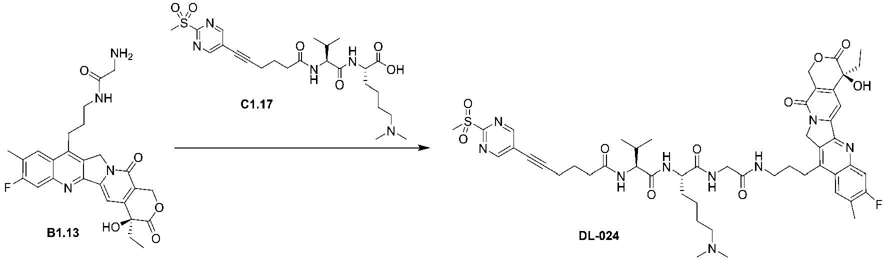

PyBOP (41 mg, 0.081) and DIPEA (20 mg, 0.16 mmol) were sequentially added to a solution of compound B1.13 (40 mg, 0.081 mmol) and compound C1.17 (42 mg, 0.081 mmol) in N,N-dimethylformamide (2 mL), and the reaction mixture was stirred at room temperature for 2 hours. LCMS showed the completion of the reaction. The reaction mixture was purified by preparative high-performance liquid chromatography (acetonitrile/0.05% trifluoroacetic acid in water) to obtain compound *N*-((*S*)-1-(((*S*)-6-(dimethylamino)-1-((2-((3-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (11.54 mg, yield: 14%) as a yellow solid.
LCMS (ESI) [M+H]⁺ =1000.6;
¹H NMR (400 MHz, DMSO-d6) δ 9.42 (s, 1H), 9.08 (s, 2H), 8.27 - 8.18 (m, 2H), 8.14 (d, *J* = 6.9 Hz, 1H), 7.96 (t, *J* = 6.9 Hz, 1H), 7.91 - 7.77 (m, 2H), 7.31 (s, 1H), 5.44 (s, 2H), 5.29 (s, 2H), 4.15 - 4.12 (m, 1H), 3.81 - 3.65 (m, 3H), 3.41 (s, 3H), 3.33 - 3.25 (m, 2H), 3.22 - 3.15 (m, 2H), 3.03 - 2.95 (m, 2H), 2.77 - 2.71 (m, 6H), 2.38 - 2.23 (m, 3H), 1.92 - 1.81 (m, 7H), 1.78 - 1.73 (m, 2H), 1.62 - 1.56 (m, 3H), 1.38 - 1.29 (m, 2H), 0.92 - 0.84 (m, 3H), 0.84 - 0.72 (m, 6H).

### Example 2.25: N-((S)-1-(((S)-6-(Diethylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-025)

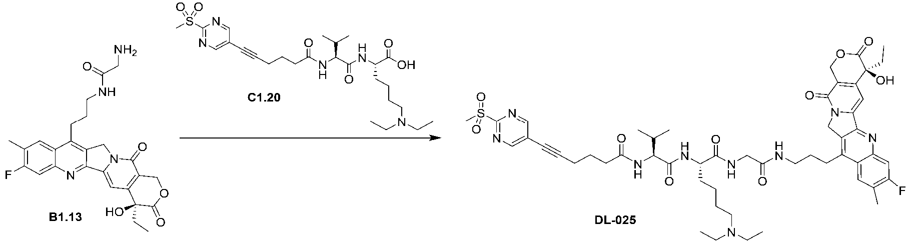

Compound C1.20 (30 mg, 0.054 mmol) and compound B1.13 (27 mg, 0.054 mmol) were dissolved in N,N-dimethylformamide (2 mL). Then HBTU (22 mg, 0.054 mmol) and N,N-diisopropylethylamine (17 mg, 0.135 mmol) were sequentially added thereto. After the addition, the reaction mixture was stirred at room temperature for 30 minutes. After the completion of the reaction was detected by LCMS, the reaction mixture was directly purified by preparative chromatography (0.01% trifluoroacetic acid in water, acetonitrile) to obtain target compound *N*-((*S*)-1-(((*S*)-6-(diethylamino)-1-((2-((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (23 mg, yield: 42%) as a yellow solid.
LCMS (ESI) [M+H]⁺ = 1028.5;
¹H NMR (400 MHz, DMSO-d6) δ 9.08 (s, 2H), 9.02 (s, 1H), 8.21 (d, *J* = 7.4 Hz, 2H), 8.13 (d, *J =* 7.1 Hz, 1H), 7.97 (t, *J =* 5.4 Hz, 1H), 7.87 (d, *J =* 10.6 Hz, 2H), 7.31 (s, 1H), 6.52 (s, 1H), 5.44 (s, 2H), 5.30 (s, 2H), 4.28 - 4.18 (m, 1H), 4.14 (t, *J* = 7.7 Hz, 1H), 3.80 - 3.65 (m, 2H), 3.41 (s, 3H), 3.31 - 3.24 (m, 2H), 3.22 - 3.15 (m, 2H), 3.12 - 3.06 (m, 4H), 3.02 - 2.91 (m, 2H), 2.36 - 2.23 (m, 2H), 1.95 - 1.68 (m, 9H), 1.68 - 1.49 (m, 4H), 1.38 - 1.28 (m, 2H), 1.13 (t, *J =* 7.2 Hz, 6H), 0.86 (t, *J =* 7.2 Hz, 3H),0.79 - 0.76 (m, 6H).

### Example 2.26: N-((S)-1-(((S)-6-(Dipropylamino)-1-((2-((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (DL-026)

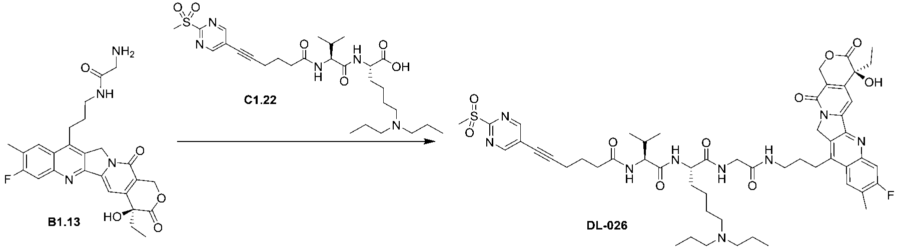

Compound C1.22 (31 mg, 0.054 mmol) and compound B1.13 (27 mg, 0.054 mmol) were dissolved in *N,N*-dimethylformamide (2 mL). Then HBTU (22 mg, 0.054 mmol) and N,N-diisopropylethylamine (17 mg, 0.135 mmol) were sequentially added thereto. The reaction mixture was stirred at room temperature for 30 minutes. After the completion of the reaction was detected by LCMS, the reaction mixture was directly purified by preparative chromatography (0.01% trifluoroacetic acid in water, acetonitrile) to obtain target compound *N*-((*S*)-1-(((*S*)-6-(dipropylamino)-1-((2-((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (25 mg, yield: 45%) as a yellow solid.
LCMS (ESI) [M+H]⁺ = 1056.6;
H NMR (400 MHz, DMSO-d6) δ 9.09 (s, 2H), 8.27 - 8.19 (m, 2H), 8.13 (d, *J* = 7.3 Hz, 1H), 7.97 (t, *J* = 5.3 Hz, 1H), 7.91 - 7.84 (m, 2H), 7.31 (s, 1H), 6.53 (s, 1H), 5.44 (s, 2H), 5.29 (s, 2H), 4.24 (d, *J* = 6.7 Hz, 1H), 4.13 (t, *J* = 7.8 Hz, 1H), 3.73 (dd, *J* = 14.7, 5.9 Hz, 2H), 3.41 (s, 3H), 3.28 (d, *J* = 6.4 Hz, 2H), 3.19 (t, *J* = 7.6 Hz, 2H), 2.99 (br, 6H), 2.33 - 2.27 (m, 2H), 1.95 - 1.70 (m, 9H), 1.62 - 1.57 (m, 8H), 1.33 - 1.30 (m, 2H), 0.96 - 0.82 (m, 9H), 0.82 - 0.73 (m, 6H).

### Example 2.27: N-((S)-1-(((S)-6-(Diethylamino)-1-((2-(((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide

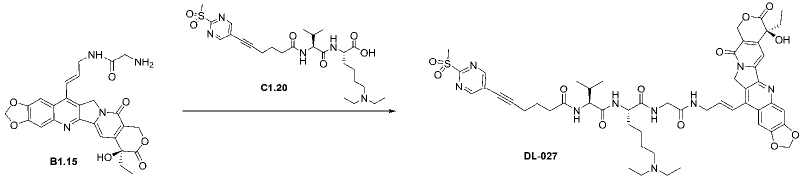

HBTU (30 mg, 0.079 mmol) and DIPEA (26 mg, 0.2 mmol) were sequentially added to a solution of compound B1.15 (40 mg, 0.074 mmol) and compound C1.20 (40 mg, 0.072 mmol) in *N,N*-dimethylformamide (4 mL). The reaction mixture was reacted at room temperature for 1 hour. LCMS showed the completion of the reaction. The reaction mixture was purified by preparative high-performance liquid chromatography (acetonitrile/0.05% trifluoroacetic acid in water) to obtain compound *N*-((*S*)-1-(((*S*)-6-(diethylamino)-1-((2-(((*E*)-3-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)allyl)amino)-2-oxoethyl)amino)-1-oxohexan-2-yl)amino)-3-methyl-1-oxobutan-2-yl)-6-(2-(methylsulfonyl)pyrimidin-5-yl)hex-5-ynamide (11.1 mg, yield: 14%).
LCMS (ESI) [M+H]⁺ =1038.6;
¹H NMR (400 MHz, DMSO-d6) δ 9.10 (s, 2H), 9.02 (s, 1H, TFA), 8.33 - 8.25 (m, 2H), 8.12 (d, *J =* 7.3 Hz, 1H), 7.92 (d, *J* = 8.4 Hz, 1H), 7.68 (d, *J* = 4.1 Hz, 1H), 7.52 (d, *J* = 2.8 Hz, 1H), 7.26 (s, 1H), 7.15 (d, *J =* 16.2 Hz, 1H), 6.52 - 6.50 (m, 2H), 6.30 (s, 2H), 5.43 (s, 2H), 5.28 - 5.24 (m, 2H), 4.27 (d, *J =* 6.2 Hz, 2H), 4.12 (br s, 2H), 3.81 (br s, 2H), 3.41 (s, 3H), 3.11 - 3.07 (m, 6H), 2.97 (br s, 2H), 2.53 - 2.50 (m, 1H), 2.39 - 2.24 (m, 4H), 1.85 - 1.79 (m, 8H), 1.16 (t, *J* = 6.4 Hz, 6H), 0.88 (t, *J* = 6.4 Hz, 3H), 0.78 (d, *J* = 6.4 Hz, 6H).

### Example 2.28: (S)-6-(Dimethylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

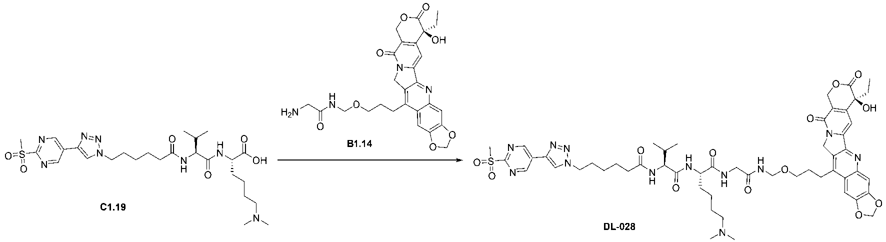

Compound C1.19 (40 mg, 0.06 mmol) was dissolved in *N,N-*dimethylformamide (1.5 mL), then HBTU (26 mg, 0.06 mmol), B1.14 (36 mg, 0.06 mmol), and DIPEA (66 mg, 0.17 mmol) were sequentially added thereto, and the reaction mixture was stirred and reacted at room temperature for 1 hour. The completion of the reaction was detected by LCMS. The reaction mixture was purified by preparative chromatography (acetonitrile/0.05% trifluoroacetic acid in water) to obtain the target compound (*S*)-6-(dimethylamino)-*N*-(2-(((3-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)propoxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (13.88 mg, TFA salt, yield: 16%) as a beige solid.
LCMS (ESI) [M+H]⁺ = 1113.7;
¹H NMR (400 MHz, DMSO-d6) δ 9.46 (s, 2H), 9.29 (s, 1H, TFA salt), 8.92 (s, 1H), 8.63 (t, *J* = 6.6 Hz, 1H), 8.18 (br s, 1H), 8.03 (d, *J =* 7.6 Hz, 1H), 7.81 (d, *J* = 8.3 Hz, 1H), 7.59 (s, 1H), 7.50 (s, 1H), 7.24 (s, 1H), 6.48 (s, 1H), 6.28 (s, 2H), 5.42 (s, 2H), 5.24 (s, 2H), 4.64 - 4.55 (m, 2H), 4.46 (t, *J* = 7.0 Hz, 2H), 4.28 - 4.20 (m, 1H), 4.15 - 4.09 (m, 1H), 3.76 - 3.71 (m, 4H), 3.53 - 3.48 (m, 4H), 3.44 (s, 3H), 3.10 - 3.08 (m, 2H), 2.97 - 2.95 (m, 2H), 2.75 (d, *J =* 4.9 Hz, 6H), 2.20 - 2.09 (m, 2H), 1.92 - 1.83 (m, 5H), 1.68 - 1.65 (m, 1H), 1.63 - 1.50 (m, 5H), 0.90 - 0.74 (m, 9H).

### Example 2.29: (S)-6-(Diethylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

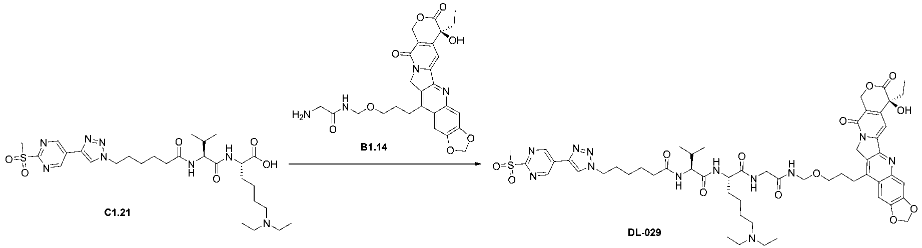

Compound C1.21 (58 mg, 0.09 mmol) and compound B1.14 (50 mg, 0.09 mmol) were dissolved in DMF (1 mL), and then HBTU (35 mg, 0.09 mmol) and*N*,*N*-diisopropylethylamine (30 mg, 0.23 mmol) were added thereto. After the addition, the reaction mixture was stirred at room temperature for 1 hour. After the completion of the reaction was detected by LCMS, the reaction mixture was purified by preparative chromatography (0.01% TFA in water, MeCN) to obtain the target compound (*S*)-6-(diethylamino)-*N*-(2-(((3-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)propoxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (14 mg, yield: 13%) as a yellow solid.
LCMS (ESI) [M+H]⁺ = 1141.7;
1H NMR (400 MHz, DMSO) δ 9.46 (s, 2H), 8.98 (s, 1H, TFA), 8.93 (s, 1H), 8.64 (t, *J =* 6.5 Hz, 1H), 8.18 (d, *J* = 5.7 Hz, 1H), 8.03 (d, *J =* 7.4 Hz, 1H), 7.81 (d, *J* = 8.5 Hz, 1H), 7.58 (s, 1H), 7.50 (d, *J* = 3.1 Hz, 1H), 7.24 (s, 1H), 6.52 (s, 1H), 6.28 (s, 2H), 5.42 (s, 2H), 5.23 (s, 2H), 4.62 - 4.55 (m, 2H), 4.46 (t, *J* = 6.9 Hz, 2H), 4.27 - 4.23 (m, 1H), 4.11 - 4.09 (m, 1H), 3.74 (d, *J =* 5.8 Hz, 2H), 3.49 (t, *J =* 5.8 Hz, 2H), 3.44 (s, 3H), 3.13 - 3.06 (m, 6H), 3.01 - 2.93 (m, 2H), 2.22 - 2.07 (m, 2H), 1.91 - 1.83 (m, 6H), 1.70- 1.51 (m, 5H), 1.33 - 1.21 (m, 6H), 1.16 (t, *J =* 7.2 Hz, 6H), 0.87 (t, *J =* 7.4 Hz, 3H), 0.82 - 0.76 (m, 6H).

### Example 2.30: (S)-6-(Dipropylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

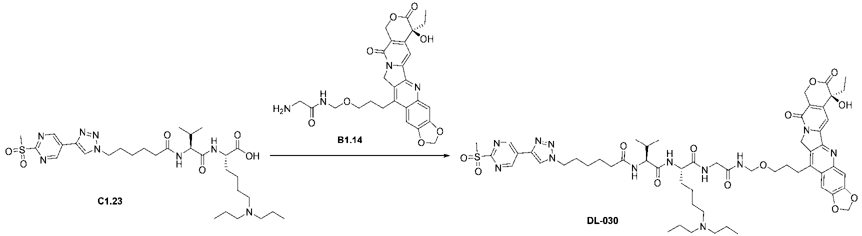

Compound C1.23 (61 mg, 0.09 mmol) and compound B1.14 (50 mg, 0.09 mmol) were dissolved in DMF (1 mL), and then HBTU (35 mg, 0.09 mmol) and*N*,*N*-diisopropylethylamine (30 mg, 0.23 mmol) were added thereto. After the addition, the reaction mixture was stirred at room temperature for 1 hour. After the completion of the reaction was detected by LCMS, the reaction mixture was concentrated to obtain a crude product, which was purified by preparative chromatography (0.01% TFA in water, MeCN) to obtain the target compound (*S*)-6-(dipropylamino)-*N*-(2-(((3-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)propoxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (21 mg, yield: 19%) as a yellow solid.
LCMS (ESI) [M+H]⁺ = 1169.8;
¹H NMR (400 MHz, DMSO) δ 9.46 (s, 2H), 9.01 (s, 1H, TFA), 8.93 (s, 1H), 8.64 (t, *J* = 6.4 Hz, 1H), 8.19 (t, *J* = 5.4 Hz, 1H), 8.02 (d, *J* = 7.3 Hz, 1H), 7.81 (d, *J* = 8.3 Hz, 1H), 7.58 (s, 1H), 7.51 (s, 1H), 7.24 (s, 1H), 6.50 (s, 1H), 6.28 (s, 2H), 5.42 (s, 2H), 5.23 (s, 2H), 4.60 - 4.58 (m, 2H), 4.46 (t, *J* = 6.9 Hz, 2H), 4.24 (d, *J* = 6.0 Hz, 1H), 4.13 - 4.09 (m, 1H), 3.74 (d, *J =* 5.7 Hz, 2H), 3.49 (t, *J* = 5.8 Hz, 2H), 3.44 (s, 3H), 3.10 (br s, 2H), 3.02 - 2.95 (m, 6H), 2.19 - 2.15 (m, 2H), 1.88 - 1.86 (m, 6H), 1.71 - 1.69 (m, 1H), 1.61 - 1.56 (m, 8H), 1.28 - 1.26 (m, 6H), 0.88 - 0.83 (m, 9H), 0.81 - 0.78 (m, 6H).

### Example 2.31: (S)-6-(Dimethylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

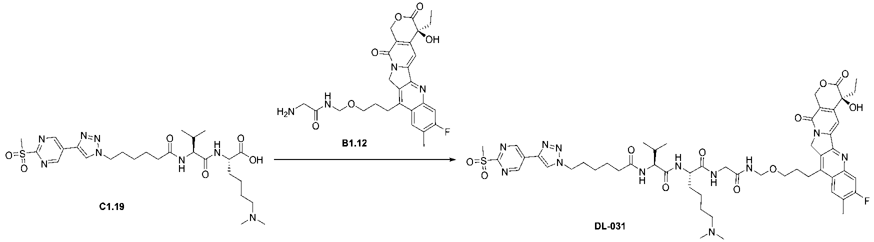

Compound C1.19 (113 mg, 0.19 mmol) and compound B1.12 (100 mg, 0.19 mmol) were dissolved in DMF (1 mL), and then HBTU (72 mg, 0.19 mmol) and *N,N-*diisopropylethylamine (61 mg, 0.48 mmol) were added thereto. After the addition, the reaction mixture was stirred at room temperature for 1 hour. After the completion of the reaction was detected by LCMS, the reaction mixture was purified by preparative chromatography (0.01% TFA in water, acetonitrile) to obtain the target compound (*S*)-6-(dimethylamino)-*N*-(2-(((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propoxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (16 mg, yield: 8%) as a white solid.
LCMS (ESI) [M+H]⁺ = 1101.6;
¹H NMR (400 MHz, DMSO) δ 9.46 (s, 2H), 8.93 (s, 1H), 8.63 (t, *J* = 6.3 Hz, 1H), 8.20 - 8.28 (m, 2H), 8.04 (d, *J* = 7.2 Hz, 1H), 7.88 (d, *J* = 10.8 Hz, 1H), 7.81 (d, *J* = 8.3 Hz, 1H), 7.31 (s, 1H), 6.53 (s, 1H), 5.44 (s, 2H), 5.29 (s, 2H), 4.63 - 4.54 (m, 2H), 4.46 (t, *J* = 7.0 Hz, 2H), 4.29 - 4.19 (m, 1H), 4.15 - 4.08 (m, 1H), 3.73 (d, *J* = 6.9 Hz, 2H), 3.50 (t, *J* = 5.8 Hz, 2H), 3.44 (s, 3H), 3.24 - 3.14 (m, 2H), 3.01 - 2.91 (m, 2H), 2.72 (s, 6H), 2.53 (s, 3H), 2.23 - 2.07 (m, 2H), 1.93 - 1.84 (m, 6H), 1.72 - 1.64 (m, 1H), 1.61 - 1.45 (m, 6H), 1.34 - 1.22 (m, 4H), 0.87 (t, *J =* 7.4 Hz, 3H), 0.82 - 0.76 (m, 6H).

### Example 2.32: (S)-6-(Diethylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

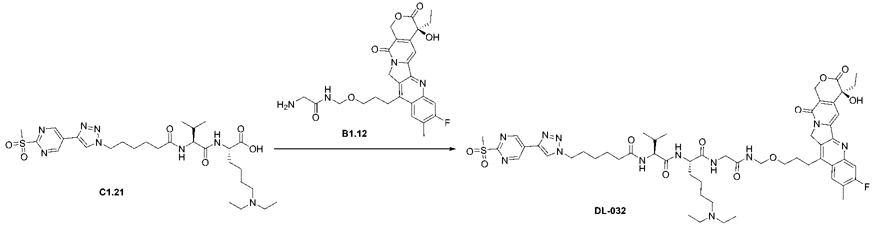

Compound C1.21 (59 mg, 0.10 mmol) and compound B1.12 (50 mg, 0.10 mmol) were dissolved in DMF (1 mL), and then HBTU (36 mg, 0.10 mmol) and*N*,*N-*diisopropylethylamine (31 mg, 0.24 mmol) were added thereto. After the addition, the reaction mixture was stirred at room temperature for 1 hour. After the completion of the reaction was detected by LCMS, the reaction mixture was purified by preparative chromatography (0.01% formic acid in water, acetonitrile) to obtain the target compound (*S*)-6-(diethylamino)-*N*-(2-(((3-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)propoxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (33.8 mg, yield: 32%) as a white solid.
LCMS (ESI) [M+H]⁺ = 1129.7;
¹H NMR (400 MHz, DMSO) δ 9.46 (s, 2H), 8.93 (s, 1H), 8.61 (t, *J* = 6.4 Hz, 1H), 8.29 (s, 1H), 8.24 - 8.15 (m, 2H), 8.00 (d, *J* = 7.2 Hz, 1H), 7.85 (d, *J* = 10.5 Hz, 2H), 7.31 (s, 1H), 5.44 (s, 2H), 5.26 (s, 2H), 4.63 - 4.55 (m, 2H), 4.46 (t, *J* = 6.8 Hz, 2H), 4.23 - 4.18 (m, 1H), 4.11 (d, *J* = 7.9 Hz, 1H), 3.77 - 3.69 (m, 2H), 3.50 (t, *J* = 5.9 Hz, 2H), 3.44 (s, 3H), 3.17 - 3.15 (m, 3H), 2.61 (q, *J* = 6.7 Hz, 4H), 2.22 - 2.10 (m, 2H), 1.92 - 1.84 (m, 6H), 1.68 (d, *J* = 6.7 Hz, 1H), 1.57 - 1.48 (m, 3H), 1.39 (d, *J=* 5.9 Hz, 2H), 1.30 - 1.22 (m, 4H), 0.97 (t, *J* = 6.9 Hz, 6H), 0.88 (t, *J =* 7.3 Hz, 3H), 0.82 - 0.77 (m, 6H).

### Example 2.33: (S)-6-(Dipropylamino)-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

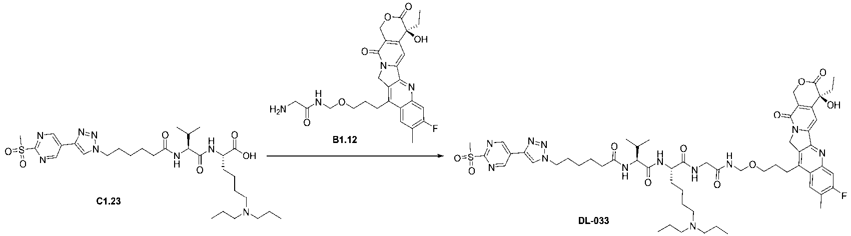

Compound C1.23 (62 mg, 0.10 mmol) and compound B1.12 (50 mg, 0.10 mmol) were dissolved in DMF (1 mL), and then HBTU (36 mg, 0.10 mmol) and*N,N*-diisopropylethylamine (31 mg, 0.24 mmol) were added thereto. After the addition, the reaction mixture was stirred at room temperature for 1 hour. After the completion of the reaction was detected by LCMS, the reaction mixture was purified by preparative chromatography (0.01% TFA in water, MeCN) to obtain the target compound (*S*)-6-(dipropylamino)-*N*-(2-(((3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propoxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (35.2 mg, yield: 32%) as a yellow solid.
LCMS (ESI) [M+H]⁺ = 1157.8;
¹H NMR (400 MHz, DMSO) δ 9.46 (s, 2H), 9.05 (s, 1H, TFA), 8.93 (s, 1H), 8.64 (t, *J* = 6.7 Hz, 1H), 8.20 (d, *J* = 7.4 Hz, 2H), 8.03 (d, *J* = 7.2 Hz, 1H), 7.88 (d, *J* = 10.8 Hz, 1H), 7.81 (d, *J* = 8.4 Hz, 1H), 7.32 (s, 1H), 6.54 (s, 1H), 5.44 (s, 2H), 5.28 (s, 2H), 4.62 - 4.55 (m, 2H), 4.46 (t, *J* = 7.0 Hz, 2H), 4.24 (m, 1H), 4.13 - 4.07 (m, 1H), 3.74 (m, 2H), 3.50 (m, 2H), 3.44 (s, 3H), 3.23 - 3.16 (m, 2H), 3.01 - 2.95 (m, 6H), 2.50 (s, 3H), 2.22 - 2.09 (m, 2H), 1.95 - 1.84 (m, 8H), 1.67 - 1.56 (m, 9H), 1.33 - 1.23 (m, 6H), 0.91 - 0.86 (m, 9H), 0.82 - 0.77 (m, 6H).

### Example 2.34: N-((11S,14S)-11-(4-(Diethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-034)

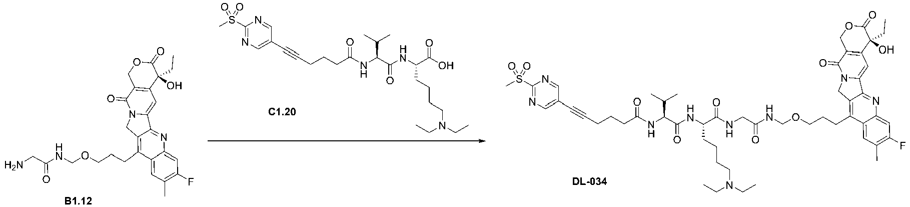

Compound (*S*)-2-amino-*N*-((3-(4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propoxy)methyl)acetamide (B1.12, 30 mg, 0.057 mmol) and *N*⁶,*N*⁶-diethyl-*N*²-((6-(2-(methylsulfonyl)pyrimidin-5-yl)hexadecyl-5-ynoyl)-*L*-propionyl)-*L*-lysine (C1.20, 31 mg, 0.057 mmol) were dissolved in DMF (1 mL). Then HBTU (22 mg, 0.057 mmol) and *N,N-*diisopropylethylamine (18 mg, 0.143 mmol) were sequentially added thereto. After the addition, the reaction mixture was stirred at room temperature for 1 hour. After the completion of the reaction was detected by LCMS, the reaction mixture was purified by preparative chromatography (0.01% trifluoroacetic acid in water, acetonitrile) to obtain the target compound trifluoroacetate of *N*-((11*S*,14*S*)-1 1-(4-(diethylamino)butyl)-1-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (3.18 mg, yield: 5%) as a yellow solid.
LCMS (ESI) [M+H]⁺ = 1058.7;
1H NMR (400 MHz, DMSO-d6) δ 9.10 (s, 2H), 8.65 (t, *J* = 6.5 Hz, 1H), 8.21 - 8.19 (m, 2H), 8.09 (d, *J* = 7.3 Hz, 1H), 7.92 (d, *J* = 8.6 Hz, 1H), 7.88 (d, *J =* 10.8 Hz, 1H), 7.32 (s, 1H), 6.53 (s, 1H), 5.44 (s, 2H), 5.28 (s, 2H), 4.64 - 4.56 (m, 2H), 4.29 - 4.22 (m, 1H), 4.15 (t, *J =* 7.4 Hz, 1H), 3.74 (d, *J =* 4.6 Hz, 2H), 3.51 - 3.49 (m, 2H), 3.24 - 3.18 (m, 2H), 3.13 - 3.07 (m, 4H), 3.01 - 2.95 (m, 2H), 2.55 (s, 3H), 2.40 - 2.31 (m, 3H), 1.99 - 1.74 (m, 8H), 1.65 - 1.49 (m, 4H), 1.38 - 1.25 (m, 2H), 1.16 (t, *J* = 7.2 Hz, 6H), 0.89 - 0.79 (m, 9H).

### Example 2.35: N-((11S,14S)-11-(4-(Dipropylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-035)

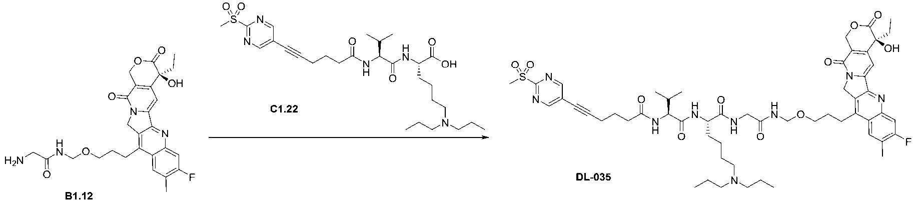

Compound (*S*)-2-amino-*N*-((3-(4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)propoxy)methyl)acetamide (B1.12, 30 mg, 0.057 mmol) and *N*²-((6-(2-(methylsulfonyl)pyrimidin-5-yl)hex-5-ynoyl)-*L*-propionyl)-*N*⁶,*N*⁶-dipropyl-*L*-lysine (C1.22, 33 mg, 0.057 mmol) were dissolved in DMF (1 mL). Then HBTU (22 mg, 0.057 mmol) and *N,N*-diisopropylethylamine (18 mg, 0.143 mmol) were sequentially added thereto. After the addition, the reaction mixture was stirred at room temperature for 1 hour. After the completion of the reaction was detected by LCMS, the reaction mixture was purified by preparative chromatography (0.01% trifluoroacetic acid in water, acetonitrile) to obtain the target compound N-((118,14S)-11-(4-(dipropylamino)butyl)-1-((S)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-035) (10.65 mg, yield: 16%) as a yellow solid.
LCMS (ESI) [M+H]⁺ = 1086.7;
¹H NMR (400 MHz, DMSO-d6) δ 9.10 (s, 2H), 8.65 (t, *J* = 6.2 Hz, 1H), 8.28 - 8.16 (m, 2H), 8.08 (d, *J* = 6.8 Hz, 1H), 7.92 (d, *J =* 8.1 Hz, 1H), 7.88 (d, *J =* 10.9 Hz, 1H), 7.32 (s, 1H), 6.53 (s, 1H), 5.44 (s, 2H), 5.29 (s, 2H), 4.63 - 4.52 (m, 2H), 4.31 - 4.19 (m, 1H), 4.19 - 4.08 (m, 1H), 3.74 (d, *J =* 4.9 Hz, 2H), 3.50 (t, *J =* 5.2 Hz, 2H), 3.41 (s, 3H), 3.24 - 3.18 (m, 2H), 3.03 - 2.94 (m, 6H), 2.55 (s, 3H), 2.46 - 2.24 (m, 3H), 2.05 - 1.67 (m, 8H), 1.66 - 1.51 (m, 8H), 1.36 - 1.22 (m, 4H), 0.91 - 0.80 (m, 15H).

### Example 2.36: N-((11S,14S)-11-(4-(Diethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-036)

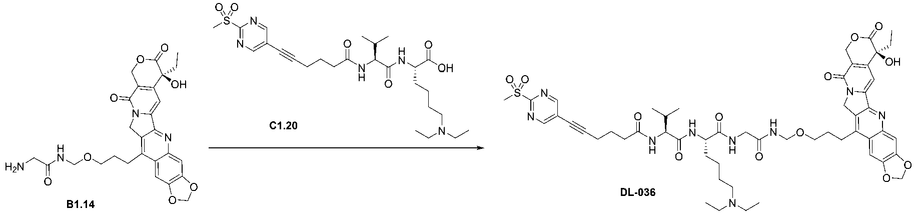

Compound C1.20 (40 mg, 0.075 mmol) and compound B1.14 (41 mg, 0.075 mmol) were dissolved in DMF (1 mL), and then HOBt (15.2 mg, 0.113 mmol) and 1-(3-dimethylaminopropyl)-3-ethylcarbodiimide hydrochloride (21.5 mg, 0.113 mmol) were added thereto, and then triethylamine (23 mg, 0.225mmol) was added thereto. After the addition, the reaction mixture was stirred at room temperature for 16 hours. After the completion of the reaction was detected by LCMS, the reaction mixture was concentrated to obtain a crude product, which was purified by preparative chromatography (0.01% TFA in water, MeCN) to obtain the target compound *N*-((11*S*,14*S*)-11-(4-(diethylamino)butyl)-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (16 mg, yield: 20%) as a yellow solid.
LCMS (ESI) [M+H]⁺ = 1070.6;
¹H NMR (400 MHz, DMSO-d6) δ 9.13 - 9.08 (m, 2H), 9.01 (s, 1H), 8.64 (t, *J* = 6.5 Hz, 1H), 8.20 (t, *J* = 5.7 Hz, 1H), 8.09 (d, *J* = 7.4 Hz, 1H), 7.92 (d, *J* = 8.4 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.65 - 4.53 (m, 2H), 4.26 (d, *J* = 6.5 Hz, 1H), 4.20 - 4.10 (m, 1H), 3.74 (d, *J* = 5.5 Hz, 2H), 3.50 (t, *J* = 5.8 Hz, 2H), 3.41 (s, 3H), 3.14 - 3.07 (m, 6H), 2.98 (s, 2H), 2.55 (d, *J =* 7.3 Hz, 2H), 2.41 - 2.29 (m, 2H), 2.03 - 1.89 (m, 2H), 1.89 - 1.77 (m, 7H), 1.61 - 1.56 (m, 2H), 1.32 (s, 2H), 1.16 (t, *J* = 7.2 Hz, 6H), 0.91 - 0.78 (m, 9H).

### Example 2.37: N-((11S,14S)-11-(4-(Dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (DL-037)

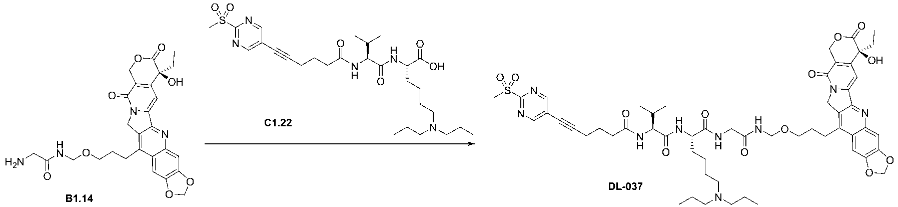

Compound C1.22 (43 mg, 0.074 mmol) and compound B1.14 (40 mg, 0.075 mmol) were dissolved in *N,N*-dimethylformamide (1 mL), and then HBTU (28 mg, 0.075 mmol) and *N,N*-diisopropylethylamine (24 mg, 0.187 mmol) were sequentially added thereto. The reaction mixture was stirred at room temperature for 1 hour. After the completion of the reaction was detected by LCMS, the reaction mixture was directly purified by preparative chromatography (0.01% trifluoroacetic acid in water, acetonitrile) to obtain the trifluoroacetate of the target compound (8.5 mg, yield: 10%) as a yellow solid.
LCMS (ESI) [M+H]⁺ = 1098.6;
¹H NMR (400 MHz, DMSO-d6) δ 9.10 (s, 2H), 9.03 (s, 1H), 8.64 (t, *J* = 6.4 Hz, 1H), 8.19 (t, *J =* 5.9 Hz, 1H), 8.08 (d, *J =* 7.4 Hz, 1H), 7.92 (d, *J* = 8.5 Hz, 1H), 7.59 (s, 1H), 7.51 (s, 1H), 7.24 (s, 1H), 6.49 (s, 1H), 6.29 (s, 2H), 5.42 (s, 2H), 5.24 (s, 2H), 4.66 - 4.52 (m, 2H), 4.31 - 4.21 (m, 1H), 4.19 - 4.10 (m, 1H), 3.74 (d, *J* = 5.5 Hz, 2H), 3.49 - 3.48 (m, 2H), 3.40 (s, 3H), 3.15 - 3.06 (m, 2H), 3.02 - 2.95 (m, 6H), 2.59 - 2.52 (m, 3H), 2.41 - 2.29 (m, 2H), 2.05 - 1.90 (m, 2H), 1.91 - 1.77 (m, 6H), 1.63 - 1.57 (m, 6H), 1.31 - 1.29 (m, 2H), 0.92 - 0.80 (m, 15H).

Using the same operation as in this example, the reaction mixture was directly purified by preparative chromatography (0.1% formic acid in water, acetonitrile) and lyophilized to obtain the formate of the target compound (32.6 mg, yield: 39%) as a white solid.
LCMS (ESI) [M+H]⁺ = 1098.6;
¹H NMR (400 MHz, DMSO-d₆) δ 9.09 (s, 2H), 8.56 (t, *J* = 6.5 Hz, 1H), 8.17 (s, 1H), 8.14 (t, *J =* 5.7 Hz, 1H), 7.97 (d, *J* = 7.3 Hz, 1H), 7.89 (d, *J* = 8.6 Hz, 1H), 7.58 (s, 1H), 7.50 (s, 1H), 7.24 (s, 1H), 6.45 (s, 1H), 6.28 (s, 2H), 5.48 - 5.35 (m, 2H), 5.24 (s, 2H), 4.64 - 4.54 (m, 2H), 4.23 - 4.07 (m, 2H), 3.79 - 3.66 (m, 2H), 3.50 (t, *J* = 5.9 Hz, 2H), 3.40 (s, 3H), 3.14 - 3.08 (t, *J =* 8.2 Hz, 2H), 2.59 - 2.52 (m, 2H), 2.36 - 2.27 (m, 8H), 2.00 - 1.76 (m, 7H), 1.72-1.62 (m, 1H), 1.58- 1.48 (m, 1H), 1.40 - 1.20 (m, 8H), 0.88 - 0.77 (m, 15H).

The formate of DL-037 (16 mg) was purified by preparative HPLC (0.1% ammonium bicarbonate in water, acetonitrile) and lyophilized to obtain the free target compound (DL-037, 10 mg, yield: 65%) as a white solid.
LCMS (ESI) [M+H]⁺ = 1098.6;
¹H NMR (400 MHz, DMSO-d6) δ 9.10 (s, 2H), 8.61 (t, *J* = 6.4 Hz, 1H), 8.21 (t,*J* = 5.6 Hz, 1H), 8.04 (d, J = 7.2 Hz, 1H), 7.95 (d, *J* = 8.5 Hz, 1H), 7.53 (s, 1H), 7.48 (s, 1H), 7.22 (s, 1H), 6.50 (s, 1H), 6.26 (s, 2H), 5.48 - 5.35 (m, 2H), 5.16 (s, 2H), 4.63 - 4.51 (m, 2H), 4.25 - 4.10 (m, 2H), 3.82 - 3.63 (m, 2H), 3.49 (t, *J* = 5.8 Hz, 2H), 3.40 (s, 3H), 3.06 (t, *J* = 8.0 Hz, 2H), 2.56 - 2.51 (m, 2H), 2.41 - 2.23 (m, 8H), 1.96 - 1.76 (m, 7H), 1.71 - 1.57 (m, 1H), 1.57 - 1.48 (m, 1H), 1.37 - 1.21 (m, 8H), 0.88 - 0.78 (m, 15H).

### Example 2.38: (S)-6-(Dipropylamino)-N-(2-(((((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide

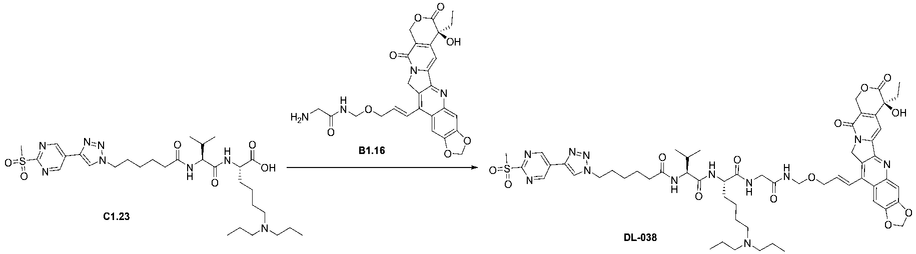

Compound B1.16 (30 mg, 0.06 mmol) and compound C1.23 (37 mg, 0.06 mmol) were dissolved in DMF (1 mL). Then HBTU (21 mg, 0.06 mmol) and *N,N*-diisopropylethylamine (16 mg, 0.12 mmol) were added thereto. After the addition, the reaction mixture was stirred at room temperature for 1 hour. After the completion of the reaction was detected by LCMS, the reaction mixture was purified by preparative chromatography (0.01% TFA in water, MeCN) to obtain the target compound (*S*)-6-(dipropylamino)-*N*-(2-(((((*E*)-3-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (4 mg, yield: 6%) as a yellow solid.
LCMS (ESI) [M+H]⁺ = 1167.6;
¹H NMR (400 MHz, DMSO) δ 9.45 (s, 2H), 8.98 (s, 1H, TFA), 8.92 (s, 1H), 8.74 (t, *J* = 6.8 Hz, 1H), 8.25 (t, *J* = 5.8 Hz, 1H), 8.05 (d, *J* = 7.3 Hz, 1H), 7.82 (d, *J* = 8.4 Hz, 1H), 7.63 (s, 1H), 7.53 (s, 1H), 7.26 (d, *J =* 12.0 Hz, 1H), 7.26 (s, 1H), 6.58 - 6.52 (m, 1H), 6.50 (s, 1H), 6.30 (s, 2H), 5.42 (s, 2H), 5.31 (s, 2H), 4.74 - 4.67 (m, 2H), 4.46 (m, 2H), 4.30 (m, 1H), 4.10 (m, 1H), 3.77 (m, 2H), 3.44 (s, 3H), 3.01 - 2.95 (m, 8H), 2.23 - 2.18 (m, 1H), 2.13 (m, 1H), 2.00 (m, 2H), 1.90 - 1.84 (m, 6H), 1.63 - 1.57 (m, 9H), 0.88 (m, 9H), 0.81 - 0.78 (m, 6H).

### Example 2.39: 4-((S)-6-Amino-2-(6-(2-(methylsulfonyl)pyrimidin-5-yl)hex-5-ynamido)hexanamido)benzyl N-ethyl-N-(2-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)carbamate

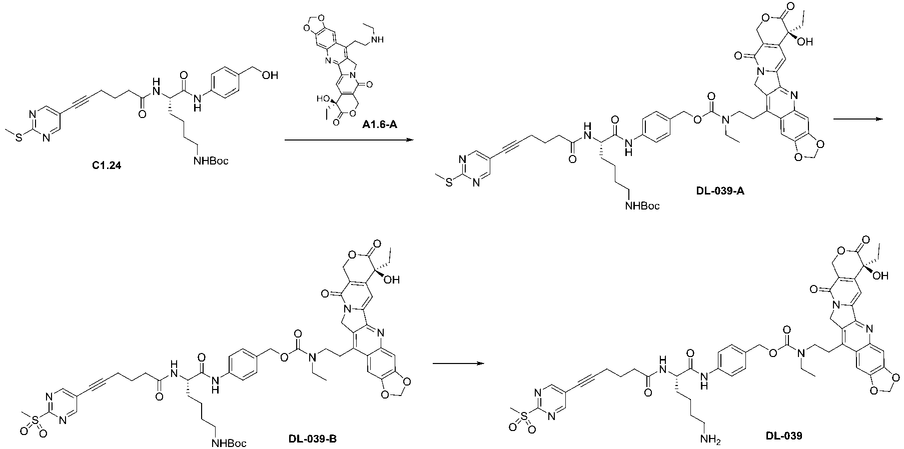

### Step 1:

*N,N*-Succinimidyl carbonate (DSC, 90 mg, 0.352 mmol) was added to a solution of C1.24 (150 mg, 0.264 mmol) and triethylamine (89 mg, 0.88 mmol) in dichloromethane (10 mL) in an ice bath at 0°C. The reaction mixture was reacted under nitrogen atmosphere at 0°C for 30 minutes. Compound A1.6-A (82 mg, 0.176 mmol) was then added dropwise thereto, and the reaction mixture was reacted at room temperature for 12 hours. The completion of the reaction was detected by LCMS. The reaction mixture was concentrated under reduced pressure to a crude product, which was purified by preparative high-performance liquid chromatography (acetonitrile/0.05% formic acid in water) to obtain compound DL-039-A (27 mg, yield: 14.4%) as a yellow oil.
LCMS (ESI) [M+H]⁺ = 1059.2;

### Step 2:

Potassium peroxymonosulfate (Oxone, 349 mg, 0.567 mmol) was added to a mixed solution of compound DL-039-A (60 mg, 0.057 mmol) in tetrahydrofuran (5 mL) and water (1 mL), and the reaction mixture was stirred at room temperature for 12 hours. The completion of the reaction was detected by TLC. The reaction mixture was added with water (10 mL) and extracted with dichloromethane (10 mL × 3). The organic phase was washed with saturated brine, dried over anhydrous sodium sulfate, subjected to suction filtration, and concentrated under reduced pressure to obtain the target compound DL-039-B (60 mg, crude product) as a yellow solid.
LCMS (ESI) [M+H]⁺ = 1091.0;

### Step 3:

Compound DL-039-B (60 mg, 0.055 mmol) was dissolved in dichloromethane (1 mL) solution, then dichloroacetic acid (1 mL) was added thereto at 0°C, and the reaction mixture was stirred at room temperature for 3 hours. LCMS showed the completion of the reaction. The filtrate was concentrated under reduced pressure to a crude product, which was purified by preparative high-performance liquid chromatography (acetonitrile/0.05% trifluoroacetic acid in water) to obtain compound 4-((S)-6-amino-2-(6-(2-(methylsulfonyl)pyrimidin-5-yl)hex-5-ynamido)hexanamido)benzyl *N*-ethyl-*N*-(2-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)ethyl)carbamate (2.44 mg, yield: 3.69%) as a yellow solid.

LCMS (ESI) [1/2M+H]⁺ = 496.3, t_{R} = 2.252 min.

### Example 2.40: 4-((S)-6-Amino-2-(6-(4-(2-(methylsulfonyl)pyrimidin-5-yl)-1H-1,2,3-triazol-1-yl)hexanamido)hexanamido)benzyl N-ethyl-N-(2-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)ethyl)carbamate (DL-040)

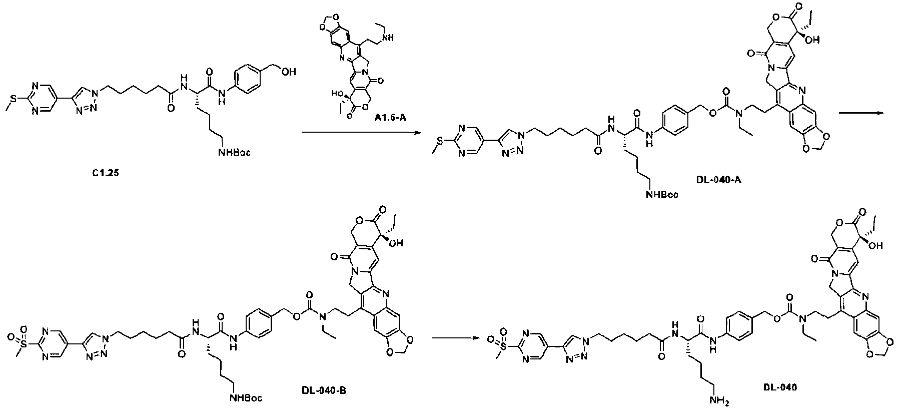

### Step 1:

*N,N*-Succinimidyl carbonate (DSC, 160 mg, 0.626 mmol) was added to a solution of C1.25 (300 mg, 0.469 mmol) and triethylamine (126 mg, 1.25 mmol) in dichloromethane (10 mL) in an ice bath at 0°C. The reaction mixture was reacted at 0°C for 30 minutes. Compound A1.6-A (145 mg, 0.313 mmol) was then added dropwise thereto, and then the reaction mixture was reacted at room temperature for 12 hours. The completion of the reaction was detected by LCMS. The reaction mixture was concentrated under reduced pressure to a crude product, which was purified by preparative high-performance liquid chromatography (acetonitrile/0.05% formic acid in water) to obtain compound DL-040-A (70 mg, yield: 19.8%) as a yellow oil. LCMS (ESI) [M+H]⁺ = 1130.1.

### Step 2:

Potassium peroxymonosulfate (Oxone, 381 mg, 0.619 mmol) was added to a mixed solution of compound DL-040-A (70 mg, 0.062 mmol) in tetrahydrofuran (5 mL) and water (1 mL), and the reaction mixture was stirred at room temperature for 12 hours. The completion of the reaction was detected by TLC. The reaction mixture was added with water (10 mL) and extracted with dichloromethane (10 mL × 3). The organic phase was washed with saturated brine, dried over anhydrous sodium sulfate, subjected to suction filtration, and concentrated under reduced pressure to obtain the target compound DL-040-B (70 mg, crude product) as a yellow solid. LCMS (ESI) [M+H]⁺ = 1162.6, t_{R} = 1.710 min.

### Step 3:

Compound DL-040-B (70 mg, 0.060 mmol) was dissolved in dichloromethane (1 mL) solution, then dichloroacetic acid (1 mL) was added thereto at 0°C, and the reaction mixture was stirred at room temperature for 3 hours. LCMS showed the completion of the reaction. The filtrate was concentrated under reduced pressure to a crude product, which was purified by preparative high-performance liquid chromatography (acetonitrile/0.05% trifluoroacetic acid in water) to obtain compound 4-((*S*)-6-amino-2-(6-(4-(2-(methylsulfonyl)pyrimidin-5-yl)-1*H*-1,2,3-triazol-1-yl)hexanamido)hexanamido)benzyl *N*-ethyl-*N*-(2-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-b]quinolin-14-yl)ethyl)carbamate (5.54 mg, yield: 8.66%) as a yellow solid.
LCMS (ESI) [1/2M+H]⁺ = 1062.5, t_{R} = 4.052 min;
¹H NMR (400 MHz, DMSO-d6) δ 10.22 (br s, 1H), 9.46 (s, 2H), 8.93 (s, 1H), 8.22 - 8.03 (m, 1H), 7.85 (br s, 3H, NH2-TFA), 7.61 - 7.45 (m, 3H), 7.32 - 7.26 (m, 1H), 7.26 - 7.17 (m, 2H), 6.54 (s, 1H), 6.35 (s, 2H), 5.91 (s, 2H), 5.47 (s, 2H), 5.37 - 5.30 (m, 1H), 5.27 - 5.20 (m, 1H), 5.10 - 5.00 (m, 1H), 4.97 - 4.78 (m, 1H), 4.52 - 4.26 (m, 3H), 3.56 - 3.46 (m, 2H), 3.44 (s, 3H), 2.83 - 2.71 (m, 2H), 2.21 - 2.11 (m, 2H), 1.98 - 1.78 (m, 4H), 1.67 - 1.48 (m, 6H), 1.37 - 1.22 (m, 4H), 1.09 - 0.96 (m, 3H), 0.93 - 0.78 (m, 3H).

### Example 2.41: (S)-6-(Dimethylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-041)

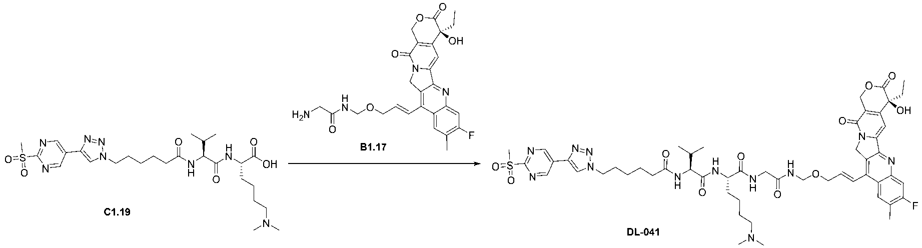

Compound B1.17 (30 mg) and compound C1.19 (37 mg) were dissolved in DMF (1 mL). Then HBTU (21 mg) and *N,N*-diisopropylethylamine (16 mg) were added thereto. After the addition, the reaction mixture was stirred at room temperature for 1 hour. After the completion of the reaction was detected by LCMS, the reaction mixture was purified by preparative chromatography (0.01% TFA in water, MeCN) to obtain the target compound (*S*)-6-(dimethylamino)-*N*-(2-(((((*E*)-3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (11 mg).

LCMS (ESI) [M+H]⁺ = 1099.5.

### Example 2.42: (S)-6-(Diethylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-042)

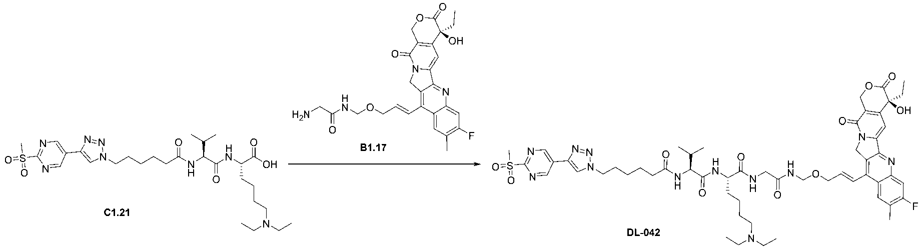

Compound B1.17 (30 mg) and compound C1.21 (37 mg) were dissolved in DMF (1 mL). Then HBTU (21 mg) and *N,N*-diisopropylethylamine (16 mg) were added thereto. After the addition, the reaction mixture was stirred at room temperature for 1 hour. After the completion of the reaction was detected by LCMS, the reaction mixture was purified by preparative chromatography (0.01% TFA in water, MeCN) to obtain the target compound (S)-6-(diethylamino)-*N*-(2-(((((*E*)-3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (7 mg).

LCMS (ESI) [M+H]⁺ = 1127.5.

### Example 2.43: (S)-6-(Dipropylamino)-N-(2-(((((E)-3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-043)

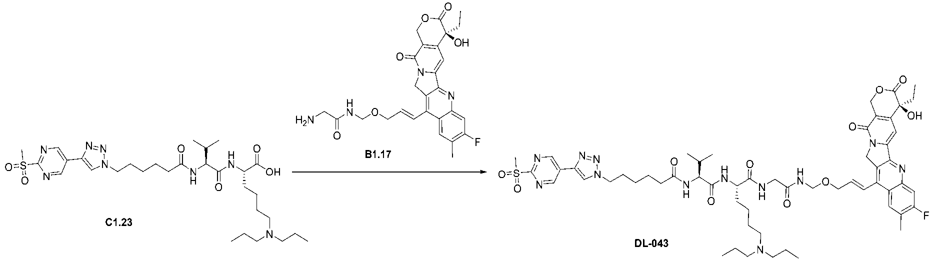

Compound B1.17 (17.5 mg, 0.033 mmol) and compound C1.23 (22 mg, 0.034 mmol) were dissolved in *N,N*-dimethylformamide (2 mL), and then HOBT (7 mg, 0.051 mmol), EDCI (10 mg, 0.051mmol), and DIPEA (9 mg, 0.068 mmol) were sequentially added thereto. After the addition, the reaction mixture was stirred at room temperature for 1 hour. The reaction mixture was directly purified by preparative high-performance liquid chromatography (0.01% trifluoroacetic acid in water, acetonitrile). The preparative solution was freeze-dried to obtain the target compound (*S*)-6-(diethylamino)-*N*-(2-(((((*E*)-3-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (7 mg, yield: 18%) as a light yellow solid product.

LCMS (ESI) [M+H]⁺ = 1155.5.

### Example 2.44: N-((11S,14S,E)-11-(4-(Dimethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (DL-044)

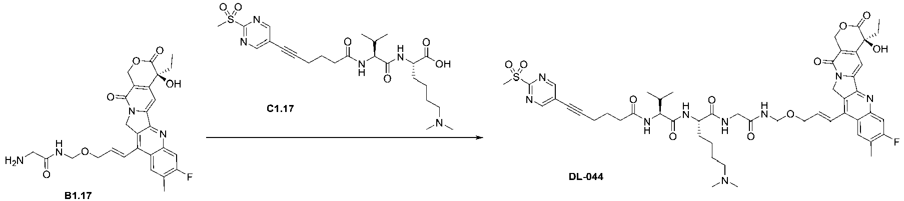

Compound B1.17 (60 mg) and compound C1.17 (80 mg) were dissolved in DMF (2 mL). Then HBTU (45 mg) and *N,N*-diisopropylethylamine (35 mg) were added thereto. After the addition, the reaction mixture was stirred at room temperature for 1 hour. After the completion of the reaction was detected by LCMS, the reaction mixture was purified by preparative chromatography (0.01% TFA in water, MeCN) to obtain the target compound N-((11*S*,14*S*,*E*)-11-(4-(dimethylamino)butyl)-1-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (21 mg).

LCMS (ESI) [M+H]⁺ = 1028.5.

### Example 2.45: N-((11S,14S,E)-11-(4-(Diethylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (DL-045)

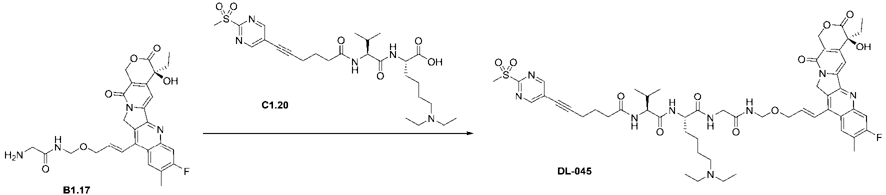

Compound B1.17 (60 mg) and compound C1.20 (80 mg) were dissolved in DMF (2 mL). Then HBTU (45 mg) and *N,N*-diisopropylethylamine (35 mg) were added thereto. After the addition, the reaction mixture was stirred at room temperature for 1 hour. After the completion of the reaction was detected by LCMS, the reaction mixture was purified by preparative chromatography (0.01% TFA in water, MeCN) to obtain the target compound *N-*((11*S,*14*S*,*E*)-11-(4-(diethylamino)butyl)-1-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (29 mg).

LCMS (ESI) [M+H]⁺ = 1056.5.

### Example 2.46: N-((11S,14S,E)-11-(4-(Dipropylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (DL-046)

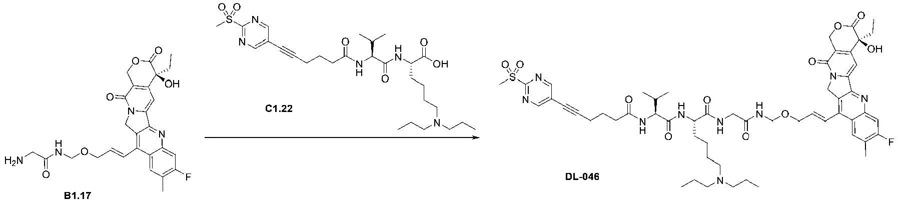

Compound B1.17 (60 mg) and compound C1.22 (80 mg) were dissolved in DMF (2 mL). Then HBTU (45 mg) and *N,N*-diisopropylethylamine (35 mg) were added thereto. After the addition, the reaction mixture was stirred at room temperature for 1 hour. After the completion of the reaction was detected by LCMS, the reaction mixture was purified by preparative chromatography (0.01% TFA in water, MeCN) to obtain the target compound *N-*((11*S*,14*S*,*E*)-11-(4-(diethylamino)butyl)-1-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)-15-methyl-7,10,13-trioxo-4-oxa-6,9,12-triazahexadec-1-en-14-yl)-6-(2-(methylsulfonyl)pyrimidin-5-yl)hex-5-ynamide (30 mg).
LCMS (ESI) [M+H]⁺ = 1084.2;
¹H NMR (400 MHz, DMSO) δ 9.09 (s, 2H), 8.73 (d, *J =* 6.3 Hz, 1H), 8.30 - 8.24 (m, 2H), 8.05 (m, 1H), 7.94 - 7.88 (m, 2H), 7.38 - 7.33 (m, 2H), 6.67 - 6.60 (m, 1H), 6.53 (s, 1H), 5.44 (s, 2H), 5.37 (s, 2H), 4.73 (t, *J* = 6.2 Hz, 2H), 4.34 (d, *J* = 4.2 Hz, 2H), 4.26 - 4.20 (m, 1H), 4.17 - 4.13 (m, 1H), 3.86 - 3.69 (m, 4H), 3.40 (s, 3H), 2.41 - 2.30 (m, 8H), 2.02 - 1.94 (m, 2H), 1.89 - 1.85 (m, 2H), 1.82 - 1.77 (m, 2H), 1.74 - 1.63 (m, 2H), 1.60 - 1.50 (m, 2H), 1.47 - 1.39 (m, 8H), 0.90 - 0.86 (m, 6H), 0.85 - 0.80 (m, 9H).

### Example 2.47: N-((11S,14S,E)-11-(4-(Dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-oxo-4-oxa-6,9,12-triazahexadec-1-en-14-yl)-6-(2-(methylsulfonyl)pyrimidin-5-yl)hex-5-ynamide (DL-047)

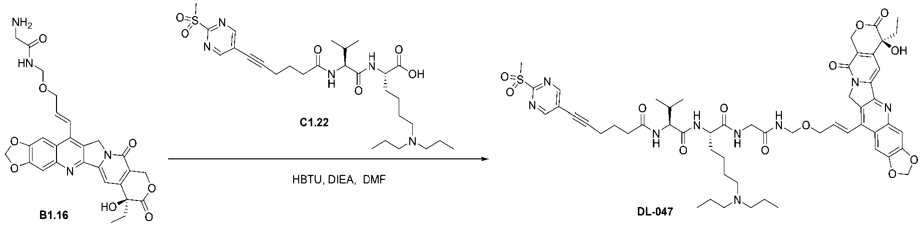

Compound B1.16 (45 mg, 0.08 mmol) and compound C1.22 (49 mg, 0.08 mmol) were dissolved in DMF (1 mL). Then HBTU (32 mg, 0.08 mmol) and *N*,*N*-diisopropylethylamine (27 mg, 0.2 mmol) were added thereto. After the addition, the reaction mixture was stirred at room temperature for 1 hour. After the completion of the reaction was detected by LCMS, the reaction mixture was purified by preparative chromatography (0.01% trifluoroacetic acid in water, acetonitrile) to obtain the target compound *N-*((11*S*,14*S*,*E*)-11-(4-(dipropylamino)butyl)-1-((*S*)-7-ethyl-7-hydroxy-8,11-dioxo-7,8,11,13-tetrahydro-10*H-*[1,3]dioxolo[4,5-*g*]pyrano[3',4':6,7]indolizino[1,2-*b*]quinolin-14-yl)-15-methyl-7,10,13-oxo-4-oxa-6,9,12-triazahexadec-1-en-14-yl)-6-(2-(methylsulfonyl)pyrimidin-5-yl)hex-5-ynamide (DL-047, 10 mg) as a yellow solid.
LCMS (ESI) [M+H]⁺ = 1096.6;
¹H NMR (400 MHz, DMSO) δ 9.10 (s, 2H), 9.05 (s, 1H, TFA), 8.75 (t, *J* = 5.3 Hz, 1H), 8.26 (t, *J* = 6.4 Hz, 1H), 8.11 (d, *J* = 7.6 Hz, 1H), 7.93 (d, *J* = 8.4 Hz, 1H), 7.63 (s, 1H), 7.53 (s, 1H), 7.26 - 2.24 (m, 2H), 6.65 - 6.43 (m, 2H), 6.30 (s, 2H), 5.42 (s, 2H), 5.30 (s, 2H), 4.74 - 4.69 (m, 1H), 4.31 - 4.29 (m, 2H), 4.26 - 424 (m, 1H), 4.19 - 4.12 (m, 1H), 3.79 - 2.76 (m, 1H), 3.40 (s, 3H), 3.02 - 2.95 (m, 8H), 2.55 (m, 2H), 2.39 - 2.32 (m, 2H), 2.01 - 1.93 (m, 2H), 1.92 - 1.77 (m, 5H), 1.62 - 1.58 (m, 6H), 1.36 - 1.30 (m, 2H), 0.91 - 0.83 (m, 15H).

### Example 2.48: N-((11S,14S)-11-(4-(Dibutylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecan-14-yl)-6-(2-(methylsulfonyl)pyrimidin-5-yl)hex-5-ynamide (DL-048)

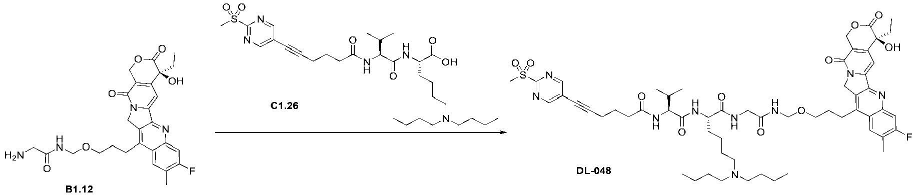

Compound C1.26 (50 mg, 0.08 mmol) and compound B1.12 (43 mg, 0.08 mmol) were dissolved in *N,N*-dimethylformamide (1.5 mL), and then DIPEA (26 mg, 0.21 mmol) and HBTU (31 mg, 0.08 mmol) were sequentially added thereto. The reaction mixture was stirred and reacted at room temperature for 1 hour. The completion of the reaction was detected by LCMS, and the reaction mixture was directly purified by preparative high-performance liquid chromatography (0.01% trifluoroacetic acid in water, acetonitrile) to obtain the target compound (13.57 mg) as a yellow solid.
LCMS (ESI) [M+H]⁺= 1114.6;
¹H NMR (400 MHz, DMSO) δ 9.09 (s, 2H), 9.05 (s, 1H, TFA), 8.64 (t, *J* = 7.2 Hz, 1H), 8.23 - 8.16 (m, 2H), 8.07 (d, *J =* 7.3 Hz, 1H), 7.93 - 7.85 (m, 2H), 7.32 (s, 1H), 6.52 (s, 1H), 5.44 (s, 2H), 5.29 (s, 2H), 4.61 - 4.59 (m, 2H), 4.24 - 4.22 (m, 1H), 4.19 - 4.10 (m, 1H), 3.73 - 3.70 (m, 2H), 3.50 (s, 3H), 3.21 - 3.18 (m, 6H), 3.00 - 2.98 (m, 8H), 2.54 (s, 3H), 1.97 - 1.78 (m, 7H), 1.56 - 1.52 (m, 8H), 1.36 - 1.25 (m, 6H), 0.93 - 0.78 (m, 15H).

### Example 2.49: (S)-6-(Dimethylamino)-N-(2-(((((E)-3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)allyl)oxy)methyl)amino)-2-oxoethyl)-2-((S)-3-methyl-2-(6-(4-(2-(methylsulfonyl)pyrimidin-5-yl)-1H-1,2,3-triazol-1-yl)hexanamido)butanamido)hexanamide (DL-049)

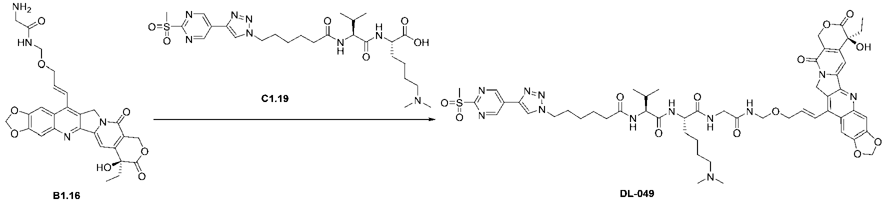

Compound B1.16 (45 mg, 0.08 mmol) and compound C1.19 (50 mg, 0.08 mmol) were dissolved in DMF (1 mL). Then *N,N*-diisopropylethylamine (27 mg, 0.2 mmol) and HBTU (32 mg, 0.08 mmol) were sequentially added thereto. After the addition, the reaction mixture was stirred at room temperature for 1 hour. After the completion of the reaction was detected by LCMS, the reaction mixture was directly purified by preparative chromatography (0.01% formic acid in water, acetonitrile) to obtain the target compound (*S*)-6-(dimethylamino)-*N*-(2-(((((*E*)-3-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)allyl)oxy)methyl)amino)-2-oxoethyl)-2-((*S*)-3-methyl-2-(6-(4-(2-(methylsulfonyl)pyrimidin-5-yl)-1*H*-1,2,3-triazol-1-yl)hexanamido)butanamido)hexanamide (DL-049, 3 mg) as a white solid.
LCMS (ESI) [M+H]⁺ = 1111.1;
¹H NMR (400 MHz, DMSO) δ 9.45 (s, 2H), 8.92 (s, 1H), 8.69 (t, *J* = 6.6 Hz, 1H), 8.25 (t, *J =* 5.9 Hz, 1H), 8.19 (s, 1H, FA), 7.99 (d, *J =* 7.0 Hz, 1H), 7.83 (d, *J =* 8.5 Hz, 1H), 7.64 (s, 1H), 7.52 (s, 1H), 7.27 (m, 2H), 6.59 - 6.48 (m, 2H), 6.29 (s, 2H), 5.42 (s, 2H), 5.31 (s, 2H), 4.74 - 4.67 (m, 2H), 4.44 (m, 2H), 4.30 (d, *J* = 4.8 Hz, 2H), 4.21 (m, 1H), 4.12 (m, 1H), 3.83 - 3.70 (m, 4H), 3.43 (s, 3H), 2.25 (m, 2H), 2.15 (s, 6H), 2.04 - 1.97 (m, 2H), 1.90 - 1.85 (m, 4H), 1.55 - 1.51 (m, 3H), 1.39 - 1.34 (m, 6H), 0.90 - 0.83 (m, 3H), 0.80 (t, *J* = 7.2 Hz, 6H).

### Example 2.50: 1-Ethyl-N-(2-(((3-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)amino)-2-oxoethyl)-4-((S)-3-methyl-2-(6-(2-(methylsulfonyl)pyrimidin-5-yl)hex-5-ynamido)butanamido)piperidine-4-carboxamide (DL-050)

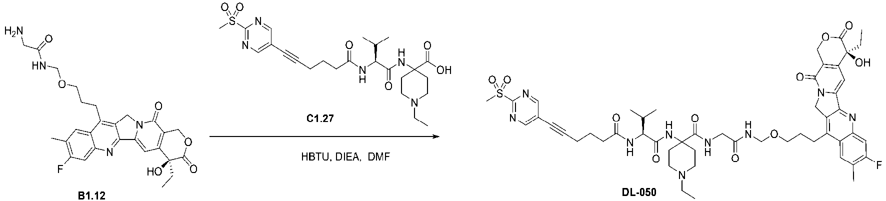

Compound B1.12 (50 mg, 0.10 mmol) and compound C1.27 (50 mg, 0.10 mmol) were dissolved in DMF (1 mL). Then *N,N*-diisopropylethylamine (30 mg, 0.24 mmol) and HBTU (36 mg, 0.10 mmol) were sequentially added thereto. After the addition, the reaction mixture was stirred at room temperature for 1 hour. After the completion of the reaction was detected by LCMS, the reaction mixture was purified by preparative chromatography (0.01% FA in water, MeCN) to obtain the target compound 1-ethyl-*N*-(2-(((3-((*S*)-4-ethyl-8-fluoro-4-hydroxy-9-methyl-3,14-dioxo-3,4,12,14-tetrahydro-1*H*-pyrano[3',4':6,7]indolizino[1,2-b]quinolin-11-yl)propoxy)methyl)amino)-2-oxoethyl)-4-((*S*)-3-methyl-2-(6-(2-(methylsulfonyl)pyrimidin-5-yl)hex-5-ynamido)butanamido)piperidine-4-carboxamide (DL-050, 10 mg) as a white solid.
LCMS (ESI) [M+H]⁺ = 1028.6;
¹H NMR (400 MHz, DMSO) δ 9.08 (s, 2H), 8.42 (s, 1H, FA), 8.18 - 8.15 (m, 4H), 7.98 (t, *J =* 5.6 Hz, 1H), 7.86 (d, *J =* 10.9 Hz, 1H), 7.30 (s, 1H), 6.51 (s, 1H), 5.43 (s, 2H), 5.28 (s, 2H), 4.72 - 4.63 (m, 1H), 4.53 - 4.45 (m, 1H), 4.07 - 4.06 (m, 1H), 3.79 - 3.69 (m, 1H), 3.58 - 3.55 (m, 2H), 3.55 - 3.48 (m, 5H), 3.21 - 3.16 (m, 3H), 2.55 (s, 3H), 2.66 - 2.64 (m, 2H), 2.40 - 2.26 (m, 5H), 1.99 - 1.71 (m, 12H), 0.96 (t, *J* = 7.1 Hz, 3H), 0.93 - 0.85 (m, 9H).

### Example 2.51: (R)-6-(Dimethylamino)-N-(4-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)phenyl)-2-((S)-3-methyl-2-(6-(4-(2-(methylsulfonyl)pyrimidin-5-yl)-1H-1,2,3-triazol-1-yl)hexanamido)butanamido)hexanamide (DL-051) and (S)-6-(dimethylamino)-N-(4-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)phenyl)-2-((S)-3-methyl-2-(6-(4-(2-(methylsulfonyl)pyrimidin-5-yl)-1H-1,2,3-triazol-1-yl)hexanamido)butanamido)hexanamide (DL-052)

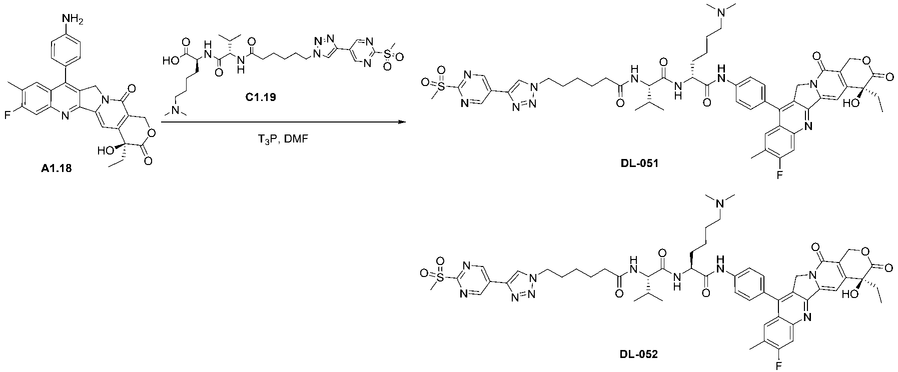

Compounds A1.18 (40 mg, 0.08 mmol) and C1.19 (71 mg, 0.12 mmol) were dissolved in *N,N*-dimethylformamide (3 mL). T₃P (254 mg, 0.40 mmol, 50% w/w mixture with ethyl acetate) was then added thereto, and the reaction mixture was stirred at room temperature for 1 hour. After the completion of the reaction was detected by LCMS, the reaction mixture was directly purified by preparative chromatography (0.01% trifluoroacetic acid in water, acetonitrile) to obtain the target compounds (*R*)-6-(dimethylamino)-*N*-(4-((*S*)-4-ethyl-8-fluoro-4-hydroxy-9-methyl-3,14-dioxo-3,4,12,14-tetrahydro-1*H-*pyrano[3',4':6,7]indolizino[1,2-*b*]quinolin-11-yl)phenyl)-2-((*S*)-3-methyl-2-(6-(4-(2-(methylsulfonyl)pyrimidin-5-yl)-1*H*-1,2,3-triazol-1-yl)hexanamido)butanamido)hexanamide (DL-051, 8.1 mg) and (*S*)-6-(dimethylamino)-*N*-(4-((*S*)-4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)phenyl)-2-((*S*)-3-methyl-2-(6-(4-(2-(methylsulfonyl)pyrimidin-5-yl)-1*H*-1,2,3-triazol-1-yl)hexanamido)butanamido)hexanamide (DL-052, 8.8 mg), which were both white solids.
DL-051:
LCMS (ESI) [M+H]⁺ = 1048.2, t_{R} = 1.258 min;
¹H NMR (400 MHz, DMSO) δ 10.12 (s, 1H), 9.40 (s, 2H), 8.84 (s, 1H), 8.55 (d,*J* = 7.4 Hz, 1H), 8.26 (s, 1H), 8.05 (d, *J* = 7.8 Hz, 1H), 7.99 - 7.89 (m, 3H), 7.73 (d, *J* = 8.4 Hz, 1H), 7.57 (d, *J* = 8.7 Hz, 2H), 7.36 (s, 1H), 5.41 (s, 2H), 5.10 (s, 2H), 4.40 (m, 3H), 4.22 - 4.10 (m, 1H), 3.44 (s, 3H), 2.40 (s, 3H), 2.30 - 2.25 (m, 2H), 2.24 - 2.20 (m, 2H), 2.19 - 2.15 (m, 6H), 2.03 - 1.93 (m, 2H), 1.92 - 1.78 (m, 6H), 1.72 - 1.66 (m, 1H), 1.61 - 1.54 (m, 2H), 1.48 - 1.42 (m, 2H), 1.30 - 1.26 (m, 2H), 0.91 - 0.86 (m, 9H).
DL-052:
LCMS (ESI) [M+H]⁺ = 1048.2, t_{R} = 1.283 min;
¹H NMR (400 MHz, DMSO) δ 10.34 (s, 1H), 9.45 (s, 2H), 8.94 (s, 1H), 8.20 (s, 1H), 7.99 - 7.85 (m, 4H), 7.75 (d, *J =* 7.4 Hz, 1H), 7.59 (m, 2H), 7.36 (s, 1H), 6.55 (s, 1H), 5.42 (s, 2H), 5.10 (s, 2H), 4.52 - 4.42 (m, 3H), 4.25 - 4.17 (m, 1H), 3.44 (s, 3H), 2.41 (s, 3H), 2.30 - 2.13 (m, 8H), 2.05 - 1.97 (m, 2H), 1.95 - 1.81 (m, 7H), 1.60 - 1.55 (m, 2H), 1.50 - 1.44 (m, 2H), 1.33 - 1.28 (m, 4H), 0.95 - 0.83 (m, 9H).

### III. Antibody preparation and identification

### Example 3. Preparation of anti-DLL3 antibody

### 3.1 Preparation of antigen

1) Antigen preparation: The full-length amino acid sequences of human DLL3, DLL1, and DLL4 were obtained from Q9NYJ7, O00548, and Q9NR61 in the UniProt protein database, respectively. The full-length amino acid sequences of rat DLL3 and mouse DLL3 were obtained from 088671 and 088516 in the UniProt protein database, respectively. The above amino acid sequences were optimized according to human codon bias and then synthesized into the lentiviral vector pLVX-puromycin (Sangon Biotech (Shanghai) Co., Ltd.). The full-length amino acid sequence of canine DLL3 was obtained from A0A8C0KSU2 in the UniProt protein database. The extracellular domain amino acid sequence of canine DLL3 ranged from positions 48 to 515. The extracellular domain amino acid sequence of canine DLL3 was optimized according to human codon bias and then separately synthesized into the pTT5-His vector. The truncated forms of human DLL3, D7 (27-492), D6 (27-428), D5 (27-390), D4 (27-352), D3 (27-311), D2 (27-250), and D1 (27-215), were fused with the EPCAM transmembrane region., then the amino acid sequences were optimized according to human codon bias and then separately synthesized into the lentiviral vector pLVX-puromycin (General Biol (Anhui) Co., Ltd.).

HEK293-EBNA cells were transiently transfected with PEImax (Polysciences, 24765-1), and canine canis DLL3-ECD-his antigen protein was obtained by purification through Ni resin (Bestchrom, Ni Bestarose FF) after 7 days of expression.

Commercialized antigen proteins: The recombinant extracellular domain protein of human DLL3 (h.DLL3-ECD-His) was from Kactus Biosystems (Catalog No.: DLL-HM103). The recombinant extracellular domain protein of cynomolgus monkey DLL3 (cyno.DLL3-ECD-His) was from ACROBiosystems (Catalog No.: DL3-C52H3). The recombinant extracellular domain protein of rat DLL3 (Rat DLL3-His) was from ACROBiosystems (Catalog No.: DL3-R52H3). The recombinant extracellular domain protein of mouse DLL3 (Mouse DLL3-His) was from Wuhan CUSABIO (Catalog No.: CSB-MP006948MO1).

2) Cell line construction: Viruses were prepared using a lentiviral packaging system. After obtaining the viruses, HEK293T cells were infected and screened with puromycin, obtaining stable cell lines overexpressing human DLL1 and DLL4, respectively, stable cell lines overexpressing human, rat, and mouse DLL3, respectively, and stable cell lines expressing various truncated forms of human DLL3. The different stable cell lines were named respectively as 293T-hDLL1, 293T-hDLL4, 293T-hDLL3, 293T-ratDLL3, 293T-mDLL3, 293T-hDLL3-D1, 293T-hDLL3-D2, 293T-hDLL3-D3, 293T-hDLL3- D4, 293T-hDLL3-D5, 293T-hDLL3-D5, and 293T-hDLL3-D7.

### 3.2 Mouse immunization and immune titer detection

Four female mice, approximately 6 weeks old, were selected for each strain: CD1 (Vital River), KM (Vital River), and Balb/c (GemPharmatech). The mice were immunized with L929-hDLL3 cells, L929-cynoDLL3 cells, and hDLL3-His.

After two booster immunizations, serum titers were assessed by ELISA. The detection method was as follows: The night before the experiment, human DLL3 antigen was coated with CBS coating solution at a coating concentration of 0.5/0.2 µg/mL in a volume of 100 µL, and incubated at 4°C overnight. Alternatively, monkey DLL3 antigen was coated with CBS coating solution at a coating concentration of 0.5/0.2 µg/mL in a volume of 100 µL, and incubated at 4°C overnight. The next day, the coating solution was removed, and each well was washed once with 300 µL of PBS. Each well was then added with 100 µL of 2% BSA (Biotopped, A6020) (in PBS), and blocked at 37°C for 2 hours. Serum samples were diluted in 2% BSA (in PBS) starting at 500-fold and diluted 3-fold for a total of 11 dilution points. After dilution, 100 µL of the serum was added to the plate and incubated at 37°C for 2 hours. The supernatant was then removed, and each well was washed three times with 300 µL of PBST. 100 µL of HRP-labeled anti-mouse secondary antibody (Jackson, 115-035-003) in 2% BSA was added to each well and incubated at 37°C for 1 hour. The secondary antibody was removed, and each well was washed five times with 300 µL of PBST. Then, 100 µL of TMB substrate (Huzhou InnoReagents, TMB-S-004) was added to each well and the color development was carried out for 5 to 20 minutes. The reaction was stopped by adding 50 µL of 2N HCl to each well, and the OD450 nM readings were measured using a microplate reader (manufacturer: MD, model: SpectraMax). The data were imported into GraphPad software for fitting. Based on the detection results, a total of four mice, Balb/c, CD1 (2 mice), and KM, were selected for hybridoma fusion.

### 3.3 Hybridoma fusion screening

Hybridoma fusion: Cell suspensions were prepared from mouse spleen and lymph nodes, mixed with SP2/0 mouse myeloma cells at a ratio of 1:1. The cells were resuspended in cell electrofusion buffer and subjected to electrofusion reaction using BTX-ECM2001. The cells were resuspended in a complete fusion medium (RPMI 160 + 15% FBS + 1× HAT) and seeded into 96-well cell culture plates at 200 million cells per well. The cells were cultured at 37°C with 5% CO₂ for 7 days before screening. A total of 2 rounds of fusion were performed, 40 plates in the first round and 81 plates in the second round.

Mother clone screening: ELISA preliminary screening was performed through human hDLL3-ECD-his antigen protein. The experimental method was as described above. Positive hybridoma clones were selected after the preliminary screening. ELISA re-screening was carried out by coating the cynomolgus monkey DLL3-ECD-his antigen protein, the cell ELISA method domain was used for re-screening and classification, and DLL3-positive tumor cells SHP77 were used for cell FACS re-screening. The cell ELISA screening method was as follows: The day before the experiment, 293T-hDLL3 D2, 293T-hDLL3 D7, or 293T-hDLL3 D4 cells were plated into 96-well plates at 5 × 10⁴ cells per well. On the day of the experiment, the supernatant was removed, and each well was washed once with 300 µL of PBS. 100 µL of 4% paraformaldehyde was added to each well and fixed at room temperature for 20 minutes. After removing the paraformaldehyde, each well was washed twice with 300 µL of PBS, added with 100 µL of 2% BSA (in PBS), and blocked at 37°C for 2 hours. After blocking, 30 µL of hybridoma supernatant was added to each well and incubated at 37°C for 2 hours. The liquid was then removed, and each well was washed three times with 300 µL of PBST. 100 µL of HRP-labeled anti-mouse secondary antibody in 2% BSA was added to each well and incubated at 37°C for 1 hour. The secondary antibody was removed, and each well was washed five times with 300 µL of PBST. Subsequently, 100 µL of TMB substrate was added to each well and the color development was carried out for 5 to 20 minutes. The reaction was stopped by adding 50 µL of 2N HCl to each well, and the OD450 nm values were measured using a microplate reader. The tumor cell FACS screening method was as follows: SHP77 cells were digested with trypsin and collected by centrifugation. The cells were washed three times with pre-cooled PBS and resuspended in 1% BSA (in PBS). The cells were plated at 2 × 10⁵ cells (50 µL) per well in a 96-well V-bottom plate. 50 µL of positive hybridoma supernatant was added to each well and incubated at 4°C for 1 hour. After washing three times with pre-cooled PBS, 100 µL of 1% BSA containing 1 µL of anti-mouse PE fluorescent secondary antibody (BioLegend, Catalog No.: 405307) was added to each well and incubated at 4°C for 0.5 hours. The cells were then washed three times with pre-cooled PBS, resuspended, and analyzed using a flow cytometer (Sony, LE-SA3800GA).

Using the above methods, 40 mother clones were selected from the first round of fusion for subcloning, and 23 mother clones were selected from the second round of fusion for subcloning.

Subclone screening: ELISA screening was performed by coating human, cynomolgus monkey, and domains, and cell FACS screening was performed by SHP77. The experimental methods were as described above. From each plate, 1 to 3 positive clones were selected, totaling 58 positive clones, which were then expanded in serum-free medium.

### 3.4 Evaluation and sequence retrieval of murine antibodies

Hybridoma cells were cultured in serum-free medium, and the collected monoclonal supernatants were purified using ProA resin to obtain murine antibodies. Murine antibodies were evaluated sequentially for affinity using ELISA by coating human DLL3-ECD-his and cynomolgus monkey DLL3-ECD-his proteins, FACS affinity evaluation with SHP77 tumor cells, ELISA classification evaluation with different truncated forms of 293T-hDLL3 cells, and FACS negative screening evaluation with 293T-hDLL1 and 293T-hDLL4 cells, following the methods described above. The murine antibodies were tested in ELISA starting at a concentration of 2 µg/mL with 3-fold dilutions for a total of 11 concentration points. In the cell ELISA, the murine antibodies were tested at a single concentration of 10 µg/mL, while in the tumor cell FACS, the murine antibodies were tested at 30 µg/mL with 3-fold dilutions for a total of 8 concentration points. Murine antibody 293T-hDLL1 and 293T-hDLL4 cells were tested using FACS negative screening at a single concentration of 20 µg/mL. A total of 58 murine antibodies were evaluated, and five monoclonal antibodies with high affinity and low positive rates for DLL4 and DLL1 were selected for sequence retrieval.

| Monoclonal number | hDLL3-ECD-his EC₅₀ (ng/mL) | cynoDLL3-ECD-his EC₅₀ (ng/mL) | 293T-DLL4 positive rate | 293T-DLL1 positive rate |
|---|---|---|---|---|
| 10F2F3 | 1.498 | 1.145 | 1.24% | 5.61% |
| 87F7F10 | 1.679 | 1.311 | 0.42% | 1.84% |
| 55C11E4 | 56.74 | 40.91 | 0.10% | 0.92% |
| 59B10D3 | 1.301 | 1.249 | 0.67% | 6.30% |
| 6F11C10 | 7.59 | 19.51 | 0.76% | 0.64% |

Murine sequence retrieval: Approximately 1 × 10⁵ candidate hybridoma cells were collected, and RNA was extracted using Trizol. cDNA was obtained through reverse transcription using the PrimeScript RT Reagent Kit with PolyA. Upstream primers were designed for the upstream region of the heavy chain and light chain, while downstream primers were designed for the CH1 region of the heavy chain and the CL region of the light chain. The products were amplified by PCR, and the fragments were recovered by an agarose gel recovery kit; the samples were sent to Beijing Tsingke Biotech Co., Ltd. for sequencing. The five clone numbers were 10F2F3, 87F7F10, 55C11E4, 59B10D3, and 6F11C10, and their murine antibodies 10F2F3, 87F7F10, 55C11E4, 59B10D3, and 6F11C10 were sequenced, respectively. The specific sequences of the variable regions and CDRs of the murine antibodies are provided in the sequence information table.

### 3.5 Antibody humanization

Using the CDR grafting method, the highest homologous human germline sequence to the original murine sequence was first identified through the conventional BLAST method as a template. The CDRs of the murine antibody were then transplanted onto the humanized template to construct a chimeric antibody. Based on structural analysis, the FR amino acids of the murine antibody that could retain their original conformation were identified, and the corresponding amino acids in the chimeric antibody were reverted to murine amino acids to maintain the original affinity. The constructed humanized antibody was subjected to computational and immunogenicity analysis to identify high-immunogenicity fragments, which were then replaced with low-immunogenicity fragments.

The murine antibody 10F2F3 was humanized, resulting in the humanized heavy chain variable region 10F2F3-58hzvh and the humanized light chain variable region 10F2F3-58hzvl. The murine antibody 55C11E4 was humanized, resulting in the humanized heavy chain variable regions 55C11E4-67hzvh and 55C11E4-67hzvh2 and humanized light chain variable region 55C11E4-67hzvl. The murine antibody 59B10D3 was humanized, resulting in the humanized heavy chain variable region 59B10D3-69hzvh and the humanized light chain variable region 59B10D3-69hzvl. The murine antibody 87F7F10 was humanized, resulting in the humanized heavy chain variable region 87F7F10hzvh and the humanized light chain variable region 87F7F10hzvl. The murine antibody 6F11C10 was humanized, resulting in the humanized heavy chain variable regions 6F11C10-84hzvh and 6F11C10-84hz2vh and humanized light chain variable region 6F11C10-84hzvl. The specific sequences of the variable regions and CDRs of each humanized antibody can be found in the sequence information table, where the CDRs are provided as defined by IMGT and Kabat.

### 3.6 Expression of anti-DLL3 humanized antibodies

The heavy chain variable region of each humanized antibody was linked to the heavy chain IgG1 constant region (SEQ NO: 82), and the light chain variable region of each humanized antibody was linked to the Kappa constant region (SEQ NO: 83). The C2 antibody (with heavy and light chain variable region sequences derived from KEGG, ID: D11117) was linked to the heavy chain IgG1 constant region (SEQ NO: 82), and the light chain variable region of each humanized antibody was linked to the Kappa constant region (SEQ NO: 83). The resulting amino acid sequences from the above linkage were submitted to General Biol for gene synthesis, followed by codon optimization and construction into the PTT5 vector. After successful plasmid synthesis, HEK293E cells were transfected using PEImax and were expressed for about 7 days. The supernatant was collected by centrifugation. The supernatant was purified using ProA resin. The purified antibodies were all ultrafiltered into PBS buffer, and the concentration was determined, and stored at - 20°C.

The humanized heavy chain variable region 55C11E4-67hzvh and the humanized light chain variable region 55C11E4-67hzvl were respectively linked to the above heavy chain IgG1 constant region and Kappa constant region. The expressed antibody was named 55C11E4-Hz1. The humanized heavy chain variable region 55C11E4-67hzvh2 and the humanized light chain variable region 55C11E4-67hzvl were respectively linked to the above heavy chain IgG1 constant region and Kappa constant region. The expressed antibody was named 55C11E4-Hz2. The humanized heavy chain variable region 59B10D3-69hzvh and the humanized light chain variable region 59B10D3-69hzvl were respectively linked to the above heavy chain IgG1 constant region and Kappa constant region. The expressed antibody was named 59B10D3-Hz antibody. The humanized heavy chain variable region 10F2F3-58hzvh and the humanized light chain variable region 10F2F3-58hzvl were respectively linked to the above heavy chain IgG1 constant region and Kappa constant region. The expressed antibody was named 10F2F3-Hz antibody. The humanized heavy chain variable region 87F7F10hzvh and the humanized light chain variable region 87F7F10hzvl were respectively linked to the above heavy chain IgG1 constant region and Kappa constant region. The expressed antibody was named 87F7F10-Hz. The humanized heavy chain variable region 6F11C10-84hzvh and the humanized light chain variable region 6F11C10-84hzvl were respectively linked to the above heavy chain IgG1 constant region and Kappa constant region. The expressed antibody was named 6F11C10-Hz1 antibody. The humanized heavy chain variable region 6F11C10-84hz2vhG and the humanized light chain variable region 6F11C10-84hzvl were respectively linked to the above heavy chain IgG1 constant region and Kappa constant region. The expressed antibody was named 6F11C10-Hz2 antibody. The constant region and heavy and light chain sequences of each humanized antibody are shown in the sequence information table.

### Example 4: Identification of anti-DLL3 antibody

### 4.1 ELISA affinity evaluation of humanized antibodies

The antigen proteins h.DLL3-ECD-his and cyno.DLL3-ECD-his were coated with CBS at a concentration of 0.2 µg/mL. The plates were blocked with 2% BSA (in PBS) at 37°C for 2 hours, followed by the addition of gradient-diluted antibodies (starting at 2 µg/mL, 3-fold dilutions, with a total of 11 concentration points) and incubated at 37°C for 2 hours. HRP-labeled anti-human secondary antibody (Jackson, 109-035-088) was then added and incubated at 37°C for 1 hour. TMB substrate was added for color development, and the reaction was terminated with 2M HCl before reading the absorbance at 450 nM.

The experimental results are shown in Table 1. The 5 humanized antibodies all have high affinity to human and cynomolgus monkey DLL3 proteins.

**Table 1. ELISA affinity evaluation of humanized antibodies**

| No. | Antibody name | hDLL3 EC₅₀ (ng/mL) | cynoDLL3 EC₅₀ (ng/mL) |
|---|---|---|---|
| 1 | 10F2F3-Hz | 3.4 | 3.5 |
| 2 | 55C11E4-Hz1 | 3.9 | 4.1 |
| 3 | 59B10D3-Hz | 5.2 | 6.3 |
| 4 | 87F7F10-Hz | 3.8 | 3.3 |
| 5 | 6F11C10-Hz1 | 4.7 | 5.4 |

### 4.2 Flow cytometry affinity evaluation of humanized antibodies

SHP77 cells were digested with trypsin and collected by centrifugation. The cells were washed three times with pre-cooled PBS and resuspended in 1% BSA (in PBS). The cells were plated at 2 × 10⁵ cells (50 µL) per well in a 96-well V-bottom plate. The DLL3 humanized antibody was gradient diluted with 1% BSA, starting at a concentration of 60 µg/mL, with 3-fold dilution gradient for a total of 8 dilution points. The diluted antibody (50 µL) was mixed homogeneously with the cells in the 96-well V-bottom plate and incubated at 4°C for 1 hour. After washing three times with pre-cooled PBS, 100 µL of 1% BSA containing 1 µL of anti-human PE fluorescent secondary antibody (BioLegend, Catalog No.: 410708) was added to each well and incubated at 4°C for 0.5 hours. The cells were then washed three times with pre-cooled PBS, resuspended, and analyzed using a flow cytometer (Beckman, cytoflex).

The experimental results show that all 5 humanized antibodies have high affinity for SHP77 cells, with 10F2F3-Hz and 6F11C10-Hz1 having higher maximum signal values than 55C11E4-Hz1, 59B10D3-Hz, 87F7F10-Hz, and antibody C2.

### 4.3 Dynamic affinity evaluation of humanized antibodies

The dynamic affinity of the humanized antibodies was determined using ForteBio. The experimental steps were as follows: 1. Sensor preparation: The ProA sensors were taken out and pre-wet in PBST diluent (pH 7.4) for 10 minutes. 2. Sample dilution: The antibodies to be immobilized were diluted to 5 µg/mL, while the antigens hDLL3-ECD-His and cynoDLL3-ECD-His were prepared starting from 500 nM, with 2-fold dilutions for a total of 5 concentration points, along with a 0 concentration point. 3. Program setup: The sensor and sample plates were put in, and the program was initiated, with sensors regenerated using a 20 mM glycine solution (pH 1.7). 4. Data analysis: The data were analyzed using Octet analysis software. The experimental results show that 10F2F3-Hz, 55C11E4-Hz1, 59B10D3-Hz, 87F7F10-Hz, and 6F11C10-Hz1 have high affinities for both human and monkey DLL3 proteins, as shown in Table 2.

**Table 2. Dynamic affinity detection of humanized antibodies**

| Antibody name | hDLL3-ECD-His | | | cynoDLL3-ECD-His | | |
|---|---|---|---|---|---|---|
| | KD (M) | ka (1/Ms) | kdis (1/s) | KD (M) | ka (1/Ms) | kdis (1/s) |
| 10F2F3-Hz | 6.07E-09 | 7.42E+04 | 4.50E-04 | 1.28E-09 | 9.51E+04 | 1.22E-04 |
| 55C11E4-Hz1 | 5.26E-09 | 1.24E+05 | 6.52E-04 | 2.37E-09 | 2.02E+05 | 4.79E-04 |
| 59B10D3-Hz | 1.40E-08 | 1.22E+05 | 1.70E-03 | 9.08E-09 | 1.00E+05 | 9.08E-04 |
| 87F7F10-Hz | 8.57E-09 | 2.54E+04 | 2.18E-04 | 2.41E-09 | 3.67E+04 | 8.84E-05 |
| 6F11C10-Hz1 | 2.43E-10 | 1.75E+05 | 4.27E-05 | 8.73E-10 | 3.75E+05 | 3.28E-04 |
| C2 | 7.41E-09 | 1.63E+05 | 1.21E-03 | 2.79E-09 | 1.45E+05 | 4.05E-04 |

### 4.4 Evaluation of non-specificity of humanized antibodies

HEK293T-hDLL1 and HEK293T-hDLL4 cells were digested with trypsin and collected by centrifugation. The cells were washed three times with pre-cooled PBS and resuspended in 1% BSA (in PBS). The cells were plated at 2 × 10⁵ cells per well in a 96-well V-bottom plate. 20 µg/mL of the antibody to be detected was added to each well and incubated at 4°C for 1 hour. After washing three times with pre-cooled PBS, 1 µL of anti-human APC fluorescent secondary antibody was added to each well and incubated at 4°C for 0.5 hours. The cells were then washed three times with pre-cooled PBS, resuspended, and analyzed using a flow cytometer.

The experimental results show that 10F2F3-Hz, 55C11E4-Hz1, 59B10D3-Hz, 87F7F10-Hz, and 6F11C10-Hz1 do not recognize human DLL1 and DLL4, indicating that the 5 humanized DLL3 antibodies have good specificity.

### 4.5 Evaluation of endocytic activity of humanized antibodies

The SHP77 cells were digested with trypsin and collected by centrifugation, and incubated with the antibody to be tested at a concentration of 20 µg/mL at 4°C for 1 hour. After washing three times with PBS, the cells were resuspended in RPMI 1640 + 10% FBS that had been pre-warmed to 37°C, and divided into three portions for incubation at 37°C for 0, 2, and 4 hours, respectively. After washing three times with pre-cooled PBS, 1.2 µL of anti-human APC fluorescent secondary antibody was added, and the cells were incubated at 4°C for 0.5 hours. After washing three times with PBS, the cells were resuspended for analysis. The endocytosis rate was calculated using the following formula: endocytosis ratio (%) = [1 - (average fluorescence value of the test sample at this time point - average fluorescence value of the negative control sample at this time point) / (average fluorescence value of the test sample at 0 hours - average fluorescence value of the negative control sample at 0 hours)] * 100.

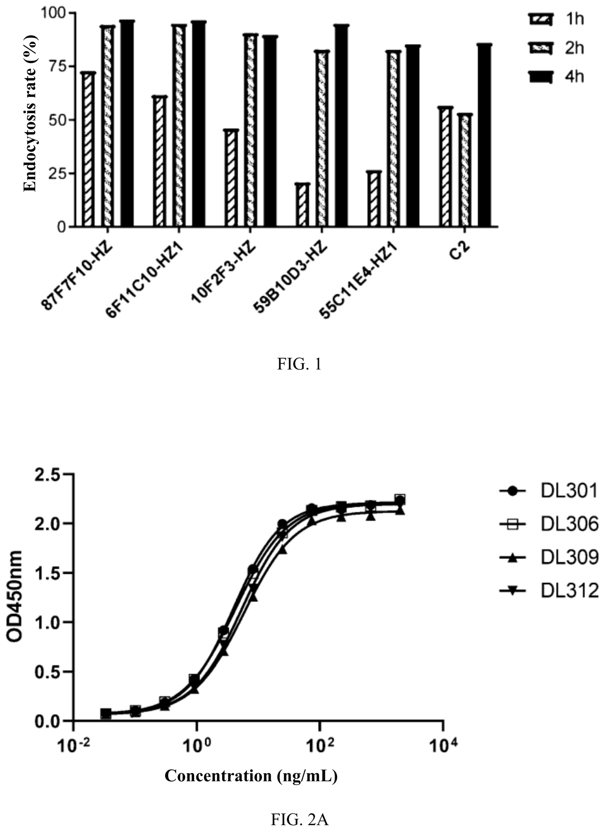

The experimental results are shown in FIG. 1. 10F2F3-Hz, 55C11E4-Hz1, 59B10D3-Hz, 87F7F10-Hz, and 6F11C10-Hz1 have strong endocytic activity and can reach an endocytosis rate of 80% or more in 4 hours, in which the endocytic activity of 87F7F10-Hz and 6F11C10-Hz1 antibodies is significantly stronger than that of C2 antibody.

### 4.6 Optimizing humanized antibody sequences

### 4.6.1 Optimizing ELISA affinity evaluation of humanized antibody proteins

### The antigen proteins h.DLL3-ECD-his, cyno.DLL3-ECD-his, Rat DLL3-his, Mouse DLL3-His, and Canis DLL3-his were coated with CBS at a concentration of 0.2 µg/mL. The plates were blocked with 2% BSA (in PBS) at 37°C for 2 hours, followed by the addition of gradient-diluted antibodies (starting at 2 µg/mL, 3-fold dilutions, with a total of 11 concentration points) and incubated at 37°C for 2 hours. HRP-labeled anti-human secondary antibody (Jackson, 109-035-088) was then added and incubated at 37°C for 1 hour. TMB substrate was added for color development, and the reaction was terminated with 2M HCl before reading the absorbance at 450 nM.

The experimental results are shown in Table 3. The two optimized humanized antibodies have high affinity to human, cynomolgus monkey DLL3, and rat DLL3 proteins, and have similar binding affinities to human, cynomolgus monkey DLL3, and rat DLL3. The binding affinity of the two optimized humanized antibodies to mouse DLL3 was reduced to a certain extent.

**Table 3. ELISA affinity evaluation of humanized antibodies**

| No. | Antibody name | hDLL3 EC₅₀ (ng/mL) | cynoDLL3 EC₅₀ (ng/mL) | RatDLL3 EC₅₀ (ng/mL) | mDLL3 EC₅₀ (ng/mL) | CanisDLL3 EC₅₀ (ng/mL) |
|---|---|---|---|---|---|---|
| 1 | 55C11E4-Hz2 | 4.952 | 4.982 | 3.67 | 21.32 | 12.95 |
| 2 | 6F11C10-Hz2 | 8.227 | 10.77 | 7.348 | 30.09 | 17.17 |

### 4.6.2 Optimizing flow cytometry affinity evaluation of humanized antibodies

SHP77 cells were digested with trypsin and collected by centrifugation. The cells were washed three times with pre-cooled PBS and resuspended in 1% BSA (in PBS). The cells were plated at 2 × 10⁵ cells (50 µL) per well in a 96-well V-bottom plate. The DLL3 optimized humanized antibody was gradient diluted with 1% BSA, starting at a concentration of 40 µg/mL, with 3-fold dilution gradient for a total of 12 dilution points. The diluted antibody (50 µL) was mixed homogeneously with the cells in the 96-well V-bottom plate and incubated at 4°C for 1 hour. After washing three times with pre-cooled PBS, 100 µL of 1% BSA containing 1 µL of anti-human APC fluorescent secondary antibody (BioLegend, Catalog No.: 410708) was added to each well and incubated at 4°C for 0.5 hours. The cells were then washed three times with pre-cooled PBS, resuspended, and analyzed using a flow cytometer (Beckman, cytoflex).

The experimental results show that the maximum signal values of the two optimized humanized antibodies 55C11E4-Hz2 and 6F11C10-Hz2 binding to SHP77 cells were higher than those of antibody C2.

### 4.6.3 Determination of Tm value of humanized antibodies

The Tm values of the humanized antibodies were determined using Differential Scanning Fluorimetry (DSF) to reflect the thermal stability of the antibodies. The experimental steps were as follows: The antibody samples to be tested were diluted to 1 mg/mL in PBS; the SYPRO Orange dye (Thermo #56651) was diluted to 40X with ddH₂O; the reaction system: 12.5 µL of sample, 2.5 µL of 40X dye, and 5 µL of ddH₂O; the mixture was sealed and briefly centrifuged; detection was performed using Q-PCR, with the following Q-PCR parameters: Target (ROX), program (25°C for 3 min; 1% rate, 95°C; 95°C, 2 min).

After analyzing the experimental data, the Tm values of the antibodies 6F11C10-Hz2 and 55C11E4-Hz2 were found to be 67.5°C and 68.6°C, respectively, indicating that they have good thermal stability.

### 4.7 Testing of humanized antibody binding epitopes

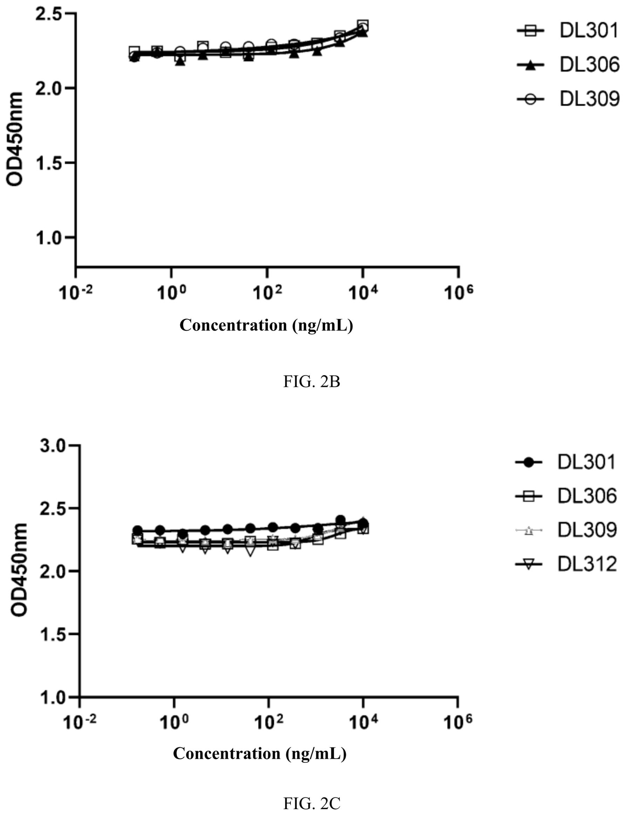

The ELISA method was used to analyze and optimize whether there was epitope competition between the humanized antibody and the four antibodies disclosed in Chugai's patent (Patent No: US9127071B2) (sequence numbers: DL301, DL306, DL309, and DL312). The night before the experiment, human DLL3 antigen was coated with CBS coating solution at a coating concentration of 0.2 µg/mL in a volume of 100 uL, and incubated at 4°C overnight. The next day, the coating solution was removed, and each well was washed once with 300 µL of PBS. Each well was then added with 100 µL of 2% BSA (Biotopped, A6020) (in PBS), and blocked at 37°C for 2 hours. Chugai's four antibodies were diluted with 6F11C10-Hz2-biotin (100 ng/mL in 2% BSA) and 55C11E4-Hz2-biotin (100 ng/mL in 2% BSA) starting from a concentration of 10 µg/mL and diluted 3-fold for a total of 11 dilution points. After dilution, 100 µL was added to the plate and incubated at room temperature for 2 hours. The supernatant was then removed, and each well was washed three times with 300 µL of PBST. 100 µL of SA-HRP secondary antibody (in 2% BSA) was added to each well and incubated at room temperature for 1 hour. The secondary antibody was removed, and each well was washed five times with 300 µL of PBST. Then, 100 µL of TMB substrate (Huzhou InnoReagents, TMB-S-004) was added to each well and the color development was carried out for 2 to 3 minutes. The reaction was stopped by adding 50 µL of 2N HCl to each well, and the OD450 nM readings were measured using a microplate reader (manufacturer: MD, model: SpectraMax). The data were imported into GraphPad software for fitting FIG. 2.

The results are shown in FIG. 2A, indicating that DL301, DL306, DL309, and DL312 all have high affinity for hDLL3. FIG. 2B shows that the DL301, DL306, and DL309 antibodies do not compete with 55C11E4-hz2 for binding to hDLL3. FIG. 2C shows that 6F11C10-Hz2 does not compete with the DL301, DL306, DL309, and DL312 antibodies for binding to hDLL3. These data suggest that 6F11C10-Hz2 and 55C11E4-hz2 bind to a novel epitope on hDLL3.

### IV. Conjugation of compounds containing cellular bioactive molecules and linkers to antibodies

### Example 5: Preparation of DLL3-ADC

### Example 5.1: Preparation of DLL3-ADC-07

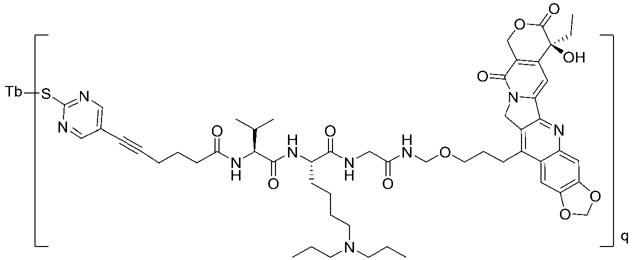

Herein, Tb is DLL3 antibody (including the following 6F11C10-hz2, 6F11C10-hz1, 55C11E4-hz1, 55C11E4-hz2, 59B10D3-hz1, 87F7F10-hz1, C2, *etc*.), q is 1, 2, 3, 4, 5, 6, 7, 8, 9 or 10.

### 5.1.1 Sample preparation of DLL3-ADC-07 (DAR8)

### 5.1.1.1 Sample preparation of 6F11C10-hz2-ADC07 (DAR8)

A total of 20 mg of anti-DLL3 antibody 6F11C10-hz2 was diluted with a diluent (20 mM PB, pH 7.5) and mixed homogeneously with a solution of sodium edetate at a final concentration of 1 mM. TCEP solution of 6.0-fold equivalents of the antibody was added, and the mixture was mixed homogeneously and left at room temperature for 60 minutes. DL-037, dissolved in dimethyl sulfoxide, was added to the above solution system at a 15-fold equivalent to the antibody, mixed homogeneously, and left at room temperature for 1 hour to obtain the conjugated sample. After the reaction was complete, the sample was exchanged into a 20 mM histidine buffer at pH 6.0 using a 30 kDa ultrafiltration tube to remove low molecular weight substances. Finally, the sample was concentrated to obtain a solution containing the anti-DLL3 antibody ADC composition 6F11C10-hz2-ADC07 (DAR8). The DAR value was determined to be 8.0 using or referencing the mass spectrometry method described in Example 5.2.

### 5.1.1.2 Sample preparation of 55C11E4-hz1-ADC07 (DAR8)

A total of 20 mg of anti-DLL3 antibody 55C11E4-hz1 was diluted with a diluent (20 mM PB, pH 7.5) and mixed homogeneously with a solution of sodium edetate at a final concentration of 1 mM. TCEP solution of 6.0-fold equivalents of the antibody was added, and the mixture was mixed homogeneously and left at room temperature for 60 minutes. DL-037, dissolved in dimethyl sulfoxide, was added to the above solution system at a 15-fold equivalent to the antibody, mixed homogeneously, and left at room temperature for 1 hour to obtain the conjugated sample. After the reaction was complete, the sample was exchanged into a 20 mM histidine buffer at pH 6.0 using a 30 kDa ultrafiltration tube to remove low molecular weight substances. Finally, the sample was concentrated to obtain a solution containing the anti-DLL3 antibody ADC composition 55C11E4-hz1-ADC07 (DAR8). The DAR value was determined to be 7.0 using or referencing the mass spectrometry method described in Example 5.2.

### 5.1.1.3 Sample preparation of 59B10D3-hz1-ADC07 (DAR8)

A total of 20 mg of anti-DLL3 antibody 59B10D3-hz1 was diluted with a diluent (20 mM PB, pH 7.5) and mixed homogeneously with a solution of sodium edetate at a final concentration of 1 mM. TCEP solution of 6.0-fold equivalents of the antibody was added, and the mixture was mixed homogeneously and left at room temperature for 60 minutes. DL-037, dissolved in dimethyl sulfoxide, was added to the above solution system at a 15-fold equivalent to the antibody, mixed homogeneously, and left at room temperature for 1 hour to obtain the conjugated sample. After the reaction was complete, the sample was exchanged into a 20 mM histidine buffer at pH 6.0 using a 30 kDa ultrafiltration tube to remove low molecular weight substances. Finally, the sample was concentrated to obtain a solution containing the anti-DLL3 antibody ADC composition 59B10D3-hz1-ADC07 (DAR8). The DAR value was determined to be 7.3 using or referencing the mass spectrometry method described in Example 5.2.

### 5.1.1.4 Sample preparation of 87F7F10-hz1-ADC07 (DAR8)

A total of 20 mg of anti-DLL3 antibody 87F7F10-hz1 was diluted with a diluent (20 mM PB, pH 7.5) and mixed homogeneously with a solution of sodium edetate at a final concentration of 1 mM. TCEP solution of 6.0-fold equivalents of the antibody was added, and the mixture was mixed homogeneously and left at room temperature for 60 minutes. DL-037, dissolved in dimethyl sulfoxide, was added to the above solution system at a 15-fold equivalent to the antibody, mixed homogeneously, and left at room temperature for 1 hour to obtain the conjugated sample. After the reaction was complete, the sample was exchanged into a 20 mM histidine buffer at pH 6.0 using a 30 kDa ultrafiltration tube to remove low molecular weight substances. Finally, the sample was concentrated to obtain a solution containing the anti-DLL3 antibody ADC composition 87F7F10-hz1-ADC07 (DAR8). The DAR value was determined to be 7.0 using or referencing the mass spectrometry method described in Example 5.2.

### 5.1.1.5 Sample preparation of C2-ADC07 (DAR8)

A total of 20 mg of anti-DLL3 antibody C2 was diluted with a diluent (20 mM PB, pH 7.5) and mixed homogeneously with a solution of sodium edetate at a final concentration of 1 mM. TCEP solution of 6.0-fold equivalents of the antibody was added, and the mixture was mixed homogeneously and left at room temperature for 60 minutes. DL-037, dissolved in dimethyl sulfoxide, was added to the above solution system at a 15-fold equivalent to the antibody, mixed homogeneously, and left at room temperature for 1 hour to obtain the conjugated sample. After the reaction was complete, the sample was exchanged into a 20 mM histidine buffer at pH 6.0 using a 30 kDa ultrafiltration tube to remove low molecular weight substances. Finally, the sample was concentrated to obtain a solution containing the anti-DLL3 antibody ADC composition C2-ADC07 (DAR8). The DAR value was determined to be 8.0 using or referencing the mass spectrometry method described in Example 5.2.

### 5.1.2 Sample preparation of DLL3-ADC-07 (DAR6)

### 5.1.2.1 Sample preparation of 6F11C10-hz1-ADC07 (DAR6)

A total of 20 mg of anti-DLL3 antibody 6F11C10-hz1 was diluted with a diluent (20 mM PB, pH 7.5) and mixed homogeneously with a solution of sodium edetate at a final concentration of 1 mM. TCEP solution of 6.0-fold equivalents of the antibody was added, and the mixture was mixed homogeneously and left at room temperature for 60 minutes. DL-037, dissolved in dimethyl sulfoxide, was added to the above solution system at a 7.5-fold equivalent to the antibody, mixed homogeneously, and left at room temperature for 1 hour to obtain the conjugated sample. After the reaction was complete, the sample was exchanged into a 20 mM histidine buffer at pH 6.0 using a 30 kDa ultrafiltration tube to remove low molecular weight substances. Finally, the sample was concentrated to obtain a solution containing the anti-DLL3 antibody ADC composition 6F11C10-hz1-ADC07 (DAR6). The DAR value was determined to be 6.6 using or referencing the mass spectrometry method described in Example 5.2.

### 5.1.2.2 Sample preparation of 55C11E4-hz2-ADC07 (DAR6)

A total of 20 mg of anti-DLL3 antibody 55C11E4-hz2 was diluted with a diluent (20 mM PB, pH 7.5) and mixed homogeneously with a solution of sodium edetate at a final concentration of 1 mM. TCEP solution of 6.0-fold equivalents of the antibody was added, and the mixture was mixed homogeneously and left at room temperature for 60 minutes. DL-037, dissolved in dimethyl sulfoxide, was added to the above solution system at a 7.5-fold equivalent to the antibody, mixed homogeneously, and left at room temperature for 1 hour to obtain the conjugated sample. After the reaction was complete, the sample was exchanged into a 20 mM histidine buffer at pH 6.0 using a 30 kDa ultrafiltration tube to remove low molecular weight substances. Finally, the sample was concentrated to obtain a solution containing the anti-DLL3 antibody ADC composition 55C11E4-hz2-ADC07 (DAR6). The DAR value was determined to be 6.2 using or referencing the mass spectrometry method described in Example 5.2.

### 5.1.3 Sample preparation of DLL3-ADC-07 (DAR4)

### 5.1.3.1 Sample preparation of 6F11C10-hz2-ADC07 (DAR4)

A total of 20 mg of anti-DLL3 antibody 6F11C10-hz2 was diluted with a diluent (20 mM PB, pH 7.5) and mixed homogeneously with a solution of sodium edetate at a final concentration of 1 mM. TCEP solution of 6.0-fold equivalents of the antibody was added, and the mixture was mixed homogeneously and left at room temperature for 60 minutes. DL-037, dissolved in dimethyl sulfoxide, was added to the above solution system at a 5.5-fold equivalent to the antibody, mixed homogeneously, and left at room temperature for 1 hour to obtain the conjugated sample. After the reaction was complete, the sample was exchanged into a 20 mM histidine buffer at pH 6.0 using a 30 kDa ultrafiltration tube to remove low molecular weight substances. Finally, the sample was concentrated to obtain a solution containing the anti-DLL3 antibody ADC composition 6F11C10-hz2-ADC07 (DAR4). The DAR value was determined to be 4.6 using or referencing the mass spectrometry method described in Example 5.2.

### 5.1.3.2 Sample preparation of 55C11E4-hz2-ADC07 (DAR4)

A total of 20 mg of anti-DLL3 antibody 55C11E4-hz2 was diluted with a diluent (20 mM PB, pH 7.5) and mixed homogeneously with a solution of sodium edetate at a final concentration of 1 mM. TCEP solution of 6.0-fold equivalents of the antibody was added, and the mixture was mixed homogeneously and left at room temperature for 60 minutes. DL-037, dissolved in dimethyl sulfoxide, was added to the above solution system at a 5.5-fold equivalent to the antibody, mixed homogeneously, and left at room temperature for 1 hour to obtain the conjugated sample. After the reaction was complete, the sample was exchanged into a 20 mM histidine buffer at pH 6.0 using a 30 kDa ultrafiltration tube to remove low molecular weight substances. Finally, the sample was concentrated to obtain a solution containing the anti-DLL3 antibody ADC composition 55C11E4-hz2-ADC07 (DAR4). The DAR value was determined to be 3.8 using or referencing the mass spectrometry method described in Example 5.2.

### Example 5.2: Preparation of DLL3-ADC-07 and determination of DAR value

### 5.2.1 DAR determination of 6F11C10-HZ1-ADC07 (DAR6)

Chromatographic conditions: chromatographic column: PLRP-S, 2.1 * 50 mm, 5 µm; mobile phase A: 0.1% FA/H₂O; mobile phase B: 0.1% FA/ACN

Column temperature: 30°C; sample chamber temperature: 8°C; flow rate: 0.6 mL/min; injection volume: 2 µL.

**Table 4. Mobile phase gradients**

| Time (min) | 1 | 5 | 5.1 | 7 | 10 |
|---|---|---|---|---|---|
| Mobile phase A | 90 | 40 | 10 | 90 | 90 |
| Mobile phase B | 10 | 60 | 90 | 10 | 10 |

Sample treatment: 50 µg of each sample was taken and added with 2 µL of 1 M DTT. The mixture was diluted to a concentration of approximately 1.0 mg/mL by adding ultrapure water to 50 µL, mixed homogeneously, and reduced at room temperature for 30 minutes.

LC/MS model: Agilent 1290-6545XT Q-TOF, mass spectrometry conditions: Gas temp: 320°C, Drying Gas: Nebulizer: 35 psi; sheath gas temp: 350°C; sheath gas flow: 11 L/min; m/z 500 to 3000.

The results are as follows:
The test results showed that the light chain of 6F11C10-HZ1-ADC07 (DAR6) sample was conjugated with 0 to 1 payload molecule (LC and DAR1 percentages of 19.8% and 71.2%, respectively), while the heavy chain was conjugated with 0 to 3 payload molecules (mAb, DAR1, DAR2, and DAR3 percentages of 0%, 18.5%, 17.0 %, and 64.5%, respectively), and thus the drug-to-antibody conjugation ratio (DAR value) of the 6F11C10-HZ1-ADC07 (DAR6) sample was calculated to be 6.6. It could be inferred from the different numbers of payload molecules conjugated to the heavy and light chains that q could be 2, 3, 4, 5, 6, 7, or 8.

### 5.2.2 DAR determination of 6F11C10-HZ2-ADC07 (DAR4)

Referring to the method for the DAR determination of the 6F11C10-HZ1-ADC07 (DAR6), it was determined that the light chain of the 6F11C10-HZ2-ADC07 (DAR4) sample was conjugated with 0 to 1 payload molecule (LC and DAR1 percentages of 19.8% and 80.2%, respectively), while the heavy chain was conjugated with 0 to 3 payload molecules (mAb, DAR1, DAR2, and DAR3 percentages of 13.6%, 45.4%, 18.2%, and 22.8%, respectively), and thus the drug-to-antibody conjugation ratio (DAR value) of the 6F11C10-HZ2-ADC07 (DAR4) sample was calculated to be 4.6. It could be inferred from the different numbers of payload molecules conjugated to the heavy and light chains that q could be 1, 2, 3, 4, 5, 6, 7, or 8.

### 5.2.3 DAR determination of 6F11C10-HZ2-ADC07 (DAR8)

Referring to the method for the DAR determination of the 6F11C10-HZ1-ADC07 (DAR6), it was determined that the light chain of the 6F11C10-HZ2-ADC07 (DAR8) sample was conjugated with 0 to 1 payload molecule (LC and DAR1 percentages of 0% and 100.0%, respectively), while the heavy chain was conjugated with 0 to 3 payload molecules (mAb, DAR1, DAR2, and DAR3 percentages of 0%, 0%, 0%, and 100.0%, respectively), and thus the drug-to-antibody conjugation ratio (DAR value) of the 6F11C10-HZ2-ADC07 (DAR8) sample was calculated to be 8.0. It could be inferred from the different numbers of payload molecules conjugated to the heavy and light chains that q is 8.

### 5.2.4 DAR determination of 55C11E4-hzl-ADC07 (DAR8)

Referring to the method for the DAR determination of the 6F11C10-HZ1-ADC07 (DAR6), it was determined that the light chain of the 55C11E4-hz1-ADC07 (DAR8) sample was conjugated with 0 to 1 payload molecule (LC and DAR1 percentages of 11.5% and 89.5%, respectively), while the heavy chain was conjugated with 0 to 3 payload molecules (mAb, DAR1, DAR2, and DAR3 percentages of 0%, 14.5%, 26.9%, and 58.6%, respectively), and thus the drug-to-antibody conjugation ratio (DAR value) of the 55C11E4-hz1-ADC07 (DAR8) sample was calculated to be 7.0. It could be inferred from the different numbers of payload molecules conjugated to the heavy and light chains that q could be 2, 3, 4, 5, 6, 7, or 8.

The DAR of other samples of the present disclosure was determined by reference to the method for DAR determination described above.

### V. In vitro bioactivity assay of bioactive molecules

### Example 6.1: Inhibitory activity assay of bioactive molecules on cell viability in vitro I

First, tumor cells were cultured; the bioactive molecules of the present disclosure were co-cultured with the tumor cells. Subsequently, CCK8 reagent (DOJINDO Chemical Technology Co., Ltd., Cat: CK04, Lot: JJ744) was added, and the absorbance was read using a microplate reader (manufacturer: Molecular Devices, model: SpectraMax M2) at a detection wavelength of 450 nm to detect the activity of dehydrogenases within the mitochondria, evaluating the inhibitory effect of the bioactive molecules on cell proliferation. The sources of tumor cells are shown in Table 5.

**Table 5.**

| Cell name | Tumor type | Source |
|---|---|---|
| NCl-H358 | Human non-small cell lung cancer | ATCC, CRL-5807 |
| HT29 | Human colon cancer cell | ATCC, HTB-38 |
| LS174T | Human colon adenocarcinoma cell | ATCC, CL-187 |
| NCl-H322M | Human non-small cell lung cancer | ATCC |
| PANC-1 | Human pancreatic cancer tumor cell | ATCC, CRL-1469 |
| HCC1806 | Breast cancer | Nanjing Cobioer Biotechnology |

*In vitro* cell viability assay method: The bioactive molecules were diluted (12 concentration gradients) in the corresponding assay medium (containing 2% FBS). Tumor cells were digested using trypsin by conventional methods, and the number of cells in a tube was collected and resuspended in the corresponding assay medium (containing 2% FBS). The diluted bioactive molecules were added to a 96-well plate, followed by the addition of cells. Then, 20 µL of CCK8 reagent was added to each well and reacted for 4 hours. The absorbance was read using a microplate reader at a detection wavelength at 450 nm. The experimental results are shown in Tables 6.1, 6.2, 6.3, 6.4, 6.5, and 6.6.

DXD was prepared with reference to CN104755494B. The specific structure is as follows:

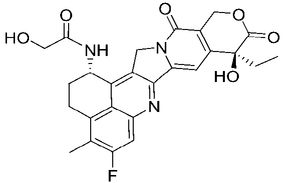

**Table 6.1 Killing results of NCl-H358 cells by bioactive molecules**

| Name | Cell name | EC₅₀ (nM) |
|---|---|---|
| A1.10 | NCl-H358 | 8.11 |
| DXD | | 39.02 |
| | | |

| Name | Cell name | EC₅₀ (nM) |
|---|---|---|
| A1.8D | | 7.919 |
| A1.15a | NCl-H358 | 3.882 |
| DXD | | 21.43 |
| | | |

| Name | Cell name | EC₅₀ (nM) |
|---|---|---|
| A1.4 | NCl-H358 | 29.91 |
| A1.8 | | 296.4 |
| A1.9 | | 60.79 |
| A1.11 | | 202.9 |
| A1.12 | | 167.1 |
| A1.13 | | 49.19 |
| A1.14 | | 122.6 |
| DXD | | 148.45 ± 10.87 (n = 4) |

**Table 6.2 Killing results of HT29 cells by bioactive molecules**

| Name | Cell name | EC₅₀ (nM) |
|---|---|---|
| A1.10 | HT29 | 5.965 |
| DXD | | 49.47 |
| | | |

| Name | Cell name | EC₅₀ (nM) |
|---|---|---|
| A1.15a | | 3.574 |
| A1.15b | HT29 | 47.52 |
| DXD | | 64.84 |
| | | |

| Name | Cell name | EC₅₀ (nM) |
|---|---|---|
| A1.4 | HT29 | 29.45 |
| A1.8 | | 367.1 |
| A1.9 | | 57.5 |
| A1.11 | | 284.5 |
| A1.12 | | 211.8 |
| A1.13 | | 96.85 |
| A1.14 | | 155.9 |
| DXD | | 301.875 ± 69.00 (n = 4) |

**Table 6.3 Killing results of LS174T cells by bioactive molecules**

| Name | Cell name | EC₅₀ (nM) |
|---|---|---|
| A1.15a | | 19.49 |
| A1.15b | LS174T | 44.62 |
| DXD | | 82.05 |

**Table 6.4 Killing results of NCl-H322M cells by bioactive molecules**

| Name | Cell name | EC₅₀ (nM) |
|---|---|---|
| A1.9 | NCl-H322M | 30.45 |
| DXD | | 76.89 |

**Table 6.5 Killing results of PANC-1 cells by bioactive molecules**

| Name | Cell name | EC₅₀ (nM) |
|---|---|---|
| A1.9 | PANC-1 | 94.54 |
| DXD | | 966 |

**Table 6.6 Killing results of HCC1806 cells by bioactive molecules**

| Name | Cell name | EC₅₀ (nM) |
|---|---|---|
| A1.9 | HCC1806 (7500 cells/well, 3 days) | 1.9 |
| Dxd | | 37 |

The test results show that the bioactive molecules of the present disclosure have a killing effect on tumor cells.

### Example 6.2: Inhibitory activity assay of bioactive molecules on cell viability in vitro II

First, tumor cells NUCG-4, PC-9, HT29, NCI-H358, KYSE520, A431, and A549 were cultured; the bioactive molecules or fragments of the present disclosure were co-cultured with the tumor cells. Subsequently, CCK8 reagent (DOJINDO Chemical Technology Co., Ltd., Cat: CK04, Lot: JJ744) was added, and the absorbance was read using a microplate reader (manufacturer: Molecular Devices, model: SpectraMax M2) at a detection wavelength of 450 nm to detect the activity of dehydrogenases within the mitochondria, evaluating the inhibitory effect of the bioactive molecules on cell proliferation. The sources of tumor cells are shown in Table 7.

**Table 7. Source of tumor cells**

| Cell name | Tumor type | Source |
|---|---|---|
| NUCG-4 | Stomach cancer | Nanjing Cobioer |
| PC-9 | Lung adenocarcinoma | ATCC |
| HT29 | Colon cancer | ATCC, HTB-38 |
| NCI-H358 | Non-small cell lung cancer | ATCC, CRL-5807 |
| KYSE520 | Esophageal cancer | ATCC |
| A431 | Human skin squamous carcinoma (human epidermal carcinoma) cell | ATCC |
| A549 | Non-small cell lung cancer | ATCC |

*In vitro* cell viability assay method: Tumor cells were digested using trypsin by conventional methods, and the number of cells in a tube was collected and resuspended in the corresponding assay medium (containing 2% FBS). 5000 to 10000 cells/well were added to a 96-well plate. 100 µL of the bioactive molecules diluted with DMSO was added to the 96-well plate, starting from a concentration of 10 µM and performing a 3-fold dilution (12 concentration gradients). The plate was incubated at 37°C under 5% CO₂ for 3 to 4 days. 20 µL of CCK8 reagent was then added to each well, and the reaction was carried out for 2 to 6 hours. The absorbance was read on a microplate reader (detection wavelength: 450 nm). The bioactive molecules and their assay results are shown in Table 8.

**Table 8 Activity determination of A1.9 and B1.14**

| Cell | A1.9 EC₅₀ nM | B1.14 EC₅₀ nM | DXD EC₅₀ nM |
|---|---|---|---|
| NUCG-4 | 8.73 | 184.8 | 43.32 |
| PC-9 | 2.06 | 34.32 | 16.69 |
| HT29 | 20.32 | 367.9 | 210.5 |
| NCI-H358 | 46.2 | 581.2 | 261.6 |
| A431 | 5.71 | 67.97 | 25.08 |
| A549 | 65.86 | 252.3 | 262.2 |

The test results show that bioactive molecules A1.9 and B1.14 have tumor cell killing effects, among which A1.9 has a stronger tumor killing effect than Dxd in multiple tumor cells. A1.9 has an EC₅₀ of 49.62 nM in an inhibitory activity assay for the *in vitro* cell viability assay for KYSE520, which has the ability to kill KYSE520.

### Example 7: In vivo activity test of anti-DLL3 antibody-drug conjugates

### 7.1 Efficacy test of DLL3 antibody-drug conjugates on SHP77 human small cell lung cancer xenografts

To verify the *in vivo* efficacy of the anti-human DLL3 ADC drug, the anti-tumor effect of the test drug was evaluated in a subcutaneous xenograft model using female Balb/c Nude mice. Female Balb/c Nude mice (5 to 6 weeks old) were purchased from Vital River Laboratories. Human small cell lung cancer SHP77 cells were grown to the logarithmic phase, digested with EDTA, and resuspended in PBS. Each mouse was subcutaneously inoculated with 5 × 10⁶ cells. When the tumors reached a size of 100 to 200 m³, intravenous administration of the test drug was performed once a week at a dose of 1 mg/kg or 3 mg/kg, as detailed in the table below. The main observational indexes of this experiment were as follows: 1) TGI (%), calculated using the formula: TGI (%) = (1 - T/C) × 100% (T and C are the relative tumor volume at a specific time point in the treatment group and control group, respectively); 2) Photographs of tumor size and tumor weight at the end of the experiment.

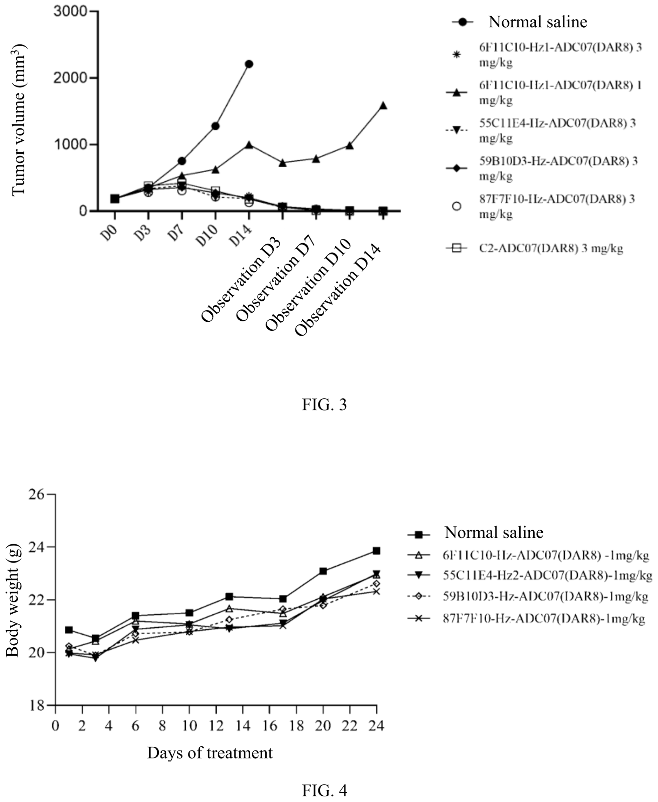

The experimental results are shown in Table 9 and FIG. 3. 55C11E4-Hz1, 59B10D3-Hz, 87F7F10-Hz, and 6F11C10-Hz1 all have good tumor inhibitory effects in mice after conjugation with drugs. The changes in the body weight of mice are shown in FIG. 4, indicating that ADC has no effect on the body weight of mice throughout the administration process.

**Table 9. In vivo efficacy of anti-human DLL3 ADC in SHP77 CDX model**

| Group | Number of mice | Sample | Dose (mg/kg) | Route of administration | Frequency of administration | Day 14 TGI (%) | P-value |
|---|---|---|---|---|---|---|---|
| 1 | 5 | Normal saline | / | i.v. | QW * 3 | / | / |
| 2 | 5 | C2-ADC07(DAR8) | 3 | i.v. | QW * 3 | 91.74 | <0.001 |
| 3 | 5 | 55C11E4-Hz1-ADC07(DAR8) | 3 | i.v. | QW * 3 | 91.23 | <0.001 |
| 4 | 5 | 59B10D3-Hz-ADC07(DAR8) | 3 | i.v. | QW * 3 | 90.74 | <0.001 |
| 5 | 5 | 87F7F10-Hz-ADC07(DAR8) | 3 | i.v. | QW * 3 | 94.12 | <0.001 |
| 6 | 5 | 6F11C10-Hz1-ADC07(DAR8) | 1 | i.v. | QW * 3 | 53.49 | 0.016 |
| 7 | 5 | 6F11C10-Hz1-ADC07(DAR8) | 3 | i.v. | QW * 3 | 89.42 | <0.001 |

### 7.2 Efficacy test of DLL3 antibody-drug conjugates on NCI-H69 human small cell lung cancer xenografts

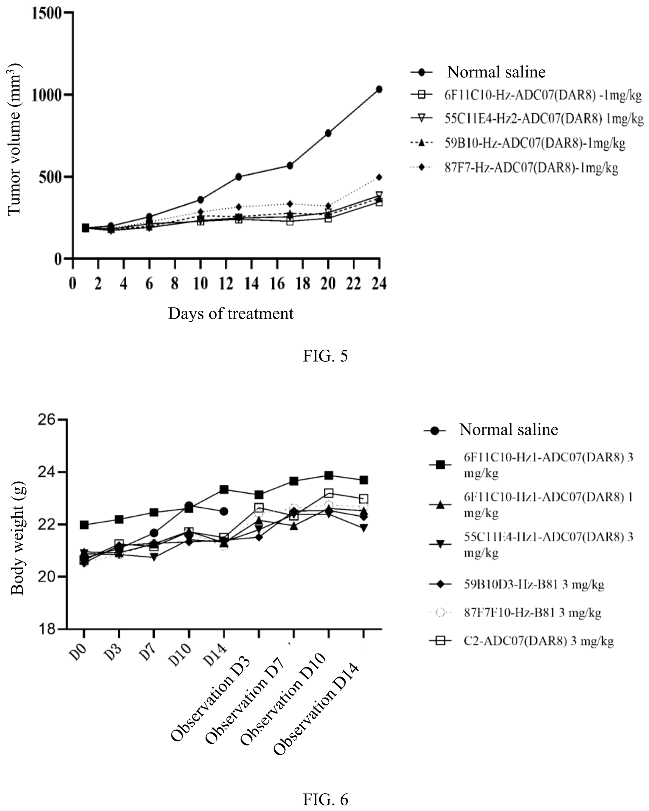

The anti-tumor effect of the DLL3 ADC candidate drug in a subcutaneous xenograft model using female BALB/c nude mice implanted with the human small cell lung cancer NCI-H69 cell line was evaluated using a similar method. The experimental results are shown in Table 10 and FIG. 5. 55C11E4-Hz2, 59B10D3-Hz, 87F7F10-Hz, and 6F11C10-Hz1 all have good tumor inhibitory effects in mice after conjugation with drugs. The changes in the body weight of mice are shown in FIG. 6, indicating that ADC has no effect on the body weight of mice throughout the administration process.

**Table 10. In vivo efficacy of anti-human DLL3 ADC in NCI-H69 CDX model**

| Group | Number of mice | Sample | Dose (mg/kg) | Route of administration | Frequency of administration | Day 24 TGI (%) | P-value |
|---|---|---|---|---|---|---|---|
| 1 | 5 | Normal saline | / | i.v. | QW * 3 | / | / |
| 2 | 5 | C2-ADC07(DAR8) | 1 | i.v. | QW * 3 | 79.3 | <0.001 |
| 3 | 5 | 55C11E4-Hz2-ADC07(DAR8) | 1 | 1.V. | QW * 3 | 62.5 | <0.001 |
| 4 | 5 | 59B10D3-Hz1-ADC07(DAR8) | 1 | 1.V. | QW * 3 | 64.0 | <0.001 |
| 5 | 5 | 87F7F10-Hz-ADC07(DAR8) | 1 | i.v. | QW * 3 | 51.6 | 0.002 |
| 6 | 5 | 6F11C10-hz1-ADC07(DAR8) | 1 | i.v. | QW * 3 | 66.4 | <0.001 |

Therefore, the ADC of the present disclosure has a wider therapeutic index and therapeutic effect. Although the specific embodiments of the present disclosure have been described in detail, those skilled in the art will understand that various modifications and changes can be made to the details based on all teachings that have been disclosed, and these changes are within the scope of protection of the present disclosure. The scope of protection of the present disclosure is given by the appended claims and any equivalents thereof.

## Claims

1. An anti-DLL3 antibody or an antigen-binding fragment thereof, and the antibody or the antigen-binding fragment thereof, comprising a heavy-chain variable region (VH) and a light-chain variable region (VL), wherein the VH and the VL are according to any one of (A1) to (A12):
(A1) the VH comprises complementarity determining regions (CDRs) HCDR1, HCDR2, and HCDR3 corresponding to HCDR1, HCDR2, and HCDR3, respectively, comprised in the heavy chain variable region VH according to SEQ ID NO: 18; and the VL comprises LCDR1, LCDR2, and LCDR3 corresponding to LCDR1, LCDR2, and LCDR3, respectively, comprised in the light chain variable region VL according to SEQ ID NO: 21; or
the VH has at least 70% sequence identity to the amino acid sequence of SEQ ID NO: 18 and the VL has at least 70% sequence identity to the amino acid of SEQ ID NO: 21;
(A2) the VH comprises HCDR1, HCDR2, and HCDR3 corresponding to HCDR1, HCDR2, and HCDR3, respectively, comprised in the heavy chain variable region VH according to SEQ ID NO: 19; and the VL comprises LCDR1, LCDR2, and LCDR3 corresponding to LCDR1, LCDR2, and LCDR3, respectively, comprised in the light chain variable region VL according to SEQ ID NO: 22; or
the VH has at least 70% sequence identity to the amino acid sequence of SEQ ID NO: 19 and the VL has at least 70% sequence identity to the amino acid of SEQ ID NO: 22;
(A3) the VH comprises HCDR1, HCDR2, and HCDR3 corresponding to HCDR1, HCDR2, and HCDR3, respectively, comprised in the heavy chain variable region VH according to SEQ ID NO: 20; and the VL comprises LCDR1, LCDR2, and LCDR3 corresponding to LCDR1, LCDR2, and LCDR3, respectively, comprised in the light chain variable region VL according to SEQ ID NO: 22; or
the VH has at least 70% sequence identity to the amino acid sequence of SEQ ID NO: 20 and the VL has at least 70% sequence identity to the amino acid of SEQ ID NO: 22;
(A4) the VH comprises HCDR1, HCDR2, and HCDR3 corresponding to HCDR1, HCDR2, and HCDR3, respectively, comprised in the heavy chain variable region VH according to SEQ ID NO: 5; and the VL comprises LCDR1, LCDR2, and LCDR3 corresponding to LCDR1, LCDR2, and LCDR3, respectively, comprised in the light chain variable region VL according to SEQ ID NO: 8; or
the VH has at least 70% sequence identity to the amino acid sequence of SEQ ID NO: 5 and the VL has at least 70% sequence identity to the amino acid of SEQ ID NO: 8;
(A5) the VH comprises HCDR1, HCDR2, and HCDR3 corresponding to HCDR1, HCDR2, and HCDR3, respectively, comprised in the heavy chain variable region VH according to SEQ ID NO: 6; and the VL comprises LCDR1, LCDR2, and LCDR3 corresponding to LCDR1, LCDR2, and LCDR3, respectively, comprised in the light chain variable region VL according to SEQ ID NO: 9; or
the VH has at least 70% sequence identity to the amino acid sequence of SEQ ID NO: 6 and the VL has at least 70% sequence identity to the amino acid of SEQ ID NO: 9;
(A6) the VH comprises HCDR1, HCDR2, and HCDR3 corresponding to HCDR1, HCDR2, and HCDR3, respectively, comprised in the heavy chain variable region VH according to SEQ ID NO: 7; and the VL comprises LCDR1, LCDR2, and LCDR3 corresponding to LCDR1, LCDR2, and LCDR3, respectively, comprised in the light chain variable region VL according to SEQ ID NO: 9; or
the VH has at least 70% sequence identity to the amino acid sequence of SEQ ID NO: 7 and the VL has at least 70% sequence identity to the amino acid of SEQ ID NO: 9;
(A7) the VH comprises HCDR1, HCDR2, and HCDR3 corresponding to HCDR1, HCDR2, and HCDR3, respectively, comprised in the heavy chain variable region VH according to SEQ ID NO: 10; and the VL comprises LCDR1, LCDR2, and LCDR3 corresponding to LCDR1, LCDR2, and LCDR3, respectively, comprised in the light chain variable region VL according to SEQ ID NO: 12; or
the VH has at least 70% sequence identity to the amino acid sequence of SEQ ID NO: 10 and the VL has at least 70% sequence identity to the amino acid of SEQ ID NO: 12;
(A8) the VH comprises HCDR1, HCDR2, and HCDR3 corresponding to HCDR1, HCDR2, and HCDR3, respectively, comprised in the heavy chain variable region VH according to SEQ ID NO: 11; and the VL comprises LCDR1, LCDR2, and LCDR3 corresponding to LCDR1, LCDR2, and LCDR3, respectively, comprised in the light chain variable region VL according to SEQ ID NO: 13; or
the VH has at least 70% sequence identity to the amino acid sequence of SEQ ID NO: 11 and the VL has at least 70% sequence identity to the amino acid of SEQ ID NO: 13;
(A9) the VH comprises HCDR1, HCDR2, and HCDR3 corresponding to HCDR1, HCDR2, and HCDR3, respectively, comprised in the heavy chain variable region VH according to SEQ ID NO: 14; and the VL comprises LCDR1, LCDR2, and LCDR3 corresponding to LCDR1, LCDR2, and LCDR3, respectively, comprised in the light chain variable region VL according to SEQ ID NO: 16; or
the VH has at least 70% sequence identity to the amino acid sequence of SEQ ID NO: 14 and the VL has at least 70% sequence identity to the amino acid of SEQ ID NO: 16;
(A10) the VH comprises HCDR1, HCDR2, and HCDR3 corresponding to HCDR1, HCDR2, and HCDR3, respectively, comprised in the heavy chain variable region VH according to SEQ ID NO: 15; and the VL comprises LCDR1, LCDR2, and LCDR3 corresponding to LCDR1, LCDR2, and LCDR3, respectively, comprised in the light chain variable region VL according to SEQ ID NO: 17; or
the VH has at least 70% sequence identity to the amino acid sequence of SEQ ID NO: 15 and the VL has at least 70% sequence identity to the amino acid of SEQ ID NO: 17;
(A11) the VH comprises HCDR1, HCDR2, and HCDR3 corresponding to HCDR1, HCDR2, and HCDR3, respectively, comprised in the heavy chain variable region VH according to SEQ ID NO: 1; and the VL comprises LCDR1, LCDR2, and LCDR3 corresponding to LCDR1, LCDR2, and LCDR3, respectively, comprised in the light chain variable region VL according to SEQ ID NO: 3; or
the VH has at least 70% sequence identity to the amino acid sequence of SEQ ID NO: 1 and the VL has at least 70% sequence identity to the amino acid of SEQ ID NO: 3; and
(A12) the VH comprises HCDR1, HCDR2, and HCDR3 corresponding to HCDR1, HCDR2, and HCDR3, respectively, comprised in the heavy chain variable region VH according to SEQ ID NO: 2; and the VL comprises LCDR1, LCDR2, and LCDR3 corresponding to LCDR1, LCDR2, and LCDR3, respectively, comprised in the light chain variable region VL according to SEQ ID NO: 4; or
the VH has at least 70% sequence identity to the amino acid sequence of SEQ ID NO: 2 and the VL has at least 70% sequence identity to the amino acid of SEQ ID NO: 4.

2. The anti-DLL3 antibody or the antigen-binding fragment of claim 1, wherein the anti-DLL3 antibody or the antigen-binding fragment is according to any one of (B1) to (B7):
(B1) CDRs are as defined by Kabat numbering system, the VH comprises: HCDR1 according to the sequence of SEQ ID NO: 68, HCDR2 according to the sequence of SEQ ID NO: 69, HCDR3 according to the sequence of SEQ ID NO: 71; and the VL comprises: LCDR1 according to the sequence of SEQ ID NO: 72, LCDR2 according to the sequence of SEQ ID NO: 73, LCDR3 according to the sequence of SEQ ID NO: 74; or
CDRs are as defined by IMGT numbering system, the VH comprises: HCDR1 according to the sequence of SEQ ID NO: 75, HCDR2 according to the sequence of SEQ ID NO: 76, HCDR3 according to the sequence of SEQ ID NO: 78; and the VL comprises: LCDR1 according to the sequence of SEQ ID NO: 79, LCDR2 according to the amino acid sequence LAS, LCDR3 according to the sequence of SEQ ID NO: 74;
(B2) CDRs are as defined by Kabat numbering system, the VH comprises: HCDR1 according to the sequence of SEQ ID NO: 68, HCDR2 according to the sequence of SEQ ID NO: 70, HCDR3 according to the sequence of SEQ ID NO: 71; and the VL comprises: LCDR1 according to the sequence of SEQ ID NO: 72, LCDR2 according to the sequence of SEQ ID NO: 73, LCDR3 according to the sequence of SEQ ID NO: 74; or
CDRs are as defined by IMGT numbering system, the VH comprises: HCDR1 according to the sequence of SEQ ID NO: 75, HCDR2 according to the sequence of SEQ ID NO: 77, HCDR3 according to the sequence of SEQ ID NO: 78; and the VL comprises: LCDR1 according to the sequence of SEQ ID NO: 79, LCDR2 according to the amino acid sequence LAS, LCDR3 according to the sequence of SEQ ID NO: 74;
(B3) CDRs are as defined by Kabat numbering system, the VH comprises: HCDR1 according to the sequence of SEQ ID NO: 34, HCDR2 according to the sequence of SEQ ID NO: 35, HCDR3 according to the sequence of SEQ ID NO: 37; and the VL comprises: LCDR1 according to the sequence of SEQ ID NO: 38, LCDR2 according to the sequence of SEQ ID NO: 39, LCDR3 according to the sequence of SEQ ID NO: 40; or
CDRs are as defined by IMGT numbering system, the VH comprises: HCDR1 according to the sequence of SEQ ID NO: 41, HCDR2 according to the sequence of SEQ ID NO: 42, HCDR3 according to the sequence of SEQ ID NO: 43; and the VL comprises: LCDR1 according to the sequence of SEQ ID NO: 44, LCDR2 according to the amino acid sequence YAS, LCDR3 according to the sequence of SEQ ID NO: 40;
(B4) CDRs are as defined by Kabat numbering system, the VH comprises: HCDR1 according to the sequence of SEQ ID NO: 34, HCDR2 according to the sequence of SEQ ID NO: 36, HCDR3 according to the sequence of SEQ ID NO: 37; and the VL comprises: LCDR1 according to the sequence of SEQ ID NO: 38, LCDR2 according to the sequence of SEQ ID NO: 39, LCDR3 according to the sequence of SEQ ID NO: 40; or
CDRs are as defined by IMGT numbering system, the VH comprises: HCDR1 according to the sequence of SEQ ID NO: 41, HCDR2 according to the sequence of SEQ ID NO: 42, HCDR3 according to the sequence of SEQ ID NO: 43; and the VL comprises: LCDR1 according to the sequence of SEQ ID NO: 44, LCDR2 according to the amino acid sequence YAS, LCDR3 according to the sequence of SEQ ID NO: 40;
(B5) CDRs are as defined by Kabat numbering system, the VH comprises: HCDR1 according to the sequence of SEQ ID NO: 46, HCDR2 according to the sequence of SEQ ID NO: 47, HCDR3 according to the sequence of SEQ ID NO: 48; and the VL comprises: LCDR1 according to the sequence of SEQ ID NO: 49, LCDR2 according to the sequence of SEQ ID NO: 50, LCDR3 according to the sequence of SEQ ID NO: 51; or
CDRs are as defined by IMGT numbering system, the VH comprises: HCDR1 according to the sequence of SEQ ID NO: 52, HCDR2 according to the sequence of SEQ ID NO: 53, HCDR3 according to the sequence of SEQ ID NO: 54; and the VL comprises: LCDR1 according to the sequence of SEQ ID NO: 55, LCDR2 according to the amino acid sequence YTS, LCDR3 according to the sequence of SEQ ID NO: 51;
(B6) CDRs are as defined by Kabat numbering system, the VH comprises: HCDR1 according to the sequence of SEQ ID NO: 57, HCDR2 according to the sequence of SEQ ID NO: 58, HCDR3 according to the sequence of SEQ ID NO: 59; and the VL comprises: LCDR1 according to the sequence of SEQ ID NO: 60, LCDR2 according to the sequence of SEQ ID NO: 61, LCDR3 according to the sequence of SEQ ID NO: 62; or
CDRs are as defined by IMGT numbering system, the VH comprises: HCDR1 according to the sequence of SEQ ID NO: 63, HCDR2 according to the sequence of SEQ ID NO: 64, HCDR3 according to the sequence of SEQ ID NO: 65; and the VL comprises: LCDR1 according to the sequence of SEQ ID NO: 66, LCDR2 according to the amino acid sequence WAS, LCDR3 according to the sequence of SEQ ID NO: 62; and
(B7) CDRs are as defined by Kabat numbering system, the VH comprises: HCDR1 according to the sequence of SEQ ID NO: 23, HCDR2 according to the sequence of SEQ ID NO: 24, HCDR3 according to the sequence of SEQ ID NO: 25; and the VL comprises: LCDR1 according to the sequence of SEQ ID NO: 26, LCDR2 according to the sequence of SEQ ID NO: 27, LCDR3 according to the sequence of SEQ ID NO: 28; or
CDRs are as defined by IMGT numbering system, the VH comprises: HCDR1 according to the sequence of SEQ ID NO: 29, HCDR2 according to the sequence of SEQ ID NO: 30, HCDR3 according to the sequence of SEQ ID NO: 31; and the VL comprises: LCDR1 according to the sequence of SEQ ID NO: 32, LCDR2 according to the amino acid sequence FAS, LCDR3 according to the sequence of SEQ ID NO: 28.

3. The anti-DLL3 antibody or the antigen-binding fragment of claim 1 or 2,
wherein CDRs are as defined by Kabat numbering system, the VH comprises: HCDR1 according to the sequence of SEQ ID NO: 68, HCDR2 according to the sequence of SEQ ID NO: 70, HCDR3 according to the sequence of SEQ ID NO: 71; and the VL comprises: LCDR1 according to the sequence of SEQ ID NO: 72, LCDR2 according to the sequence of SEQ ID NO: 73, LCDR3 according to the sequence of SEQ ID NO: 74; or
wherein CDRs are as defined by IMGT numbering system, the VH comprises: HCDR1 with the sequence of SEQ ID NO: 75, HCDR2 with the sequence of SEQ ID NO: 76 or 77, HCDR3 with the sequence of SEQ ID NO: 78; and the VL comprises: LCDR1 according to the sequence of SEQ ID NO: 79, LCDR2 according to the amino acid sequence of LAS, LCDR3 according to the sequence of SEQ ID NO: 74.

4. The anti-DLL3 antibody or the antigen-binding fragment of any one of claims 1 to 3, wherein the VH comprises HCDR1, HCDR2, and HCDR3 corresponding to HCDR1, HCDR2, and HCDR3, respectively, comprised in the heavy chain variable region VH according to SEQ ID NO: 20; and the VL comprises LCDR1, LCDR2, and LCDR3 corresponding to LCDR1, LCDR2, and LCDR3, respectively, comprised in the light chain variable region VL according to SEQ ID NO: 22.

5. The anti-DLL3 antibody or the antigen-binding fragment of claim 1, wherein the VH comprises an amino acid sequence according to SEQ ID NO: 18 or a humanized variant thereof, and the VL comprises an amino acid sequence according to SEQ ID NO: 21 or a humanized variant thereof.

6. The anti-DLL3 antibody or the antigen-binding fragment of any one of claims 1 to 4, wherein the VH comprises an amino acid sequence of SEQ ID NO: 20, and the VL comprises an amino acid sequence of SEQ ID NO: 22; preferably, the anti-DLL3 antibody or the antigen-binding fragment comprises a heavy chain comprising an amino sequence of SEQ ID NO: 80 and a light chain comprising an amino acid of SEQ ID NO: 81.

7. The anti-DLL3 antibody or the antigen-binding fragment of any one of claims 1 to 6, wherein the antibody or the antigen-binding fragment is at least one selected from Fab, Fab', (Fab')₂, Fd, Fv selected from scFv and disulfide linked Fv (dsFv), dAb, a complementarity determining region fragment, a non-human antibody, a humanized antibody, a chimeric antibody, a fully human antibody, a probody, a monoclonal antibody, a bispecific antibody, and a multi-specific antibody.

8. An isolated nucleic acid molecule encoding the anti-DLL3 antibody or the antigen-binding fragment according to any one of claims 1-7.

9. A vector comprising the isolated nucleic acid molecule according to claim 8; preferably, the vector is a cloning vector or an expression vector.

10. A host cell comprising the isolated nucleic acid molecule according to claim 8 or the vector according to claim 9.

11. A method for preparing the anti-DLL3 antibody or the antigen-binding fragment according to any one of claims 1-7, comprising culturing the host cell according to claim 10 under conditions that allow the expression of the antibody or the antigen-binding fragment thereof, and recovering the antibody or the antigen-binding fragment thereof from a cultured host cell culture.

12. An antibody-drug conjugate of formula XV, a stereoisomer of the antibody-drug conjugate, a pharmaceutically acceptable salt of the antibody-drug conjugate or the stereoisomer, or a pharmaceutically acceptable solvate of the antibody-drug conjugate or the stereoisomer, 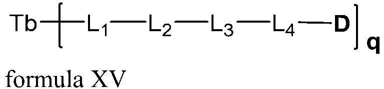
wherein Tb is selected from the anti-DLL3 antibody or the antigen-binding fragment thereof according to any one of claims 1-7,
q is drug-to-antibody coupling ratio selected from any value between 0.1 and 16.0;
D is a bioactive molecule fragment, wherein the bioactive molecule is a DNA topoisomerase inhibitor;
L₁ is selected from 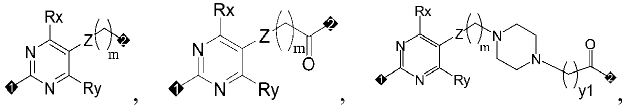 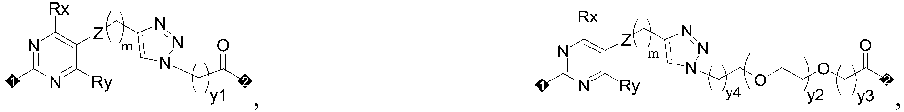 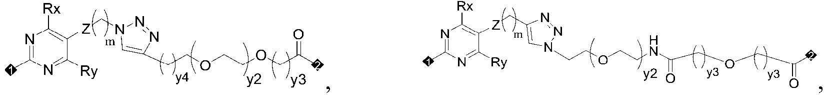 and 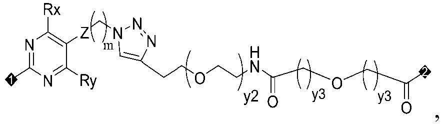 each Z is independently selected from a direct bond, a carbon-carbon triple bond, and a carbon-carbon double bond;
L₂ is absent;
L₃ is selected from Val-AA¹-Gly, Val-AA¹, Ala-AA¹, Gly-AA¹, AA¹-Gly, Ala-AA¹-Gly, Gly-AA¹-Gly, AA¹-Ala-Asn, and AA¹; an amino acid residue represented by the AA¹ has a structure as follows, 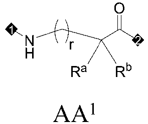
wherein R^{a} and R^{b} are each independently selected from H, 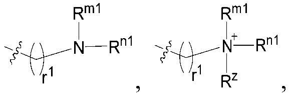 and 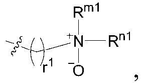 and R^{a} and R^{b} are not both H;
r and r¹ are each independently selected from any integer between 0 and 20;
R^{m1} and Rⁿ¹ are each independently selected from H, C1-6 alkyl, and C3-6 cycloalkyl; and
L₄ is 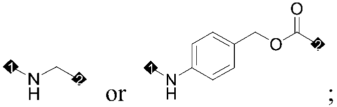 position 1 is attached to L₃ and position 2 is attached to D.

13. The antibody-drug conjugate, the stereoisomer, the pharmaceutically acceptable salt, or the pharmaceutically acceptable solvate of claim 12, wherein the antibody-drug conjugate is:

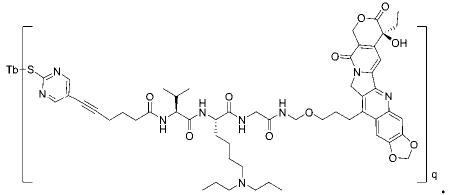

14. A pharmaceutical composition comprising one or more selected from: the anti-DLL3 antibody or the antigen-binding fragment according to any one of claims 1-7, the nucleic acid molecule according to claim 8, the vector according to claim 9, the host cell according to claim 10, the antibody-drug conjugate, the stereoisomer, the pharmaceutically acceptable salt, or the pharmaceutically acceptable solvate according to claim 12 or 13; preferably further comprising a pharmaceutically acceptable carrier and/or excipient.

15. The anti-DLL3 antibody or the antigen-binding fragment according to any one of claims 1-7, the nucleic acid molecule according to claim 8, the vector according to claim 9, the host cell according to claim 10, the antibody-drug conjugate, the stereoisomer, the pharmaceutically acceptable salt, the pharmaceutically acceptable solvate according to claim 12 or 13, or the pharmaceutical composition according to claim 14, for use in treating or preventing a DLL3-associated cancer disease, preferably the DLL3-associated cancer disease is esophageal cancer, brain tumor, lung cancer, squamous cell carcinoma, bladder cancer, gastric cancer, ovarian cancer, peritoneal cancer, pancreatic cancer, breast cancer, head and neck cancer, cervical cancer, endometrial cancer, colon cancer, rectal cancer, colorectal cancer, liver cancer, kidney cancer, urothelial cancer, epidermal carcinoma, non-Hodgkin lymphoma, central nervous system tumor, prostate cancer, or thyroid cancer.
